# EUROPEAN PATENT APPLICATION

(11) **EP 4 574 170 A1**
(43) Date of publication of application: **25.06.2025**
(21) Application number: 23855218.6
(22) Date of filing: 18.08.2023
(51) Int. Cl.: A61K 39/395, C07K 16/28, A61P 13/12, A61P 1/00, A61P 35/00, A61P 37/00, A61P 9/00

(54) **METHOD FOR TREATING A T-LYMPHOCYTE-MEDIATED DISEASE**

(30) Priority: 18.08.2022 RU 2022122378; 21.04.2023 RU 2023110336; 17.08.2023 RU 2023121536
(71) Applicant: Joint Stock Company "Biocad", Saint Petersburg 198515 (RU)
(72) Inventor: ZINKINA-ORIKHAN, Arina Valerievna, Saint Petersburg, 197374 (RU); MOROZOVA, Mariia Andreevna, Moscow, 119334 (RU); LINKOVA, Yulia Nikolaevna, Saint Petersburg, 198323 (RU); USTIUGOV, Iakov Urievich, Saint Petersburg, 196084 (RU); ALEKSANDROV, Aleksei Aleksandrovich, Moscow, 109518 (RU); OGANOVA, Marina Albertovna, Pyatigorsk, 357524 (RU); MOROZOV, Dmitry Valentinovich, Saint Petersburg, 190000 (RU)
(74) Representative: Meissner Bolte Partnerschaft mbB
(86) International application number: PCT/RU2023/050196
(87) International publication number: WO 2024/039268

(57) **Abstract**

The present invention relates to the field of medicine, and more particularly to a method for treating a disease or disorder mediated by T-lymphocytes bearing a TRBV9 segment within the T-cell receptor, which includes administering an anti-TRBV9 antibody in specific doses and dosage regimens, and further relates to the use of an anti-TRBV9 antibody to treat a disease or disorder mediated by T-lymphocytes bearing a TRBV9 segment within the T-cell receptor, where the anti-TRBV9 antibody is administered in specific doses and dosage regimens.

## Description

### Field of the invention

The present invention relates to the field of medicine, specifically to a method of treatment of a disease or disorder mediated by T-lymphocytes bearing the TRBV9 segment within the T cell receptor, comprising administering an anti-TRBV9 antibody using defined doses, as well as dosing regimens; to the use of an anti-TRBV9 antibody for the treatment of a disease or disorder mediated by T-lymphocytes bearing the TRBV9 segment within the T cell receptor, wherein the anti-TRBV9 antibody is administered using defined doses, as well as dosing regimens.

### Background of the invention

Autoimmune diseases are caused by autoreactive T-lymphocytes (Haroon N et al., Arthritis Rheum. 2013 Oct; 65(10):2645-54., Duarte J. et al., PloS One 2010 May 10; 5(5):e10558; Konig M. et al., Front Immunol 2016 Jan 25; 7:11). The major role in the appearance of autoreactive T-lymphocyte clones is played by the interaction of the antigen-recognizing T cell receptor (TCR) with proteins of the major histocompatibility complex (MHC, HLA), which present on their surface the processed peptides of intracellular proteins or those of proteins of pathogenic organisms. A number of autoimmune diseases are associated with the presence of a certain variant of the HLA gene in humans. Accordingly, the HLA-B27 allele is associated with ankylosing spondylitis, reactive arthritis, and Crohn's disease. The risk of developing autoimmune diseases in carriers of certain HLA allelic variants can be explained by the fact that these alleles present preferentially certain peptides that are autoantigens, an immune response against which triggers the development of an autoimmune disease. One of the possible mechanisms underlying an autoimmune reaction is presenting, by the histocompatibility complex molecules, the peptides from proteins of bacterial or viral origin, which are homologous to the organism's own peptides, which fact may lead to an immune response against self-antigens as a result of cross-reactivity.

The prior art provides that a T cell receptor (TCR) sequence is a marker allowing to identify a T-lymphocyte clone involved in the pathogenesis of an autoimmune disease. T cell receptor subunits belong structurally to the immunoglobulin superfamily and are formed from several gene segments. The variable regions of TCR form the antigen-binding center of TCR. This means that they are clone-specific, that is, they are different in those T-lymphocytes that react to different antigens.

T lymphocytes (T cells) are stimulated when antigens bind to T cell receptors (TCRs) thereof. The TCR, a defining structure of T cells, is a transmembrane heterodimer consisting of either an alpha and beta chain or delta and gamma chain linked by a disulphide bond. Within these chains there are complementary determining regions (CDRs) which determine the antigen to which the TCR will bind. TCR development occurs through a lymphocyte specific process of gene recombination, which assembles a final sequence from a large number of potential segments. This genetic recombination of TCR gene segments in somatic T cells occurs during the early stages of development in the thymus. The TCRα gene locus contains variable (V) and joining (J) gene segments (Vβ and Jβ), whereas the TCRβ locus contains a D gene segment in addition to Vα and Jα segments. Accordingly, the α chain is generated from VJ recombination and the β chain is involved in VDJ recombination.

The TCR α chain gene locus consists of 46 variable segments (TRAV), 8 joining segments (TRAJ) and the constant region. The TCR β chain gene locus consists of 48 variable segments (TRBV) followed by two diversity segments (TRBD), 12 joining segments (TRBJ) and two constant regions. (Bio-Rad. Mini-review | An overview of T cell receptors [Electronic resource] // Bio-Rad. URL: https://www.bio-rad-antibodies.com/t-cell-receptorminireview.html (accessed: 24.04.2020)).

To date, a significant amount of data has been accumulated demonstrating that the development of HLA-B27-associated diseases is due to the expansion of antigen-specific T-lymphocyte clones.

The consensus variant of autoimmune TCRs has been described in ankylosing spondylitis (radiographic axial spondyloarthritis); it has been shown that it is present in synovial fluid and peripheral blood in patients with ankylosing spondylitis and is absent with the same analysis depth in healthy donors, regardless of the HLA*B27 allele status (Faham M. et al., Arthritis Rheumatol. 2017; 69(4):774-784; Komech E et al. 12th EJI-EFIS Tatra Immunology Conference; 2016 Sep 3-7; Strbske Pleso, Slovakia. Abstract book p. 39). Said TCRs belong to the TRBV9 family (according to the IMGT nomenclature).

It has been shown that T cell receptors bearing beta chains of the TRBV9 family are also involved in the development of such an autoimmune disease as celiac disease (Petersen J et al., J Immunol. 2015; 194(12): 6112-22). They are also found on the surface of T cells subject to malignization in T cell lymphomas and T cell leukemias, including T cell lymphoma caused by the Epstein-Barr virus (EBV) (Toyabe S et al., Clin Exp Immunol. 2003; 134 (1): 92-97).

Considering the above, a TRBV9 protein may serve as a target for a cytotoxic monoclonal antibody that will induce depletion of TRBV9+ T-lymphocytes (TRBV9-positive T-lymphocytes), including pathogenic autoreactive T-lymphocyte clones.

Accordingly, there is a need to develop a method of treatment of a disease or disorder mediated by the human T-cell receptor bearing TRBV9, comprising administering an anti-TRBV9 antibody using defined doses, as well as dosing regimens.

### Brief description of drawings

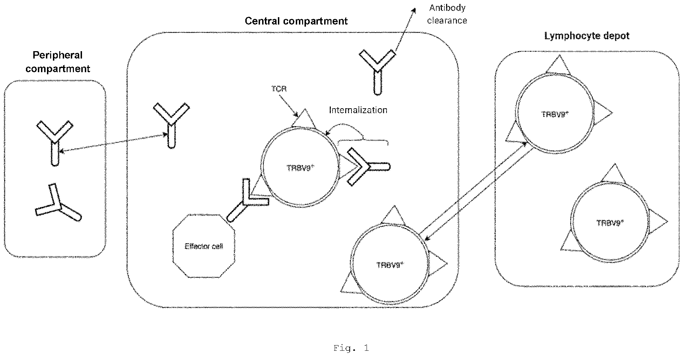

Figure 1 is a diagram of the main processes considered in the mathematical model
TCR - T-Cell Receptor,
Effector cell - effector cell.

### Detailed description of the invention

### Definitions

Unless defined otherwise herein, all technical and scientific terms used in connection with the present invention will have the same meaning as is commonly understood by those skilled in the art.

Furthermore, unless otherwise required by context, singular terms shall include plural terms, and the plural terms shall include the singular terms.

As used in the present description and claims that follow, unless otherwise dictated by the context, the words "have", "include," and "comprise" or variations thereof such as "has", "having," "includes", "including", "comprises," or "comprising," will be understood to imply the inclusion of a stated integer or group of integers but not the exclusion of any other integer or group of integers.

The term "antibody" or "immunoglobulin" (Ig) includes full-length antibodies or any antigen binding fragment (i.e, "antigen-binding portion") or individual chains thereof. The term "antibody" within the scope of the present invention is used in the broadest sense and may include, without limitation, monoclonal antibodies (including full-length monoclonal antibodies), polyclonal antibodies, humanized, fully human antibodies and chimeric antibodies.

A full-length antibody refers to a glycoprotein comprising at least two heavy (H) chains and two light (L) chains interconnected by disulfide bonds. Each heavy chain comprises a heavy chain variable region (abbreviated referred to in the present description as VH) and a heavy chain constant region. The constant region is identical in all antibodies of the same isotype, but differs in antibodies of different isotypes. Heavy chains γ, α and δ have a constant region composed of three constant domains CH1, CH2 and CH3 (in a line), and a hinge region for added flexibility (Woof J., Burton D., Nat Rev Immunol 4, 2004, pp.89-99). In mammals, known are only two types of light chains denoted by lambda (λ) and kappa (κ). Each light chain consists of a light chain variable region (abbreviated referred to in the present description as VL) and light chain constant region. The approximate length of a light chain is 211 to 217 amino acids. Preferably, the light chain is a lambda (λ) light chain, and the constant domain CL is preferably C lambda (λ).

VL and VH regions may be further subdivided into hypervariability regions called complementarity determining regions (CDRs), located between regions that are more conserved, termed framework regions (FRs). Each VH and VL is composed of three CDRs and four FRs, arranged from amino-terminus to carboxy-terminus in the following order: FR1, CDR1, FR2, CDR2, FR3, CDR3, FR4. The variable regions of heavy and light chains contain a binding domain that interacts with an antigen. The constant regions of antibodies may mediate the binding of immunoglobulin to host tissues or factors, including various cells of the immune system (e.g. effector cells) and the first component (C1q) of the classical complement system.

The term "antigen-binding portion" of antibody or "antigen-binding fragment", as used in the present description, refers to one or more antibody fragments that retain the ability to specifically bind to an antigen. It has been shown that the antigen-binding function of antibody can be performed by fragments of a full-length antibody. As used in the present invention, the term "antigen-binding fragment" means a Fabfragment, i.e. a monovalent fragment, consisting of VL, VH, CL and CH1 domains, which is linked with the Fc-fragment monomer.

The term "variable" refers to the fact that certain portions of the variable domains greatly differ in sequence among antibodies. The V domain mediates antigen binding and determines specificity of each particular antibody for its particular antigen. However, the variability is not evenly distributed across the 110-amino acid span of the variable domains. Instead, the V regions consist of invariant fragments termed framework regions (FRs) of 15-30 amino acids separated by shorter regions of extreme variability termed "hypervariable regions" or CDRs. The variable domains of native heavy and light chains each comprise four FRs, largely adopting a beta-sheet configuration, connected by three hypervariable regions, which form loops connecting, and in some cases forming part of, the beta-sheet structure. The hypervariable regions in each chain are held together in close proximity by FRs and, with the hypervariable regions from the other chain, contribute to the formation of the antigen-binding site of antibodies (see Kabat et al., Sequences of Proteins of Immunological Interest. 5th Ed. Public Health Service, National Institutes of Health, Bethesda, MD. (1991)). The constant domains are not involved directly in binding of antibody to antigen, but exhibit various effector functions, such as participation of antibody in antibody-dependent cellular cytotoxicity (ADCC).

The term "hypervariable region" according to the present description refers to the amino acid residues of antibody which are responsible for antigen binding. The hypervariable region typically comprises amino acid residues from a "complementarity determining region" or "CDR" and/or those residues from a "hypervariable loop".

"Kabat numbering scheme" or "numbering according to Kabat" refers in the present invention to a system for numbering of amino acid residues that are more variable (i.e. hypervariable) than other amino acid residues in variable regions of heavy and light chains of antibody (Kabat et al. Ann. N.Y. Acad. Sci., 190:382-93 (1971); Kabat et al. Sequences of Proteins of Immunological Interest, Fifth Edition, U.S. Department of Health and Human Services, NIH Publication No. 91-3242 (1991)).

The antibody of the present invention "which binds" a target antigen refers to an antibody that binds the antigen with sufficient affinity such that the antibody can be used as a diagnostic and/or therapeutic agent targeting a protein or cell or tissue expressing the antigen, and slightly cross-reacts with other proteins. According to analytical methods: fluorescenceactivated cell sorting (FACS), radioimmunoassay (RIA) or ELISA, in such embodiments, the degree of antibody binding to a nontarget protein is less than 10 % of antibody binding to a specific target protein. With regard to the binding of antibody to a target molecule, the term "specific binding" or phrases "specifically binds to" or "is specific for" a particular polypeptide or an epitope on a particular target polypeptide means binding that is significantly (measurably) different from a non-specific interaction.

Specific binding may be measured, for example, by determining binding of a molecule as compared to binding of a control molecule. For example, specific binding may be determined by competition with another molecule that is similar to the target, for example, an excess of non-labeled target. In this case, specific binding is indicated if the binding of the labeled target to a probe is competitively inhibited by the excess of unlabeled target. As used in the present description, the term "specific binding" or phrases "specifically binds to" or " is specific for" a particular polypeptide or an epitope on a particular target polypeptide may be described by example of a molecule having a KD (affinity constant) for the target of at least about 200 nM, or at least about 150 nM, or at least about 100 nM, or at least about 60 nM, or at least about 50 nM, or at least about 40 nM, or at least about 30 nM, or at least about 20 nM, or at least about 10 nM, or at least about 8 nM, or at least about 6 nM, or at least about 4 nM, or at least about 2 nM, or at least about 1 nM, or greater. In one embodiment, the term "specific binding" refers to binding where a molecule binds to a particular polypeptide or epitope on a particular polypeptide without substantially binding to any other polypeptide or epitope on a polypeptide.

The term "monoclonal antibody" or "mAb" refers to an antibody that is synthesized and isolated as an individual clonal population of cells.

The term "recombinant antibody" refers to an antibody that is expressed in a cell or cell line comprising nucleotide sequence(s) encoding antibodies, wherein said nucleotide sequence(s) is (are) not associated with the cell in nature.

The term "isolated" applied to describe various antibodies according to the present invention refers to an antibody which has been identified and isolated and/or regenerated from a cell or cell culture, in which the antibody is expressed. Impurities (contaminant components) from natural environment are materials which typically interfere with diagnostic or therapeutic uses of the polypeptide, and may include enzymes, hormones, and other proteinaceous or nonproteinaceous solutes. The isolated polypeptide is typically prepared by at least one purification step.

The terms "anti-TRBV9 antibody", "antibody to TRBV9", "antibody specifically binding to the TRBV9 family beta-chain" and "antibody against the TRBV9 family beta-chain" are interchangeable within the scope of the present invention and relate to an antibody that specifically binds to the epitope of TRBV9 family beta-chain of human T cell receptor.

The term "main dose" refers to the recommended dose of the anti-TRBV9 antibody product, or the full recommended dose of the anti-TRBV9 antibody product, or the recommended dose of the anti-TRBV9 antibody product in the form of intravenous infusion, or the full recommended dose of the anti-TRBV9 antibody product in the form of intravenous infusion.

The term "half of the main dose" refers to half of the recommended dose of the anti-TRBV9 antibody product, or half of the full recommended dose of the anti-TRBV9 antibody product, or half of the recommended dose of the anti-TRBV9 antibody product in the form of intravenous infusion, or half of the full recommended dose of the anti-TRBV9 antibody product in the form of intravenous infusion.

The term "at least" in the context of administration refers to the minimum administration interval ± 2 days. "At least 3 months" refers to administration after 3 months ± 2 days, or 6 months ±2 days, or 9 months ±2 days, or 12 months ± 2 days, etc. in increments of 3 months ± 2 days. "At least 6 months" refers to administration after 6 months ± 2 days, or 9 months ±2 days, or 12 months ± 2 days, etc. in increments of 3 months ± 2 days. "At least 9 months" refers to administration after 9 months ± 2 days, or 12 months ±2 days, or 15 months ± 2 days, etc. in increments of 3 months ± 2 days. "At least 12 months" refers to administration after 12 months ± 2 days, or 15 months ±2 days, or 18 months ± 2 days, etc. in increments of 3 months ± 2 days.

The term "administration interval" refers to the time interval between administrations and subsequent administrations are counted from the date of the second or third infusion, and subsequently from each previous administration.

The term "first administration" or "first infusion" refers to the initial dose of the anti-TRBV9 antibody product or half of the main dose.

The term "second administration" or "second infusion" refers to the administration of the main dose of the anti-TRBV9 antibody product after at least 3 months from the first administration.

The term "third administration" or "third infusion" refers to the administration of the main dose of the anti-TRBV9 antibody product after at least 3 months from the second administration.

The term "subsequent administration" refers to the administration of the main dose of the anti-TRBV9 antibody product after at least 3 months from each previous administration.

The term "use" applies to the possibility to use of the anti-TRBV9 antibody according to the present invention or a pharmaceutical composition comprising thereof to treat, relief the course of the disease or disorders, expedite the remission, reduce the recurrence rate for the diseases or disorders.

The term "disease or disorder mediated by T-lymphocytes bearing the TRBV9 segment within the T cell receptor" refers to any disease or disorder that is either directly, or indirectly associated with T-lymphocytes bearing the TRBV9 segment within the T cell receptor, including etiology, development, progression, persistence or pathology of a disease or disorder.

The term "method of treatment" applies to the ability of using an antibody of the present invention or a pharmaceutical composition containing thereof to treat, relief the course of the disease, expedite the remission or reduce the recurrence rate for the disease or disorders associated with the activity of T-lymphocytes bearing the TRBV9 segment within the T-cell receptor. "Treat" or "treatment" of a disease, disorder or condition may comprise the prevention or delay of the onset of clinical symptoms of a disease, disorder or condition developing in human, the inhibition of a disease, disorder or condition, i.e. stop, reduction or delay of the development of a disease or a relapse thereof (in case of maintenance therapy) or at least one clinical or subclinical symptom thereof, or the alleviation or easement of a disease, i.e. the causing of regression of a disease, disorder or condition. As used herein, to "alleviate" a disease, disorder or condition means reducing the severity and/or occurrence frequency of the symptoms of the disease, disorder, or condition.

The present invention discloses methods of treatment of a disease or disorder mediated by T-lymphocytes bearing the TRBV9 segment within the T-cell receptor, comprising administering an anti-TRBV9 antibody using defined doses, as well as dosing regimens. Furthermore, the present invention discloses uses of an anti-TRBV9 antibody for the treatment of a disease or disorder mediated by T-lymphocytes bearing the TRBV9 segment within the T-cell receptor, wherein the anti-TRBV9 antibody is administered using defined doses, as well as dosing regimens.

In one aspect, the present invention relates to a method of treatment of a disease or disorder mediated by T-lymphocytes bearing the TRBV9 segment within the T-cell receptor, comprising administering to a subject in need thereof an anti-TRBV9 antibody at a dose of 0.03-10 mg/kg.

In one aspect, the present invention relates to a method of treatment of a disease or disorder mediated by T-lymphocytes bearing the TRBV9 segment within the T-cell receptor, comprising administering to a subject in need thereof an anti-TRBV9 antibody at a dose of 0.03-7 mg/kg.

In one aspect, the present invention relates to a method of treatment of a disease or disorder mediated by T-lymphocytes bearing the TRBV9 segment within the T-cell receptor, comprising administering to a subject in need thereof an anti-TRBV9 antibody at a dose of 0.3-7 mg/kg.

In one aspect, the present invention relates to a method of treatment of a disease or disorder mediated by T-lymphocytes bearing the TRBV9 segment within the T-cell receptor, comprising administering to a subject in need thereof an anti-TRBV9 antibody at a dose of 1-7 mg/kg.

In some embodiments of the invention, the anti-TRBV9 antibody is administered at a dose of 2-7 mg/kg.

In some embodiments of the invention, the anti-TRBV9 antibody is administered at a dose of 3-7 mg/kg.

In some embodiments of the invention, the anti-TRBV9 antibody is administered at a dose of 4-7 mg/kg.

In some embodiments of the invention, the anti-TRBV9 antibody is administered at a dose of 5-7 mg/kg.

In some embodiments of the invention, the anti-TRBV9 antibody is administered at a dose of 6-7 mg/kg.

In some embodiments of the invention, the anti-TRBV9 antibody is administered at a dose of 1 mg/kg, or 2 mg/kg, or 3 mg/kg, or 4 mg/kg, or 5 mg/kg, or 6 mg/kg, or 7 mg/kg.

In some embodiments of the invention, the anti-TRBV9 antibody is administered at a dose of 7.5 mg/kg, or 8 mg/kg, or 8.5 mg/kg, or 9 mg/kg, or 9.5 mg/kg, or 10 mg/kg.

In some embodiments of the invention, the anti-TRBV9 antibody is administered at least every 3-12 months.

In some embodiments of the invention, the anti-TRBV9 antibody is administered every 3-12 months.

In some embodiments of the invention, the anti-TRBV9 antibody is administered at least every 6-12 months.

In some embodiments of the invention, the anti-TRBV9 antibody is administered every 6-12 months.

In some embodiments of the invention, the anti-TRBV9 antibody is administered at least every 3 months; or the anti-TRBV9 antibody is administered at least every 6 months; or the anti-TRBV9 antibody is administered at least every 9 months; or the anti-TRBV9 antibody is administered at least every 12 months.

In some embodiments of the invention, the anti-TRBV9 antibody is administered every 3 months; or the anti-TRBV9 antibody is administered every 6 months; or the anti-TRBV9 antibody is administered every 9 months; or the anti-TRBV9 antibody is administered every 12 months.

In one aspect, the present invention relates to a method of treatment of a disease or disorder mediated by T-lymphocytes bearing the TRBV9 segment within the T-cell receptor, comprising administering to a subject in need thereof an anti-TRBV9 antibody at a dose of 75-675 mg.

In one aspect, the present invention relates to a method of treatment of a disease or disorder mediated by T-lymphocytes bearing the TRBV9 segment within the T-cell receptor, comprising administering to a subject in need thereof an anti-TRBV9 antibody at a dose of 100-650 mg.

In one aspect, the present invention relates to a method of treatment of a disease or disorder mediated by T-lymphocytes bearing the TRBV9 segment within the T-cell receptor, comprising administering to a subject in need thereof an anti-TRBV9 antibody at a dose of 75-175 mg; or 200-300 mg; or 325-425 mg; or 450-650 mg; or 575-675 mg.

In one aspect, the present invention relates to a method of treatment of a disease or disorder mediated by T-lymphocytes bearing the TRBV9 segment within the T-cell receptor, comprising administering to a subject in need thereof an anti-TRBV9 antibody at a dose of 100-150 mg; or 225-275 mg; or 350-400 mg; or 475-525 mg; or 600-650mg.

In some embodiments of the invention, the anti-TRBV9 antibody is administered at a dose of 115-135 **mg;** or 240-260 **mg;** or 365-385 **mg;** or 490- 510 **mg;** or 615-635mg.

In some embodiments of the invention, the anti-TRBV9 antibody is administered at a dose of 120-130 **mg;** or 245-255 **mg;** or 370-380 **mg;** or 495- 505 **mg;** or 620-630 **mg.**

In some embodiments of the invention, the anti-TRBV9 antibody is administered at a dose of 120 mg; or 125 mg; or 130 mg; or 245 mg; or 250 mg; or 255 mg; or 370 mg; or 375 mg; or 380 mg; or 495 mg; or 500 mg; or 505 mg; or 620 mg; or 625 mg; or 630 mg.

In some embodiments of the invention, the anti-TRBV9 antibody is administered at least every 3-12 months.

In some embodiments of the invention, the anti-TRBV9 antibody is administered every 3-12 months.

In some embodiments of the invention, the anti-TRBV9 antibody is administered at least every 6-12 months.

In some embodiments of the invention, the anti-TRBV9 antibody is administered every 6-12 months.

In some embodiments of the invention, the anti-TRBV9 antibody is administered at least every 3 months; or at least every 6 months; or at least every 9 months; or at least every 12 months.

In some embodiments of the invention, the anti-TRBV9 antibody is administered every 3 months; or every 6 months; or every 9 months; or every 12 months.

In one aspect, the present invention relates to a method of treatment of a disease or disorder mediated by T-lymphocytes bearing the TRBV9 segment within the T-cell receptor, comprising the first administration of an anti-TRBV9 antibody to a subject in need thereof at a dose equal to half of the main dose, 0.015-5 mg/kg, the second administration of an anti-TRBV9 antibody at the main dose of 0.03-10 mg/kg after at least 3 months, and the subsequent administrations of an anti-TRBV9 antibody at the main dose of 0.03-10 mg/kg at least every 6 months.

In some embodiments of the invention, the method comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 3 months, and the subsequent administrations of an anti-TRBV9 antibody at the main dose of 0.03-10 mg/kg every 6 months; or every 9 months; or every 12 months.

In some embodiments of the invention, the method comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 6 months, and the subsequent administrations of an anti-TRBV9 antibody at the main dose of 0.03-10 mg/kg every 6 months; or every 9 months; or every 12 months.

In some embodiments of the invention, the method comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 9 months, and the subsequent administrations of an anti-TRBV9 antibody at the main dose of 0.03-10 mg/kg every 9 months; or every 12 months.

In some embodiments of the invention, the method comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 12 months, and the subsequent administrations of an anti-TRBV9 antibody at the main dose of 0.03-10 mg/kg every 12 months.

In one aspect, the present invention relates to a method of treatment of a disease or disorder mediated by T-lymphocytes bearing the TRBV9 segment within the T-cell receptor, comprising the first administration of an anti-TRBV9 antibody to a subject in need thereof at a dose equal to half of the main dose, 0.015-3.5 mg/kg, the second administration of an anti-TRBV9 antibody at the main dose of 0.03-7 mg/kg after at least 3 months, and the subsequent administrations of an anti-TRBV9 antibody at the main dose of 0.03-7 mg/kg at least every 6 months.

In some embodiments of the invention, the method comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 3 months, and the subsequent administrations of an anti-TRBV9 antibody at the main dose of 0.03-7 mg/kg every 6 months; or every 9 months; or every 12 months.

In some embodiments of the invention, the method comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 6 months, and the subsequent administrations of an anti-TRBV9 antibody at the main dose of 0.03-7 mg/kg every 6 months; or every 9 months; or every 12 months.

In some embodiments of the invention, the method comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 9 months, and the subsequent administrations of an anti-TRBV9 antibody at the main dose of 0.03-7 mg/kg every 9 months; or every 12 months.

In some embodiments of the invention, the method comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 12 months, and the subsequent administrations of an anti-TRBV9 antibody at the main dose of 0.03-7 mg/kg every 12 months.

In one aspect, the present invention relates to a method of treatment of a disease or disorder mediated by T-lymphocytes bearing the TRBV9 segment within the T-cell receptor, comprising the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after at least 3 months, and the subsequent administrations of an anti-TRBV9 antibody at the main dose of 0.3-10 mg/kg at least every 6 months.

In some embodiments of the invention, the method comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 3 months, and the subsequent administrations of an anti-TRBV9 antibody at the main dose of 0.3-10 mg/kg every 6 months; or every 9 months; or every 12 months.

In some embodiments of the invention, the method comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 6 months, and the subsequent administrations of an anti-TRBV9 antibody at the main dose of 0.3-10 mg/kg every 6 months; or every 9 months; or every 12 months.

In some embodiments of the invention, the method comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 9 months, and the subsequent administrations of an anti-TRBV9 antibody at the main dose of 0.3-10 mg/kg every 9 months; or every 12 months.

In some embodiments of the invention, the method comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 12 months, and the subsequent administrations of an anti-TRBV9 antibody at the main dose of 0.3-10 mg/kg every 12 months.

In one aspect, the present invention relates to a method of treatment of a disease or disorder mediated by T-lymphocytes bearing the TRBV9 segment within the T-cell receptor, comprising the first administration of an anti-TRBV9 antibody to a subject in need thereof at a dose equal to half of the main dose, 0.15-3.5 mg/kg, the second administration of an anti-TRBV9 antibody at the main dose of 1-7 mg/kg after at least 3 months, and the subsequent administrations of an anti-TRBV9 antibody at the main dose of 0.3-7 mg/kg at least every 6 months.

In some embodiments of the invention, the method comprises the first administration to a subject in need thereof of an anti-TRBV9 antibody at a dose equal to half of the main dose, 0.15-3.5 mg/kg, the second administration of an anti-TRBV9 antibody at the main dose of 1-7 mg/kg after 3 months, and the subsequent administrations of an anti-TRBV9 antibody at the main dose of 0.3-7 mg/kg every 6 months; or every 9 months; or every 12 months.

In some embodiments of the invention, the method comprises the first administration to a subject in need thereof of an anti-TRBV9 antibody at a dose equal to half of the main dose, 0.15-3.5 mg/kg, the second administration of an anti-TRBV9 antibody at the main dose of 1-7 mg/kg after 6 months, and the subsequent administrations of an anti-TRBV9 antibody at the main dose of 0.3-7 mg/kg every 6 months; or every 9 months; or every 12 months.

In some embodiments of the invention, the method comprises the first administration to a subject in need thereof of an anti-TRBV9 antibody at a dose equal to half of the main dose, 0.15-3.5 mg/kg, the second administration of an anti-TRBV9 antibody at the main dose of 1-7 mg/kg after 9 months, and the subsequent administrations of an anti-TRBV9 antibody at the main dose of 0.3-7 mg/kg every 9 months; or every 12 months.

In some embodiments of the invention, the method comprises the first administration to a subject in need thereof of an anti-TRBV9 antibody at a dose equal to half of the main dose, 0.15-3.5 mg/kg, the second administration of an anti-TRBV9 antibody at the main dose of 1-7 mg/kg after 12 months, and the subsequent administrations of an anti-TRBV9 antibody at the main dose of 0.3-7 mg/kg every 12 months.

In one aspect, the present invention relates to a method of treatment of a disease or disorder mediated by T-lymphocytes bearing the TRBV9 segment within the T-cell receptor, comprising the first administration of an anti-TRBV9 antibody to a subject in need thereof at a dose equal to half of the main dose, 0.5-3.5 mg/kg, the second administration of an anti-TRBV9 antibody at the main dose of 1-7 mg/kg after at least 3 months, and the subsequent administrations of an anti-TRBV9 antibody at the main dose of 1-7 mg/kg at least every 6 months.

In some embodiments, the present invention relates to a method of treatment of a disease or disorder mediated by T-lymphocytes bearing the TRBV9 segment within the T-cell receptor, comprising the first administration to a subject in need thereof of an anti-TRBV9 antibody at a dose equal to half of the main dose, 0.5-3.5 mg/kg, the second administration of an anti-TRBV9 antibody at the main dose of 1-7 mg/kg after 3 months, and the subsequent administrations of an anti-TRBV9 antibody at the main dose of 1-7 mg/kg every 6 months; or every 9 months; or every 12 months.

In some embodiments of the invention, the method comprises the first administration to a subject in need thereof of an anti-TRBV9 antibody at a dose equal to half of the main dose, 0.15-3.5 mg/kg, the second administration of an anti-TRBV9 antibody at the main dose of 1-7 mg/kg after 6 months, and the subsequent administrations of an anti-TRBV9 antibody at the main dose of 1-7 mg/kg every 6 months; or every 9 months; or every 12 months.

In some embodiments of the invention, the method comprises the first administration to a subject in need thereof of an anti-TRBV9 antibody at a dose equal to half of the main dose, 0.15-3.5 mg/kg, the second administration of an anti-TRBV9 antibody at the main dose of 1-7 mg/kg after 9 months, and the subsequent administrations of an anti-TRBV9 antibody at the main dose of 1-7 mg/kg every 9 months; or every 12 months.

In some embodiments of the invention, the method comprises the first administration to a subject in need thereof of an anti-TRBV9 antibody at a dose equal to half of the main dose, 0.15-3.5 mg/kg, the second administration of an anti-TRBV9 antibody at the main dose of 1-7 mg/kg after 12 months, and the subsequent administrations of an anti-TRBV9 antibody at the main dose of 1-7 mg/kg every 12 months.

In some embodiments of the invention, the method comprises the first administration of an anti-TRBV9 antibody at a dose of 0.5 mg/kg, the second administration of an anti-TRBV9 antibody at a dose of 1 mg/kg after at least 3 months, and the subsequent administrations of an anti-TRBV9 antibody at a dose of 1 mg/kg at least every 6 months.

In some embodiments of the invention, the method comprises the first administration of an anti-TRBV9 antibody at a dose of 0.5 mg/kg, the second administration of an anti-TRBV9 antibody at a dose of 1 mg/kg after 3 months, and the subsequent administrations of an anti-TRBV9 antibody at a dose of 1 mg/kg every 6 months; or every 9 months; or every 12 months.

In some embodiments of the invention, the method comprises the first administration of an anti-TRBV9 antibody at a dose of 0.5 mg/kg, the second administration of an anti-TRBV9 antibody at a dose of 1 mg/kg after 6 months, and the subsequent administrations of an anti-TRBV9 antibody at a dose of 1 mg/kg every 6 months; or every 9 months; or every 12 months.

In some embodiments of the invention, the method comprises the first administration of an anti-TRBV9 antibody at a dose of 0.5 mg/kg, the second administration of an anti-TRBV9 antibody at a dose of 1 mg/kg after 9 months, and the subsequent administrations of an anti-TRBV9 antibody at a dose of 1 mg/kg every 9 months; or every 12 months.

In some embodiments of the invention, the method comprises the first administration of an anti-TRBV9 antibody at a dose of 0.5 mg/kg, the second administration of an anti-TRBV9 antibody at a dose of 1 mg/kg after 12 months, and the subsequent administrations of an anti-TRBV9 antibody at a dose of 1 mg/kg every 12 months.

In some embodiments of the invention, the method comprises the first administration of an anti-TRBV9 antibody at a dose of 1 mg/kg, the second administration of an anti-TRBV9 antibody at a dose of 2 mg/kg after at least 3 months, and the subsequent administrations of an anti-TRBV9 antibody at a dose of 2 mg/kg at least every 6 months.

In some embodiments of the invention, the method comprises the first administration of an anti-TRBV9 antibody at a dose of 1 mg/kg, the second administration of an anti-TRBV9 antibody at a dose of 2 mg/kg after 3 months, and the subsequent administrations of an anti-TRBV9 antibody at a dose of 2 mg/kg every 6 months; or every 9 months; or every 12 months.

In some embodiments of the invention, the method comprises the first administration of an anti-TRBV9 antibody at a dose of 1 mg/kg, the second administration of an anti-TRBV9 antibody at a dose of 2 mg/kg after 6 months, and the subsequent administrations of an anti-TRBV9 antibody at a dose of 2 mg/kg every 6 months; or every 9 months; or every 12 months.

In some embodiments of the invention, the method comprises the first administration of an anti-TRBV9 antibody at a dose of 1 mg/kg, the second administration of an anti-TRBV9 antibody at a dose of 2 mg/kg after 9 months, and the subsequent administrations of an anti-TRBV9 antibody at a dose of 2 mg/kg every 9 months; or every 12 months.

In some embodiments of the invention, the method comprises the first administration of an anti-TRBV9 antibody at a dose of 1 mg/kg, the second administration of an anti-TRBV9 antibody at a dose of 2 mg/kg after 12 months, and the subsequent administrations of an anti-TRBV9 antibody at a dose of 2 mg/kg every 12 months.

In some embodiments of the invention, the method comprises the first administration of an anti-TRBV9 antibody at a dose of 1.5 mg/kg, the second administration of an anti-TRBV9 antibody at a dose of 3 mg/kg after at least 3 months, and the subsequent administrations of an anti-TRBV9 antibody at a dose of 3 mg/kg at least every 6 months.

In some embodiments of the invention, the method comprises the first administration of an anti-TRBV9 antibody at a dose of 1.5 mg/kg, the second administration of an anti-TRBV9 antibody at a dose of 3 mg/kg after 3 months, and the subsequent administrations of an anti-TRBV9 antibody at a dose of 3 mg/kg every 6 months; or every 9 months; or every 12 months.

In some embodiments of the invention, the method comprises the first administration of an anti-TRBV9 antibody at a dose of 1.5 mg/kg, the second administration of an anti-TRBV9 antibody at a dose of 3 mg/kg after 6 months, and the subsequent administrations of an anti-TRBV9 antibody at a dose of 3 mg/kg every 6 months; or every 9 months; or every 12 months.

In some embodiments of the invention, the method comprises the first administration of an anti-TRBV9 antibody at a dose of 1.5 mg/kg, the second administration of an anti-TRBV9 antibody at a dose of 3 mg/kg after 9 months, and the subsequent administrations of an anti-TRBV9 antibody at a dose of 3 mg/kg every 9 months; or every 12 months.

In some embodiments of the invention, the method comprises the first administration of an anti-TRBV9 antibody at a dose of 1.5 mg/kg, the second administration of an anti-TRBV9 antibody at a dose of 3 mg/kg after 12 months, and the subsequent administrations of an anti-TRBV9 antibody at a dose of 3 mg/kg every 12 months.

In some embodiments of the invention, the method comprises the first administration of an anti-TRBV9 antibody at a dose of 2 mg/kg, the second administration of an anti-TRBV9 antibody at a dose of 4 mg/kg after at least 3 months, and the subsequent administrations of an anti-TRBV9 antibody at a dose of 4 mg/kg at least every 6 months.

In some embodiments of the invention, the method comprises the first administration of an anti-TRBV9 antibody at a dose of 2 mg/kg, the second administration of an anti-TRBV9 antibody at a dose of 4 mg/kg after 3 months, and the subsequent administrations of an anti-TRBV9 antibody at a dose of 4 mg/kg every 6 months; or every 9 months; or every 12 months.

In some embodiments of the invention, the method comprises the first administration of an anti-TRBV9 antibody at a dose of 2 mg/kg, the second administration of an anti-TRBV9 antibody at a dose of 4 mg/kg after 6 months, and the subsequent administrations of an anti-TRBV9 antibody at a dose of 4 mg/kg every 6 months; or every 9 months; or every 12 months.

In some embodiments of the invention, the method comprises the first administration of an anti-TRBV9 antibody at a dose of 2 mg/kg, the second administration of an anti-TRBV9 antibody at a dose of 4 mg/kg after 9 months, and the subsequent administrations of an anti-TRBV9 antibody at a dose of 4 mg/kg every 9 months; or every 12 months.

In some embodiments of the invention, the method comprises the first administration of an anti-TRBV9 antibody at a dose of 2 mg/kg, the second administration of an anti-TRBV9 antibody at a dose of 4 mg/kg after 12 months, and the subsequent administrations of an anti-TRBV9 antibody at a dose of 4 mg/kg every 12 months.

In some embodiments of the invention, the method comprises the first administration of an anti-TRBV9 antibody at a dose of 2.5 mg/kg, the second administration of an anti-TRBV9 antibody at a dose of 5 mg/kg after at least 3 months, and the subsequent administrations of an anti-TRBV9 antibody at a dose of 5 mg/kg at least every 6 months.

In some embodiments of the invention, the method comprises the first administration of an anti-TRBV9 antibody at a dose of 2,5 mg/kg, the second administration of an anti-TRBV9 antibody at a dose of 5 mg/kg after 3 months, and the subsequent administrations of an anti-TRBV9 antibody at a dose of 5 mg/kg every 6 months; or every 9 months; or every 12 months.

In some embodiments of the invention, the method comprises the first administration of an anti-TRBV9 antibody at a dose of 2,5 mg/kg, the second administration of an anti-TRBV9 antibody at a dose of 5 mg/kg after 6 months, and the subsequent administrations of an anti-TRBV9 antibody at a dose of 5 mg/kg every 6 months; or every 9 months; or every 12 months.

In some embodiments of the invention, the method comprises the first administration of an anti-TRBV9 antibody at a dose of 2,5 mg/kg, the second administration of an anti-TRBV9 antibody at a dose of 5 mg/kg after 9 months, and the subsequent administrations of an anti-TRBV9 antibody at a dose of 5 mg/kg every 9 months; or every 12 months.

In some embodiments of the invention, the method comprises the first administration of an anti-TRBV9 antibody at a dose of 2,5 mg/kg, the second administration of an anti-TRBV9 antibody at a dose of 5 mg/kg after 12 months, and the subsequent administrations of an anti-TRBV9 antibody at a dose of 5 mg/kg every 12 months.

In some embodiments of the invention, the method comprises the first administration of an anti-TRBV9 antibody at a dose of 3 mg/kg, the second administration of an anti-TRBV9 antibody at a dose of 6 mg/kg after at least 3 months, and the subsequent administrations of an anti-TRBV9 antibody at a dose of 6 mg/kg at least every 6 months.

In some embodiments of the invention, the method comprises the first administration of an anti-TRBV9 antibody at a dose of 3 mg/kg, the second administration of an anti-TRBV9 antibody at a dose of 6 mg/kg after 3 months, and the subsequent administrations of an anti-TRBV9 antibody at a dose of 6 mg/kg every 6 months; or every 9 months; or every 12 months.

In some embodiments of the invention, the method comprises the first administration of an anti-TRBV9 antibody at a dose of 3 mg/kg, the second administration of an anti-TRBV9 antibody at a dose of 6 mg/kg after 6 months, and the subsequent administrations of an anti-TRBV9 antibody at a dose of 6 mg/kg every 6 months; or every 9 months; or every 12 months.

In some embodiments of the invention, the method comprises the first administration of an anti-TRBV9 antibody at a dose of 3 mg/kg, the second administration of an anti-TRBV9 antibody at a dose of 6 mg/kg after 9 months, and the subsequent administrations of an anti-TRBV9 antibody at a dose of 6 mg/kg every 9 months; or every 12 months.

In some embodiments of the invention, the method comprises the first administration of an anti-TRBV9 antibody at a dose of 3 mg/kg, the second administration of an anti-TRBV9 antibody at a dose of 6 mg/kg after 12 months, and the subsequent administrations of an anti-TRBV9 antibody at a dose of 6 mg/kg every 12 months.

In some embodiments of the invention, the method comprises the first administration of an anti-TRBV9 antibody at a dose of 3.5 mg/kg, the second administration of an anti-TRBV9 antibody at a dose of 7 mg/kg after at least 3 months, and the subsequent administrations of an anti-TRBV9 antibody at a dose of 7 mg/kg at least every 6 months.

In some embodiments of the invention, the method comprises the first administration of an anti-TRBV9 antibody at a dose of 3.5 mg/kg, the second administration of an anti-TRBV9 antibody at a dose of 7 mg/kg after 3 months, and the subsequent administrations of an anti-TRBV9 antibody at a dose of 7 mg/kg every 6 months; or every 9 months; or every 12 months.

In some embodiments of the invention, the method comprises the first administration of an anti-TRBV9 antibody at a dose of 3.5 mg/kg, the second administration of an anti-TRBV9 antibody at a dose of 7 mg/kg after 6 months, and the subsequent administrations of an anti-TRBV9 antibody at a dose of 7 mg/kg every 6 months; or every 9 months; or every 12 months.

In some embodiments of the invention, the method comprises the first administration of an anti-TRBV9 antibody at a dose of 3.5 mg/kg, the second administration of an anti-TRBV9 antibody at a dose of 7 mg/kg after 9 months, and the subsequent administrations of an anti-TRBV9 antibody at a dose of 7 mg/kg every 9 months; or every 12 months.

In some embodiments of the invention, the method comprises the first administration of an anti-TRBV9 antibody at a dose of 3.5 mg/kg, the second administration of an anti-TRBV9 antibody at a dose of 7 mg/kg after 12 months, and the subsequent administrations of an anti-TRBV9 antibody at a dose of 7 mg/kg every 12 months.

In some embodiments of the invention, the method comprises the first administration of an anti-TRBV9 antibody performed at a dose of 4 mg/kg, the second administration of an anti-TRBV9 antibody is performed at a dose of 8 mg/kg after 3 months, and the subsequent administrations of an anti-TRBV9 antibody are performed at a dose of 8 mg/kg every 6 months; or every 9 months; or every 12 months.

In some embodiments of the invention, the method comprises the first administration of an anti-TRBV9 antibody at a dose of 4 mg/kg, the second administration of an anti-TRBV9 antibody at a dose of 8 mg/kg after 6 months, and the subsequent administrations of an anti-TRBV9 antibody at a dose of 8 mg/kg every 6 months; or every 9 months; or every 12 months.

In some embodiments of the invention, the method comprises the first administration of an anti-TRBV9 antibody at a dose of 4 mg/kg, the second administration of an anti-TRBV9 antibody at a dose of 8 mg/kg after 9 months, and the subsequent administrations of an anti-TRBV9 antibody at a dose of 8 mg/kg every 9 months; or every 12 months.

In some embodiments of the invention, the method comprises the first administration of an anti-TRBV9 antibody at a dose of 4 mg/kg, the second administration of an anti-TRBV9 antibody at a dose of 8 mg/kg after 12 months, and the subsequent administrations of an anti-TRBV9 antibody at a dose of 8 mg/kg every 12 months.

In some embodiments of the invention, the method comprises the first administration of an anti-TRBV9 antibody performed at a dose of 4.5 mg/kg, the second administration of an anti-TRBV9 antibody is performed at a dose of 9 mg/kg after 3 months, and the subsequent administrations of an anti-TRBV9 antibody are performed at a dose of 9 mg/kg every 6 months; or every 9 months; or every 12 months.

In some embodiments of the invention, the method comprises the first administration of an anti-TRBV9 antibody at a dose of 4.5 mg/kg, the second administration of an anti-TRBV9 antibody at a dose of 9 mg/kg after 6 months, and the subsequent administrations of an anti-TRBV9 antibody at a dose of 9 mg/kg every 6 months; or every 9 months; or every 12 months.

In some embodiments of the invention, the method comprises the first administration of an anti-TRBV9 antibody at a dose of 4.5 mg/kg, the second administration of an anti-TRBV9 antibody at a dose of 9 mg/kg after 9 months, and the subsequent administrations of an anti-TRBV9 antibody at a dose of 9 mg/kg every 9 months; or every 12 months.

In some embodiments of the invention, the method comprises the first administration of an anti-TRBV9 antibody at a dose of 4.5 mg/kg, the second administration of an anti-TRBV9 antibody at a dose of 9 mg/kg after 12 months, and the subsequent administrations of an anti-TRBV9 antibody at a dose of 9 mg/kg every 12 months.

In some embodiments of the invention, the method comprises the first administration of an anti-TRBV9 antibody performed at a dose of 5 mg/kg, the second administration of an anti-TRBV9 antibody is performed at a dose of 10 mg/kg after 3 months, and the subsequent administrations of an anti-TRBV9 antibody are performed at a dose of 10 mg/kg every 6 months; or every 9 months; or every 12 months.

In some embodiments of the invention, the method comprises the first administration of an anti-TRBV9 antibody at a dose of 5 mg/kg, the second administration of an anti-TRBV9 antibody at a dose of 10 mg/kg after 6 months, and the subsequent administrations of an anti-TRBV9 antibody at a dose of 10 mg/kg every 6 months; or every 9 months; or every 12 months.

In some embodiments of the invention, the method comprises the first administration of an anti-TRBV9 antibody at a dose of 5 mg/kg, the second administration of an anti-TRBV9 antibody at a dose of 10 mg/kg after 9 months, and the subsequent administrations of an anti-TRBV9 antibody at a dose of 10 mg/kg every 9 months; or every 12 months;

In some embodiments of the invention, the method comprises the first administration of an anti-TRBV9 antibody at a dose of 5 mg/kg, the second administration of an anti-TRBV9 antibody at a dose of 10 mg/kg after 12 months, and the subsequent administrations of an anti-TRBV9 antibody at a dose of 10 mg/kg every 12 months.

In one aspect, the present invention relates to a method of treatment of a disease or disorder mediated by T-lymphocytes bearing the TRBV9 segment within the T-cell receptor, comprising the first administration to a subject in need thereof of an anti-TRBV9 antibody at a dose of 75-300 mg, the second administration of an anti-TRBV9 antibody at the main dose of 325-675 mg after at least 3 months, and the subsequent administrations of an anti-TRBV9 antibody at the main dose of 325-675 mg at least every 6 months.

In some embodiments of the invention, the method comprises the first administration to a subject in need thereof of an anti-TRBV9 antibody at a dose of 75-300 mg, the second administration of an anti-TRBV9 antibody at the main dose of 325-675 mg after 3 months, and the subsequent administrations of an anti-TRBV9 antibody at the main dose of 325-675 mg every 6 months; or every 9 months; or every 12 months.

In some embodiments of the invention, the method comprises the first administration to a subject in need thereof of an anti-TRBV9 antibody at a dose of 75-300 mg, the second administration of an anti-TRBV9 antibody at the main dose of 325-675 mg after 6 months, and the subsequent administrations of an anti-TRBV9 antibody at the main dose of 325-675 mg every 6 months; or every 9 months; or every 12 months.

In some embodiments of the invention, the method comprises the first administration to a subject in need thereof of an anti-TRBV9 antibody at a dose of 75-300 mg, the second administration of an anti-TRBV9 antibody at the main dose of 325-675 mg after 9 months, and the subsequent administrations of an anti-TRBV9 antibody at the main dose of 325-675 mg every 9 months; or every 12 months.

In some embodiments of the invention, the method comprises the first administration to a subject in need thereof of an anti-TRBV9 antibody at a dose of 75-300 mg, the second administration of an anti-TRBV9 antibody at the main dose of 325-675 mg after 12 months, and the subsequent administrations of an anti-TRBV9 antibody at the main dose of 325-675 mg every 12 months.

In one aspect, the present invention relates to a method of treatment of a disease or disorder mediated by T-lymphocytes bearing the TRBV9 segment within the T-cell receptor, comprising the first administration to a subject in need thereof of an anti-TRBV9 antibody at a dose of 100-275 mg, the second administration of an anti-TRBV9 antibody at the main dose of 350-650 mg after at least 3 months, and the subsequent administrations of an anti-TRBV9 antibody at the main dose of 350-650 mg at least every 6 months.

In some embodiments of the invention, the method comprises the first administration to a subject in need thereof of an anti-TRBV9 antibody at a dose of 100-275 mg, the second administration of an anti-TRBV9 antibody at the main dose of 350-650 mg after 3 months, and the subsequent administrations of an anti-TRBV9 antibody at the main dose of 350-650 mg every 6 months; or every 9 months; or every 12 months.

In some embodiments of the invention, the method comprises the first administration to a subject in need thereof of an anti-TRBV9 antibody at a dose of 100-275 mg, the second administration of an anti-TRBV9 antibody at the main dose of 350-650 mg after 6 months, and the subsequent administrations of an anti-TRBV9 antibody at the main dose of 350-650 mg every 6 months; or every 9 months; or every 12 months

In some embodiments of the invention, the method comprises the first administration to a subject in need thereof of an anti-TRBV9 antibody at a dose of 100-275 mg, the second administration of an anti-TRBV9 antibody at the main dose of 350-650 mg after 9 months, and the subsequent administrations of an anti-TRBV9 antibody at the main dose of 350-650 mg every 9 months; or every 12 months.

In some embodiments of the invention, the method comprises the first administration to a subject in need thereof of an anti-TRBV9 antibody at a dose of 100-275 mg, the second administration of an anti-TRBV9 antibody at the main dose of 350-650 mg after 12 months, and the subsequent administrations of an anti-TRBV9 antibody at the main dose of 350-650 mg every 12 months.

In one aspect, the present invention relates to a method of treatment of a disease or disorder mediated by T-lymphocytes bearing the TRBV9 segment within the T-cell receptor, comprising the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after at least 3 months, and the subsequent administrations of an anti-TRBV9 antibody at the main dose of 350-400 mg; or 475- 525 mg; or 600-650 mg at least every 6 months.

In some embodiments of the invention, the method comprises
(i) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 3 months, and the subsequent administrations of an anti-TRBV9 antibody at the main dose of 350-400 mg; or 475- 525 mg; or 600-650 mg every 6 months; or
(ii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 3 months, and the subsequent administrations of an anti-TRBV9 antibody at the main dose of 350-400 mg; or 475- 525 mg; or 600-650 mg every 9 months; or
(iii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 3 months, and the subsequent administrations of an anti-TRBV9 antibody at the main dose of 350-400 mg; or 475- 525 mg; or 600-650 mg every 12 months; or
(iv) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 6 months, and the subsequent administrations of an anti-TRBV9 antibody at the main dose of 350-400 mg; or 475- 525 mg; or 600-650 mg every 6 months; or
(v) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 6 months, and the subsequent administrations of an anti-TRBV9 antibody at the main dose of 350-400 mg; or 475- 525 mg; or 600-650 mg every 9 months; or
(vi) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 6 months, and the subsequent administrations of an anti-TRBV9 antibody at the main dose of 350-400 mg; or 475- 525 mg; or 600-650 mg every 12 months; or
(vii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 9 months, and the subsequent administrations of an anti-TRBV9 antibody at the main dose of 350-400 mg; or 475- 525 mg; or 600-650 mg every 9 months; or
(viii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 9 months, and the subsequent administrations of an anti-TRBV9 antibody at the main dose of 350-400 mg; or 475- 525 mg; or 600-650 mg every 12 months; or
(ix) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 12 months, and the subsequent administrations of an anti-TRBV9 antibody at the main dose of 350-400 mg; or 475- 525 mg; or 600-650 mg every 12 months.

In one aspect, the present invention relates to a method of treatment of a disease or disorder mediated by T-lymphocytes bearing the TRBV9 segment within the T-cell receptor, comprising the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after at least 3 months, and the subsequent administrations of an anti-TRBV9 antibody at the main dose of 365-385 mg; or 490- 510 mg; or 615-635 mg at least every 6 months.

In some embodiments of the invention, the method comprises
(i) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 3 months, and the subsequent administrations of an anti-TRBV9 antibody at the main dose of 365-385 mg; or 490- 510 mg; or 615-635 mg every 6 months; or
(ii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 3 months, and the subsequent administrations of an anti-TRBV9 antibody at the main dose of 365-385 mg; or 490- 510 mg; or 615-635 mg every 9 months; or
(iii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 3 months, and the subsequent administrations of an anti-TRBV9 antibody at the main dose of 365-385 mg; or 490- 510 mg; or 615-635 mg every 12 months; or
(iv) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 6 months, and the subsequent administrations of an anti-TRBV9 antibody at the main dose of 365-385 mg; or 490- 510 mg; or 615-635 mg every 6 months; or
(v) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 6 months, and the subsequent administrations of an anti-TRBV9 antibody at the main dose of 365-385 mg; or 490- 510 mg; or 615-635 mg every 9 months; or
(vi) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 6 months, and the subsequent administrations of an anti-TRBV9 antibody at the main dose of 365-385 mg; or 490- 510 mg; or 615-635 mg every 12 months; or
(vii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 9 months, and the subsequent administrations of an anti-TRBV9 antibody at the main dose of 365-385 mg; or 490- 510 mg; or 615-635 mg every 9 months; or
(viii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 9 months, and the subsequent administrations of an anti-TRBV9 antibody at the main dose of 365-385 mg; or 490- 510 mg; or 615-635 mg every 12 months; or
(ix) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 12 months, and the subsequent administrations of an anti-TRBV9 antibody at the main dose of 365-385 mg; or 490- 510 mg; or 615-635 mg every 12 months.

In one aspect, the present invention relates to a method of treatment of a disease or disorder mediated by T-lymphocytes bearing the TRBV9 segment within the T-cell receptor, comprising the first administration to a subject in need thereof of an anti-TRBV9 antibody at a dose of 120 mg; or 125 mg; or 130 mg; or 245 mg; or 250 mg; or 255 mg, the second administration of an anti-TRBV9 antibody at the main dose of 370 mg; or 375 mg; or 380 mg; or 495 mg; or 500 mg; or 505 mg; or 620 mg; or 625 mg; or 630 mg after at least 3 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg at least every 6 months.

In some embodiments, the present invention relates to a method of treatment of a disease or disorder mediated by T-lymphocytes bearing the TRBV9 segment within the T-cell receptor, comprising (i) the first administration to a subject in need thereof of an anti-TRBV9 antibody at a dose of 120 mg; or 125 mg; or 130 mg; or 245 mg; or 250 mg; or 255 mg, the second administration of an anti-TRBV9 antibody at the main dose of 370 mg; or 375 mg; or 380 mg; or 495 mg; or 500 mg; or 505 mg; or 620 mg; or 625 mg; or 630 mg after 3 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 6 months; or
(ii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 3 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 9 months; or
(iii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 3 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months; or
(iv) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 6 months; or
(v) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 9 months; or
(vi) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months; or
(vii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 9 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 9 months; or
(viii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 9 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months; or
(ix) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 12 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months.

In some embodiments of the invention, the method comprises the first administration of an anti-TRBV9 antibody at a dose of 120 mg, the second administration of an anti-TRBV9 antibody at a dose of 370 mg after at least 3 months, and the subsequent administrations of an anti-TRBV9 antibody at a dose of 370 mg at least every 6 months.

In some embodiments of the invention, the method comprises the first administration of an anti-TRBV9 antibody at a dose of 120 mg, the second administration of an anti-TRBV9 antibody at a dose of 370 mg after 3 months; and the subsequent administrations of an anti-TRBV9 antibody at a dose of 370 mg every 6 months; or every 9 months; or every 12 months.

In some embodiments of the invention, the method comprises the first administration of an anti-TRBV9 antibody at a dose of 120 mg, the second administration of an anti-TRBV9 antibody at a dose of 370 mg after 6 months; and the subsequent administrations of an anti-TRBV9 antibody at a dose of 370 mg every 6 months; or every 9 months; or every 12 months.

In some embodiments of the invention, the method comprises the first administration of an anti-TRBV9 antibody at a dose of 120 mg, the second administration of an anti-TRBV9 antibody at a dose of 370 mg after 9 months; and the subsequent administrations of an anti-TRBV9 antibody at a dose of 370 mg every 9 months; or every 12 months.

In some embodiments of the invention, the method comprises the first administration of an anti-TRBV9 antibody at a dose of 120 mg, the second administration of an anti-TRBV9 antibody at a dose of 370 mg after 12 months, and the subsequent administrations of an anti-TRBV9 antibody at 370 mg every 12 months.

In some embodiments of the invention, the method comprises the first administration of an anti-TRBV9 antibody at a dose of 125 mg, the second administration of an anti-TRBV9 antibody at a dose of 375 mg after at least 3 months, and the subsequent administrations of an anti-TRBV9 antibody at a dose of 375 mg at least every 6 months.

In some embodiments of the invention, the method comprises the first administration of an anti-TRBV9 antibody at a dose of 125 mg, the second administration of an anti-TRBV9 antibody at a dose of 375 mg after 3 months; and the subsequent administrations of an anti-TRBV9 antibody at a dose of 375 mg every 6 months; or every 9 months; or every 12 months.

In some embodiments of the invention, the method comprises the first administration of an anti-TRBV9 antibody at a dose of 125 mg, the second administration of an anti-TRBV9 antibody at a dose of 375 mg after 6 months; and the subsequent administrations of an anti-TRBV9 antibody at a dose of 375 mg every 6 months; or every 9 months; or every 12 months.

In some embodiments of the invention, the method comprises the first administration of an anti-TRBV9 antibody at a dose of 125 mg, the second administration of an anti-TRBV9 antibody at a dose of 375 mg after 9 months; and the subsequent administrations of an anti-TRBV9 antibody at a dose of 375 mg every 9 months; or every 12 months.

In some embodiments of the invention, the method comprises the first administration of an anti-TRBV9 antibody at a dose of 125 mg, the second administration of an anti-TRBV9 antibody at a dose of 375 mg after 12 months, and the subsequent administrations of an anti-TRBV9 antibody at 375 mg every 12 months.

In some embodiments of the invention, the method comprises the first administration of an anti-TRBV9 antibody at a dose of 130 mg, the second administration of an anti-TRBV9 antibody at a dose of 380 mg after at least 3 months, and the subsequent administrations of an anti-TRBV9 antibody at a dose of 380 mg at least every 6 months.

In some embodiments of the invention, the method comprises the first administration of an anti-TRBV9 antibody at a dose of 130 mg, the second administration of an anti-TRBV9 antibody at a dose of 380 mg after 3 months; and the subsequent administrations of an anti-TRBV9 antibody at a dose of 380 mg every 6 months; or every 9 months; or every 12 months.

In some embodiments of the invention, the method comprises the first administration of an anti-TRBV9 antibody at a dose of 130 mg, the second administration of an anti-TRBV9 antibody at a dose of 380 mg after 6 months; and the subsequent administrations of an anti-TRBV9 antibody at a dose of 380 mg every 6 months; or every 9 months; or every 12 months.

In some embodiments of the invention, the method comprises the first administration of an anti-TRBV9 antibody at a dose of 130 mg, the second administration of an anti-TRBV9 antibody at a dose of 380 mg after 9 months; and the subsequent administrations of an anti-TRBV9 antibody at a dose of 380 mg every 9 months; or every 12 months.

In some embodiments of the invention, the method comprises the first administration of an anti-TRBV9 antibody at a dose of 130 mg, the second administration of an anti-TRBV9 antibody at a dose of 380 mg after 12 months, and the subsequent administrations of an anti-TRBV9 antibody at 380 mg every 12 months.

In some embodiments of the invention, the method comprises the first administration of an anti-TRBV9 antibody at a dose of 120 mg, the second administration of an anti-TRBV9 antibody at a dose of 370 mg after at least 3 months, and the subsequent administrations of an anti-TRBV9 antibody at a dose of 370 mg at least every 6 months based on subject's body weight from 30 to 50 kg.

In some embodiments of the invention, the method comprises the first administration of an anti-TRBV9 antibody at a dose of 120 mg, the second administration of an anti-TRBV9 antibody at a dose of 370 mg after 3 months; and the subsequent administrations of an anti-TRBV9 antibody at a dose of 370 mg every 6 months; or every 9 months; or every 12 months based on subject's body weight from 30 to 50 kg.

In some embodiments of the invention, the method comprises the first administration of an anti-TRBV9 antibody at a dose of 120 mg, the second administration of an anti-TRBV9 antibody at a dose of 370 mg after 6 months; and the subsequent administrations of an anti-TRBV9 antibody at a dose of 370 mg every 6 months; or every 9 months; or every 12 months based on subject's body weight from 30 to 50 kg.

In some embodiments of the invention, the method comprises the first administration of an anti-TRBV9 antibody at a dose of 120 mg, the second administration of an anti-TRBV9 antibody at a dose of 370 mg after 9 months; and the subsequent administrations of an anti-TRBV9 antibody at a dose of 370 mg every 9 months; or every 12 months based on subject's body weight from 30 to 50 kg.

In some embodiments of the invention, the method comprises the first administration of an anti-TRBV9 antibody at a dose of 120 mg, the second administration of an anti-TRBV9 antibody at a dose of 370 mg after 12 months, and the subsequent administrations of an anti-TRBV9 antibody at 370 mg every 12 months based on subject's body weight from 30 to 50 kg.

In some embodiments of the invention, the method comprises the first administration of an anti-TRBV9 antibody at a dose of 125 mg, the second administration of an anti-TRBV9 antibody at a dose of 375 mg after at least 3 months, and the subsequent administrations of an anti-TRBV9 antibody at a dose of 375 mg at least every 6 months based on subject's body weight from 30 to 50 kg.

In some embodiments of the invention, the method comprises the first administration of an anti-TRBV9 antibody at a dose of 125 mg, the second administration of an anti-TRBV9 antibody at a dose of 375 mg after 3 months; and the subsequent administrations of an anti-TRBV9 antibody at a dose of 375 mg every 6 months; or every 9 months; or every 12 months based on subject's body weight from 30 to 50 kg.

In some embodiments of the invention, the method comprises the first administration of an anti-TRBV9 antibody at a dose of 125 mg, the second administration of an anti-TRBV9 antibody at a dose of 375 mg after 6 months; and the subsequent administrations of an anti-TRBV9 antibody at a dose of 375 mg every 6 months; or every 9 months; or every 12 months based on subject's body weight from 30 to 50 kg.

In some embodiments of the invention, the method comprises the first administration of an anti-TRBV9 antibody at a dose of 125 mg, the second administration of an anti-TRBV9 antibody at a dose of 375 mg after 9 months; and the subsequent administrations of an anti-TRBV9 antibody at a dose of 375 mg every 9 months; or every 12 months based on subject's body weight from 30 to 50 kg.

In some embodiments of the invention, the method comprises the first administration of an anti-TRBV9 antibody at a dose of 125 mg, the second administration of an anti-TRBV9 antibody at a dose of 375 mg after 12 months, and the subsequent administrations of an anti-TRBV9 antibody at 375 mg every 12 months based on subject's body weight from 30 to 50 kg.

In some embodiments of the invention, the method comprises the first administration of an anti-TRBV9 antibody at a dose of 130 mg, the second administration of an anti-TRBV9 antibody at a dose of 380 mg after at least 3 months, and the subsequent administrations of an anti-TRBV9 antibody at a dose of 380 mg at least every 6 months based on subject's body weight from 30 to 50 kg.

In some embodiments of the invention, the method comprises the first administration of an anti-TRBV9 antibody at a dose of 130 mg, the second administration of an anti-TRBV9 antibody at a dose of 380 mg after 3 months; and the subsequent administrations of an anti-TRBV9 antibody at a dose of 380 mg every 6 months; or every 9 months; or every 12 months based on subject's body weight from 30 to 50 kg.

In some embodiments of the invention, the method comprises the first administration of an anti-TRBV9 antibody at a dose of 130 mg, the second administration of an anti-TRBV9 antibody at a dose of 380 mg after 6 months; and the subsequent administrations of an anti-TRBV9 antibody at a dose of 380 mg every 6 months; or every 9 months; or every 12 months based on subject's body weight from 30 to 50 kg.

In some embodiments of the invention, the method comprises the first administration of an anti-TRBV9 antibody at a dose of 130 mg, the second administration of an anti-TRBV9 antibody at a dose of 380 mg after 9 months; and the subsequent administrations of an anti-TRBV9 antibody at a dose of 380 mg every 9 months; or every 12 months based on subject's body weight from 30 to 50 kg.

In some embodiments of the invention, the method comprises the first administration of an anti-TRBV9 antibody at a dose of 130 mg, the second administration of an anti-TRBV9 antibody at a dose of 380 mg after 12 months, and the subsequent administrations of an anti-TRBV9 antibody at 380 mg every 12 months based on subject's body weight from 30 to 50 kg.

In some embodiments of the invention, the method comprises the first administration of an anti-TRBV9 antibody at a dose of 245 mg, the second administration of an anti-TRBV9 antibody at a dose of 495 mg after at least 3 months, and the subsequent administrations of an anti-TRBV9 antibody at a dose of 495 mg at least every 6 months.

In some embodiments of the invention, the method comprises the first administration of an anti-TRBV9 antibody at a dose of 245 mg, the second administration of an anti-TRBV9 antibody at a dose of 495 mg after 3 months; and the subsequent administrations of an anti-TRBV9 antibody at a dose of 495 mg every 6 months; or every 9 months; or every 12 months.

In some embodiments of the invention, the method comprises the first administration of an anti-TRBV9 antibody at a dose of 245 mg, the second administration of an anti-TRBV9 antibody at a dose of 495 mg after 6 months; and the subsequent administrations of an anti-TRBV9 antibody at a dose of 495 mg every 6 months; or every 9 months; or every 12 months.

In some embodiments of the invention, the method comprises the first administration of an anti-TRBV9 antibody at a dose of 245 mg, the second administration of an anti-TRBV9 antibody at a dose of 495 mg after 9 months; and the subsequent administrations of an anti-TRBV9 antibody at a dose of 495 mg every 9 months; or every 12 months.

In some embodiments of the invention, the method comprises the first administration of an anti-TRBV9 antibody at a dose of 245 mg, the second administration of an anti-TRBV9 antibody at a dose of 495 mg after 12 months, and the subsequent administrations of an anti-TRBV9 antibody at 495 mg every 12 months.

In some embodiments of the invention, the method comprises the first administration of an anti-TRBV9 antibody at a dose of 250 mg, the second administration of an anti-TRBV9 antibody at a dose of 500 mg after at least 3 months, and the subsequent administrations of an anti-TRBV9 antibody at a dose of 500 mg at least every 6 months.

In some embodiments of the invention, the method comprises the first administration of an anti-TRBV9 antibody at a dose of 250 mg, the second administration of an anti-TRBV9 antibody at a dose of 500 mg after 3 months; and the subsequent administrations of an anti-TRBV9 antibody at a dose of 500 mg every 6 months; or every 9 months; or every 12 months.

In some embodiments of the invention, the method comprises the first administration of an anti-TRBV9 antibody at a dose of 250 mg, the second administration of an anti-TRBV9 antibody at a dose of 500 mg after 6 months; and the subsequent administrations of an anti-TRBV9 antibody at a dose of 500 mg every 6 months; or every 9 months; or every 12 months.

In some embodiments of the invention, the method comprises the first administration of an anti-TRBV9 antibody at a dose of 250 mg, the second administration of an anti-TRBV9 antibody at a dose of 500 mg after 9 months; and the subsequent administrations of an anti-TRBV9 antibody at a dose of 500 mg every 9 months; or every 12 months.

In some embodiments of the invention, the method comprises the first administration of an anti-TRBV9 antibody at a dose of 250 mg, the second administration of an anti-TRBV9 antibody at a dose of 500 mg after 12 months, and the subsequent administrations of an anti-TRBV9 antibody at 500 mg every 12 months.

In some embodiments of the invention, the method comprises the first administration of an anti-TRBV9 antibody at a dose of 255 mg, the second administration of an anti-TRBV9 antibody at a dose of 505 mg after at least 3 months, and the subsequent administrations of an anti-TRBV9 antibody at a dose of 505 mg at least every 6 months.

In some embodiments of the invention, the method comprises the first administration of an anti-TRBV9 antibody at a dose of 255 mg, the second administration of an anti-TRBV9 antibody at a dose of 505 mg after 3 months; and the subsequent administrations of an anti-TRBV9 antibody at a dose of 505 mg every 6 months; or every 9 months; or every 12 months.

In some embodiments of the invention, the method comprises the first administration of an anti-TRBV9 antibody at a dose of 255 mg, the second administration of an anti-TRBV9 antibody at a dose of 505 mg after 6 months; and the subsequent administrations of an anti-TRBV9 antibody at a dose of 505 mg every 6 months; or every 9 months; or every 12 months.

In some embodiments of the invention, the method comprises the first administration of an anti-TRBV9 antibody at a dose of 255 mg, the second administration of an anti-TRBV9 antibody at a dose of 505 mg after 9 months; and the subsequent administrations of an anti-TRBV9 antibody at a dose of 505 mg every 9 months; or every 12 months.

In some embodiments of the invention, the method comprises the first administration of an anti-TRBV9 antibody at a dose of 255 mg, the second administration of an anti-TRBV9 antibody at a dose of 505 mg after 12 months, and the subsequent administrations of an anti-TRBV9 antibody at 505 mg every 12 months.

In some embodiments of the invention, the method comprises the first administration of an anti-TRBV9 antibody at a dose of 245 mg, the second administration of an anti-TRBV9 antibody at a dose of 495 mg after at least 3 months, and the subsequent administrations of an anti-TRBV9 antibody at a dose of 495 mg at least every 6 months based on subject's body weight from 51 to 89 kg.

In some embodiments of the invention, the method comprises the first administration of an anti-TRBV9 antibody at a dose of 245 mg, the second administration of an anti-TRBV9 antibody at a dose of 495 mg after 3 months; and the subsequent administrations of an anti-TRBV9 antibody at a dose of 495 mg every 6 months; or every 9 months; or every 12 months based on subject's body weight from 51 to 89 kg.

In some embodiments of the invention, the method comprises the first administration of an anti-TRBV9 antibody at a dose of 245 mg, the second administration of an anti-TRBV9 antibody at a dose of 495 mg after 6 months; and the subsequent administrations of an anti-TRBV9 antibody at a dose of 495 mg every 6 months; or every 9 months; or every 12 months based on subject's body weight from 51 to 89 kg.

In some embodiments of the invention, the method comprises the first administration of an anti-TRBV9 antibody at a dose of 245 mg, the second administration of an anti-TRBV9 antibody at a dose of 495 mg after 9 months; and the subsequent administrations of an anti-TRBV9 antibody at a dose of 495 mg every 9 months; or every 12 months based on subject's body weight from 51 to 89 kg.

In some embodiments of the invention, the method comprises the first administration of an anti-TRBV9 antibody at a dose of 245 mg, the second administration of an anti-TRBV9 antibody at a dose of 495 mg after 12 months, and the subsequent administrations of an anti-TRBV9 antibody at 495 mg every 12 months based on subject's body weight from 51 to 89 kg.

In some embodiments of the invention, the method comprises the first administration of an anti-TRBV9 antibody at a dose of 250 mg, the second administration of an anti-TRBV9 antibody at a dose of 500 mg after at least 3 months, and the subsequent administrations of an anti-TRBV9 antibody at a dose of 500 mg at least every 6 months based on subject's body weight from 51 to 89 kg.

In some embodiments of the invention, the method comprises the first administration of an anti-TRBV9 antibody at a dose of 250 mg, the second administration of an anti-TRBV9 antibody at a dose of 500 mg after 3 months; and the subsequent administrations of an anti-TRBV9 antibody at a dose of 500 mg every 6 months; or every 9 months; or every 12 months based on subject's body weight from 51 to 89 kg.

In some embodiments of the invention, the method comprises the first administration of an anti-TRBV9 antibody at a dose of 250 mg, the second administration of an anti-TRBV9 antibody at a dose of 500 mg after 6 months; and the subsequent administrations of an anti-TRBV9 antibody at a dose of 500 mg every 6 months; or every 9 months; or every 12 months based on subject's body weight from 51 to 89 kg.

In some embodiments of the invention, the method comprises the first administration of an anti-TRBV9 antibody at a dose of 250 mg, the second administration of an anti-TRBV9 antibody at a dose of 500 mg after 9 months; and the subsequent administrations of an anti-TRBV9 antibody at a dose of 500 mg every 9 months; or every 12 months based on subject's body weight from 51 to 89 kg.

In some embodiments of the invention, the method comprises the first administration of an anti-TRBV9 antibody at a dose of 250 mg, the second administration of an anti-TRBV9 antibody at a dose of 500 mg after 12 months, and the subsequent administrations of an anti-TRBV9 antibody at 500 mg every 12 months based on subject's body weight from 51 to 89 kg.

In some embodiments of the invention, the method comprises the first administration of an anti-TRBV9 antibody at a dose of 255 mg, the second administration of an anti-TRBV9 antibody at a dose of 505 mg after at least 3 months, and the subsequent administrations of an anti-TRBV9 antibody at a dose of 505 mg at least every 6 months based on subject's body weight from 51 to 89 kg.

In some embodiments of the invention, the method comprises the first administration of an anti-TRBV9 antibody at a dose of 255 mg, the second administration of an anti-TRBV9 antibody at a dose of 505 mg after 3 months; and the subsequent administrations of an anti-TRBV9 antibody at a dose of 505 mg every 6 months; or every 9 months; or every 12 months based on subject's body weight from 51 to 89 kg.

In some embodiments of the invention, the method comprises the first administration of an anti-TRBV9 antibody at a dose of 255 mg, the second administration of an anti-TRBV9 antibody at a dose of 505 mg after 6 months; and the subsequent administrations of an anti-TRBV9 antibody at a dose of 505 mg every 6 months; or every 9 months; or every 12 months based on subject's body weight from 51 to 89 kg.

In some embodiments of the invention, the method comprises the first administration of an anti-TRBV9 antibody at a dose of 255 mg, the second administration of an anti-TRBV9 antibody at a dose of 505 mg after 9 months; and the subsequent administrations of an anti-TRBV9 antibody at a dose of 505 mg every 9 months; or every 12 months based on subject's body weight from 51 to 89 kg.

In some embodiments of the invention, the method comprises the first administration of an anti-TRBV9 antibody at a dose of 255 mg, the second administration of an anti-TRBV9 antibody at a dose of 505 mg after 12 months, and the subsequent administrations of an anti-TRBV9 antibody at 505 mg every 12 months based on subject's body weight from 51 to 89 kg.

In some embodiments of the invention, the method comprises the first administration of an anti-TRBV9 antibody at a dose of 245 mg, the second administration of an anti-TRBV9 antibody at a dose of 620 mg after at least 3 months, and the subsequent administrations of an anti-TRBV9 antibody at a dose of 620 mg at least every 6 months.

In some embodiments of the invention, the method comprises the first administration of an anti-TRBV9 antibody at a dose of 245 mg, the second administration of an anti-TRBV9 antibody at a dose of 620 mg after 3 months; and the subsequent administrations of an anti-TRBV9 antibody at a dose of 620 mg every 6 months; or every 9 months; or every 12 months.

In some embodiments of the invention, the method comprises the first administration of an anti-TRBV9 antibody at a dose of 245 mg, the second administration of an anti-TRBV9 antibody at a dose of 620 mg after 6 months; and the subsequent administrations of an anti-TRBV9 antibody at a dose of 620 mg every 6 months; or every 9 months; or every 12 months.

In some embodiments of the invention, the method comprises the first administration of an anti-TRBV9 antibody at a dose of 245 mg, the second administration of an anti-TRBV9 antibody at a dose of 620 mg after 9 months; and the subsequent administrations of an anti-TRBV9 antibody at a dose of 620 mg every 9 months; or every 12 months.

In some embodiments of the invention, the method comprises the first administration of an anti-TRBV9 antibody at a dose of 245 mg, the second administration of an anti-TRBV9 antibody at a dose of 620 mg after 12 months, and the subsequent administrations of an anti-TRBV9 antibody at 620 mg every 12 months.

In some embodiments of the invention, the method comprises the first administration of an anti-TRBV9 antibody at a dose of 250 mg, the second administration of an anti-TRBV9 antibody at a dose of 625 mg after at least 3 months, and the subsequent administrations of an anti-TRBV9 antibody at a dose of 625 mg at least every 6 months.

In some embodiments of the invention, the method comprises the first administration of an anti-TRBV9 antibody at a dose of 250 mg, the second administration of an anti-TRBV9 antibody at a dose of 625 mg after 3 months; and the subsequent administrations of an anti-TRBV9 antibody at a dose of 625 mg every 6 months; or every 9 months; or every 12 months.

In some embodiments of the invention, the method comprises the first administration of an anti-TRBV9 antibody at a dose of 250 mg, the second administration of an anti-TRBV9 antibody at a dose of 625 mg after 6 months; and the subsequent administrations of an anti-TRBV9 antibody at a dose of 625 mg every 6 months; or every 9 months; or every 12 months.

In some embodiments of the invention, the method comprises the first administration of an anti-TRBV9 antibody at a dose of 250 mg, the second administration of an anti-TRBV9 antibody at a dose of 625 mg after 9 months; and the subsequent administrations of an anti-TRBV9 antibody at a dose of 625 mg every 9 months; or every 12 months.

In some embodiments of the invention, the method comprises the first administration of an anti-TRBV9 antibody at a dose of 250 mg, the second administration of an anti-TRBV9 antibody at a dose of 625 mg after 12 months, and the subsequent administrations of an anti-TRBV9 antibody at 625 mg every 12 months.

In some embodiments of the invention, the method comprises the first administration of an anti-TRBV9 antibody at a dose of 255 mg, the second administration of an anti-TRBV9 antibody at a dose of 630 mg after at least 3 months, and the subsequent administrations of an anti-TRBV9 antibody at a dose of 630 mg at least every 6 months.

In some embodiments of the invention, the method comprises the first administration of an anti-TRBV9 antibody at a dose of 255 mg, the second administration of an anti-TRBV9 antibody at a dose of 630 mg after 3 months; and the subsequent administrations of an anti-TRBV9 antibody at a dose of 630 mg every 6 months; or every 9 months; or every 12 months.

In some embodiments of the invention, the method comprises the first administration of an anti-TRBV9 antibody at a dose of 255 mg, the second administration of an anti-TRBV9 antibody at a dose of 630 mg after 6 months; and the subsequent administrations of an anti-TRBV9 antibody at a dose of 630 mg every 6 months; or every 9 months; or every 12 months.

In some embodiments of the invention, the method comprises the first administration of an anti-TRBV9 antibody at a dose of 255 mg, the second administration of an anti-TRBV9 antibody at a dose of 630 mg after 9 months; and the subsequent administrations of an anti-TRBV9 antibody at a dose of 630 mg every 9 months; or every 12 months.

In some embodiments of the invention, the method comprises the first administration of an anti-TRBV9 antibody at a dose of 255 mg, the second administration of an anti-TRBV9 antibody at a dose of 630 mg after 12 months, and the subsequent administrations of an anti-TRBV9 antibody at 630 mg every 12 months.

In some embodiments of the invention, the method comprises the first administration of an anti-TRBV9 antibody at a dose of 245 mg, the second administration of an anti-TRBV9 antibody at a dose of 620 mg after at least 3 months, and the subsequent administrations of an anti-TRBV9 antibody at a dose of 620 mg at least every 6 months based on subject's body weight from 90 to 150 kg.

In some embodiments of the invention, the method comprises the first administration of an anti-TRBV9 antibody at a dose of 245 mg, the second administration of an anti-TRBV9 antibody at a dose of 620 mg after 3 months; and the subsequent administrations of an anti-TRBV9 antibody at a dose of 620 mg every 6 months; or every 9 months; or every 12 months based on subject's body weight from 90 to 150 kg.

In some embodiments of the invention, the method comprises the first administration of an anti-TRBV9 antibody at a dose of 245 mg, the second administration of an anti-TRBV9 antibody at a dose of 620 mg after 6 months; and the subsequent administrations of an anti-TRBV9 antibody at a dose of 620 mg every 6 months; or every 9 months; or every 12 months based on subject's body weight from 90 to 150 kg.

In some embodiments of the invention, the method comprises the first administration of an anti-TRBV9 antibody at a dose of 245 mg, the second administration of an anti-TRBV9 antibody at a dose of 620 mg after 9 months; and the subsequent administrations of an anti-TRBV9 antibody at a dose of 620 mg every 9 months; or every 12 months based on subject's body weight from 90 to 150 kg.

In some embodiments of the invention, the method comprises the first administration of an anti-TRBV9 antibody at a dose of 245 mg, the second administration of an anti-TRBV9 antibody at a dose of 620 mg after 12 months, and the subsequent administrations of an anti-TRBV9 antibody at 620 mg every 12 months based on subject's body weight from 90 to 150 kg.

In some embodiments of the invention, the method comprises the first administration of an anti-TRBV9 antibody at a dose of 250 mg, the second administration of an anti-TRBV9 antibody at a dose of 625 mg after at least 3 months, and the subsequent administrations of an anti-TRBV9 antibody at a dose of 625 mg at least every 6 months based on subject's body weight from 90 to 150 kg.

In some embodiments of the invention, the method comprises the first administration of an anti-TRBV9 antibody at a dose of 250 mg, the second administration of an anti-TRBV9 antibody at a dose of 625 mg after 3 months; and the subsequent administrations of an anti-TRBV9 antibody at a dose of 625 mg every 6 months; or every 9 months; or every 12 months based on subject's body weight from 90 to 150 kg.

In some embodiments of the invention, the method comprises the first administration of an anti-TRBV9 antibody at a dose of 250 mg, the second administration of an anti-TRBV9 antibody at a dose of 625 mg after 6 months; and the subsequent administrations of an anti-TRBV9 antibody at a dose of 625 mg every 6 months; or every 9 months; or every 12 months based on subject's body weight from 90 to 150 kg.

In some embodiments of the invention, the method comprises the first administration of an anti-TRBV9 antibody at a dose of 250 mg, the second administration of an anti-TRBV9 antibody at a dose of 625 mg after 9 months; and the subsequent administrations of an anti-TRBV9 antibody at a dose of 625 mg every 9 months; or every 12 months based on subject's body weight from 90 to 150 kg.

In some embodiments of the invention, the method comprises the first administration of an anti-TRBV9 antibody at a dose of 250 mg, the second administration of an anti-TRBV9 antibody at a dose of 625 mg after 12 months, and the subsequent administrations of an anti-TRBV9 antibody at 625 mg every 12 months based on subject's body weight from 90 to 150 kg.

In some embodiments of the invention, the method comprises the first administration of an anti-TRBV9 antibody at a dose of 255 mg, the second administration of an anti-TRBV9 antibody at a dose of 630 mg after at least 3 months, and the subsequent administrations of an anti-TRBV9 antibody at a dose of 630 mg at least every 6 months based on subject's body weight from 90 to 150 kg.

In some embodiments of the invention, the method comprises the first administration of an anti-TRBV9 antibody at a dose of 255 mg, the second administration of an anti-TRBV9 antibody at a dose of 630 mg after 3 months; and the subsequent administrations of an anti-TRBV9 antibody at a dose of 630 mg every 6 months; or every 9 months; or every 12 months based on subject's body weight from 90 to 150 kg.

In some embodiments of the invention, the method comprises the first administration of an anti-TRBV9 antibody at a dose of 255 mg, the second administration of an anti-TRBV9 antibody at a dose of 630 mg after 6 months; and the subsequent administrations of an anti-TRBV9 antibody at a dose of 630 mg every 6 months; or every 9 months; or every 12 months based on subject's body weight from 90 to 150 kg.

In some embodiments of the invention, the method comprises the first administration of an anti-TRBV9 antibody at a dose of 255 mg, the second administration of an anti-TRBV9 antibody at a dose of 630 mg after 9 months; and the subsequent administrations of an anti-TRBV9 antibody at a dose of 630 mg every 9 months; or every 12 months based on subject's body weight from 90 to 150 kg.

In some embodiments of the invention, the method comprises the first administration of an anti-TRBV9 antibody at a dose of 255 mg, the second administration of an anti-TRBV9 antibody at a dose of 630 mg after 12 months, and the subsequent administrations of an anti-TRBV9 antibody at 630 mg every 12 months based on subject's body weight from 90 to 150 kg.

In one aspect, the present invention relates to a method of treatment of a disease or disorder mediated by T-lymphocytes bearing the TRBV9 segment within the T-cell receptor, comprising the first administration of an anti-TRBV9 antibody to a subject in need thereof at a dose equal to half of the main dose, 0.015-5 mg/kg, the second administration of an anti-TRBV9 antibody at the main dose of 0.03-10 mg/kg after at least 3 months, the third administration of an anti-TRBV9 antibody at the main dose of 0.03-10 mg/kg after at least 6 months, and the subsequent administrations of an anti-TRBV9 antibody at the main dose of 0.03-10 mg/kg at least every 12 months.

In some embodiments of the invention, the method comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 3 months, the third administration of an anti-TRBV9 antibody at the main dose of 0.03-10 mg/kg after 6 months; or after 9 months; or after 12 months; and the subsequent administrations of an anti-TRBV9 antibody at the main dose of 0.03-10 mg/kg every 12 months.

In some embodiments of the invention, the method comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg [] 6 months, the third administration of an anti-TRBV9 antibody at the main dose of 0.03-10 mg/kg after 6 months; or after 9 months; or after 12 months; and the subsequent administrations of an anti-TRBV9 antibody at the main dose of 0.03-10 mg/kg every 12 months.

In some embodiments of the invention, the method comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 9 months, the third administration of an anti-TRBV9 antibody at the main dose of 0.03-10 mg/kg after 9 months; or after 12 months; and the subsequent administrations of an anti-TRBV9 antibody at the main dose of 0.03-10 mg/kg every 12 months.

In some embodiments of the invention, the method comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 12 months, the third administration of an anti-TRBV9 antibody at the main dose of 0.03-10 mg/kg after 12 months, and the subsequent administrations of an anti-TRBV9 antibody at the main dose of 0.03-10 mg/kg every 12 months.

In one aspect, the present invention relates to a method of treatment of a disease or disorder mediated by T-lymphocytes bearing the TRBV9 segment within the T-cell receptor, comprising the first administration of an anti-TRBV9 antibody to a subject in need thereof at a dose equal to half of the main dose, 0.015-3.5 mg/kg, the second administration of an anti-TRBV9 antibody at the main dose of 0.03-7 mg/kg after at least 3 months, the third administration of an anti-TRBV9 antibody at the main dose of 0.03-7 mg/kg after at least 6 months, and the subsequent administrations of an anti-TRBV9 antibody at the main dose of 0.03-7 mg/kg at least every 12 months.

In some embodiments of the invention, the method comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 3 months, the third administration of an anti-TRBV9 antibody at the main dose of 0.03-7 mg/kg after 6 months; or after 9 months; or after 12 months; and the subsequent administrations of an anti-TRBV9 antibody at the main dose of 0.03-7 mg/kg every 12 months.

In some embodiments of the invention, the method comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 6 months, the third administration of an anti-TRBV9 antibody at the main dose of 0.03-7 mg/kg after 6 months; or after 9 months; or after 12 months; and the subsequent administrations of an anti-TRBV9 antibody at the main dose of 0.03-7 mg/kg every 12 months.

In some embodiments of the invention, the method comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 9 months, the third administration of an anti-TRBV9 antibody at the main dose of 0.03-7 mg/kg after 9 months; or after 12 months; and the subsequent administrations of an anti-TRBV9 antibody at the main dose of 0.03-7 mg/kg every 12 months.

In some embodiments of the invention, the method comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 12 months, the third administration of an anti-TRBV9 antibody at the main dose of 0.03-7 mg/kg after 12 months, and the subsequent administrations of an anti-TRBV9 antibody at the main dose of 0.03-7 mg/kg every 12 months.

In one aspect, the present invention relates to a method of treatment of a disease or disorder mediated by T-lymphocytes bearing the TRBV9 segment within the T-cell receptor, comprising the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after at least 3 months, the third administration of an anti-TRBV9 antibody at the main dose of 0.3-10 mg/kg after at least 6 months, and the subsequent administrations of an anti-TRBV9 antibody at the main dose of 0.3-10 mg/kg at least every 12 months.

In some embodiments of the invention, the method comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 3 months, the third administration of an anti-TRBV9 antibody at the main dose of 0.3-10 mg/kg after 6 months; or after 9 months; or after 12 months; and the subsequent administrations of an anti-TRBV9 antibody at the main dose of 0.3-10 mg/kg every 12 months.

In some embodiments of the invention, the method comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 6 months, the third administration of an anti-TRBV9 antibody at the main dose of 0.3-10 mg/kg after 6 months; or after 9 months; or after 12 months; and the subsequent administrations of an anti-TRBV9 antibody at the main dose of 0.3-10 mg/kg every 12 months.

In some embodiments of the invention, the method comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 9 months, the third administration of an anti-TRBV9 antibody at the main dose of 0.3-10 mg/kg after 9 months; or after 12 months; and the subsequent administrations of an anti-TRBV9 antibody at the main dose of 0.3-10 mg/kg every 12 months.

In some embodiments of the invention, the method comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 12 months, the third administration of an anti-TRBV9 antibody at the main dose of 0.3-10 mg/kg after 12 months, and the subsequent administrations of an anti-TRBV9 antibody at the main dose of 0.3-10 mg/kg every 12 months.

In one aspect, the present invention relates to a method of treatment of a disease or disorder mediated by T-lymphocytes bearing the TRBV9 segment within the T-cell receptor, comprising the first administration of an anti-TRBV9 antibody to a subject in need thereof at a dose equal to half of the main dose, 0.15-3.5 mg/kg, the second administration of an anti-TRBV9 antibody at the main dose of 0.3-7 mg/kg after at least 3 months, the third administration of an anti-TRBV9 antibody at the main dose of 0.3-7 mg/kg after at least 6 months, and the subsequent administrations of an anti-TRBV9 antibody at the main dose of 0.3-7 mg/kg at least every 12 months.

In some embodiments of the invention, the method comprises the first administration to a subject in need thereof of an anti-TRBV9 antibody at a dose equal to half of the main dose, 0.15-3.5 mg/kg, the second administration of an anti-TRBV9 antibody at the main dose of 0.3-7 mg/kg after 3 months, the third administration of an anti-TRBV9 antibody at the main dose of 0.3-7 mg/kg after 6 months; or after 9 months; or after 12 months; and the subsequent administrations of an anti-TRBV9 antibody at the main dose of 0.3-7 mg/kg every 12 months.

In some embodiments of the invention, the method comprises the first administration to a subject in need thereof of an anti-TRBV9 antibody at a dose equal to half of the main dose, 0.15-3.5 mg/kg, the second administration of an anti-TRBV9 antibody at the main dose of 0.3-7 mg/kg after 6 months, the third administration of an anti-TRBV9 antibody at the main dose of 0.3-7 mg/kg after 6 months; or after 9 months; or after 12 months; and the subsequent administrations of an anti-TRBV9 antibody at the main dose of 0.3-7 mg/kg every 12 months.

In some embodiments of the invention, the method comprises the first administration to a subject in need thereof of an anti-TRBV9 antibody at a dose equal to half of the main dose, 0.15-3.5 mg/kg, the second administration of an anti-TRBV9 antibody at the main dose of 0.3-7 mg/kg after 9 months, the third administration of an anti-TRBV9 antibody at the main dose of 0.3-7 mg/kg after 9 months; or after 12 months; and the subsequent administrations of an anti-TRBV9 antibody at the main dose of 0.3-7 mg/kg every 12 months.

In some embodiments of the invention, the method comprises the first administration to a subject in need thereof of an anti-TRBV9 antibody at a dose equal to half of the main dose, 0.15-3.5 mg/kg, the second administration of an anti-TRBV9 antibody at the main dose of 0.3-7 mg/kg after 12 months, the third administration of an anti-TRBV9 antibody at the main dose of 0.3-7 mg/kg after 12 months, and the subsequent administrations of an anti-TRBV9 antibody at the main dose of 0.3-7 mg/kg every 12 months.

In one aspect, the present invention relates to a method of treatment of a disease or disorder mediated by T-lymphocytes bearing the TRBV9 segment within the T-cell receptor, comprising the first administration of an anti-TRBV9 antibody to a subject in need thereof at a dose equal to half of the main dose, 0.5-3.5 mg/kg, the second administration of an anti-TRBV9 antibody at the main dose of 1-7 mg/kg after at least 3 months, the third administration of an anti-TRBV9 antibody at the main dose of 1-7 mg/kg after at least 6 months, and the subsequent administrations of an anti-TRBV9 antibody at the main dose of 1-7 mg/kg at least every 12 months.

In one aspect, the present invention relates to a method of treatment of a disease or disorder mediated by T-lymphocytes bearing the TRBV9 segment within the T-cell receptor, comprising the first administration of an anti-TRBV9 antibody to a subject in need thereof at a dose equal to half of the main dose, 0.5-3.5 mg/kg, the second administration of an anti-TRBV9 antibody at the main dose of 1-7 mg/kg after 3 months, the third administration of an anti-TRBV9 antibody at the main dose of 1-7 mg/kg after 6 months; or after 9 months; or after 12 months; and the subsequent administrations of an anti-TRBV9 antibody at the main dose of 1-7 mg/kg every 12 months.

In some embodiments of the invention, the method comprises the first administration to a subject in need thereof of an anti-TRBV9 antibody at a dose equal to half of the main dose, 0.5-3.5 mg/kg, the second administration of an anti-TRBV9 antibody at the main dose of 1-7 mg/kg after 6 months, the third administration of an anti-TRBV9 antibody at the main dose of 1-7 mg/kg after 6 months; or after 9 months; or after 12 months; and the subsequent administrations of an anti-TRBV9 antibody at the main dose of 1-7 mg/kg every 12 months.

In some embodiments of the invention, the method comprises the first administration to a subject in need thereof of an anti-TRBV9 antibody at a dose equal to half of the main dose, 0.5-3.5 mg/kg, the second administration of an anti-TRBV9 antibody at the main dose of 1-7 mg/kg after 9 months, the third administration of an anti-TRBV9 antibody at the main dose of 1-7 mg/kg after 9 months; or after 12 months; and the subsequent administrations of an anti-TRBV9 antibody at the main dose of 1-7 mg/kg every 12 months.

In some embodiments of the invention, the method comprises the first administration to a subject in need thereof of an anti-TRBV9 antibody at a dose equal to half of the main dose, 0.5-3.5 mg/kg, the second administration of an anti-TRBV9 antibody at the main dose of 1-7 mg/kg after 12 months, the third administration of an anti-TRBV9 antibody at the main dose of 1-7 mg/kg after 12 months, and the subsequent administrations of an anti-TRBV9 antibody at the main dose of 1-7 mg/kg every 12 months.

In some embodiments of the invention, the method comprises the first administration of an anti-TRBV9 antibody at a dose of 0.5 mg/kg, the second administration of an anti-TRBV9 antibody at a dose of 1 mg/kg after at least 3 months, the third administration of an anti-TRBV9 antibody at a dose of 1 mg/kg after at least 6 months, and the subsequent administrations of an anti-TRBV9 antibody at a dose of 1 mg/kg at least every 12 months.

In some embodiments of the invention, the method comprises the first administration of an anti-TRBV9 antibody at a dose of 0.5 mg/kg, the second administration of an anti-TRBV9 antibody at a dose of 1 mg/kg after 3 months, the third administration of an anti-TRBV9 antibody at a dose of 1 mg/kg after 6 months; or after 9 months; or after 12 months; and the subsequent administrations of an anti-TRBV9 antibody at a dose of 1 mg/kg every 12 months.

In some embodiments of the invention, the method comprises the first administration of an anti-TRBV9 antibody at a dose of 0.5 mg/kg, the second administration of an anti-TRBV9 antibody at a dose of 1 mg/kg after 6 months, the third administration of an anti-TRBV9 antibody at a dose of 1 mg/kg after 6 months; or after 9 months; or after 12 months; and the subsequent administrations of an anti-TRBV9 antibody at a dose of 1 mg/kg every 12 months.

In some embodiments of the invention, the method comprises the first administration of an anti-TRBV9 antibody at a dose of 0.5 mg/kg, the second administration of an anti-TRBV9 antibody at a dose of 1 mg/kg after 9 months, the third administration of an anti-TRBV9 antibody at a dose of 1 mg/kg after 9 months; or after 12 months; and the subsequent administrations of an anti-TRBV9 antibody at a dose of 1 mg/kg every 12 months.

In some embodiments of the invention, the method comprises the first administration of an anti-TRBV9 antibody at a dose of 0.5 mg/kg, the second administration of an anti-TRBV9 antibody at a dose of 1 mg/kg after 12 months, the third administration of an anti-TRBV9 antibody at a dose of 1 mg/kg after 12 months, and the subsequent administrations of an anti-TRBV9 antibody at a dose of 1 mg/kg every 12 months.

In some embodiments of the invention, the method comprises the first administration of an anti-TRBV9 antibody at a dose of 1 mg/kg, the second administration of an anti-TRBV9 antibody at a dose of 2 mg/kg after at least 3 months, the third administration of an anti-TRBV9 antibody at a dose of 2 mg/kg after at least 6 months, and the subsequent administrations of an anti-TRBV9 antibody at a dose of 2 mg/kg at least every 12 months.

In some embodiments of the invention, the method comprises the first administration of an anti-TRBV9 antibody at a dose of 1 mg/kg, the second administration of an anti-TRBV9 antibody at a dose of 2 mg/kg after 3 months, the third administration of an anti-TRBV9 antibody at a dose of 2 mg/kg after 6 months; or after 9 months; or after 12 months; and the subsequent administrations of an anti-TRBV9 antibody at a dose of 2 mg/kg every 12 months.

In some embodiments of the invention, the method comprises the first administration of an anti-TRBV9 antibody at a dose of 1 mg/kg, the second administration of an anti-TRBV9 antibody at a dose of 2 mg/kg after 6 months, the third administration of an anti-TRBV9 antibody at a dose of 2 mg/kg after 6 months; or after 9 months; or after 12 months; and the subsequent administrations of an anti-TRBV9 antibody at a dose of 2 mg/kg every 12 months.

In some embodiments of the invention, the method comprises the first administration of an anti-TRBV9 antibody at a dose of 1 mg/kg, the second administration of an anti-TRBV9 antibody at a dose of 2 mg/kg after 9 months, the third administration of an anti-TRBV9 antibody at a dose of 2 mg/kg after 9 months; or after 12 months; and the subsequent administrations of an anti-TRBV9 antibody at a dose of 2 mg/kg every 12 months.

In some embodiments of the invention, the method comprises the first administration of an anti-TRBV9 antibody at a dose of 1 mg/kg, the second administration of an anti-TRBV9 antibody at a dose of 2 mg/kg after 12 months, the third administration of an anti-TRBV9 antibody at a dose of 2 mg/kg after 12 months, and the subsequent administrations of an anti-TRBV9 antibody at a dose of 2 mg/kg every 12 months.

In some embodiments of the invention, the method comprises the first administration of an anti-TRBV9 antibody at a dose of 1.5 mg/kg, the second administration of an anti-TRBV9 antibody at a dose of 3 mg/kg after at least 3 months, the third administration of an anti-TRBV9 antibody at a dose of 3 mg/kg after at least 6 months, and the subsequent administrations of an anti-TRBV9 antibody at a dose of 3 mg/kg at least every 12 months.

In some embodiments of the invention, the method comprises the first administration of an anti-TRBV9 antibody at a dose of 1.5 mg/kg, the second administration of an anti-TRBV9 antibody at a dose of 3 mg/kg after 3 months, the third administration of an anti-TRBV9 antibody at a dose of 3 mg/kg after 6 months; or after 9 months; or after 12 months; and the subsequent administrations of an anti-TRBV9 antibody at a dose of 3 mg/kg every 12 months.

In some embodiments of the invention, the method comprises the first administration of an anti-TRBV9 antibody at a dose of 1.5 mg/kg, the second administration of an anti-TRBV9 antibody at a dose of 3 mg/kg after 6 months, the third administration of an anti-TRBV9 antibody at a dose of 3 mg/kg after 6 months; or after 9 months; or after 12 months; and the subsequent administrations of an anti-TRBV9 antibody at a dose of 3 mg/kg every 12 months.

In some embodiments of the invention, the method comprises the first administration of an anti-TRBV9 antibody at a dose of 1.5 mg/kg, the second administration of an anti-TRBV9 antibody at a dose of 3 mg/kg after 9 months, the third administration of an anti-TRBV9 antibody at a dose of 3 mg/kg after 9 months; or after 12 months; and the subsequent administrations of an anti-TRBV9 antibody at a dose of 3 mg/kg every 12 months.

In some embodiments of the invention, the method comprises the first administration of an anti-TRBV9 antibody at a dose of 1.5 mg/kg, the second administration of an anti-TRBV9 antibody at a dose of 3 mg/kg after 12 months, the third administration of an anti-TRBV9 antibody at a dose of 3 mg/kg after 12 months, and the subsequent administrations of an anti-TRBV9 antibody at a dose of 3 mg/kg every 12 months.

In some embodiments of the invention, the method comprises the first administration of an anti-TRBV9 antibody at a dose of 2 mg/kg, the second administration of an anti-TRBV9 antibody at a dose of 4 mg/kg after at least 3 months, the third administration of an anti-TRBV9 antibody at a dose of 4 mg/kg after at least 6 months, and the subsequent administrations of an anti-TRBV9 antibody at a dose of 4 mg/kg at least every 12 months.

In some embodiments of the invention, the method comprises the first administration of an anti-TRBV9 antibody at a dose of 2 mg/kg, the second administration of an anti-TRBV9 antibody at a dose of 4 mg/kg after 3 months, the third administration of an anti-TRBV9 antibody at a dose of 4 mg/kg after 6 months; or after 9 months; or after 12 months; and the subsequent administrations of an anti-TRBV9 antibody at a dose of 4 mg/kg every 12 months.

In some embodiments of the invention, the method comprises the first administration of an anti-TRBV9 antibody at a dose of 2 mg/kg, the second administration of an anti-TRBV9 antibody at a dose of 4 mg/kg after 6 months, the third administration of an anti-TRBV9 antibody at a dose of 4 mg/kg after 6 months; or after 9 months; or after 12 months; and the subsequent administrations of an anti-TRBV9 antibody at a dose of 4 mg/kg every 12 months.

In some embodiments of the invention, the method comprises the first administration of an anti-TRBV9 antibody at a dose of 2 mg/kg, the second administration of an anti-TRBV9 antibody at a dose of 4 mg/kg after 9 months, the third administration of an anti-TRBV9 antibody at a dose of 4 mg/kg after 9 months; or after 12 months; and the subsequent administrations of an anti-TRBV9 antibody at a dose of 4 mg/kg every 12 months.

In some embodiments of the invention, the method comprises the first administration of an anti-TRBV9 antibody at a dose of 2 mg/kg, the second administration of an anti-TRBV9 antibody at a dose of 4 mg/kg after 12 months, the third administration of an anti-TRBV9 antibody at a dose of 4 mg/kg after 12 months, and the subsequent administrations of an anti-TRBV9 antibody at a dose of 4 mg/kg every 12 months.

In some embodiments of the invention, the method comprises the first administration of an anti-TRBV9 antibody at a dose of 2,5 mg/kg, the second administration of an anti-TRBV9 antibody at a dose of 5 mg/kg after at least 3 months, the third administration of an anti-TRBV9 antibody at a dose of 5 mg/kg after at least 6 months, and the subsequent administrations of an anti-TRBV9 antibody at a dose of 5 mg/kg at least every 12 months.

In some embodiments of the invention, the method comprises the first administration of an anti-TRBV9 antibody at a dose of 2,5 mg/kg, the second administration of an anti-TRBV9 antibody at a dose of 5 mg/kg after 3 months, the third administration of an anti-TRBV9 antibody at a dose of 5 mg/kg after 6 months; or after 9 months; or after 12 months; and the subsequent administrations of an anti-TRBV9 antibody at a dose of 5 mg/kg every 12 months.

In some embodiments of the invention, the method comprises the first administration of an anti-TRBV9 antibody at a dose of 2,5 mg/kg, the second administration of an anti-TRBV9 antibody at a dose of 5 mg/kg after 6 months, the third administration of an anti-TRBV9 antibody at a dose of 5 mg/kg after 6 months; or after 9 months; or after 12 months; and the subsequent administrations of an anti-TRBV9 antibody at a dose of 5 mg/kg every 12 months.

In some embodiments of the invention, the method comprises the first administration of an anti-TRBV9 antibody at a dose of 2,5 mg/kg, the second administration of an anti-TRBV9 antibody at a dose of 5 mg/kg after 9 months, the third administration of an anti-TRBV9 antibody at a dose of 5 mg/kg after 9 months; or after 12 months; and the subsequent administrations of an anti-TRBV9 antibody at a dose of 5 mg/kg every 12 months.

In some embodiments of the invention, the method comprises the first administration of an anti-TRBV9 antibody at a dose of 2,5 mg/kg, the second administration of an anti-TRBV9 antibody at a dose of 5 mg/kg after 12 months, the third administration of an anti-TRBV9 antibody at a dose of 5 mg/kg after 6 months, and the subsequent administrations of an anti-TRBV9 antibody at a dose of 5 mg/kg every 12 months.

In some embodiments of the invention, the method comprises the first administration of an anti-TRBV9 antibody at a dose of 3 mg/kg, the second administration of an anti-TRBV9 antibody at a dose of 6 mg/kg after at least 3 months, the third administration of an anti-TRBV9 antibody at a dose of 6 mg/kg after at least 6 months, and the subsequent administrations of an anti-TRBV9 antibody at a dose of 6 mg/kg at least every 12 months.

In some embodiments of the invention, the method comprises the first administration of an anti-TRBV9 antibody at a dose of 3 mg/kg, the second administration of an anti-TRBV9 antibody at a dose of 6 mg/kg after 3 months, the third administration of an anti-TRBV9 antibody at a dose of 6 mg/kg after 6 months; or after 9 months; or after 12 months; and the subsequent administrations of an anti-TRBV9 antibody at a dose of 6 mg/kg every 12 months.

In some embodiments of the invention, the method comprises the first administration of an anti-TRBV9 antibody at a dose of 3 mg/kg, the second administration of an anti-TRBV9 antibody at a dose of 6 mg/kg after 6 months, the third administration of an anti-TRBV9 antibody at a dose of 6 mg/kg after 6 months; or after 9 months; or after 12 months; and the subsequent administrations of an anti-TRBV9 antibody at a dose of 6 mg/kg every 12 months.

In some embodiments of the invention, the method comprises the first administration of an anti-TRBV9 antibody at a dose of 3 mg/kg, the second administration of an anti-TRBV9 antibody at a dose of 6 mg/kg after 9 months, the third administration of an anti-TRBV9 antibody at a dose of 6 mg/kg after 9 months; or after 12 months; and the subsequent administrations of an anti-TRBV9 antibody at a dose of 6 mg/kg every 12 months.

In some embodiments of the invention, the method comprises the first administration of an anti-TRBV9 antibody at a dose of 3 mg/kg, the second administration of an anti-TRBV9 antibody at a dose of 6 mg/kg after 12 months, the third administration of an anti-TRBV9 antibody at a dose of 6 mg/kg after 12 months, and the subsequent administrations of an anti-TRBV9 antibody at a dose of 6 mg/kg every 12 months.

In some embodiments of the invention, the method comprises the first administration to a subject in need thereof of an anti-TRBV9 antibody at a dose of 3.5 mg/kg, the second administration of an anti-TRBV9 antibody at a dose of 7 mg/kg after at least 3 months, the third administration of an anti-TRBV9 antibody at a dose of 7 mg/kg after at least 6 months, and the subsequent administrations of an anti-TRBV9 antibody at a dose of 7 mg/kg at least every 12 months.

In some embodiments of the invention, the method comprises the first administration to a subject in need thereof of an anti-TRBV9 antibody at a dose of 3.5 mg/kg, the second administration of an anti-TRBV9 antibody at a dose of 7 mg/kg after 3 months, the third administration of an anti-TRBV9 antibody at a dose of 7 mg/kg after 6 months; or after 9 months; or after 12 months; and the subsequent administrations of an anti-TRBV9 antibody at a dose of 7 mg/kg every 12 months.

In some embodiments of the invention, the method comprises the first administration to a subject in need thereof of an anti-TRBV9 antibody at a dose of 3.5 mg/kg, the second administration of an anti-TRBV9 antibody at a dose of 7 mg/kg after 6 months, the third administration of an anti-TRBV9 antibody at a dose of 7 mg/kg after 6 months; or after 9 months; or after 12 months; and the subsequent administrations of an anti-TRBV9 antibody at a dose of 7 mg/kg every 12 months.

In some embodiments of the invention, the method comprises the first administration to a subject in need thereof of an anti-TRBV9 antibody at a dose of 3.5 mg/kg, the second administration of an anti-TRBV9 antibody at a dose of 7 mg/kg after 9 months, the third administration of an anti-TRBV9 antibody at a dose of 7 mg/kg after 9 months; or after 12 months; and the subsequent administrations of an anti-TRBV9 antibody at a dose of 7 mg/kg every 12 months.

In some embodiments of the invention, the method comprises the first administration to a subject in need thereof of an anti-TRBV9 antibody at a dose of 3.5 mg/kg, the second administration of an anti-TRBV9 antibody at a dose of 7 mg/kg after 12 months, the third administration of an anti-TRBV9 antibody at a dose of 7 mg/kg after 12 months, and the subsequent administrations of an anti-TRBV9 antibody at a dose of 7 mg/kg every 12 months.

In some embodiments of the invention, the method comprises the first administration of an anti-TRBV9 antibody performed at a dose of 4 mg/kg, the second administration of an anti-TRBV9 antibody is performed at a dose of 8 mg/kg after 3 months, and the subsequent administrations of an anti-TRBV9 antibody are performed at a dose of 8 mg/kg every 6 months; or every 9 months; or every 12 months.

In some embodiments of the invention, the method comprises the first administration of an anti-TRBV9 antibody at a dose of 4 mg/kg, the second administration of an anti-TRBV9 antibody at a dose of 8 mg/kg after 6 months, and the subsequent administrations of an anti-TRBV9 antibody at a dose of 8 mg/kg every 6 months; or every 9 months; or every 12 months.

In some embodiments of the invention, the method comprises the first administration of an anti-TRBV9 antibody at a dose of 4 mg/kg, the second administration of an anti-TRBV9 antibody at a dose of 8 mg/kg after 9 months, and the subsequent administrations of an anti-TRBV9 antibody at a dose of 8 mg/kg every 9 months; or every 12 months.

In some embodiments of the invention, the method comprises the first administration of an anti-TRBV9 antibody at a dose of 4 mg/kg, the second administration of an anti-TRBV9 antibody at a dose of 8 mg/kg after 12 months, and the subsequent administrations of an anti-TRBV9 antibody at a dose of 8 mg/kg every 12 months.

In some embodiments of the invention, the method comprises the first administration of an anti-TRBV9 antibody performed at a dose of 4.5 mg/kg, the second administration of an anti-TRBV9 antibody is performed at a dose of 9 mg/kg after 3 months, and the subsequent administrations of an anti-TRBV9 antibody are performed at a dose of 9 mg/kg every 6 months; or every 9 months; or every 12 months.

In some embodiments of the invention, the method comprises the first administration of an anti-TRBV9 antibody at a dose of 4.5 mg/kg, the second administration of an anti-TRBV9 antibody at a dose of 9 mg/kg after 6 months, and the subsequent administrations of an anti-TRBV9 antibody at a dose of 9 mg/kg every 6 months; or every 9 months; or every 12 months.

In some embodiments of the invention, the method comprises the first administration of an anti-TRBV9 antibody at a dose of 4.5 mg/kg, the second administration of an anti-TRBV9 antibody at a dose of 9 mg/kg after 9 months, and the subsequent administrations of an anti-TRBV9 antibody at a dose of 9 mg/kg every 9 months; or every 12 months.

In some embodiments of the invention, the method comprises the first administration of an anti-TRBV9 antibody at a dose of 4.5 mg/kg, the second administration of an anti-TRBV9 antibody at a dose of 9 mg/kg after 12 months, and the subsequent administrations of an anti-TRBV9 antibody at a dose of 9 mg/kg every 12 months.

In some embodiments of the invention, the method comprises the first administration of an anti-TRBV9 antibody performed at a dose of 5 mg/kg, the second administration of an anti-TRBV9 antibody is performed at a dose of 10 mg/kg after 3 months, and the subsequent administrations of an anti-TRBV9 antibody are performed at a dose of 10 mg/kg every 6 months; or every 9 months; or every 12 months.

In some embodiments of the invention, the method comprises the first administration of an anti-TRBV9 antibody at a dose of 5 mg/kg, the second administration of an anti-TRBV9 antibody at a dose of 10 mg/kg after 6 months, and the subsequent administrations of an anti-TRBV9 antibody at a dose of 10 mg/kg every 6 months; or every 9 months; or every 12 months.

In some embodiments of the invention, the method comprises the first administration of an anti-TRBV9 antibody at a dose of 5 mg/kg, the second administration of an anti-TRBV9 antibody at a dose of 10 mg/kg after 9 months, and the subsequent administrations of an anti-TRBV9 antibody at a dose of 10 mg/kg every 9 months; or every 12 months.

In some embodiments of the invention, the method comprises the first administration of an anti-TRBV9 antibody at a dose of 5 mg/kg, the second administration of an anti-TRBV9 antibody at a dose of 10 mg/kg after 12 months, and the subsequent administrations of an anti-TRBV9 antibody at a dose of 10 mg/kg every 12 months.

In one aspect, the present invention relates to a method of treatment of a disease or disorder mediated by T-lymphocytes bearing the TRBV9 segment within the T-cell receptor, comprising the first administration to a subject in need thereof of an anti-TRBV9 antibody at a dose of 75-300 mg, the second administration of an anti-TRBV9 antibody at the main dose of 325-675 mg after at least 3 months, the third administration of an anti-TRBV9 antibody at the main dose of 325-675 mg after at least 6 months, and the subsequent administrations of an anti-TRBV9 antibody at the main dose of 325-675 mg at least every 12 months.

In some embodiments of the invention, the method comprises the first administration to a subject in need thereof of an anti-TRBV9 antibody at a dose of 75-300 mg, the second administration of an anti-TRBV9 antibody at the main dose of 325-675 mg after 3 months, the third administration of an anti-TRBV9 antibody at the main dose of 325-675 mg after 6 months; or after 9 months; or after 12 months; and the subsequent administrations of an anti-TRBV9 antibody at the main dose of 325-675 mg every 12 months.

In some embodiments of the invention, the method comprises the first administration to a subject in need thereof of an anti-TRBV9 antibody at a dose of 75-300 mg, the second administration of an anti-TRBV9 antibody at the main dose of 325-675 mg [] 6 months, the third administration of an anti-TRBV9 antibody at the main dose of 325-675 mg after 6 months; or after 9 months; or after 12 months; and the subsequent administrations of an anti-TRBV9 antibody at the main dose of 325-675 mg every 12 months.

In some embodiments of the invention, the method comprises the first administration to a subject in need thereof of an anti-TRBV9 antibody at a dose of 75-300 mg, the second administration of an anti-TRBV9 antibody at the main dose of 325-675 mg after 9 months, the third administration of an anti-TRBV9 antibody at the main dose of 325-675 mg after 9 months; or after 12 months; and the subsequent administrations of an anti-TRBV9 antibody at the main dose of 325-675 mg every 12 months.

In some embodiments of the invention, the method comprises the first administration to a subject in need thereof of an anti-TRBV9 antibody at a dose of 75-300 mg, the second administration of an anti-TRBV9 antibody at the main dose of 325-675 mg after 12 months, the third administration of an anti-TRBV9 antibody at the main dose of 325-675 mg after 12 months, and the subsequent administrations of an anti-TRBV9 antibody at the main dose of 325-675 mg every 12 months.

In one aspect, the present invention relates to a method of treatment of a disease or disorder mediated by T-lymphocytes bearing the TRBV9 segment within the T-cell receptor, comprising the first administration to a subject in need thereof of an anti-TRBV9 antibody at a dose of 100-275 mg, the second administration of an anti-TRBV9 antibody at the main dose of 350-650 mg after at least 3 months, the third administration of an anti-TRBV9 antibody at the main dose of 350-650 mg after at least 6 months, and the subsequent administrations of an anti-TRBV9 antibody at the main dose of 350-650 mg at least every 12 months.

In some embodiments of the invention, the method comprises the first administration to a subject in need thereof of an anti-TRBV9 antibody at a dose of 100-275 mg, the second administration of an anti-TRBV9 antibody at the main dose of 350-650 mg after 3 months, the third administration of an anti-TRBV9 antibody at the main dose of 350-650 mg after 6 months; or after 9 months; or after 12 months; and the subsequent administrations of an anti-TRBV9 antibody at the main dose of 350-650 mg every 12 months.

In some embodiments of the invention, the method comprises the first administration to a subject in need thereof of an anti-TRBV9 antibody at a dose of 100-275 mg, the second administration of an anti-TRBV9 antibody at the main dose of 350-650 mg after 6 months, the third administration of an anti-TRBV9 antibody at the main dose of 350-650 mg after 6 months; or after 9 months; or 12 months; and the subsequent administrations of an anti-TRBV9 antibody at the main dose of 350-650 mg every 12 months.

In some embodiments of the invention, the method comprises the first administration to a subject in need thereof of an anti-TRBV9 antibody at a dose of 100-275 mg, the second administration of an anti-TRBV9 antibody at the main dose of 350-650 mg after 9 months, the third administration of an anti-TRBV9 antibody at the main dose of 350-650 mg after 9 months; or after 12 months; and the subsequent administrations of an anti-TRBV9 antibody at the main dose of 350-650 mg every 12 months.

In some embodiments of the invention, the method comprises the first administration to a subject in need thereof of an anti-TRBV9 antibody at a dose of 100-275 mg, the second administration of an anti-TRBV9 antibody at the main dose of 350-650 mg after 12 months, the third administration of an anti-TRBV9 antibody at the main dose of 350-650 mg after 12 months, and the subsequent administrations of an anti-TRBV9 antibody at the main dose of 350-650 mg every 12 months.

In one aspect, the present invention relates to a method of treatment of a disease or disorder mediated by T-lymphocytes bearing the TRBV9 segment within the T-cell receptor, comprising the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after at least 3 months, the third administration of an anti-TRBV9 antibody at the main dose of 350-400 mg; or 475- 525 mg; or 600-650 mg after at least 6 months, and the subsequent administrations of an anti-TRBV9 antibody at the main dose of 350-400 mg; or 475- 525 mg; or 600-650 mg at least every 12 months.

In some embodiments of the invention, the method comprises (i) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 3 months, the third administration of an anti-TRBV9 antibody at the main dose of 350-400 mg; or 475- 525 mg; or 600-650 mg after 6 months, and the subsequent administrations of an anti-TRBV9 antibody at the main dose of 350-400 mg; or 475- 525 mg; or 600-650 mg every 12 months, or
(ii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 3 months, the third administration of an anti-TRBV9 antibody at the main dose of 350-400 mg; or 475- 525 mg; or 600-650 mg after 9 months, and the subsequent administrations of an anti-TRBV9 antibody at the main dose of 350-400 mg; or 475- 525 mg; or 600-650 mg every 12 months, or
(iii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 3 months, the third administration of an anti-TRBV9 antibody at the main dose of 350-400 mg; or 475- 525 mg; or 600-650 mg after 12 months, and the subsequent administrations of an anti-TRBV9 antibody at the main dose of 350-400 mg; or 475- 525 mg; or 600-650 mg every 12 months, or
(iv) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 6 months, the third administration of an anti-TRBV9 antibody at the main dose of 350-400 mg; or 475- 525 mg; or 600-650 mg after 6 months, and the subsequent administrations of an anti-TRBV9 antibody at the main dose of 350-400 mg; or 475- 525 mg; or 600-650 mg every 12 months, or
(v) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 6 months, the third administration of an anti-TRBV9 antibody at the main dose of 350-400 mg; or 475- 525 mg; or 600-650 mg after 9 months, and the subsequent administrations of an anti-TRBV9 antibody at the main dose of 350-400 mg; or 475- 525 mg; or 600-650 mg every 12 months, or
(vi) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 6 months, the third administration of an anti-TRBV9 antibody at the main dose of 350-400 mg; or 475- 525 mg; or 600-650 mg after 12 months, and the subsequent administrations of an anti-TRBV9 antibody at the main dose of 350-400 mg; or 475- 525 mg; or 600-650 mg every 12 months, or
(vii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 9 months, the third administration of an anti-TRBV9 antibody at the main dose of 350-400 mg; or 475- 525 mg; or 600-650 mg after 9 months, and the subsequent administrations of an anti-TRBV9 antibody at the main dose of 350-400 mg; or 475- 525 mg; or 600-650 mg every 12 months, or
(viii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 9 months, the third administration of an anti-TRBV9 antibody at the main dose of 350-400 mg; or 475- 525 mg; or 600-650 mg after 12 months, and the subsequent administrations of an anti-TRBV9 antibody at the main dose of 350-400 mg; or 475- 525 mg; or 600-650 mg every 12 months, or
(ix)the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 12 months, the third administration of an anti-TRBV9 antibody at the main dose of 350-400 mg; or 475- 525 mg; or 600-650 mg after 12 months, and the subsequent administrations of an anti-TRBV9 antibody at the main dose of 350-400 mg; or 475- 525 mg; or 600-650 mg every 12 months.

In one aspect, the present invention relates to a method of treatment of a disease or disorder mediated by T-lymphocytes bearing the TRBV9 segment within the T-cell receptor, comprising the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after at least 3 months, the third administration of an anti-TRBV9 antibody at the main dose of 365-385 mg; or 490- 510 mg; or 615-635 mg after at least 6 months, and the subsequent administrations of an anti-TRBV9 antibody at the main dose of 365-385 mg; or 490- 510 mg; or 615-635 mg at least every 12 months.

In some embodiments of the invention, the method comprises (i) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 3 months, the third administration of an anti-TRBV9 antibody at the main dose of 365-385 mg; or 490- 510 mg; or 615-635 mg after 6 months, and the subsequent administrations of an anti-TRBV9 antibody at the main dose of 365-385 mg; or 490- 510 mg; or 615-635 mg every 12 months, or
(ii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 3 months, the third administration of an anti-TRBV9 antibody at the main dose of 365-385 mg; or 490- 510 mg; or 615-635 mg after 9 months, and the subsequent administrations of an anti-TRBV9 antibody at the main dose of 365-385 mg; or 490- 510 mg; or 615-635 mg every 12 months, or
(iii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 3 months, the third administration of an anti-TRBV9 antibody at the main dose of 365-385 mg; or 490- 510 mg; or 615-635 mg after 12 months, and the subsequent administrations of an anti-TRBV9 antibody at the main dose of 365-385 mg; or 490- 510 mg; or 615-635 mg every 12 months, or
(iv) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 6 months, the third administration of an anti-TRBV9 antibody at the main dose of 365-385 mg; or 490- 510 mg; or 615-635 mg after 6 months, and the subsequent administrations of an anti-TRBV9 antibody at the main dose of 365-385 mg; or 490- 510 mg; or 615-635 mg every 12 months, or
(v) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 6 months, the third administration of an anti-TRBV9 antibody at the main dose of 365-385 mg; or 490- 510 mg; or 615-635 mg after 9 months, and the subsequent administrations of an anti-TRBV9 antibody at the main dose of 365-385 mg; or 490- 510 mg; or 615-635 mg every 12 months, or
(vi) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 6 months, the third administration of an anti-TRBV9 antibody at the main dose of 365-385 mg; or 490- 510 mg; or 615-635 mg after 12 months, and the subsequent administrations of an anti-TRBV9 antibody at the main dose of 365-385 mg; or 490- 510 mg; or 615-635 mg every 12 months, or
(vii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 9 months, the third administration of an anti-TRBV9 antibody at the main dose of 365-385 mg; or 490- 510 mg; or 615-635 mg after 9 months, and the subsequent administrations of an anti-TRBV9 antibody at the main dose of 365-385 mg; or 490- 510 mg; or 615-635 mg every 12 months, or
(viii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 9 months, the third administration of an anti-TRBV9 antibody at the main dose of 365-385 mg; or 490- 510 mg; or 615-635 mg after 12 months, and the subsequent administrations of an anti-TRBV9 antibody at the main dose of 365-385 mg; or 490- 510 mg; or 615-635 mg every 12 months, or
(ix)the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 12 months, the third administration of an anti-TRBV9 antibody at the main dose of 365-385 mg; or 490- 510 mg; or 615-635 mg after 12 months, and the subsequent administrations of an anti-TRBV9 antibody at the main dose of 365-385 mg; or 490- 510 mg; or 615-635 mg every 12 months.

In one aspect, the present invention relates to a method of treatment of a disease or disorder mediated by T-lymphocytes bearing the TRBV9 segment within the T-cell receptor, comprising the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after at least 3 months, the third administration of an anti-TRBV9 antibody at the main dose of 370 mg; or 375 mg; or 380 mg; or 495 mg; or 500 mg; or 505 mg; or 620 mg; or 625 mg; or 630 mg after at least 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg at least every 12 months.

In one aspect, the present invention relates to a method of treatment of a disease or disorder mediated by T-lymphocytes bearing the TRBV9 segment within the T-cell receptor, comprising (i) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 3 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(ii) the first administration to a subject in need thereof of an anti-TRBV9 antibody at a dose of 120 mg; or 125 mg; or 130 mg; or 245 mg; or 250 mg; or 255 mg, the second administration of an anti-TRBV9 antibody at the main dose of 370 mg; or 375 mg; or 380 mg; or 495 mg; or 500 mg; or 505 mg; or 620 mg; or 625 mg; or 630 mg after 3 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 9 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(iii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 3 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 12 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(iv) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 6 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(v) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 6 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 9 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(vi) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 6 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 12 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(vii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 9 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 9 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(viii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 9 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 12 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(ix) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 12 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 12 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months.

In some embodiments of the invention, the method comprises the first administration of an anti-TRBV9 antibody at a dose of 120 mg, the second administration of an anti-TRBV9 antibody at a dose of 370 mg after at least 3 months, the third administration of an anti-TRBV9 antibody at a dose of 370 mg after at least 6 months, and the subsequent administrations of an anti-TRBV9 antibody at a dose of 370 mg at least every 12 months.

In some embodiments of the invention, the method comprises the first administration of an anti-TRBV9 antibody at a dose of 120 mg, the second administration of an anti-TRBV9 antibody at a dose of 370 mg after 3 months; the third administration of an anti-TRBV9 antibody at a dose of 370 mg after 6 months; or after 9 months; or after 12 months; and the subsequent administrations of an anti-TRBV9 antibody at a dose of 370 mg every 12 months.

In some embodiments of the invention, the method comprises the first administration of an anti-TRBV9 antibody at a dose of 120 mg, the second administration of an anti-TRBV9 antibody at a dose of 370 mg after 6 months; the third administration of an anti-TRBV9 antibody at a dose of 370 mg after 6 months; or after 9 months; or after 12 months; and the subsequent administrations of an anti-TRBV9 antibody at a dose of 370 mg every 12 months.

In some embodiments of the invention, the method comprises the first administration of an anti-TRBV9 antibody at a dose of 120 mg, the second administration of an anti-TRBV9 antibody at a dose of 370 mg after 9 months; the third administration of an anti-TRBV9 antibody at a dose of 370 mg after 9 months; or after 12 months; and the subsequent administrations of an anti-TRBV9 antibody at a dose of 370 mg every 12 months.

In some embodiments of the invention, the method comprises the first administration of an anti-TRBV9 antibody at a dose of 120 mg, the second administration of an anti-TRBV9 antibody at a dose of 370 mg after 12 months, the third administration of an anti-TRBV9 antibody at a dose of 370 mg after 12 months, and the subsequent administrations of an anti-TRBV9 antibody at a dose of 370 mg every 12 months.

In some embodiments of the invention, the method comprises the first administration of an anti-TRBV9 antibody at a dose of 125 mg, the second administration of an anti-TRBV9 antibody at a dose of 375 mg after at least 3 months, the third administration of an anti-TRBV9 antibody at a dose of 375 mg after at least 6 months, and the subsequent administrations of an anti-TRBV9 antibody at a dose of 375 mg at least every 12 months.

In some embodiments of the invention, the method comprises the first administration of an anti-TRBV9 antibody at a dose of 125 mg, the second administration of an anti-TRBV9 antibody at a dose of 375 mg after 3 months; the third administration of an anti-TRBV9 antibody at a dose of 375 mg after 6 months; or after 9 months; or after 12 months; and the subsequent administrations of an anti-TRBV9 antibody at a dose of 375 mg every 12 months.

In some embodiments of the invention, the method comprises the first administration of an anti-TRBV9 antibody at a dose of 125 mg, the second administration of an anti-TRBV9 antibody at a dose of 375 mg after 6 months; the third administration of an anti-TRBV9 antibody at a dose of 375 mg after 6 months; or after 9 months; or after 12 months; and the subsequent administrations of an anti-TRBV9 antibody at a dose of 375 mg every 12 months.

In some embodiments of the invention, the method comprises the first administration of an anti-TRBV9 antibody at a dose of 125 mg, the second administration of an anti-TRBV9 antibody at a dose of 375 mg after 9 months; the third administration of an anti-TRBV9 antibody at a dose of 375 mg after 9 months; or after 12 months; and the subsequent administrations of an anti-TRBV9 antibody at a dose of 375 mg every 12 months.

In some embodiments of the invention, the method comprises the first administration of an anti-TRBV9 antibody at a dose of 125 mg, the second administration of an anti-TRBV9 antibody at a dose of 375 mg after 12 months, the third administration of an anti-TRBV9 antibody at a dose of 375 mg after 12 months, and the subsequent administrations of an anti-TRBV9 antibody at a dose of 375 mg every 12 months.

In some embodiments of the invention, the method comprises the first administration of an anti-TRBV9 antibody at a dose of 130 mg, the second administration of an anti-TRBV9 antibody at a dose of 380 mg after at least 3 months, the third administration of an anti-TRBV9 antibody at a dose of 380 mg after at least 6 months, and the subsequent administrations of an anti-TRBV9 antibody at a dose of 380 mg at least every 12 months.

In some embodiments of the invention, the method comprises the first administration of an anti-TRBV9 antibody at a dose of 130 mg, the second administration of an anti-TRBV9 antibody at a dose of 380 mg after 3 months; the third administration of an anti-TRBV9 antibody at a dose of 380 mg after 6 months; or after 9 months; or after 12 months; and the subsequent administrations of an anti-TRBV9 antibody at a dose of 380 mg every 12 months.

In some embodiments of the invention, the method comprises the first administration of an anti-TRBV9 antibody at a dose of 130 mg, the second administration of an anti-TRBV9 antibody at a dose of 380 mg after 6 months; the third administration of an anti-TRBV9 antibody at a dose of 380 mg after 6 months; or after 9 months; or after 12 months; and the subsequent administrations of an anti-TRBV9 antibody at a dose of 380 mg every 12 months.

In some embodiments of the invention, the method comprises the first administration of an anti-TRBV9 antibody at a dose of 130 mg, the second administration of an anti-TRBV9 antibody at a dose of 380 mg after 9 months; the third administration of an anti-TRBV9 antibody at a dose of 380 mg after 9 months; or after 12 months; and the subsequent administrations of an anti-TRBV9 antibody at a dose of 380 mg every 12 months.

In some embodiments of the invention, the method comprises the first administration of an anti-TRBV9 antibody at a dose of 130 mg, the second administration of an anti-TRBV9 antibody at a dose of 380 mg after 12 months, the third administration of an anti-TRBV9 antibody at a dose of 380 mg after 12 months, and the subsequent administrations of an anti-TRBV9 antibody at a dose of 380 mg every 12 months.

In some embodiments of the invention, the method comprises the first administration of an anti-TRBV9 antibody at a dose of 120 mg, the second administration of an anti-TRBV9 antibody at a dose of 370 mg after at least 3 months, the third administration of an anti-TRBV9 antibody at a dose of 370 mg after at least 6 months, and the subsequent administrations of an anti-TRBV9 antibody at a dose of 370 mg at least every 12 months based on subject's body weight from 30 to 50 kg.

In some embodiments of the invention, the method comprises the first administration of an anti-TRBV9 antibody at a dose of 120 mg, the second administration of an anti-TRBV9 antibody at a dose of 370 mg after 3 months; the third administration of an anti-TRBV9 antibody at a dose of 370 mg after 6 months; or after 9 months; or after 12 months; and the subsequent administrations of an anti-TRBV9 antibody at a dose of 370 mg every 12 months based on subject's body weight from 30 to 50 kg.

In some embodiments of the invention, the method comprises the first administration of an anti-TRBV9 antibody at a dose of 120 mg, the second administration of an anti-TRBV9 antibody at a dose of 370 mg after 6 months; the third administration of an anti-TRBV9 antibody at a dose of 370 mg after 6 months; or after 9 months; or after 12 months; and the subsequent administrations of an anti-TRBV9 antibody at a dose of 370 mg every 12 months based on subject's body weight from 30 to 50 kg.

In some embodiments of the invention, the method comprises the first administration of an anti-TRBV9 antibody at a dose of 120 mg, the second administration of an anti-TRBV9 antibody at a dose of 370 mg after 9 months; the third administration of an anti-TRBV9 antibody at a dose of 370 mg after 9 months; or after 12 months; and the subsequent administrations of an anti-TRBV9 antibody at a dose of 370 mg every 12 months based on subject's body weight from 30 to 50 kg.

In some embodiments of the invention, the method comprises the first administration of an anti-TRBV9 antibody at a dose of 120 mg, the second administration of an anti-TRBV9 antibody at a dose of 370 mg after 12 months, the third administration of an anti-TRBV9 antibody at a dose of 370 mg after 12 months, and the subsequent administrations of an anti-TRBV9 antibody at a dose of 370 mg every 12 months based on subject's body weight from 30 to 50 kg.

In some embodiments of the invention, the method comprises the first administration of an anti-TRBV9 antibody at a dose of 125 mg, the second administration of an anti-TRBV9 antibody at a dose of 375 mg after at least 3 months, the third administration of an anti-TRBV9 antibody at a dose of 375 mg after at least 6 months, and the subsequent administrations of an anti-TRBV9 antibody at a dose of 375 mg at least every 12 months based on subject's body weight from 30 to 50 kg.

In some embodiments of the invention, the method comprises the first administration of an anti-TRBV9 antibody at a dose of 125 mg, the second administration of an anti-TRBV9 antibody at a dose of 375 mg after 3 months; the third administration of an anti-TRBV9 antibody at a dose of 375 mg after 6 months; or after 9 months; or after 12 months; and the subsequent administrations of an anti-TRBV9 antibody at a dose of 375 mg every 12 months based on subject's body weight from 30 to 50 kg.

In some embodiments of the invention, the method comprises the first administration of an anti-TRBV9 antibody at a dose of 125 mg, the second administration of an anti-TRBV9 antibody at a dose of 375 mg after 6 months; the third administration of an anti-TRBV9 antibody at a dose of 375 mg after 6 months; or after 9 months; or after 12 months; and the subsequent administrations of an anti-TRBV9 antibody at a dose of 375 mg every 12 months based on subject's body weight from 30 to 50 kg.

In some embodiments of the invention, the method comprises the first administration of an anti-TRBV9 antibody at a dose of 125 mg, the second administration of an anti-TRBV9 antibody at a dose of 375 mg after 9 months; the third administration of an anti-TRBV9 antibody at a dose of 375 mg after 9 months; or after 12 months; and the subsequent administrations of an anti-TRBV9 antibody at a dose of 375 mg every 12 months based on subject's body weight from 30 to 50 kg.

In some embodiments of the invention, the method comprises the first administration of an anti-TRBV9 antibody at a dose of 125 mg, the second administration of an anti-TRBV9 antibody at a dose of 375 mg after 12 months, the third administration of an anti-TRBV9 antibody at a dose of 375 mg after 12 months, and the subsequent administrations of an anti-TRBV9 antibody at a dose of 375 mg every 12 months based on subject's body weight from 30 to 50 kg.

In some embodiments of the invention, the method comprises the first administration of an anti-TRBV9 antibody at a dose of 130 mg, the second administration of an anti-TRBV9 antibody at a dose of 380 mg after at least 3 months, the third administration of an anti-TRBV9 antibody at a dose of 380 mg after at least 6 months, and the subsequent administrations of an anti-TRBV9 antibody at a dose of 380 mg at least every 12 months based on subject's body weight from 30 to 50 kg.

In some embodiments of the invention, the method comprises the first administration of an anti-TRBV9 antibody at a dose of 130 mg, the second administration of an anti-TRBV9 antibody at a dose of 380 mg after 3 months; the third administration of an anti-TRBV9 antibody at a dose of 380 mg after 6 months; or after 9 months; or after 12 months; and the subsequent administrations of an anti-TRBV9 antibody at a dose of 380 mg every 12 months based on subject's body weight from 30 to 50 kg.

In some embodiments of the invention, the method comprises the first administration of an anti-TRBV9 antibody at a dose of 130 mg, the second administration of an anti-TRBV9 antibody at a dose of 380 mg after 6 months; the third administration of an anti-TRBV9 antibody at a dose of 380 mg after 6 months; or after 9 months; or after 12 months; and the subsequent administrations of an anti-TRBV9 antibody at a dose of 380 mg every 12 months based on subject's body weight from 30 to 50 kg.

In some embodiments of the invention, the method comprises the first administration of an anti-TRBV9 antibody at a dose of 130 mg, the second administration of an anti-TRBV9 antibody at a dose of 380 mg after 9 months; the third administration of an anti-TRBV9 antibody at a dose of 380 mg after 9 months; or after 12 months; and the subsequent administrations of an anti-TRBV9 antibody at a dose of 380 mg every 12 months based on subject's body weight from 30 to 50 kg.

In some embodiments of the invention, the method comprises the first administration of an anti-TRBV9 antibody at a dose of 130 mg, the second administration of an anti-TRBV9 antibody at a dose of 380 mg after 12 months, the third administration of an anti-TRBV9 antibody at a dose of 380 mg after 12 months, and the subsequent administrations of an anti-TRBV9 antibody at a dose of 380 mg every 12 months based on subject's body weight from 30 to 50 kg.

In some embodiments of the invention, the method comprises the first administration of an anti-TRBV9 antibody at a dose of 245 mg, the second administration of an anti-TRBV9 antibody at a dose of 495 mg after at least 3 months, the third administration of an anti-TRBV9 antibody at a dose of 495 mg after at least 6 months, and the subsequent administrations of an anti-TRBV9 antibody at a dose of 495 mg at least every 12 months.

In some embodiments of the invention, the method comprises the first administration of an anti-TRBV9 antibody at a dose of 245 mg, the second administration of an anti-TRBV9 antibody at a dose of 495 mg after 3 months; the third administration of an anti-TRBV9 antibody at a dose of 495 mg after 6 months; or after 9 months; or after 12 months; and the subsequent administrations of an anti-TRBV9 antibody at a dose of 495 mg every 12 months.

In some embodiments of the invention, the method comprises the first administration of an anti-TRBV9 antibody at a dose of 245 mg, the second administration of an anti-TRBV9 antibody at a dose of 495 mg after 6 months; the third administration of an anti-TRBV9 antibody at a dose of 495 mg after 6 months; or after 9 months; or after 12 months; and the subsequent administrations of an anti-TRBV9 antibody at a dose of 495 mg every 12 months.

In some embodiments of the invention, the method comprises the first administration of an anti-TRBV9 antibody at a dose of 245 mg, the second administration of an anti-TRBV9 antibody at a dose of 495 mg after 9 months; the third administration of an anti-TRBV9 antibody at a dose of 495 mg after 9 months; or after 12 months; and the subsequent administrations of an anti-TRBV9 antibody at a dose of 495 mg every 12 months.

In some embodiments of the invention, the method comprises the first administration of an anti-TRBV9 antibody at a dose of 245 mg, the second administration of an anti-TRBV9 antibody at a dose of 495 mg after 12 months, the third administration of an anti-TRBV9 antibody at a dose of 495 mg after 12 months, and the subsequent administrations of an anti-TRBV9 antibody at a dose of 495 mg every 12 months.

In some embodiments of the invention, the method comprises the first administration of an anti-TRBV9 antibody at a dose of 250 mg, the second administration of an anti-TRBV9 antibody at a dose of 500 mg after at least 3 months, the third administration of an anti-TRBV9 antibody at a dose of 500 mg after at least 6 months, and the subsequent administrations of an anti-TRBV9 antibody at a dose of 500 mg at least every 12 months.

In some embodiments of the invention, the method comprises the first administration of an anti-TRBV9 antibody at a dose of 250 mg, the second administration of an anti-TRBV9 antibody at a dose of 500 mg after 3 months; the third administration of an anti-TRBV9 antibody at a dose of 500 mg after 6 months; or after 9 months; or after 12 months; and the subsequent administrations of an anti-TRBV9 antibody at a dose of 500 mg every 12 months.

In some embodiments of the invention, the method comprises the first administration of an anti-TRBV9 antibody at a dose of 250 mg, the second administration of an anti-TRBV9 antibody at a dose of 500 mg after 6 months; the third administration of an anti-TRBV9 antibody at a dose of 500 mg after 6 months; or after 9 months; or after 12 months; and the subsequent administrations of an anti-TRBV9 antibody at a dose of 500 mg every 12 months.

In some embodiments of the invention, the method comprises the first administration of an anti-TRBV9 antibody at a dose of 250 mg, the second administration of an anti-TRBV9 antibody at a dose of 500 mg after 9 months; the third administration of an anti-TRBV9 antibody at a dose of 500 mg after 9 months; or after 12 months; and the subsequent administrations of an anti-TRBV9 antibody at a dose of 500 mg every 12 months.

In some embodiments of the invention, the method comprises the first administration of an anti-TRBV9 antibody at a dose of 250 mg, the second administration of an anti-TRBV9 antibody at a dose of 500 mg after 12 months, the third administration of an anti-TRBV9 antibody at a dose of 500 mg after 12 months, and the subsequent administrations of an anti-TRBV9 antibody at a dose of 500 mg every 12 months.

In some embodiments of the invention, the method comprises the first administration of an anti-TRBV9 antibody at a dose of 255 mg, the second administration of an anti-TRBV9 antibody at a dose of 505 mg after at least 3 months, the third administration of an anti-TRBV9 antibody at a dose of 505 mg after at least 6 months, and the subsequent administrations of an anti-TRBV9 antibody at a dose of 505 mg at least every 12 months.

In some embodiments of the invention, the method comprises the first administration of an anti-TRBV9 antibody at a dose of 255 mg, the second administration of an anti-TRBV9 antibody at a dose of 505 mg after 3 months; the third administration of an anti-TRBV9 antibody at a dose of 505 mg after 6 months; or after 9 months; or after 12 months; and the subsequent administrations of an anti-TRBV9 antibody at a dose of 505 mg every 12 months.

In some embodiments of the invention, the method comprises the first administration of an anti-TRBV9 antibody at a dose of 255 mg, the second administration of an anti-TRBV9 antibody at a dose of 505 mg after 6 months; the third administration of an anti-TRBV9 antibody at a dose of 505 mg after 6 months; or after 9 months; or after 12 months; and the subsequent administrations of an anti-TRBV9 antibody at a dose of 505 mg every 12 months.

In some embodiments of the invention, the method comprises the first administration of an anti-TRBV9 antibody at a dose of 255 mg, the second administration of an anti-TRBV9 antibody at a dose of 505 mg after 9 months; the third administration of an anti-TRBV9 antibody at a dose of 505 mg after 9 months; or after 12 months; and the subsequent administrations of an anti-TRBV9 antibody at a dose of 505 mg every 12 months.

In some embodiments of the invention, the method comprises the first administration of an anti-TRBV9 antibody at a dose of 255 mg, the second administration of an anti-TRBV9 antibody at a dose of 505 mg after 12 months, the third administration of an anti-TRBV9 antibody at a dose of 505 mg after 12 months, and the subsequent administrations of an anti-TRBV9 antibody at a dose of 505 mg every 12 months.

In some embodiments of the invention, the method comprises the first administration of an anti-TRBV9 antibody at a dose of 245 mg, the second administration of an anti-TRBV9 antibody at a dose of 495 mg after at least 3 months, the third administration of an anti-TRBV9 antibody at a dose of 495 mg after at least 6 months, and the subsequent administrations of an anti-TRBV9 antibody at a dose of 495 mg at least every 12 months based on subject's body weight from 51 to 89 kg.

In some embodiments of the invention, the method comprises the first administration of an anti-TRBV9 antibody at a dose of 245 mg, the second administration of an anti-TRBV9 antibody at a dose of 495 mg after 3 months; the third administration of an anti-TRBV9 antibody at a dose of 495 mg after 6 months; or after 9 months; or after 12 months; and the subsequent administrations of an anti-TRBV9 antibody at a dose of 495 mg every 12 months based on subject's body weight from 51 to 89 kg.

In some embodiments of the invention, the method comprises the first administration of an anti-TRBV9 antibody at a dose of 245 mg, the second administration of an anti-TRBV9 antibody at a dose of 495 mg after 6 months; the third administration of an anti-TRBV9 antibody at a dose of 495 mg after 6 months; or after 9 months; or after 12 months; and the subsequent administrations of an anti-TRBV9 antibody at a dose of 495 mg every 12 months based on subject's body weight from 51 to 89 kg.

In some embodiments of the invention, the method comprises the first administration of an anti-TRBV9 antibody at a dose of 245 mg, the second administration of an anti-TRBV9 antibody at a dose of 495 mg after 9 months; the third administration of an anti-TRBV9 antibody at a dose of 495 mg after 9 months; or after 12 months; and the subsequent administrations of an anti-TRBV9 antibody at a dose of 495 mg every 12 months based on subject's body weight from 51 to 89 kg.

In some embodiments of the invention, the method comprises the first administration of an anti-TRBV9 antibody at a dose of 245 mg, the second administration of an anti-TRBV9 antibody at a dose of 495 mg after 12 months, the third administration of an anti-TRBV9 antibody at a dose of 495 mg after 12 months, and the subsequent administrations of an anti-TRBV9 antibody at a dose of 495 mg every 12 months based on subject's body weight from 51 to 89 kg.

In some embodiments of the invention, the method comprises the first administration of an anti-TRBV9 antibody at a dose of 250 mg, the second administration of an anti-TRBV9 antibody at a dose of 500 mg after at least 3 months, the third administration of an anti-TRBV9 antibody at a dose of 500 mg after at least 6 months, and the subsequent administrations of an anti-TRBV9 antibody at a dose of 500 mg at least every 12 months based on subject's body weight from 51 to 89 kg.

In some embodiments of the invention, the method comprises the first administration of an anti-TRBV9 antibody at a dose of 250 mg, the second administration of an anti-TRBV9 antibody at a dose of 500 mg after 3 months; the third administration of an anti-TRBV9 antibody at a dose of 500 mg after 6 months; or after 9 months; or after 12 months; and the subsequent administrations of an anti-TRBV9 antibody at a dose of 500 mg every 12 months based on subject's body weight from 51 to 89 kg.

In some embodiments of the invention, the method comprises the first administration of an anti-TRBV9 antibody at a dose of 250 mg, the second administration of an anti-TRBV9 antibody at a dose of 500 mg after 6 months; the third administration of an anti-TRBV9 antibody at a dose of 500 mg after 6 months; or after 9 months; or after 12 months; and the subsequent administrations of an anti-TRBV9 antibody at a dose of 500 mg every 12 months based on subject's body weight from 51 to 89 kg.

In some embodiments of the invention, the method comprises the first administration of an anti-TRBV9 antibody at a dose of 250 mg, the second administration of an anti-TRBV9 antibody at a dose of 500 mg after 9 months; the third administration of an anti-TRBV9 antibody at a dose of 500 mg after 9 months; or after 12 months; and the subsequent administrations of an anti-TRBV9 antibody at a dose of 500 mg every 12 months based on subject's body weight from 51 to 89 kg.

In some embodiments of the invention, the method comprises the first administration of an anti-TRBV9 antibody at a dose of 250 mg, the second administration of an anti-TRBV9 antibody at a dose of 500 mg after 12 months, the third administration of an anti-TRBV9 antibody at a dose of 500 mg after 12 months, and the subsequent administrations of an anti-TRBV9 antibody at a dose of 500 mg every 12 months based on subject's body weight from 51 to 89 kg.

In some embodiments of the invention, the method comprises the first administration of an anti-TRBV9 antibody at a dose of 255 mg, the second administration of an anti-TRBV9 antibody at a dose of 505 mg after at least 3 months, the third administration of an anti-TRBV9 antibody at a dose of 505 mg after at least 6 months, and the subsequent administrations of an anti-TRBV9 antibody at a dose of 505 mg at least every 12 months based on subject's body weight from 51 to 89 kg.

In some embodiments of the invention, the method comprises the first administration of an anti-TRBV9 antibody at a dose of 255 mg, the second administration of an anti-TRBV9 antibody at a dose of 505 mg after 3 months; the third administration of an anti-TRBV9 antibody at a dose of 505 mg after 6 months; or after 9 months; or after 12 months; and the subsequent administrations of an anti-TRBV9 antibody at a dose of 505 mg every 12 months based on subject's body weight from 51 to 89 kg.

In some embodiments of the invention, the method comprises the first administration of an anti-TRBV9 antibody at a dose of 255 mg, the second administration of an anti-TRBV9 antibody at a dose of 505 mg after 6 months; the third administration of an anti-TRBV9 antibody at a dose of 505 mg after 6 months; or after 9 months; or after 12 months; and the subsequent administrations of an anti-TRBV9 antibody at a dose of 505 mg every 12 months based on subject's body weight from 51 to 89 kg.

In some embodiments of the invention, the method comprises the first administration of an anti-TRBV9 antibody at a dose of 255 mg, the second administration of an anti-TRBV9 antibody at a dose of 505 mg after 9 months; the third administration of an anti-TRBV9 antibody at a dose of 505 mg after 9 months; or after 12 months; and the subsequent administrations of an anti-TRBV9 antibody at a dose of 505 mg every 12 months based on subject's body weight from 51 to 89 kg.

In some embodiments of the invention, the method comprises the first administration of an anti-TRBV9 antibody at a dose of 255 mg, the second administration of an anti-TRBV9 antibody at a dose of 505 mg after 12 months, the third administration of an anti-TRBV9 antibody at a dose of 505 mg after 12 months, and the subsequent administrations of an anti-TRBV9 antibody at a dose of 505 mg every 12 months based on subject's body weight from 51 to 89 kg.

In some embodiments of the invention, the method comprises the first administration of an anti-TRBV9 antibody at a dose of 245 mg, the second administration of an anti-TRBV9 antibody at a dose of 620 mg after at least 3 months, the third administration of an anti-TRBV9 antibody at a dose of 620 mg after at least 6 months, and the subsequent administrations of an anti-TRBV9 antibody at a dose of 620 mg at least every 12 months.

In some embodiments of the invention, the method comprises the first administration of an anti-TRBV9 antibody at a dose of 245 mg, the second administration of an anti-TRBV9 antibody at a dose of 620 mg after 3 months; the third administration of an anti-TRBV9 antibody at a dose of 620 mg after 6 months; or after 9 months; or after 12 months; and the subsequent administrations of an anti-TRBV9 antibody at a dose of 620 mg every 12 months.

In some embodiments of the invention, the method comprises the first administration of an anti-TRBV9 antibody at a dose of 245 mg, the second administration of an anti-TRBV9 antibody at a dose of 620 mg after 6 months; the third administration of an anti-TRBV9 antibody at a dose of 620 mg after 6 months; or after 9 months; or after 12 months; and the subsequent administrations of an anti-TRBV9 antibody at a dose of 620 mg every 12 months.

In some embodiments of the invention, the method comprises the first administration of an anti-TRBV9 antibody at a dose of 245 mg, the second administration of an anti-TRBV9 antibody at a dose of 620 mg after 9 months; the third administration of an anti-TRBV9 antibody at a dose of 620 mg after 9 months; or after 12 months; and the subsequent administrations of an anti-TRBV9 antibody at a dose of 620 mg every 12 months.

In some embodiments of the invention, the method comprises the first administration of an anti-TRBV9 antibody at a dose of 245 mg, the second administration of an anti-TRBV9 antibody at a dose of 620 mg after 12 months, the third administration of an anti-TRBV9 antibody at a dose of 620 mg after 12 months, and the subsequent administrations of an anti-TRBV9 antibody at a dose of 620 mg every 12 months.

In some embodiments of the invention, the method comprises the first administration of an anti-TRBV9 antibody at a dose of 250 mg, the second administration of an anti-TRBV9 antibody at a dose of 625 mg after at least 3 months, the third administration of an anti-TRBV9 antibody at a dose of 625 mg after at least 6 months, and the subsequent administrations of an anti-TRBV9 antibody at a dose of 625 mg at least every 12 months.

In some embodiments of the invention, the method comprises the first administration of an anti-TRBV9 antibody at a dose of 250 mg, the second administration of an anti-TRBV9 antibody at a dose of 625 mg after 3 months; the third administration of an anti-TRBV9 antibody at a dose of 625 mg after 6 months; or after 9 months; or after 12 months; and the subsequent administrations of an anti-TRBV9 antibody at a dose of 625 mg every 12 months.

In some embodiments of the invention, the method comprises the first administration of an anti-TRBV9 antibody at a dose of 250 mg, the second administration of an anti-TRBV9 antibody at a dose of 625 mg after 6 months; the third administration of an anti-TRBV9 antibody at a dose of 625 mg after 6 months; or after 9 months; or after 12 months; and the subsequent administrations of an anti-TRBV9 antibody at a dose of 625 mg every 12 months.

In some embodiments of the invention, the method comprises the first administration of an anti-TRBV9 antibody at a dose of 250 mg, the second administration of an anti-TRBV9 antibody at a dose of 625 mg after 9 months; the third administration of an anti-TRBV9 antibody at a dose of 625 mg after 9 months; or after 12 months; and the subsequent administrations of an anti-TRBV9 antibody at a dose of 625 mg every 12 months.

In some embodiments of the invention, the method comprises the first administration of an anti-TRBV9 antibody at a dose of 250 mg, the second administration of an anti-TRBV9 antibody at a dose of 625 mg after 12 months, the third administration of an anti-TRBV9 antibody at a dose of 625 mg after 12 months, and the subsequent administrations of an anti-TRBV9 antibody at a dose of 625 mg every 12 months.

In some embodiments of the invention, the method comprises the first administration of an anti-TRBV9 antibody at a dose of 255 mg, the second administration of an anti-TRBV9 antibody at a dose of 630 mg after at least 3 months, the third administration of an anti-TRBV9 antibody at a dose of 630 mg after at least 6 months, and the subsequent administrations of an anti-TRBV9 antibody at a dose of 630 mg at least every 12 months.

In some embodiments of the invention, the method comprises the first administration of an anti-TRBV9 antibody at a dose of 255 mg, the second administration of an anti-TRBV9 antibody at a dose of 630 mg after 3 months; the third administration of an anti-TRBV9 antibody at a dose of 630 mg after 6 months; or after 9 months; or after 12 months; and the subsequent administrations of an anti-TRBV9 antibody at a dose of 630 mg every 12 months.

In some embodiments of the invention, the method comprises the first administration of an anti-TRBV9 antibody at a dose of 255 mg, the second administration of an anti-TRBV9 antibody at a dose of 630 mg after 6 months; the third administration of an anti-TRBV9 antibody at a dose of 630 mg after 6 months; or after 9 months; or after 12 months; and the subsequent administrations of an anti-TRBV9 antibody at a dose of 630 mg every 12 months.

In some embodiments of the invention, the method comprises the first administration of an anti-TRBV9 antibody at a dose of 255 mg, the second administration of an anti-TRBV9 antibody at a dose of 630 mg after 9 months; the third administration of an anti-TRBV9 antibody at a dose of 630 mg after 9 months; or after 12 months; and the subsequent administrations of an anti-TRBV9 antibody at a dose of 630 mg every 12 months.

In some embodiments of the invention, the method comprises the first administration of an anti-TRBV9 antibody at a dose of 255 mg, the second administration of an anti-TRBV9 antibody at a dose of 630 mg after 12 months, the third administration of an anti-TRBV9 antibody at a dose of 630 mg after 12 months, and the subsequent administrations of an anti-TRBV9 antibody at a dose of 630 mg every 12 months.

In some embodiments of the invention, the method comprises the first administration of an anti-TRBV9 antibody at a dose of 245 mg, the second administration of an anti-TRBV9 antibody at a dose of 620 mg after at least 3 months, the third administration of an anti-TRBV9 antibody at a dose of 620 mg after at least 6 months, and the subsequent administrations of an anti-TRBV9 antibody at a dose of 620 mg at least every 12 months based on subject's body weight from 90 to 150 kg.

In some embodiments of the invention, the method comprises the first administration of an anti-TRBV9 antibody at a dose of 245 mg, the second administration of an anti-TRBV9 antibody at a dose of 620 mg after 3 months; the third administration of an anti-TRBV9 antibody at a dose of 620 mg after 6 months; or after 9 months; or after 12 months; and the subsequent administrations of an anti-TRBV9 antibody at a dose of 620 mg every 12 months based on subject's body weight from 90 to 150 kg.

In some embodiments of the invention, the method comprises the first administration of an anti-TRBV9 antibody at a dose of 245 mg, the second administration of an anti-TRBV9 antibody at a dose of 620 mg after 6 months; the third administration of an anti-TRBV9 antibody at a dose of 620 mg after 6 months; or after 9 months; or after 12 months; and the subsequent administrations of an anti-TRBV9 antibody at a dose of 620 mg every 12 months based on subject's body weight from 90 to 150 kg.

In some embodiments of the invention, the method comprises the first administration of an anti-TRBV9 antibody at a dose of 245 mg, the second administration of an anti-TRBV9 antibody at a dose of 620 mg after 9 months; the third administration of an anti-TRBV9 antibody at a dose of 620 mg after 9 months; or after 12 months; and the subsequent administrations of an anti-TRBV9 antibody at a dose of 620 mg every 12 months based on subject's body weight from 90 to 150 kg.

In some embodiments of the invention, the method comprises the first administration of an anti-TRBV9 antibody at a dose of 245 mg, the second administration of an anti-TRBV9 antibody at a dose of 620 mg after 12 months, the third administration of an anti-TRBV9 antibody at a dose of 620 mg after 12 months, and the subsequent administrations of an anti-TRBV9 antibody at a dose of 620 mg every 12 months based on subject's body weight from 90 to 150 kg.

In some embodiments of the invention, the method comprises the first administration of an anti-TRBV9 antibody at a dose of 250 mg, the second administration of an anti-TRBV9 antibody at a dose of 625 mg after at least 3 months, the third administration of an anti-TRBV9 antibody at a dose of 625 mg after at least 6 months, and the subsequent administrations of an anti-TRBV9 antibody at a dose of 625 mg at least every 12 months based on subject's body weight from 90 to 150 kg.

In some embodiments of the invention, the method comprises the first administration of an anti-TRBV9 antibody at a dose of 250 mg, the second administration of an anti-TRBV9 antibody at a dose of 625 mg after 3 months; the third administration of an anti-TRBV9 antibody at a dose of 625 mg after 6 months; or after 9 months; or after 12 months; and the subsequent administrations of an anti-TRBV9 antibody at a dose of 625 mg every 12 months based on subject's body weight from 90 to 150 kg.

In some embodiments of the invention, the method comprises the first administration of an anti-TRBV9 antibody at a dose of 250 mg, the second administration of an anti-TRBV9 antibody at a dose of 625 mg after 6 months; the third administration of an anti-TRBV9 antibody at a dose of 625 mg after 6 months; or after 9 months; or after 12 months; and the subsequent administrations of an anti-TRBV9 antibody at a dose of 625 mg every 12 months based on subject's body weight from 90 to 150 kg.

In some embodiments of the invention, the method comprises the first administration of an anti-TRBV9 antibody at a dose of 250 mg, the second administration of an anti-TRBV9 antibody at a dose of 625 mg after 9 months; the third administration of an anti-TRBV9 antibody at a dose of 625 mg after 9 months; or after 12 months; and the subsequent administrations of an anti-TRBV9 antibody at a dose of 625 mg every 12 months based on subject's body weight from 90 to 150 kg.

In some embodiments of the invention, the method comprises the first administration of an anti-TRBV9 antibody at a dose of 250 mg, the second administration of an anti-TRBV9 antibody at a dose of 625 mg after 12 months, the third administration of an anti-TRBV9 antibody at a dose of 625 mg after 12 months, and the subsequent administrations of an anti-TRBV9 antibody at a dose of 625 mg every 12 months based on subject's body weight from 90 to 150 kg.

In some embodiments of the invention, the method comprises the first administration of an anti-TRBV9 antibody at a dose of 255 mg, the second administration of an anti-TRBV9 antibody at a dose of 630 mg after at least 3 months, the third administration of an anti-TRBV9 antibody at a dose of 630 mg after at least 6 months, and the subsequent administrations of an anti-TRBV9 antibody at a dose of 630 mg at least every 12 months based on subject's body weight from 90 to 150 kg.

In some embodiments of the invention, the method comprises the first administration of an anti-TRBV9 antibody at a dose of 255 mg, the second administration of an anti-TRBV9 antibody at a dose of 630 mg after 3 months; the third administration of an anti-TRBV9 antibody at a dose of 630 mg after 6 months; or after 9 months; or after 12 months; and the subsequent administrations of an anti-TRBV9 antibody at a dose of 630 mg every 12 months based on subject's body weight from 90 to 150 kg.

In some embodiments of the invention, the method comprises the first administration of an anti-TRBV9 antibody at a dose of 255 mg, the second administration of an anti-TRBV9 antibody at a dose of 630 mg after 6 months; the third administration of an anti-TRBV9 antibody at a dose of 630 mg after 6 months; or after 9 months; or after 12 months; and the subsequent administrations of an anti-TRBV9 antibody at a dose of 630 mg every 12 months based on subject's body weight from 90 to 150 kg.

In some embodiments of the invention, the method comprises the first administration of an anti-TRBV9 antibody at a dose of 255 mg, the second administration of an anti-TRBV9 antibody at a dose of 630 mg after 9 months; the third administration of an anti-TRBV9 antibody at a dose of 630 mg after 9 months; or after 12 months; and the subsequent administrations of an anti-TRBV9 antibody at a dose of 630 mg every 12 months based on subject's body weight from 90 to 150 kg.

In some embodiments of the invention, the method comprises the first administration of an anti-TRBV9 antibody at a dose of 255 mg, the second administration of an anti-TRBV9 antibody at a dose of 630 mg after 12 months, the third administration of an anti-TRBV9 antibody at a dose of 630 mg after 12 months, and the subsequent administrations of an anti-TRBV9 antibody at a dose of 630 mg every 12 months based on subject's body weight from 90 to 150 kg.

In one aspect, the present invention relates to use of an anti-TRBV9 antibody for the treatment of a disease or disorder mediated by T-lymphocytes bearing the TRBV9 segment within the T-cell receptor, wherein the anti-TRBV9 antibody is administered to a subject in need thereof at a dose of 0.03-10 mg/kg.

In one aspect, the present invention relates to use of an anti-TRBV9 antibody for the treatment of a disease or disorder mediated by T-lymphocytes bearing the TRBV9 segment within the T-cell receptor, wherein the anti-TRBV9 antibody is administered to a subject in need thereof at a dose of 0.03-7 mg/kg.

In one aspect, the present invention relates to use of an anti-TRBV9 antibody for the treatment of a disease or disorder mediated by T-lymphocytes bearing the TRBV9 segment within the T-cell receptor, wherein the anti-TRBV9 antibody is administered to a subject in need thereof at a dose of 0.3-10 mg/kg.

In one aspect, the present invention relates to use of an anti-TRBV9 antibody for the treatment of a disease or disorder mediated by T-lymphocytes bearing the TRBV9 segment within the T-cell receptor, wherein the anti-TRBV9 antibody is administered to a subject in need thereof at a dose of 0.3-7 mg/kg.

In one aspect, the present invention relates to use of an anti-TRBV9 antibody for the treatment of a disease or disorder mediated by T-lymphocytes bearing the TRBV9 segment within the T-cell receptor, wherein the anti-TRBV9 antibody is administered to a subject in need thereof at a dose of 1-7 mg/kg.

In some embodiments of the invention, the anti-TRBV9 antibody is administered at a dose of 2-7 mg/kg.

In some embodiments of the invention, the anti-TRBV9 antibody is administered at a dose of 3-7 mg/kg.

In some embodiments of the invention, the anti-TRBV9 antibody is administered at a dose of 4-7 mg/kg.

In some embodiments of the invention, the anti-TRBV9 antibody is administered at a dose of 5-7 mg/kg.

In some embodiments of the invention, the anti-TRBV9 antibody is administered at a dose of 6-7 mg/kg.

In some embodiments of the invention, the anti-TRBV9 antibody is administered at a dose of 1 mg/kg, or 2 mg/kg, or 3 mg/kg, or 4 mg/kg, or 5 mg/kg, or 6 mg/kg, or 7 mg/kg.

In some embodiments of the invention, the anti-TRBV9 antibody is administered at a dose of 7.5 mg/kg, or 8 mg/kg, or 8.5 mg/kg, or 9 mg/kg, or 9.5 mg/kg, or 10 mg/kg.

In some embodiments of the invention, the anti-TRBV9 antibody is administered at least every 3-12 months.

In some embodiments of the invention, the anti-TRBV9 antibody is administered every 3-12 months.

In some embodiments of the invention, the anti-TRBV9 antibody is administered at least every 6-12 months.

In some embodiments of the invention, the anti-TRBV9 antibody is administered every 6-12 months.

In some embodiments of the invention, the anti-TRBV9 antibody is administered at least every 3 months; or at least every 6 months; or at least every 9 months; or at least every 12 months.

In some embodiments of the invention, the anti-TRBV9 antibody is administered every 3 months; or every 6 months; or every 9 months; or every 12 months.

In one aspect, the present invention relates to use of an anti-TRBV9 antibody for the treatment of a disease or disorder mediated by T-lymphocytes bearing the TRBV9 segment within the T-cell receptor, wherein the anti-TRBV9 antibody is administered to a subject in need thereof at a dose of 75-675 mg.

In one aspect, the present invention relates to use of an anti-TRBV9 antibody for the treatment of a disease or disorder mediated by T-lymphocytes bearing the TRBV9 segment within the T-cell receptor, wherein the anti-TRBV9 antibody is administered to a subject in need thereof at a dose of 100-650 mg.

In one aspect, the present invention relates to use of an anti-TRBV9 antibody for the treatment of a disease or disorder mediated by T-lymphocytes bearing the TRBV9 segment within the T-cell receptor, wherein the anti-TRBV9 antibody is administered to a subject in need thereof at a dose of 75-175 mg; or 200-300mg; or 325-425 mg; or 450- 650 mg; or 575-675 mg.

In one aspect, the present invention relates to use of an anti-TRBV9 antibody for the treatment of a disease or disorder mediated by T-lymphocytes bearing the TRBV9 segment within the T-cell receptor, wherein the anti-TRBV9 antibody is administered to a subject in need thereof at a dose of 100-150 mg; or 225-275 mg; or 350-400 mg; or 475- 525 mg; or 600-650mg.

In one aspect, the present invention relates to use of an anti-TRBV9 antibody for the treatment of a disease or disorder mediated by T-lymphocytes bearing the TRBV9 segment within the T-cell receptor, wherein the anti-TRBV9 antibody is administered to a subject in need thereof at a dose of 115-135 mg; or 240-260 mg; or 365-385 mg; or 490- 510 mg; or 615-635mg.

In some embodiments of the invention, the anti-TRBV9 antibody is administered at a dose of 120 mg; or 125 mg; or 130 mg; or 245 mg; or 250 mg; or 255 mg; or 370 mg; or 375 mg; or 380 mg; or 495 mg; or 500 mg; or 505 mg; or 620 mg; or 625 mg; or 630 mg.

In some embodiments of the invention, the anti-TRBV9 antibody is administered at least every 3-12 months.

In some embodiments of the invention, the anti-TRBV9 antibody is administered every 3-12 months.

In some embodiments of the invention, the anti-TRBV9 antibody is administered at least every 6-12 months.

In some embodiments of the invention, the anti-TRBV9 antibody is administered every 6-12 months.

In some embodiments of the invention, the anti-TRBV9 antibody is administered at least every 3 months; or at least every 6 months; or at least every 9 months; or at least every 12 months.

In some embodiments of the invention, the anti-TRBV9 antibody is administered every 3 months; or every 6 months; or every 9 months; or every 12 months.

In one aspect, the present invention relates to use of an anti-TRBV9 antibody for the treatment of a disease or disorder mediated by T-lymphocytes bearing the TRBV9 segment within the T-cell receptor, wherein the first administration of an anti-TRBV9 antibody to a subject in need thereof is performed at a dose equal to half of the main dose, 0.015-5 mg/kg, the second administration of an anti-TRBV9 antibody is performed at the main dose of 0.03-10 mg/kg after at least 3 months, and the subsequent administrations of an anti-TRBV9 antibody are performed at the main dose of 0.03-10 mg/kg at least every 6 months.

In some embodiments of the invention, the use comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 3 months, and the subsequent administrations of an anti-TRBV9 antibody at the main dose of 0.03-10 mg/kg every 6 months; or every 9 months; or every 12 months.

In some embodiments of the invention, the use comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 6 months, and the subsequent administrations of an anti-TRBV9 antibody at the main dose of 0.03-10 mg/kg every 6 months; or every 9 months; or every 12 months.

In some embodiments of the invention, the use comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 9 months, and the subsequent administrations of an anti-TRBV9 antibody at the main dose of 0.03-10 mg/kg every 9 months; or every 12 months.

In some embodiments of the invention, the use comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 12 months, and the subsequent administrations of an anti-TRBV9 antibody at the main dose of 0.03-10 mg/kg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration of an anti-TRBV9 antibody to a subject in need thereof is performed at a dose equal to half of the main dose, 0.015-3.5 mg/kg, the second administration of an anti-TRBV9 antibody is performed at the main dose of 0.03-7 mg/kg after at least 3 months, and the subsequent administrations of an anti-TRBV9 antibody are performed at the main dose of 0.03-7 mg/kg at least every 6 months.

In some embodiments of the invention, the use comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 3 months, and the subsequent administrations of an anti-TRBV9 antibody at the main dose of 0.03-7 mg/kg every 6 months; or every 9 months; or every 12 months.

In some embodiments of the invention, the use comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 6 months, and the subsequent administrations of an anti-TRBV9 antibody at the main dose of 0.03-7 mg/kg every 6 months; or every 9 months; or every 12 months.

In some embodiments of the invention, the use comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 9 months, and the subsequent administrations of an anti-TRBV9 antibody at the main dose of 0.03-7 mg/kg every 9 months; or every 12 months.

In some embodiments of the invention, the use comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 12 months, and the subsequent administrations of an anti-TRBV9 antibody at the main dose of 0.03-7 mg/kg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration of an anti-TRBV9 antibody to a subject in need thereof is performed at a dose equal to half of the main dose, 0.15-5 mg/kg, the second administration of an anti-TRBV9 antibody is performed at the main dose of 0.3-10 mg/kg after at least 3 months, and the subsequent administrations of an anti-TRBV9 antibody are performed at the main dose of 0.3-10 mg/kg at least every 6 months.

In some embodiments of the invention, the use comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 3 months, and the subsequent administrations of an anti-TRBV9 antibody at the main dose of 0.3-10 mg/kg every 6 months; or every 9 months; or every 12 months.

In some embodiments of the invention, the use comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 6 months, and the subsequent administrations of an anti-TRBV9 antibody at the main dose of 0.3-10 mg/kg every 6 months; or every 9 months; or every 12 months.

In some embodiments of the invention, the use comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 9 months, and the subsequent administrations of an anti-TRBV9 antibody at the main dose of 0.3-10 mg/kg every 9 months; or every 12 months.

In some embodiments of the invention, the use comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 12 months, and the subsequent administrations of an anti-TRBV9 antibody at the main dose of 0.3-10 mg/kg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration of an anti-TRBV9 antibody to a subject in need thereof is performed at a dose equal to half of the main dose, 0.15-3.5 mg/kg, the second administration of an anti-TRBV9 antibody is performed at the main dose of 0.3-7 mg/kg after at least 3 months, and the subsequent administrations of an anti-TRBV9 antibody are performed at the main dose of 0.3-7 mg/kg at least every 6 months.

In some embodiments of the invention, the use comprises the first administration to a subject in need thereof of an anti-TRBV9 antibody performed at a dose equal to half of the main dose, 0.15-3.5 mg/kg, the second administration of an anti-TRBV9 antibody is performed at the main dose of 0.3-7 mg/kg after 3 months, and the subsequent administrations of an anti-TRBV9 antibody are performed at the main dose of 0.3-7 mg/kg every 6 months; or every 9 months; or every 12 months.

In some embodiments of the invention, the use comprises the first administration to a subject in need thereof of an anti-TRBV9 antibody performed at a dose equal to half of the main dose, 0.15-3.5 mg/kg, the second administration of an anti-TRBV9 antibody is performed at the main dose of 0.3-7 mg/kg after 6 months, and the subsequent administrations of an anti-TRBV9 antibody are performed at the main dose of 0.3-7 mg/kg every 6 months; or every 9 months; or every 12 months.

In some embodiments of the invention, the use comprises the first administration to a subject in need thereof of an anti-TRBV9 antibody performed at a dose equal to half of the main dose, 0.15-3.5 mg/kg, the second administration of an anti-TRBV9 antibody is performed at the main dose of 0.3-7 mg/kg after 9 months, and the subsequent administrations of an anti-TRBV9 antibody are performed at the main dose of 0.3-7 mg/kg every 9 months; or every 12 months.

In some embodiments of the invention, the use comprises the first administration to a subject in need thereof of an anti-TRBV9 antibody performed at a dose equal to half of the main dose, 0.15-3.5 mg/kg, the second administration of an anti-TRBV9 antibody is performed at the main dose of 0.3-7 mg/kg after 12 months, and the subsequent administrations of an anti-TRBV9 antibody are performed at the main dose of 0.3-7 mg/kg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration of an anti-TRBV9 antibody to a subject in need thereof is performed at a dose equal to half of the main dose, 0.5-3.5 mg/kg, the second administration of an anti-TRBV9 antibody is performed at the main dose of 1-7 mg/kg after at least 3 months, and the subsequent administrations of an anti-TRBV9 antibody are performed at the main dose of 1-7 mg/kg at least every 6 months.

In some embodiments, the invention relates to use of an anti-TRBV9 antibody for the treatment of a disease or disorder mediated by T-lymphocytes bearing the TRBV9 segment within the T-cell receptor, wherein the first administration to a subject in need thereof of an anti-TRBV9 antibody is performed at a dose equal to half of the main dose, 0.5-3.5 mg/kg, the second administration of an anti-TRBV9 antibody is performed at the main dose of 1-7 mg/kg after 3 months, and the subsequent administrations of an anti-TRBV9 antibody are performed at the main dose of 1-7 mg/kg every 6 months; or every 9 months; or every 12 months.

In some embodiments of the invention, the use comprises the first administration to a subject in need thereof of an anti-TRBV9 antibody at a dose equal to half of the main dose, 0.15-3.5 mg/kg, the second administration of an anti-TRBV9 antibody at the main dose of 1-7 mg/kg after 6 months, and the subsequent administrations of an anti-TRBV9 antibody at the main dose of 1-7 mg/kg every 6 months; or every 9 months; or every 12 months.

In some embodiments of the invention, the use comprises the first administration to a subject in need thereof of an anti-TRBV9 antibody at a dose equal to half of the main dose, 0.15-3.5 mg/kg, the second administration of an anti-TRBV9 antibody at the main dose of 1-7 mg/kg after 9 months, and the subsequent administrations of an anti-TRBV9 antibody at the main dose of 1-7 mg/kg every 9 months; or every 12 months.

In some embodiments of the invention, the use comprises the first administration to a subject in need thereof of an anti-TRBV9 antibody at a dose equal to half of the main dose, 0.15-3.5 mg/kg, the second administration of an anti-TRBV9 antibody at the main dose of 1-7 mg/kg after 12 months, and the subsequent administrations of an anti-TRBV9 antibody at the main dose of 1-7 mg/kg every 12 months.

In some embodiments of the invention, the first administration of an anti-TRBV9 antibody is performed at a dose of 0.5 mg/kg, the second administration of an anti-TRBV9 antibody is performed at a dose of 1 mg/kg after at least 3 months, and the subsequent administrations of an anti-TRBV9 antibody are performed at a dose of 1 mg/kg at least every 6 months.

In some embodiments of the invention, the first administration of an anti-TRBV9 antibody is performed at a dose of 0.5 mg/kg, the second administration of an anti-TRBV9 antibody is performed at a dose of 1 mg/kg after 3 months, and the subsequent administrations of an anti-TRBV9 antibody are performed at a dose of 1 mg/kg every 6 months; or every 9 months; or every 12 months.

In some embodiments of the invention, the first administration of an anti-TRBV9 antibody at a dose of 0.5 mg/kg, the second administration of an anti-TRBV9 antibody at a dose of 1 mg/kg after 6 months, and the subsequent administrations of an anti-TRBV9 antibody at a dose of 1 mg/kg every 6 months; or every 9 months; or every 12 months.

In some embodiments of the invention, the first administration of an anti-TRBV9 antibody at a dose of 0.5 mg/kg, the second administration of an anti-TRBV9 antibody at a dose of 1 mg/kg after 9 months, and the subsequent administrations of an anti-TRBV9 antibody at a dose of 1 mg/kg every 9 months; or every 12 months.

In some embodiments of the invention, the first administration of an anti-TRBV9 antibody at a dose of 0.5 mg/kg, the second administration of an anti-TRBV9 antibody at a dose of 1 mg/kg after 12 months, and the subsequent administrations of an anti-TRBV9 antibody at a dose of 1 mg/kg every 12 months.

In some embodiments of the invention, the first administration of an anti-TRBV9 antibody is performed at a dose of 1 mg/kg, the second administration of an anti-TRBV9 antibody is performed at a dose of 2 mg/kg after at least 3 months, and the subsequent administrations of an anti-TRBV9 antibody are performed at a dose of 2 mg/kg at least every 6 months.

In some embodiments of the invention, the first administration of an anti-TRBV9 antibody is performed at a dose of 1 mg/kg, the second administration of an anti-TRBV9 antibody is performed at a dose of 2 mg/kg after 3 months, and the subsequent administrations of an anti-TRBV9 antibody are performed at a dose of 2 mg/kg every 6 months; or every 9 months; or every 12 months.

In some embodiments of the invention, the first administration of an anti-TRBV9 antibody at a dose of 1 mg/kg, the second administration of an anti-TRBV9 antibody at a dose of 2 mg/kg after 6 months, and the subsequent administrations of an anti-TRBV9 antibody at a dose of 2 mg/kg every 6 months; or every 9 months; or every 12 months.

In some embodiments of the invention, the first administration of an anti-TRBV9 antibody at a dose of 1 mg/kg, the second administration of an anti-TRBV9 antibody at a dose of 2 mg/kg after 9 months, and the subsequent administrations of an anti-TRBV9 antibody at a dose of 2 mg/kg every 9 months; or every 12 months.

In some embodiments of the invention, the first administration of an anti-TRBV9 antibody at a dose of 1 mg/kg, the second administration of an anti-TRBV9 antibody at a dose of 2 mg/kg after 12 months, and the subsequent administrations of an anti-TRBV9 antibody at a dose of 2 mg/kg every 12 months.

In some embodiments of the invention, the first administration of an anti-TRBV9 antibody is performed at a dose of 1.5 mg/kg, the second administration of an anti-TRBV9 antibody is performed at a dose of 3 mg/kg after at least 3 months, and the subsequent administrations of an anti-TRBV9 antibody are performed at a dose of 3 mg/kg at least every 6 months.

In some embodiments of the invention, the first administration of an anti-TRBV9 antibody is performed at a dose of 1.5 mg/kg, the second administration of an anti-TRBV9 antibody is performed at a dose of 3 mg/kg after 3 months, and the subsequent administrations of an anti-TRBV9 antibody are performed at a dose of 3 mg/kg every 6 months; or every 9 months; or every 12 months.

In some embodiments of the invention, the first administration of an anti-TRBV9 antibody at a dose of 1.5 mg/kg, the second administration of an anti-TRBV9 antibody at a dose of 3 mg/kg after 6 months, and the subsequent administrations of an anti-TRBV9 antibody at a dose of 3 mg/kg every 6 months; or every 9 months; or every 12 months.

In some embodiments of the invention, the first administration of an anti-TRBV9 antibody at a dose of 1.5 mg/kg, the second administration of an anti-TRBV9 antibody at a dose of 3 mg/kg after 9 months, and the subsequent administrations of an anti-TRBV9 antibody at a dose of 3 mg/kg every 9 months; or every 12 months.

In some embodiments of the invention, the first administration of an anti-TRBV9 antibody at a dose of 1.5 mg/kg, the second administration of an anti-TRBV9 antibody at a dose of 3 mg/kg after 12 months, and the subsequent administrations of an anti-TRBV9 antibody at a dose of 3 mg/kg every 12 months.

In some embodiments of the invention, the first administration of an anti-TRBV9 antibody is performed at a dose of 2 mg/kg, the second administration of an anti-TRBV9 antibody is performed at a dose of 4 mg/kg after at least 3 months, and the subsequent administrations of an anti-TRBV9 antibody are performed at a dose of 4 mg/kg at least every 6 months.

In some embodiments of the invention, the first administration of an anti-TRBV9 antibody is performed at a dose of 2 mg/kg, the second administration of an anti-TRBV9 antibody is performed at a dose of 4 mg/kg after 3 months, and the subsequent administrations of an anti-TRBV9 antibody at are performed at a dose of 4 mg/kg every 6 months; or every 9 months; or every 12 months.

In some embodiments of the invention, the first administration of an anti-TRBV9 antibody at a dose of 2 mg/kg, the second administration of an anti-TRBV9 antibody at a dose of 4 mg/kg after 6 months, and the subsequent administrations of an anti-TRBV9 antibody at a dose of 4 mg/kg every 6 months; or every 9 months; or every 12 months.

In some embodiments of the invention, the first administration of an anti-TRBV9 antibody at a dose of 2 mg/kg, the second administration of an anti-TRBV9 antibody at a dose of 4 mg/kg after 9 months, and the subsequent administrations of an anti-TRBV9 antibody at a dose of 4 mg/kg every 9 months; or every 12 months.

In some embodiments of the invention, the first administration of an anti-TRBV9 antibody at a dose of 2 mg/kg, the second administration of an anti-TRBV9 antibody at a dose of 4 mg/kg after 12 months, and the subsequent administrations of an anti-TRBV9 antibody at a dose of 4 mg/kg every 12 months.

In some embodiments of the invention, the first administration of an anti-TRBV9 antibody is performed at a dose of 2.5 mg/kg, the second administration of an anti-TRBV9 antibody is performed at a dose of 5 mg/kg after at least 3 months, and the subsequent administrations of an anti-TRBV9 antibody are performed at a dose of 5 mg/kg at least every 6 months.

In some embodiments of the invention, the first administration of an anti-TRBV9 antibody is performed at a dose of 2.5 mg/kg, the second administration of an anti-TRBV9 antibody is performed at a dose of 5 mg/kg after 3 months, and the subsequent administrations of an anti-TRBV9 antibody are performed at a dose of 5 mg/kg every 6 months; or every 9 months; or every 12 months.

In some embodiments of the invention, the first administration of an anti-TRBV9 antibody at a dose of 2,5 mg/kg, the second administration of an anti-TRBV9 antibody at a dose of 5 mg/kg after 6 months, and the subsequent administrations of an anti-TRBV9 antibody at a dose of 5 mg/kg every 6 months; or every 9 months; or every 12 months.

In some embodiments of the invention, the first administration of an anti-TRBV9 antibody at a dose of 2,5 mg/kg, the second administration of an anti-TRBV9 antibody at a dose of 5 mg/kg after 9 months, and the subsequent administrations of an anti-TRBV9 antibody at a dose of 5 mg/kg every 9 months; or every 12 months.

In some embodiments of the invention, the first administration of an anti-TRBV9 antibody at a dose of 2,5 mg/kg, the second administration of an anti-TRBV9 antibody at a dose of 5 mg/kg after 12 months, and the subsequent administrations of an anti-TRBV9 antibody at a dose of 5 mg/kg every 12 months.

In some embodiments of the invention, the first administration of an anti-TRBV9 antibody is performed at a dose of 3 mg/kg, the second administration of an anti-TRBV9 antibody is performed at a dose of 6 mg/kg after at least 3 months, and the subsequent administrations of an anti-TRBV9 antibody are performed at a dose of 6 mg/kg at least every 6 months.

In some embodiments of the invention, the first administration of an anti-TRBV9 antibody is performed at a dose of 3 mg/kg, the second administration of an anti-TRBV9 antibody is performed at a dose of 6 mg/kg after 3 months, and the subsequent administrations of an anti-TRBV9 antibody are performed at a dose of 6 mg/kg every 6 months; or every 9 months; or every 12 months.

In some embodiments of the invention, the first administration of an anti-TRBV9 antibody at a dose of 3 mg/kg, the second administration of an anti-TRBV9 antibody at a dose of 6 mg/kg after 6 months, and the subsequent administrations of an anti-TRBV9 antibody at a dose of 6 mg/kg every 6 months; or every 9 months; or every 12 months.

In some embodiments of the invention, the first administration of an anti-TRBV9 antibody at a dose of 3 mg/kg, the second administration of an anti-TRBV9 antibody at a dose of 6 mg/kg after 9 months, and the subsequent administrations of an anti-TRBV9 antibody at a dose of 6 mg/kg every 9 months; or every 12 months.

In some embodiments of the invention, the first administration of an anti-TRBV9 antibody at a dose of 3 mg/kg, the second administration of an anti-TRBV9 antibody at a dose of 6 mg/kg after 12 months, and the subsequent administrations of an anti-TRBV9 antibody at a dose of 6 mg/kg every 12 months.

In some embodiments of the invention, the first administration of an anti-TRBV9 antibody is performed at a dose of 3.5 mg/kg, the second administration of an anti-TRBV9 antibody is performed at a dose of 7 mg/kg after at least 3 months, and the subsequent administrations of an anti-TRBV9 antibody are performed at a dose of 7 mg/kg at least every 6 months.

In some embodiments of the invention, the first administration of an anti-TRBV9 antibody is performed at a dose of 3.5 mg/kg, the second administration of an anti-TRBV9 antibody is performed at a dose of 7 mg/kg after 3 months, and the subsequent administrations of an anti-TRBV9 antibody are performed at a dose of 7 mg/kg every 6 months; or every 9 months; or every 12 months.

In some embodiments of the invention, the first administration of an anti-TRBV9 antibody at a dose of 3.5 mg/kg, the second administration of an anti-TRBV9 antibody at a dose of 7 mg/kg after 6 months, and the subsequent administrations of an anti-TRBV9 antibody at a dose of 7 mg/kg every 6 months; or every 9 months; or every 12 months.

In some embodiments of the invention, the first administration of an anti-TRBV9 antibody at a dose of 3.5 mg/kg, the second administration of an anti-TRBV9 antibody at a dose of 7 mg/kg after 9 months, and the subsequent administrations of an anti-TRBV9 antibody at a dose of 7 mg/kg every 9 months; or every 12 months.

In some embodiments of the invention, the first administration of an anti-TRBV9 antibody at a dose of 3.5 mg/kg, the second administration of an anti-TRBV9 antibody at a dose of 7 mg/kg after 12 months, and the subsequent administrations of an anti-TRBV9 antibody at a dose of 7 mg/kg every 12 months.

In some embodiments of the invention, the first administration of an anti-TRBV9 antibody is performed at a dose of 4 mg/kg, the second administration of an anti-TRBV9 antibody is performed at a dose of 8 mg/kg after 3 months, and the subsequent administrations of an anti-TRBV9 antibody are performed at a dose of 8 mg/kg every 6 months; or every 9 months; or every 12 months.

In some embodiments of the invention, the first administration of an anti-TRBV9 antibody at a dose of 4 mg/kg, the second administration of an anti-TRBV9 antibody at a dose of 8 mg/kg after 6 months, and the subsequent administrations of an anti-TRBV9 antibody at a dose of 8 mg/kg every 6 months; or every 9 months; or every 12 months.

In some embodiments of the invention, the first administration of an anti-TRBV9 antibody at a dose of 4 mg/kg, the second administration of an anti-TRBV9 antibody at a dose of 8 mg/kg after 9 months, and the subsequent administrations of an anti-TRBV9 antibody at a dose of 8 mg/kg every 9 months; or every 12 months.

In some embodiments of the invention, the first administration of an anti-TRBV9 antibody at a dose of 4 mg/kg, the second administration of an anti-TRBV9 antibody at a dose of 8 mg/kg after 12 months, and the subsequent administrations of an anti-TRBV9 antibody at a dose of 8 mg/kg every 12 months.

In some embodiments of the invention, the first administration of an anti-TRBV9 antibody is performed at a dose of 4.5 mg/kg, the second administration of an anti-TRBV9 antibody is performed at a dose of 9 mg/kg after 3 months, and the subsequent administrations of an anti-TRBV9 antibody are performed at a dose of 9 mg/kg every 6 months; or every 9 months; or every 12 months.

In some embodiments of the invention, the first administration of an anti-TRBV9 antibody at a dose of 4.5 mg/kg, the second administration of an anti-TRBV9 antibody at a dose of 9 mg/kg after 6 months, and the subsequent administrations of an anti-TRBV9 antibody at a dose of 9 mg/kg every 6 months; or every 9 months; or every 12 months.

In some embodiments of the invention, the first administration of an anti-TRBV9 antibody at a dose of 4.5 mg/kg, the second administration of an anti-TRBV9 antibody at a dose of 9 mg/kg after 9 months, and the subsequent administrations of an anti-TRBV9 antibody at a dose of 9 mg/kg every 9 months; or every 12 months.

In some embodiments of the invention, the first administration of an anti-TRBV9 antibody at a dose of 4.5 mg/kg, the second administration of an anti-TRBV9 antibody at a dose of 9 mg/kg after 12 months, and the subsequent administrations of an anti-TRBV9 antibody at a dose of 9 mg/kg every 12 months.

In some embodiments of the invention, the first administration of an anti-TRBV9 antibody is performed at a dose of 5 mg/kg, the second administration of an anti-TRBV9 antibody is performed at a dose of 10 mg/kg after 3 months, and the subsequent administrations of an anti-TRBV9 antibody are performed at a dose of 10 mg/kg every 6 months; or every 9 months; or every 12 months.

In some embodiments of the invention, the first administration of an anti-TRBV9 antibody at a dose of 5 mg/kg, the second administration of an anti-TRBV9 antibody at a dose of 10 mg/kg after 6 months, and the subsequent administrations of an anti-TRBV9 antibody at a dose of 10 mg/kg every 6 months; or every 9 months; or every 12 months.

In some embodiments of the invention, the first administration of an anti-TRBV9 antibody at a dose of 5 mg/kg, the second administration of an anti-TRBV9 antibody at a dose of 10 mg/kg after 9 months, and the subsequent administrations of an anti-TRBV9 antibody at a dose of 10 mg/kg every 9 months; or every 12 months.
(viii)the first administration of an anti-TRBV9 antibody at a dose of 5 mg/kg, the second In some embodiments of the invention, the first administration of an anti-TRBV9 antibody at a dose of 5 mg/kg, the second administration of an anti-TRBV9 antibody at a dose of 10 mg/kg after 12 months, and the subsequent administrations of an anti-TRBV9 antibody at a dose of 10 mg/kg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration to a subject in need thereof of an anti-TRBV9 antibody is performed at a dose of 75-300 mg, the second administration of an anti-TRBV9 antibody at the main dose of 325-675 mg after at least 3 months, and the subsequent administrations of an anti-TRBV9 antibody at the main dose of 325-675 mg at least every 6 months.

In some embodiments of the invention, the use comprises the first administration to a subject in need thereof of an anti-TRBV9 antibody at a dose of 75-300 mg, the second administration of an anti-TRBV9 antibody at the main dose of 325-675 mg after 3 months, and the subsequent administrations of an anti-TRBV9 antibody at the main dose of 325-675 mg every 6 months; or every 9 months; or every 12 months.

In some embodiments of the invention, the use comprises the first administration to a subject in need thereof of an anti-TRBV9 antibody at a dose of 75-300 mg, the second administration of an anti-TRBV9 antibody at the main dose of 325-675 mg after 6 months, and the subsequent administrations of an anti-TRBV9 antibody at the main dose of 325-675 mg every 6 months; or every 9 months; or every 12 months.

In some embodiments of the invention, the use comprises the first administration to a subject in need thereof of an anti-TRBV9 antibody at a dose of 75-300 mg, the second administration of an anti-TRBV9 antibody at the main dose of 325-675 mg after 9 months, and the subsequent administrations of an anti-TRBV9 antibody at the main dose of 325-675 mg every 9 months; or every 12 months.

In some embodiments of the invention, the use comprises the first administration to a subject in need thereof of an anti-TRBV9 antibody at a dose of 75-300 **mg,** the second administration of an anti-TRBV9 antibody at the main dose of 325-675 mg after 12 months, and the subsequent administrations of an anti-TRBV9 antibody at the main dose of 325-675 mg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration to a subject in need thereof of an anti-TRBV9 antibody is performed at a dose of 100-275 mg, the second administration of an anti-TRBV9 antibody at the main dose of 350-650 mg after at least 3 months, and the subsequent administrations of an anti-TRBV9 antibody at the main dose of 350-650 mg at least every 6 months.

In some embodiments of the invention, the use comprises the first administration to a subject in need thereof of an anti-TRBV9 antibody at a dose of 100-275 mg, the second administration of an anti-TRBV9 antibody at the main dose of 350-650 mg after 3 months, and the subsequent administrations of an anti-TRBV9 antibody at the main dose of 350-650 mg every 6 months; or every 9 months; or every 12 months.

In some embodiments of the invention, the use comprises the first administration to a subject in need thereof of an anti-TRBV9 antibody at a dose of 100-275 mg, the second administration of an anti-TRBV9 antibody at the main dose of 350-650 mg after 6 months, and the subsequent administrations of an anti-TRBV9 antibody at the main dose of 350-650 mg every 6 months; or every 9 months; or every 12 months.

In some embodiments of the invention, the use comprises the first administration to a subject in need thereof of an anti-TRBV9 antibody at a dose of 100-275 mg, the second administration of an anti-TRBV9 antibody at the main dose of 350-650 mg after 9 months, and the subsequent administrations of an anti-TRBV9 antibody at the main dose of 350-650 mg every 9 months; or every 12 months.

In some embodiments of the invention, the use comprises the first administration to a subject in need thereof of an anti-TRBV9 antibody at a dose of 100-275 mg, the second administration of an anti-TRBV9 antibody at the main dose of 350-650 mg after 12 months, and the subsequent administrations of an anti-TRBV9 antibody at the main dose of 350-650 mg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration to a subject in need thereof of an anti-TRBV9 antibody is performed at a dose of 100-150 mg; or 225-275 mg, the second administration of an anti-TRBV9 antibody at the main dose of 350-400 mg; or 475- 525 mg; or 600-650 mg after at least 3 months, and the subsequent administrations of an anti-TRBV9 antibody at the main dose of 350-400 mg; or 475- 525 mg; or 600-650 mg at least every 6 months.

In some embodiments of the invention, the use comprises (i) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 3 months, and the subsequent administrations of an anti-TRBV9 antibody at the main dose of 350-400 mg; or 475- 525 mg; or 600-650 mg every 6 months; or
(ii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 3 months, and the subsequent administrations of an anti-TRBV9 antibody at the main dose of 350-400 mg; or 475- 525 mg; or 600-650 mg every 9 months; or
(iii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 3 months, and the subsequent administrations of an anti-TRBV9 antibody at the main dose of 350-400 mg; or 475- 525 mg; or 600-650 mg every 12 months; or
(iv) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 6 months, and the subsequent administrations of an anti-TRBV9 antibody at the main dose of 350-400 mg; or 475- 525 mg; or 600-650 mg every 6 months; or
(v) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 6 months, and the subsequent administrations of an anti-TRBV9 antibody at the main dose of 350-400 mg; or 475- 525 mg; or 600-650 mg every 9 months; or
(vi) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 6 months, and the subsequent administrations of an anti-TRBV9 antibody at the main dose of 350-400 mg; or 475- 525 mg; or 600-650 mg every 12 months; or
(vii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 9 months, and the subsequent administrations of an anti-TRBV9 antibody at the main dose of 350-400 mg; or 475- 525 mg; or 600-650 mg every 9 months; or
(viii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 9 months, and the subsequent administrations of an anti-TRBV9 antibody at the main dose of 350-400 mg; or 475- 525 mg; or 600-650 mg every 12 months; or
(ix) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 12 months, and the subsequent administrations of an anti-TRBV9 antibody at the main dose of 350-400 mg; or 475- 525 mg; or 600-650 mg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration to a subject in need thereof of an anti-TRBV9 antibody is performed at a dose of 115-135 mg; or 240-260 mg, the second administration of an anti-TRBV9 antibody at the main dose of 365-385 mg; or 490- 510 mg; or 615-635 mg after at least 3 months, and the subsequent administrations of an anti-TRBV9 antibody at the main dose of 365-385 mg; or 490- 510 mg; or 615-635 mg at least every 6 months.

In some embodiments of the invention, the use comprises (i) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 3 months, and the subsequent administrations of an anti-TRBV9 antibody at the main dose of 365-385 mg; or 490- 510 mg; or 615-635 mg every 6 months; or
(ii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 3 months, and the subsequent administrations of an anti-TRBV9 antibody at the main dose of 365-385 mg; or 490- 510 mg; or 615-635 mg every 9 months; or
(iii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 3 months, and the subsequent administrations of an anti-TRBV9 antibody at the main dose of 365-385 mg; or 490- 510 mg; or 615-635 mg every 12 months; or
(iv) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 6 months, and the subsequent administrations of an anti-TRBV9 antibody at the main dose of 365-385 mg; or 490- 510 mg; or 615-635 mg every 6 months; or
(v) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 6 months, and the subsequent administrations of an anti-TRBV9 antibody at the main dose of 365-385 mg; or 490- 510 mg; or 615-635 mg every 9 months; or
(vi) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 6 months, and the subsequent administrations of an anti-TRBV9 antibody at the main dose of 365-385 mg; or 490- 510 mg; or 615-635 mg every 12 months; or
(vii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 9 months, and the subsequent administrations of an anti-TRBV9 antibody at the main dose of 365-385 mg; or 490- 510 mg; or 615-635 mg every 9 months; or
(viii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 9 months, and the subsequent administrations of an anti-TRBV9 antibody at the main dose of 365-385 mg; or 490- 510 mg; or 615-635 mg every 12 months; or
(ix) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 12 months, and the subsequent administrations of an anti-TRBV9 antibody at the main dose of 365-385 mg; or 490- 510 mg; or 615-635 mg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration to a subject in need thereof of an anti-TRBV9 antibody is performed at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after at least 3 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg at least every 6 months.

In some embodiments, the invention relates to use of an anti-TRBV9 antibody for the treatment of a disease or disorder mediated by T-lymphocytes bearing the TRBV9 segment within the T-cell receptor, wherein (i) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 3 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 6 months; or
(ii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 3 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 9 months; or
(iii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 3 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months; or
(iv) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 6 months; or
(v) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 9 months; or
(vi) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months; or
(vii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 9 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 9 months; or
(viii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 9 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months; or
(ix) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 12 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months.

In some embodiments of the invention, the use comprises the first administration of an anti-TRBV9 antibody at a dose of 120 mg, the second administration of an anti-TRBV9 antibody at a dose of 370 mg after at least 3 months, and the subsequent administrations of an anti-TRBV9 antibody at a dose of 370 mg at least every 6 months.

In some embodiments of the invention, the use comprises the first administration of an anti-TRBV9 antibody at a dose of 120 mg, the second administration of an anti-TRBV9 antibody at a dose of 370 mg after at least 3 months, and the subsequent administrations of an anti-TRBV9 antibody at a dose of 370 mg at least every 6 months.

In some embodiments of the invention, the use comprises the first administration of an anti-TRBV9 antibody at a dose of 120 mg, the second administration of an anti-TRBV9 antibody at a dose of 370 mg after 3 months; and the subsequent administrations of an anti-TRBV9 antibody at a dose of 370 mg every 6 months; or every 9 months; or every 12 months.

In some embodiments of the invention, the use comprises the first administration of an anti-TRBV9 antibody at a dose of 120 mg, the second administration of an anti-TRBV9 antibody at a dose of 370 mg after 6 months; and the subsequent administrations of an anti-TRBV9 antibody at a dose of 370 mg every 6 months; or every 9 months; or every 12 months.

In some embodiments of the invention, the use comprises the first administration of an anti-TRBV9 antibody at a dose of 120 mg, the second administration of an anti-TRBV9 antibody at a dose of 370 mg after 9 months; and the subsequent administrations of an anti-TRBV9 antibody at a dose of 370 mg every 9 months; or every 12 months.

In some embodiments of the invention, the use comprises the first administration of an anti-TRBV9 antibody at a dose of 120 mg, the second administration of an anti-TRBV9 antibody at a dose of 370 mg after 12 months, and the subsequent administrations of an anti-TRBV9 antibody at 370 mg every 12 months.

In some embodiments of the invention, the use comprises the first administration of an anti-TRBV9 antibody at a dose of 125 mg, the second administration of an anti-TRBV9 antibody at a dose of 375 mg after at least 3 months, and the subsequent administrations of an anti-TRBV9 antibody at a dose of 375 mg at least every 6 months.

In some embodiments of the invention, the use comprises the first administration of an anti-TRBV9 antibody at a dose of 125 mg, the second administration of an anti-TRBV9 antibody at a dose of 375 mg after 3 months; and the subsequent administrations of an anti-TRBV9 antibody at a dose of 375 mg every 6 months; or every 9 months; or every 12 months.

In some embodiments of the invention, the use comprises the first administration of an anti-TRBV9 antibody at a dose of 125 mg, the second administration of an anti-TRBV9 antibody at a dose of 375 mg after 6 months; and the subsequent administrations of an anti-TRBV9 antibody at a dose of 375 mg every 6 months; or every 9 months; or every 12 months.

In some embodiments of the invention, the use comprises the first administration of an anti-TRBV9 antibody at a dose of 125 mg, the second administration of an anti-TRBV9 antibody at a dose of 375 mg after 9 months; and the subsequent administrations of an anti-TRBV9 antibody at a dose of 375 mg every 9 months; or every 12 months.

In some embodiments of the invention, the use comprises the first administration of an anti-TRBV9 antibody at a dose of 125 mg, the second administration of an anti-TRBV9 antibody at a dose of 375 mg after 12 months, and the subsequent administrations of an anti-TRBV9 antibody at 375 mg every 12 months.

In some embodiments of the invention, the use comprises the first administration of an anti-TRBV9 antibody at a dose of 130 mg, the second administration of an anti-TRBV9 antibody at a dose of 380 mg after at least 3 months, and the subsequent administrations of an anti-TRBV9 antibody at a dose of 380 mg at least every 6 months.

In some embodiments of the invention, the use comprises the first administration of an anti-TRBV9 antibody at a dose of 130 mg, the second administration of an anti-TRBV9 antibody at a dose of 380 mg after 3 months; and the subsequent administrations of an anti-TRBV9 antibody at a dose of 380 mg every 6 months; or every 9 months; or every 12 months.

In some embodiments of the invention, the use comprises the first administration of an anti-TRBV9 antibody at a dose of 130 mg, the second administration of an anti-TRBV9 antibody at a dose of 380 mg after 6 months; and the subsequent administrations of an anti-TRBV9 antibody at a dose of 380 mg every 6 months; or every 9 months; or every 12 months.

In some embodiments of the invention, the use comprises the first administration of an anti-TRBV9 antibody at a dose of 130 mg, the second administration of an anti-TRBV9 antibody at a dose of 380 mg after 9 months; and the subsequent administrations of an anti-TRBV9 antibody at a dose of 380 mg every 9 months; or every 12 months.

In some embodiments of the invention, the use comprises the first administration of an anti-TRBV9 antibody at a dose of 130 mg, the second administration of an anti-TRBV9 antibody at a dose of 380 mg after 12 months, and the subsequent administrations of an anti-TRBV9 antibody at 380 mg every 12 months.

In some embodiments of the invention, the use comprises the first administration of an anti-TRBV9 antibody at a dose of 120 mg, the second administration of an anti-TRBV9 antibody at a dose of 370 mg after at least 3 months, and the subsequent administrations of an anti-TRBV9 antibody at a dose of 370 mg at least every 6 months based on subject's body weight from 30 to 50 kg.

In some embodiments of the invention, the use comprises the first administration of an anti-TRBV9 antibody at a dose of 120 mg, the second administration of an anti-TRBV9 antibody at a dose of 370 mg after 3 months; and the subsequent administrations of an anti-TRBV9 antibody at a dose of 370 mg every 6 months; or every 9 months; or every 12 months based on subject's body weight from 30 to 50 kg.

In some embodiments of the invention, the use comprises the first administration of an anti-TRBV9 antibody at a dose of 120 mg, the second administration of an anti-TRBV9 antibody at a dose of 370 mg after 6 months; and the subsequent administrations of an anti-TRBV9 antibody at a dose of 370 mg every 6 months; or every 9 months; or every 12 months based on subject's body weight from 30 to 50 kg.

In some embodiments of the invention, the use comprises the first administration of an anti-TRBV9 antibody at a dose of 120 mg, the second administration of an anti-TRBV9 antibody at a dose of 370 mg after 9 months; and the subsequent administrations of an anti-TRBV9 antibody at a dose of 370 mg every 9 months; or every 12 months based on subject's body weight from 30 to 50 kg.

In some embodiments of the invention, the use comprises the first administration of an anti-TRBV9 antibody at a dose of 120 mg, the second administration of an anti-TRBV9 antibody at a dose of 370 mg after 12 months, and the subsequent administrations of an anti-TRBV9 antibody at 370 mg every 12 months based on subject's body weight from 30 to 50 kg.

In some embodiments of the invention, the use comprises the first administration of an anti-TRBV9 antibody at a dose of 125 mg, the second administration of an anti-TRBV9 antibody at a dose of 375 mg after at least 3 months, and the subsequent administrations of an anti-TRBV9 antibody at a dose of 375 mg at least every 6 months based on subject's body weight from 30 to 50 kg.

In some embodiments of the invention, the use comprises the first administration of an anti-TRBV9 antibody at a dose of 125 mg, the second administration of an anti-TRBV9 antibody at a dose of 375 mg after 3 months; and the subsequent administrations of an anti-TRBV9 antibody at a dose of 375 mg every 6 months; or every 9 months; or every 12 months based on subject's body weight from 30 to 50 kg.

In some embodiments of the invention, the use comprises the first administration of an anti-TRBV9 antibody at a dose of 125 mg, the second administration of an anti-TRBV9 antibody at a dose of 375 mg after 6 months; and the subsequent administrations of an anti-TRBV9 antibody at a dose of 375 mg every 6 months; or every 9 months; or every 12 months based on subject's body weight from 30 to 50 kg.

In some embodiments of the invention, the use comprises the first administration of an anti-TRBV9 antibody at a dose of 125 mg, the second administration of an anti-TRBV9 antibody at a dose of 375 mg after 9 months; and the subsequent administrations of an anti-TRBV9 antibody at a dose of 375 mg every 9 months; or every 12 months based on subject's body weight from 30 to 50 kg.

In some embodiments of the invention, the use comprises the first administration of an anti-TRBV9 antibody at a dose of 125 mg, the second administration of an anti-TRBV9 antibody at a dose of 375 mg after 12 months, and the subsequent administrations of an anti-TRBV9 antibody at 375 mg every 12 months based on subject's body weight from 30 to 50 kg.

In some embodiments of the invention, the use comprises the first administration of an anti-TRBV9 antibody at a dose of 130 mg, the second administration of an anti-TRBV9 antibody at a dose of 380 mg after at least 3 months, and the subsequent administrations of an anti-TRBV9 antibody at a dose of 380 mg at least every 6 months based on subject's body weight from 30 to 50 kg.

In some embodiments of the invention, the use comprises the first administration of an anti-TRBV9 antibody at a dose of 130 mg, the second administration of an anti-TRBV9 antibody at a dose of 380 mg after 3 months; and the subsequent administrations of an anti-TRBV9 antibody at a dose of 380 mg every 6 months; or every 9 months; or every 12 months based on subject's body weight from 30 to 50 kg.

In some embodiments of the invention, the use comprises the first administration of an anti-TRBV9 antibody at a dose of 130 mg, the second administration of an anti-TRBV9 antibody at a dose of 380 mg after 6 months; and the subsequent administrations of an anti-TRBV9 antibody at a dose of 380 mg every 6 months; or every 9 months; or every 12 months based on subject's body weight from 30 to 50 kg.

In some embodiments of the invention, the use comprises the first administration of an anti-TRBV9 antibody at a dose of 130 mg, the second administration of an anti-TRBV9 antibody at a dose of 380 mg after 9 months; and the subsequent administrations of an anti-TRBV9 antibody at a dose of 380 mg every 9 months; or every 12 months based on subject's body weight from 30 to 50 kg.

In some embodiments of the invention, the use comprises the first administration of an anti-TRBV9 antibody at a dose of 130 mg, the second administration of an anti-TRBV9 antibody at a dose of 380 mg after 12 months, and the subsequent administrations of an anti-TRBV9 antibody at 380 mg every 12 months based on subject's body weight from 30 to 50 kg.

In some embodiments of the invention, the use comprises the first administration of an anti-TRBV9 antibody at a dose of 245 mg, the second administration of an anti-TRBV9 antibody at a dose of 495 mg after at least 3 months, and the subsequent administrations of an anti-TRBV9 antibody at a dose of 495 mg at least every 6 months.

In some embodiments of the invention, the use comprises the first administration of an anti-TRBV9 antibody at a dose of 245 mg, the second administration of an anti-TRBV9 antibody at a dose of 495 mg after 3 months; and the subsequent administrations of an anti-TRBV9 antibody at a dose of 495 mg every 6 months; or every 9 months; or every 12 months.

In some embodiments of the invention, the use comprises the first administration of an anti-TRBV9 antibody at a dose of 245 mg, the second administration of an anti-TRBV9 antibody at a dose of 495 mg after 6 months; and the subsequent administrations of an anti-TRBV9 antibody at a dose of 495 mg every 6 months; or every 9 months; or every 12 months.

In some embodiments of the invention, the use comprises the first administration of an anti-TRBV9 antibody at a dose of 245 mg, the second administration of an anti-TRBV9 antibody at a dose of 495 mg after 9 months; and the subsequent administrations of an anti-TRBV9 antibody at a dose of 495 mg every 9 months; or every 12 months.

In some embodiments of the invention, the use comprises the first administration of an anti-TRBV9 antibody at a dose of 245 **mg,** the second administration of an anti-TRBV9 antibody at a dose of 495 mg after 12 months, and the subsequent administrations of an anti-TRBV9 antibody at 495 mg every 12 months.

In some embodiments of the invention, the use comprises the first administration of an anti-TRBV9 antibody at a dose of 250 **mg,** the second administration of an anti-TRBV9 antibody at a dose of 500 mg after at least 3 months, and the subsequent administrations of an anti-TRBV9 antibody at a dose of 500 mg at least every 6 months.

In some embodiments of the invention, the use comprises the first administration of an anti-TRBV9 antibody at a dose of 250 **mg,** the second administration of an anti-TRBV9 antibody at a dose of 500 mg after 3 months; and the subsequent administrations of an anti-TRBV9 antibody at a dose of 500 mg every 6 months; or every 9 months; or every 12 months.

In some embodiments of the invention, the use comprises the first administration of an anti-TRBV9 antibody at a dose of 250 mg, the second administration of an anti-TRBV9 antibody at a dose of 500 mg after 6 months; and the subsequent administrations of an anti-TRBV9 antibody at a dose of 500 mg every 6 months; or every 9 months; or every 12 months.

In some embodiments of the invention, the use comprises the first administration of an anti-TRBV9 antibody at a dose of 250 mg, the second administration of an anti-TRBV9 antibody at a dose of 500 mg after 9 months; and the subsequent administrations of an anti-TRBV9 antibody at a dose of 500 mg every 9 months; or every 12 months.

In some embodiments of the invention, the use comprises the first administration of an anti-TRBV9 antibody at a dose of 250 mg, the second administration of an anti-TRBV9 antibody at a dose of 500 mg after 12 months, and the subsequent administrations of an anti-TRBV9 antibody at 500 mg every 12 months.

In some embodiments of the invention, the use comprises the first administration of an anti-TRBV9 antibody at a dose of 255 mg, the second administration of an anti-TRBV9 antibody at a dose of 505 mg after at least 3 months, and the subsequent administrations of an anti-TRBV9 antibody at a dose of 505 mg at least every 6 months.

In some embodiments of the invention, the use comprises the first administration of an anti-TRBV9 antibody at a dose of 255 mg, the second administration of an anti-TRBV9 antibody at a dose of 505 mg after 3 months; and the subsequent administrations of an anti-TRBV9 antibody at a dose of 505 mg every 6 months; or every 9 months; or every 12 months.

In some embodiments of the invention, the use comprises the first administration of an anti-TRBV9 antibody at a dose of 255 **mg,** the second administration of an anti-TRBV9 antibody at a dose of 505 mg after 6 months; and the subsequent administrations of an anti-TRBV9 antibody at a dose of 505 mg every 6 months; or every 9 months; or every 12 months.

In some embodiments of the invention, the use comprises the first administration of an anti-TRBV9 antibody at a dose of 255 **mg,** the second administration of an anti-TRBV9 antibody at a dose of 505 mg after 9 months; and the subsequent administrations of an anti-TRBV9 antibody at a dose of 505 mg every 9 months; or every 12 months.

In some embodiments of the invention, the use comprises the first administration of an anti-TRBV9 antibody at a dose of 255 **mg,** the second administration of an anti-TRBV9 antibody at a dose of 505 mg after 12 months, and the subsequent administrations of an anti-TRBV9 antibody at 505 mg every 12 months.

In some embodiments of the invention, the use comprises the first administration of an anti-TRBV9 antibody at a dose of 245 **mg,** the second administration of an anti-TRBV9 antibody at a dose of 495 mg after at least 3 months, and the subsequent administrations of an anti-TRBV9 antibody at a dose of 495 mg at least every 6 months based on subject's body weight from 51 to 89 **kg.**

In some embodiments of the invention, the use comprises the first administration of an anti-TRBV9 antibody at a dose of 245 **mg,** the second administration of an anti-TRBV9 antibody at a dose of 495 mg after 3 months; and the subsequent administrations of an anti-TRBV9 antibody at a dose of 495 mg every 6 months; or every 9 months; or every 12 months based on subject's body weight from 51 to 89 **kg.**

In some embodiments of the invention, the use comprises the first administration of an anti-TRBV9 antibody at a dose of 245 **mg,** the second administration of an anti-TRBV9 antibody at a dose of 495 mg after 6 months; and the subsequent administrations of an anti-TRBV9 antibody at a dose of 495 mg every 6 months; or every 9 months; or every 12 months based on subject's body weight from 51 to 89 **kg.**

In some embodiments of the invention, the use comprises the first administration of an anti-TRBV9 antibody at a dose of 245 **mg,** the second administration of an anti-TRBV9 antibody at a dose of 495 mg after 9 months; and the subsequent administrations of an anti-TRBV9 antibody at a dose of 495 mg every 9 months; or every 12 months based on subject's body weight from 51 to 89 **kg.**

In some embodiments of the invention, the use comprises the first administration of an anti-TRBV9 antibody at a dose of 245 **mg,** the second administration of an anti-TRBV9 antibody at a dose of 495 mg after 12 months, and the subsequent administrations of an anti-TRBV9 antibody at 495 mg every 12 months based on subject's body weight from 51 to 89 **kg.**

In some embodiments of the invention, the use comprises the first administration of an anti-TRBV9 antibody at a dose of 250 **mg,** the second administration of an anti-TRBV9 antibody at a dose of 500 mg after at least 3 months, and the subsequent administrations of an anti-TRBV9 antibody at a dose of 500 mg at least every 6 months based on subject's body weight from 51 to 89 **kg.**

In some embodiments of the invention, the use comprises the first administration of an anti-TRBV9 antibody at a dose of 250 **mg,** the second administration of an anti-TRBV9 antibody at a dose of 500 mg after 3 months; and the subsequent administrations of an anti-TRBV9 antibody at a dose of 500 mg every 6 months; or every 9 months; or every 12 months based on subject's body weight from 51 to 89 kg.

In some embodiments of the invention, the use comprises the first administration of an anti-TRBV9 antibody at a dose of 250 mg, the second administration of an anti-TRBV9 antibody at a dose of 500 mg after 6 months; and the subsequent administrations of an anti-TRBV9 antibody at a dose of 500 mg every 6 months; or every 9 months; or every 12 months based on subject's body weight from 51 to 89 kg.

In some embodiments of the invention, the use comprises the first administration of an anti-TRBV9 antibody at a dose of 250 mg, the second administration of an anti-TRBV9 antibody at a dose of 500 mg after 9 months; and the subsequent administrations of an anti-TRBV9 antibody at a dose of 500 mg every 9 months; or every 12 months based on subject's body weight from 51 to 89 kg.

In some embodiments of the invention, the use comprises the first administration of an anti-TRBV9 antibody at a dose of 250 mg, the second administration of an anti-TRBV9 antibody at a dose of 500 mg after 12 months, and the subsequent administrations of an anti-TRBV9 antibody at 500 mg every 12 months based on subject's body weight from 51 to 89 kg.

In some embodiments of the invention, the use comprises the first administration of an anti-TRBV9 antibody at a dose of 255 mg, the second administration of an anti-TRBV9 antibody at a dose of 505 mg after at least 3 months, and the subsequent administrations of an anti-TRBV9 antibody at a dose of 505 mg at least every 6 months based on subject's body weight from 51 to 89 kg.

In some embodiments of the invention, the use comprises the first administration of an anti-TRBV9 antibody at a dose of 255 mg, the second administration of an anti-TRBV9 antibody at a dose of 505 mg after 3 months; and the subsequent administrations of an anti-TRBV9 antibody at a dose of 505 mg every 6 months; or every 9 months; or every 12 months based on subject's body weight from 51 to 89 kg.

In some embodiments of the invention, the use comprises the first administration of an anti-TRBV9 antibody at a dose of 255 mg, the second administration of an anti-TRBV9 antibody at a dose of 505 mg after 6 months; and the subsequent administrations of an anti-TRBV9 antibody at a dose of 505 mg every 6 months; or every 9 months; or every 12 months based on subject's body weight from 51 to 89 kg.

In some embodiments of the invention, the use comprises the first administration of an anti-TRBV9 antibody at a dose of 255 mg, the second administration of an anti-TRBV9 antibody at a dose of 505 mg after 9 months; and the subsequent administrations of an anti-TRBV9 antibody at a dose of 505 mg every 9 months; or every 12 months based on subject's body weight from 51 to 89 **kg.**

In some embodiments of the invention, the use comprises the first administration of an anti-TRBV9 antibody at a dose of 255 **mg,** the second administration of an anti-TRBV9 antibody at a dose of 505 mg after 12 months, and the subsequent administrations of an anti-TRBV9 antibody at 505 mg every 12 months based on subject's body weight from 51 to 89 **kg.**

In some embodiments of the invention, the use comprises the first administration of an anti-TRBV9 antibody at a dose of 245 mg, the second administration of an anti-TRBV9 antibody at a dose of 620 mg after at least 3 months, and the subsequent administrations of an anti-TRBV9 antibody at a dose of 620 mg at least every 6 months.

In some embodiments of the invention, the use comprises the first administration of an anti-TRBV9 antibody at a dose of 245 mg, the second administration of an anti-TRBV9 antibody at a dose of 620 mg after 3 months; and the subsequent administrations of an anti-TRBV9 antibody at a dose of 620 mg every 6 months; or every 9 months; or every 12 months.

In some embodiments of the invention, the use comprises the first administration of an anti-TRBV9 antibody at a dose of 245 mg, the second administration of an anti-TRBV9 antibody at a dose of 620 mg after 6 months; and the subsequent administrations of an anti-TRBV9 antibody at a dose of 620 mg every 6 months; or every 9 months; or every 12 months.

In some embodiments of the invention, the use comprises the first administration of an anti-TRBV9 antibody at a dose of 245 mg, the second administration of an anti-TRBV9 antibody at a dose of 620 mg after 9 months; and the subsequent administrations of an anti-TRBV9 antibody at a dose of 620 mg every 9 months; or every 12 months.

In some embodiments of the invention, the use comprises the first administration of an anti-TRBV9 antibody at a dose of 245 mg, the second administration of an anti-TRBV9 antibody at a dose of 620 mg after 12 months, and the subsequent administrations of an anti-TRBV9 antibody at 620 mg every 12 months.

In some embodiments of the invention, the use comprises the first administration of an anti-TRBV9 antibody at a dose of 250 mg, the second administration of an anti-TRBV9 antibody at a dose of 625 mg after at least 3 months, and the subsequent administrations of an anti-TRBV9 antibody at a dose of 625 mg at least every 6 months.

In some embodiments of the invention, the use comprises the first administration of an anti-TRBV9 antibody at a dose of 250 mg, the second administration of an anti-TRBV9 antibody at a dose of 625 mg after 3 months; and the subsequent administrations of an anti-TRBV9 antibody at a dose of 625 mg every 6 months; or every 9 months; or every 12 months.

In some embodiments of the invention, the use comprises the first administration of an anti-TRBV9 antibody at a dose of 250 mg, the second administration of an anti-TRBV9 antibody at a dose of 625 mg after 6 months; and the subsequent administrations of an anti-TRBV9 antibody at a dose of 625 mg every 6 months; or every 9 months; or every 12 months.

In some embodiments of the invention, the use comprises the first administration of an anti-TRBV9 antibody at a dose of 250 mg, the second administration of an anti-TRBV9 antibody at a dose of 625 mg after 9 months; and the subsequent administrations of an anti-TRBV9 antibody at a dose of 625 mg every 9 months; or every 12 months.

In some embodiments of the invention, the use comprises the first administration of an anti-TRBV9 antibody at a dose of 250 mg, the second administration of an anti-TRBV9 antibody at a dose of 625 mg after 12 months, and the subsequent administrations of an anti-TRBV9 antibody at 625 mg every 12 months.

In some embodiments of the invention, the use comprises the first administration of an anti-TRBV9 antibody at a dose of 255 **mg,** the second administration of an anti-TRBV9 antibody at a dose of 630 mg after at least 3 months, and the subsequent administrations of an anti-TRBV9 antibody at a dose of 630 mg at least every 6 months.

In some embodiments of the invention, the use comprises the first administration of an anti-TRBV9 antibody at a dose of 255 mg, the second administration of an anti-TRBV9 antibody at a dose of 630 mg after 3 months; and the subsequent administrations of an anti-TRBV9 antibody at a dose of 630 mg every 6 months; or every 9 months; or every 12 months.

In some embodiments of the invention, the use comprises the first administration of an anti-TRBV9 antibody at a dose of 255 mg, the second administration of an anti-TRBV9 antibody at a dose of 630 mg after 6 months; and the subsequent administrations of an anti-TRBV9 antibody at a dose of 630 mg every 6 months; or every 9 months; or every 12 months.

In some embodiments of the invention, the use comprises the first administration of an anti-TRBV9 antibody at a dose of 255 mg, the second administration of an anti-TRBV9 antibody at a dose of 630 mg after 9 months; and the subsequent administrations of an anti-TRBV9 antibody at a dose of 630 mg every 9 months; or every 12 months.

In some embodiments of the invention, the use comprises the first administration of an anti-TRBV9 antibody at a dose of 255 **mg,** the second administration of an anti-TRBV9 antibody at a dose of 630 mg after 12 months, and the subsequent administrations of an anti-TRBV9 antibody at 630 mg every 12 months.

In some embodiments of the invention, the use comprises the first administration of an anti-TRBV9 antibody at a dose of 245 **mg,** the second administration of an anti-TRBV9 antibody at a dose of 620 mg after at least 3 months, and the subsequent administrations of an anti-TRBV9 antibody at a dose of 620 mg at least every 6 months based on subject's body weight from 90 to 150 kg.

In some embodiments of the invention, the use comprises the first administration of an anti-TRBV9 antibody at a dose of 245 **mg,** the second administration of an anti-TRBV9 antibody at a dose of 620 mg after 3 months; and the subsequent administrations of an anti-TRBV9 antibody at a dose of 620 mg every 6 months; or every 9 months; or every 12 months based on subject's body weight from 90 to 150 kg.

In some embodiments of the invention, the use comprises the first administration of an anti-TRBV9 antibody at a dose of 245 **mg,** the second administration of an anti-TRBV9 antibody at a dose of 620 mg after 6 months; and the subsequent administrations of an anti-TRBV9 antibody at a dose of 620 mg every 6 months; or every 9 months; or every 12 months based on subject's body weight from 90 to 150 kg.

In some embodiments of the invention, the use comprises the first administration of an anti-TRBV9 antibody at a dose of 245 **mg,** the second administration of an anti-TRBV9 antibody at a dose of 620 mg after 9 months; and the subsequent administrations of an anti-TRBV9 antibody at a dose of 620 mg every 9 months; or every 12 months based on subject's body weight from 90 to 150 kg.

In some embodiments of the invention, the use comprises the first administration of an anti-TRBV9 antibody at a dose of 245 **mg,** the second administration of an anti-TRBV9 antibody at a dose of 620 mg after 12 months, and the subsequent administrations of an anti-TRBV9 antibody at 620 mg every 12 months based on subject's body weight from 90 to 150 kg.

In some embodiments of the invention, the use comprises the first administration of an anti-TRBV9 antibody at a dose of 250 **mg,** the second administration of an anti-TRBV9 antibody at a dose of 625 mg after at least 3 months, and the subsequent administrations of an anti-TRBV9 antibody at a dose of 625 mg at least every 6 months based on subject's body weight from 90 to 150 kg.

In some embodiments of the invention, the use comprises the first administration of an anti-TRBV9 antibody at a dose of 250 **mg,** the second administration of an anti-TRBV9 antibody at a dose of 625 mg after 3 months; and the subsequent administrations of an anti-TRBV9 antibody at a dose of 625 mg every 6 months; or every 9 months; or every 12 months based on subject's body weight from 90 to 150 kg.

In some embodiments of the invention, the use comprises the first administration of an anti-TRBV9 antibody at a dose of 250 **mg,** the second administration of an anti-TRBV9 antibody at a dose of 625 mg after 6 months; and the subsequent administrations of an anti-TRBV9 antibody at a dose of 625 mg every 6 months; or every 9 months; or every 12 months based on subject's body weight from 90 to 150 kg.

In some embodiments of the invention, the use comprises the first administration of an anti-TRBV9 antibody at a dose of 250 mg, the second administration of an anti-TRBV9 antibody at a dose of 625 mg after 9 months; and the subsequent administrations of an anti-TRBV9 antibody at a dose of 625 mg every 9 months; or every 12 months based on subject's body weight from 90 to 150 kg.

In some embodiments of the invention, the use comprises the first administration of an anti-TRBV9 antibody at a dose of 250 mg, the second administration of an anti-TRBV9 antibody at a dose of 625 mg after 12 months, and the subsequent administrations of an anti-TRBV9 antibody at 625 mg every 12 months based on subject's body weight from 90 to 150 kg.

In some embodiments of the invention, the use comprises the first administration of an anti-TRBV9 antibody at a dose of 255 mg, the second administration of an anti-TRBV9 antibody at a dose of 630 mg after at least 3 months, and the subsequent administrations of an anti-TRBV9 antibody at a dose of 630 mg at least every 6 months based on subject's body weight from 90 to 150 kg.

In some embodiments of the invention, the use comprises the first administration of an anti-TRBV9 antibody at a dose of 255 mg, the second administration of an anti-TRBV9 antibody at a dose of 630 mg after 3 months; and the subsequent administrations of an anti-TRBV9 antibody at a dose of 630 mg every 6 months; or every 9 months; or every 12 months based on subject's body weight from 90 to 150 kg.

In some embodiments of the invention, the use comprises the first administration of an anti-TRBV9 antibody at a dose of 255 mg, the second administration of an anti-TRBV9 antibody at a dose of 630 mg after 6 months; and the subsequent administrations of an anti-TRBV9 antibody at a dose of 630 mg every 6 months; or every 9 months; or every 12 months based on subject's body weight from 90 to 150 kg.

In some embodiments of the invention, the use comprises the first administration of an anti-TRBV9 antibody at a dose of 255 mg, the second administration of an anti-TRBV9 antibody at a dose of 630 mg after 9 months; and the subsequent administrations of an anti-TRBV9 antibody at a dose of 630 mg every 9 months; or every 12 months based on subject's body weight from 90 to 150 kg.

In some embodiments of the invention, the use comprises the first administration of an anti-TRBV9 antibody at a dose of 255 mg, the second administration of an anti-TRBV9 antibody at a dose of 630 mg after 12 months, and the subsequent administrations of an anti-TRBV9 antibody at 630 mg every 12 months based on subject's body weight from 90 to 150 kg.

In one aspect, the present invention relates to use of an anti-TRBV9 antibody for the treatment of a disease or disorder mediated by T-lymphocytes bearing the TRBV9 segment within the T-cell receptor, wherein the first administration to a subject in need thereof of an anti-TRBV9 antibody is performed at a dose equal to half of the main dose, 0.015-5 mg/kg, the second administration of an anti-TRBV9 antibody is performed at the main dose of 0.03-10 mg/kg after at least 3 months, the third administration of an anti-TRBV9 antibody is performed at the main dose of 0.03-10 mg/kg after at least 6 months, and the subsequent administrations of an anti-TRBV9 antibody are performed at the main dose of 1-10 mg/kg at least every 12 months.

In some embodiments of the invention, the use comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 3 months, the third administration of an anti-TRBV9 antibody at the main dose of 0.03-10 mg/kg after 6 months; or after 9 months; or after 12 months; and the subsequent administrations of an anti-TRBV9 antibody at the main dose of 0.03-10 mg/kg every 12 months.

In some embodiments of the invention, the use comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg [] 6 months, the third administration of an anti-TRBV9 antibody at the main dose of 0.03-10 mg/kg after 6 months; or after 9 months; or after 12 months; and the subsequent administrations of an anti-TRBV9 antibody at the main dose of 0.03-10 mg/kg every 12 months.

In some embodiments of the invention, the use comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 9 months, the third administration of an anti-TRBV9 antibody at the main dose of 0.03-10 mg/kg after 9 months; or after 12 months; and the subsequent administrations of an anti-TRBV9 antibody at the main dose of 0.03-10 mg/kg every 12 months.

In some embodiments of the invention, the use comprises the first administration to a subject in need thereof of an anti-TRBV9 antibody at a dose equal to half of the main dose, 0.015-5 mg/kg, the second administration of an anti-TRBV9 antibody at the main dose of 0.03-10 mg/kg after 12 months, the third administration of an anti-TRBV9 antibody at the main dose of 0.03-10 mg/kg after 12 months, and the subsequent administrations of an anti-TRBV9 antibody at the main dose of 0.03-10 mg/kg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration to a subject in need thereof of an anti-TRBV9 antibody is performed at a dose equal to half of the main dose, 0.015-3.5 mg/kg, the second administration of an anti-TRBV9 antibody is performed at the main dose of 0.03-7 mg/kg after at least 3 months, the third administration of an anti-TRBV9 antibody is performed at the main dose of 0.03-7 mg/kg after at least 6 months, and the subsequent administrations of an anti-TRBV9 antibody are performed at the main dose of 1-7 mg/kg at least every 12 months.

In some embodiments of the invention, the use comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 3 months, the third administration of an anti-TRBV9 antibody at the main dose of 0.03-7 mg/kg after 6 months; or after 9 months; or after 12 months; and the subsequent administrations of an anti-TRBV9 antibody at the main dose of 1-7 mg/kg every 12 months.

In some embodiments of the invention, the use comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 6 months, the third administration of an anti-TRBV9 antibody at the main dose of 0.03-7 mg/kg after 6 months; or after 9 months; or after 12 months; and the subsequent administrations of an anti-TRBV9 antibody at the main dose of 1-7 mg/kg every 12 months.

In some embodiments of the invention, the use comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 9 months, the third administration of an anti-TRBV9 antibody at the main dose of 0.03-7 mg/kg after 9 months; or after 12 months; and the subsequent administrations of an anti-TRBV9 antibody at the main dose of 1-7 mg/kg every 12 months.

In some embodiments of the invention, the use comprises the first administration to a subject in need thereof of an anti-TRBV9 antibody at a dose equal to half of the main dose, 0.015-3.5 mg/kg, the second administration of an anti-TRBV9 antibody at the main dose of 0.03-7 mg/kg after 12 months, the third administration of an anti-TRBV9 antibody at the main dose of 0.03-7 mg/kg after 12 months, and the subsequent administrations of an anti-TRBV9 antibody at the main dose of 1-7 mg/kg every 12 months.

In one aspect, the present invention relates to use of an anti-TRBV9 antibody for the treatment of a disease or disorder mediated by T-lymphocytes bearing the TRBV9 segment within the T-cell receptor, comprising the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after at least 3 months, the third administration of an anti-TRBV9 antibody at the main dose of 0.3-7 mg/kg after at least 6 months, and the subsequent administrations of an anti-TRBV9 antibody at the main dose of 0.3-10 mg/kg at least every 12 months.

In some embodiments of the invention, the use comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 3 months, the third administration of an anti-TRBV9 antibody at the main dose of 0.3-10 mg/kg after 6 months; or after 9 months; or after 12 months; and the subsequent administrations of an anti-TRBV9 antibody at the main dose of 0.3-10 mg/kg every 12 months.

In some embodiments of the invention, the use comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 6 months, the third administration of an anti-TRBV9 antibody at the main dose of 0.3-10 mg/kg after 6 months; or after 9 months; or after 12 months; and the subsequent administrations of an anti-TRBV9 antibody at the main dose of 0.3-10 mg/kg every 12 months.

In some embodiments of the invention, the use comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 9 months, the third administration of an anti-TRBV9 antibody at the main dose of 0.3-10 mg/kg after 9 months; or after 12 months; and the subsequent administrations of an anti-TRBV9 antibody at the main dose of 0.3-10 mg/kg every 12 months.

In some embodiments of the invention, the use comprises the first administration to a subject in need thereof of an anti-TRBV9 antibody at a dose equal to half of the main dose, 0.15-5 mg/kg, the second administration of an anti-TRBV9 antibody at the main dose of 0.3-10 mg/kg after 12 months, the third administration of an anti-TRBV9 antibody at the main dose of 0.3-10 mg/kg after 12 months, and the subsequent administrations of an anti-TRBV9 antibody at the main dose of 0.3-10 mg/kg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration to a subject in need thereof of an anti-TRBV9 antibody is performed at a dose equal to half of the main dose, 0.15-3.5 mg/kg, the second administration of an anti-TRBV9 antibody is performed at the main dose of 0.3-7 mg/kg after at least 3 months, the third administration of an anti-TRBV9 antibody is performed at the main dose of 0.3-7 mg/kg after at least 6 months, and the subsequent administrations of an anti-TRBV9 antibody are performed at the main dose of 0.3-7 mg/kg at least every 12 months.

In some embodiments of the invention, the use comprises the first administration to a subject in need thereof of an anti-TRBV9 antibody performed at a dose equal to half of the main dose, 0.15-3.5 mg/kg, the second administration of an anti-TRBV9 antibody is performed at the main dose of 0.3-7 mg/kg after 3 months, the third administration of an anti-TRBV9 antibody is performed at the main dose of 0.3-7 mg/kg after 6 months; or after 9 months; or after 12 months; and the subsequent administrations of an anti-TRBV9 antibody are performed at the main dose of 0.3-7 mg/kg every 12 months.

In some embodiments of the invention, the use comprises the first administration to a subject in need thereof of an anti-TRBV9 antibody performed at a dose equal to half of the main dose, 0.15-3.5 mg/kg, the second administration of an anti-TRBV9 antibody is performed at the main dose of 0.3-7 mg/kg after 6 months, the third administration of an anti-TRBV9 antibody is performed at the main dose of 0.3-7 mg/kg after 6 months; or after 9 months; or after 12 months; and the subsequent administrations of an anti-TRBV9 antibody are performed at the main dose of 0.3-7 mg/kg every 12 months.

In some embodiments of the invention, the use comprises the first administration to a subject in need thereof of an anti-TRBV9 antibody performed at a dose equal to half of the main dose, 0.15-3.5 mg/kg, the second administration of an anti-TRBV9 antibody is performed at the main dose of 0.3-7 mg/kg after 9 months, the third administration of an anti-TRBV9 antibody is performed at the main dose of 0.3-7 mg/kg after 9 months; or after 12 months; and the subsequent administrations of an anti-TRBV9 antibody are performed at the main dose of 0.3-7 mg/kg every 12 months.

In some embodiments of the invention, the use comprises the first administration to a subject in need thereof of an anti-TRBV9 antibody performed at a dose equal to half of the main dose, 0.15-3.5 mg/kg, the second administration of an anti-TRBV9 antibody is performed at the main dose of 0.3-7 mg/kg after 12 months, the third administration of an anti-TRBV9 antibody is performed at the main dose of 0.3-7 mg/kg after 12 months, and the subsequent administrations of an anti-TRBV9 antibody are performed at the main dose of 0.3-7 mg/kg every 12 months.

In one aspect, the present invention relates to use of an anti-TRBV9 antibody for the treatment of a disease or disorder mediated by T-lymphocytes bearing the TRBV9 segment within the **T-**cell receptor, wherein the first administration to a subject in need thereof of an anti-TRBV9 antibody is performed at a dose equal to half of the main dose, 0.5-3.5 mg/kg, the second administration of an anti-TRBV9 antibody is performed at the main dose of 1-7 mg/kg after at least 3 months, the third administration of an anti-TRBV9 antibody is performed at the main dose of 1-7 mg/kg after at least 6 months, and the subsequent administrations of an anti-TRBV9 antibody are performed at the main dose of 1-7 mg/kg at least every 12 months.

In one aspect, the present invention relates to use of an anti-TRBV9 antibody for the treatment of a disease or disorder mediated by T-lymphocytes bearing the TRBV9 segment within the **T-**cell receptor, wherein the first administration to a subject in need thereof of an anti-TRBV9 antibody is performed at a dose equal to half of the main dose, **0.5-3.5** mg/kg, the second administration of an anti-TRBV9 antibody is performed at the main dose of 1-7 mg/kg after 3 months, the third administration of an anti-TRBV9 antibody is performed at the main dose of 1-7 mg/kg after 6 months; or after 9 months; or after 12 months; and the subsequent administrations of an anti-TRBV9 antibody are performed at the main dose of 1-7 mg/kg every 12 months.

In some embodiments of the invention, the use comprises the first administration to a subject in need thereof of an anti-TRBV9 antibody at a dose equal to half of the main dose, 0.5-3.5 mg/kg, the second administration of an anti-TRBV9 antibody at the main dose of 1-7 mg/kg after 6 months, the third administration of an anti-TRBV9 antibody at the main dose of 1-7 mg/kg after 6 months; or after 9 months; or after 12 months; and the subsequent administrations of an anti-TRBV9 antibody at the main dose of 1-7 mg/kg every 12 months.

In some embodiments of the invention, the use comprises the first administration to a subject in need thereof of an anti-TRBV9 antibody at a dose equal to half of the main dose, **0.5-3.5** mg/kg, the second administration of an anti-TRBV9 antibody at the main dose of 1-7 mg/kg after 9 months, the third administration of an anti-TRBV9 antibody at the main dose of 1-7 mg/kg after 9 months; or after 12 months; and the subsequent administrations of an anti-TRBV9 antibody at the main dose of 1-7 mg/kg every 12 months.

In some embodiments of the invention, the use comprises the first administration to a subject in need thereof of an anti-TRBV9 antibody at a dose equal to half of the main dose, **0.5-3.5** mg/kg, the second administration of an anti-TRBV9 antibody at the main dose of 1-7 mg/kg after 12 months, the third administration of an anti-TRBV9 antibody at the main dose of 1-7 mg/kg after 12 months, and the subsequent administrations of an anti-TRBV9 antibody at the main dose of 1-7 mg/kg every 12 months.

In some embodiments of the invention, the first administration of an anti-TRBV9 antibody is performed at a dose of 0.5 mg/kg, the second administration of an anti-TRBV9 antibody is performed at a dose of 1 mg/kg after at least 3 months, the third administration of an anti-TRBV9 antibody is performed at a dose of 1 mg/kg after at least 6 months, and the subsequent administrations of an anti-TRBV9 antibody are performed at a dose of 1 mg/kg at least every 12 months.

In some embodiments of the invention, the use comprises the first administration of an anti-TRBV9 antibody at a dose of 0.5 mg/kg, the second administration of an anti-TRBV9 antibody at a dose of 1 mg/kg after 3 months, the third administration of an anti-TRBV9 antibody at a dose of 1 mg/kg after 6 months; or after 9 months; or after 12 months; and the subsequent administrations of an anti-TRBV9 antibody at a dose of 1 mg/kg every 12 months.

In some embodiments of the invention, the use comprises the first administration of an anti-TRBV9 antibody at a dose of 0.5 mg/kg, the second administration of an anti-TRBV9 antibody at a dose of 1 mg/kg after 6 months, the third administration of an anti-TRBV9 antibody at a dose of 1 mg/kg after 6 months; or after 9 months; or after 12 months; and the subsequent administrations of an anti-TRBV9 antibody at a dose of 1 mg/kg every 12 months.

In some embodiments of the invention, the use comprises the first administration of an anti-TRBV9 antibody at a dose of 0.5 mg/kg, the second administration of an anti-TRBV9 antibody at a dose of 1 mg/kg after 9 months, the third administration of an anti-TRBV9 antibody at a dose of 1 mg/kg after 9 months; or after 12 months; and the subsequent administrations of an anti-TRBV9 antibody at a dose of 1 mg/kg every 12 months.

In some embodiments of the invention, the use comprises the first administration of an anti-TRBV9 antibody at a dose of 0.5 mg/kg, the second administration of an anti-TRBV9 antibody at a dose of 1 mg/kg after 12 months, the third administration of an anti-TRBV9 antibody at a dose of 1 mg/kg after 12 months, and the subsequent administrations of an anti-TRBV9 antibody at a dose of 1 mg/kg every 12 months.

In some embodiments of the invention, the first administration of an anti-TRBV9 antibody is performed at a dose of 1 mg/kg, the second administration of an anti-TRBV9 antibody is performed at a dose of 2 mg/kg after at least 3 months, the third administration of an anti-TRBV9 antibody is performed at a dose of 2 mg/kg after at least 6 months, and the subsequent administrations of an anti-TRBV9 antibody are performed at a dose of 2 mg/kg at least every 12 months.

In some embodiments of the invention, the first administration of an anti-TRBV9 antibody is performed at a dose of 1 mg/kg, the second administration of an anti-TRBV9 antibody is performed at a dose of 2 mg/kg after 3 months, the third administration of an anti-TRBV9 antibody is performed at a dose of 2 mg/kg after 6 months; or after 9 months; or after 12 months; and the subsequent administrations of an anti-TRBV9 antibody are performed at a dose of 2 mg/kg every 12 months.

In some embodiments of the invention, the first administration of an anti-TRBV9 antibody at a dose of 1 mg/kg, the second administration of an anti-TRBV9 antibody at a dose of 2 mg/kg after 6 months, the third administration of an anti-TRBV9 antibody at a dose of 2 mg/kg after 6 months; or after 9 months; or after 12 months; and the subsequent administrations of an anti-TRBV9 antibody at a dose of 2 mg/kg every 12 months.

In some embodiments of the invention, the first administration of an anti-TRBV9 antibody at a dose of 1 mg/kg, the second administration of an anti-TRBV9 antibody at a dose of 2 mg/kg after 9 months, the third administration of an anti-TRBV9 antibody at a dose of 2 mg/kg after 9 months; or after 12 months; and the subsequent administrations of an anti-TRBV9 antibody at a dose of 2 mg/kg every 12 months.

In some embodiments of the invention, the first administration of an anti-TRBV9 antibody at a dose of 1 mg/kg, the second administration of an anti-TRBV9 antibody at a dose of 2 mg/kg after 12 months, the third administration of an anti-TRBV9 antibody at a dose of 2 mg/kg after 12 months, and the subsequent administrations of an anti-TRBV9 antibody at a dose of 2 mg/kg every 12 months.

In some embodiments of the invention, the first administration of an anti-TRBV9 antibody is performed at a dose of 1.5 mg/kg, the second administration of an anti-TRBV9 antibody is performed at a dose of 3 mg/kg after at least 3 months, the third administration of an anti-TRBV9 antibody is performed at a dose of 3 mg/kg after at least 6 months and the subsequent administrations of an anti-TRBV9 antibody are performed at a dose of 3 mg/kg at least every 12 months.

In some embodiments of the invention, the first administration of an anti-TRBV9 antibody is performed at a dose of 1.5 mg/kg, the second administration of an anti-TRBV9 antibody is performed at a dose of 3 mg/kg after 3 months, the third administration of an anti-TRBV9 antibody is performed at a dose of 3 mg/kg after 6 months; or after 9 months; or after 12 months; and the subsequent administrations of an anti-TRBV9 antibody are performed at a dose of 3 mg/kg every 12 months.

In some embodiments of the invention, the first administration of an anti-TRBV9 antibody at a dose of 1.5 mg/kg, the second administration of an anti-TRBV9 antibody at a dose of 3 mg/kg after 6 months, the third administration of an anti-TRBV9 antibody at a dose of 3 mg/kg after 6 months; or after 9 months; or after 12 months; and the subsequent administrations of an anti-TRBV9 antibody at a dose of 3 mg/kg every 12 months.

In some embodiments of the invention, the first administration of an anti-TRBV9 antibody at a dose of 1.5 mg/kg, the second administration of an anti-TRBV9 antibody at a dose of 3 mg/kg after 9 months, the third administration of an anti-TRBV9 antibody at a dose of 3 mg/kg after 9 months; or after 12 months; and the subsequent administrations of an anti-TRBV9 antibody at a dose of 3 mg/kg every 12 months.

In some embodiments of the invention, the first administration of an anti-TRBV9 antibody at a dose of 1.5 mg/kg, the second administration of an anti-TRBV9 antibody at a dose of 3 mg/kg after 12 months, the third administration of an anti-TRBV9 antibody at a dose of 3 mg/kg after 12 months, and the subsequent administrations of an anti-TRBV9 antibody at a dose of 3 mg/kg every 12 months.

In some embodiments of the invention, the first administration of an anti-TRBV9 antibody is performed at a dose of 2 mg/kg, the second administration of an anti-TRBV9 antibody is performed at a dose of 4 mg/kg after at least 3 months, the third administration of an anti-TRBV9 antibody is performed at a dose of 4 mg/kg after at least 6 months, and the subsequent administrations of an anti-TRBV9 antibody are performed at a dose of 4 mg/kg at least every 12 months.

In some embodiments of the invention, the first administration of an anti-TRBV9 antibody is performed at a dose of 2 mg/kg, the second administration of an anti-TRBV9 antibody is performed at a dose of 4 mg/kg after 3 months, the third administration of an anti-TRBV9 antibody is performed at a dose of 4 mg/kg after 6 months; or after 9 months; or after 12 months; and the subsequent administrations of an anti-TRBV9 antibody are performed at a dose of 4 mg/kg every 12 months.

In some embodiments of the invention, the first administration of an anti-TRBV9 antibody at a dose of 2 mg/kg, the second administration of an anti-TRBV9 antibody at a dose of 4 mg/kg after 6 months, the third administration of an anti-TRBV9 antibody at a dose of 4 mg/kg after 6 months; or after 9 months; or after 12 months; and the subsequent administrations of an anti-TRBV9 antibody at a dose of 4 mg/kg every 12 months.

In some embodiments of the invention, the first administration of an anti-TRBV9 antibody at a dose of 2 mg/kg, the second administration of an anti-TRBV9 antibody at a dose of 4 mg/kg after 9 months, the third administration of an anti-TRBV9 antibody at a dose of 4 mg/kg after 9 months; or after 12 months; and the subsequent administrations of an anti-TRBV9 antibody at a dose of 4 mg/kg every 12 months.

In some embodiments of the invention, the first administration of an anti-TRBV9 antibody at a dose of 2 mg/kg, the second administration of an anti-TRBV9 antibody at a dose of 4 mg/kg after 12 months, the third administration of an anti-TRBV9 antibody at a dose of 4 mg/kg after 12 months, and the subsequent administrations of an anti-TRBV9 antibody at a dose of 4 mg/kg every 12 months.

In some embodiments of the invention, the first administration of an anti-TRBV9 antibody is performed at a dose of 2.5 mg/kg, the second administration of an anti-TRBV9 antibody is performed at a dose of 5 mg/kg after at least 3 months, the third administration of an anti-TRBV9 antibody is performed at a dose of 5 mg/kg after at least 6 months, and the subsequent administrations of an anti-TRBV9 antibody are performed at a dose of 5 mg/kg at least every 12 months.

In some embodiments of the invention, the first administration of an anti-TRBV9 antibody is performed at a dose of 2,5 mg/kg, the second administration of an anti-TRBV9 antibody is performed at a dose of 5 mg/kg after 3 months, the third administration of an anti-TRBV9 antibody is performed at a dose of 5 mg/kg after 6 months; or after 9 months; or after 12 months; and the subsequent administrations of an anti-TRBV9 antibody are performed at a dose of 5 mg/kg every 12 months.

In some embodiments of the invention, the first administration of an anti-TRBV9 antibody at a dose of 2,5 mg/kg, the second administration of an anti-TRBV9 antibody at a dose of 5 mg/kg after 6 months, the third administration of an anti-TRBV9 antibody at a dose of 5 mg/kg after 6 months; or after 9 months; or after 12 months; and the subsequent administrations of an anti-TRBV9 antibody at a dose of 5 mg/kg every 12 months.

In some embodiments of the invention, the first administration of an anti-TRBV9 antibody at a dose of 2,5 mg/kg, the second administration of an anti-TRBV9 antibody at a dose of 5 mg/kg after 9 months, the third administration of an anti-TRBV9 antibody at a dose of 5 mg/kg after 9 months; or after 12 months; and the subsequent administrations of an anti-TRBV9 antibody at a dose of 5 mg/kg every 12 months.

In some embodiments of the invention, the first administration of an anti-TRBV9 antibody at a dose of 2,5 mg/kg, the second administration of an anti-TRBV9 antibody at a dose of 5 mg/kg after 12 months, the third administration of an anti-TRBV9 antibody at a dose of 5 mg/kg after 6 months, and the subsequent administrations of an anti-TRBV9 antibody at a dose of 5 mg/kg every 12 months.

In some embodiments of the invention, the first administration of an anti-TRBV9 antibody is performed at a dose of 3 mg/kg, the second administration of an anti-TRBV9 antibody is performed at a dose of 6 mg/kg after at least 3 months, the third administration of an anti-TRBV9 antibody is performed at a dose of 6 mg/kg after at least 6 months, and the subsequent administrations of an anti-TRBV9 antibody are performed at a dose of 6 mg/kg at least every 12 months.

In some embodiments of the invention, the first administration of an anti-TRBV9 antibody is performed at a dose of 3 mg/kg, the second administration of an anti-TRBV9 antibody is performed at a dose of 6 mg/kg after 3 months, the third administration of an anti-TRBV9 antibody is performed at a dose of 6 mg/kg after 6 months; or after 9 months; or after 12 months; and the subsequent administrations of an anti-TRBV9 antibody are performed at a dose of 6 mg/kg every 12 months.

In some embodiments of the invention, the first administration of an anti-TRBV9 antibody at a dose of 3 mg/kg, the second administration of an anti-TRBV9 antibody at a dose of 6 mg/kg after 6 months, the third administration of an anti-TRBV9 antibody at a dose of 6 mg/kg after 6 months; or after 9 months; or after 12 months; and the subsequent administrations of an anti-TRBV9 antibody at a dose of 6 mg/kg every 12 months.

In some embodiments of the invention, the first administration of an anti-TRBV9 antibody at a dose of 3 mg/kg, the second administration of an anti-TRBV9 antibody at a dose of 6 mg/kg after 9 months, the third administration of an anti-TRBV9 antibody at a dose of 6 mg/kg after 9 months; or after 12 months; and the subsequent administrations of an anti-TRBV9 antibody at a dose of 6 mg/kg every 12 months.

In some embodiments of the invention, the first administration of an anti-TRBV9 antibody at a dose of 3 mg/kg, the second administration of an anti-TRBV9 antibody at a dose of 6 mg/kg after 12 months, the third administration of an anti-TRBV9 antibody at a dose of 6 mg/kg after 12 months, and the subsequent administrations of an anti-TRBV9 antibody at a dose of 6 mg/kg every 12 months.

In some embodiments of the invention, the first administration to a subject in need thereof of an anti-TRBV9 antibody is performed at a dose of 3.5 mg/kg, the second administration of an anti-TRBV9 antibody is performed at a dose of 7 mg/kg after at least 3 months, the third administration of an anti-TRBV9 antibody is performed at a dose of 7 mg/kg after at least 6 months, and the subsequent administrations of an anti-TRBV9 antibody are performed at a dose of 7 mg/kg at least every 12 months.

In some embodiments of the invention, the first administration to a subject in need thereof of an anti-TRBV9 antibody is performed at a dose of 3.5 mg/kg, the second administration of an anti-TRBV9 antibody is performed at a dose of 7 mg/kg after 3 months, the third administration of an anti-TRBV9 antibody is performed at a dose of 7 mg/kg after 6 months; or after 9 months; or after 12 months; and the subsequent administrations of an anti-TRBV9 antibody are performed at a dose of 7 mg/kg every 12 months.

In some embodiments of the invention, the first administration to a subject in need thereof of an anti-TRBV9 antibody at a dose of 3.5 mg/kg, the second administration of an anti-TRBV9 antibody at a dose of 7 mg/kg after 6 months, the third administration of an anti-TRBV9 antibody at a dose of 7 mg/kg after 6 months; or after 9 months; or after 12 months; and the subsequent administrations of an anti-TRBV9 antibody at a dose of 7 mg/kg every 12 months.

In some embodiments of the invention, the first administration to a subject in need thereof of an anti-TRBV9 antibody at a dose of 3.5 mg/kg, the second administration of an anti-TRBV9 antibody at a dose of 7 mg/kg after 9 months, the third administration of an anti-TRBV9 antibody at a dose of 7 mg/kg after 9 months; or after 12 months; and the subsequent administrations of an anti-TRBV9 antibody at a dose of 7 mg/kg every 12 months.

In some embodiments of the invention, the first administration to a subject in need thereof of an anti-TRBV9 antibody at a dose of 3.5 mg/kg, the second administration of an anti-TRBV9 antibody at a dose of 7 mg/kg after 12 months, the third administration of an anti-TRBV9 antibody at a dose of 7 mg/kg after 12 months, and the subsequent administrations of an anti-TRBV9 antibody at a dose of 7 mg/kg every 12 months.

In some embodiments of the invention, the first administration to a subject in need thereof of an anti-TRBV9 antibody is performed at a dose of 4 mg/kg, the second administration of an anti-TRBV9 antibody is performed at a dose of 8 mg/kg after at least 3 months, the third administration of an anti-TRBV9 antibody is performed at a dose of 8 mg/kg after at least 6 months, and the subsequent administrations of an anti-TRBV9 antibody are performed at a dose of 8 mg/kg at least every 12 months.

In some embodiments of the invention, the first administration to a subject in need thereof of an anti-TRBV9 antibody is performed at a dose of 4 mg/kg, the second administration of an anti-TRBV9 antibody is performed at a dose of 8 mg/kg after 3 months, the third administration of an anti-TRBV9 antibody is performed at a dose of 8 mg/kg after 6 months; or after 9 months; or after 12 months; and the subsequent administrations of an anti-TRBV9 antibody are performed at a dose of 8 mg/kg every 12 months .

In some embodiments of the invention, the first administration to a subject in need thereof of an anti-TRBV9 antibody at a dose of 4 mg/kg, the second administration of an anti-TRBV9 antibody at a dose of 8 mg/kg after 6 months, the third administration of an anti-TRBV9 antibody at a dose of 8 mg/kg after 6 months; or after 9 months; or after 12 months; and the subsequent administrations of an anti-TRBV9 antibody at a dose of 8 mg/kg every 12 months.

In some embodiments of the invention, the first administration to a subject in need thereof of an anti-TRBV9 antibody at a dose of 4 mg/kg, the second administration of an anti-TRBV9 antibody at a dose of 8 mg/kg after 9 months, the third administration of an anti-TRBV9 antibody at a dose of 8 mg/kg after 9 months; or after 12 months; and the subsequent administrations of an anti-TRBV9 antibody at a dose of 8 mg/kg every 12 months.

In some embodiments of the invention, the first administration to a subject in need thereof of an anti-TRBV9 antibody at a dose of 4 mg/kg, the second administration of an anti-TRBV9 antibody at a dose of 8 mg/kg after 12 months, the third administration of an anti-TRBV9 antibody at a dose of 8 mg/kg after 12 months, and the subsequent administrations of an anti-TRBV9 antibody at a dose of 8 mg/kg every 12 months.

In some embodiments of the invention, the first administration to a subject in need thereof of an anti-TRBV9 antibody is performed at a dose of 4.5 mg/kg, the second administration of an anti-TRBV9 antibody is performed at a dose of 9 mg/kg after at least 3 months, the third administration of an anti-TRBV9 antibody is performed at a dose of 9 mg/kg after at least 6 months, and the subsequent administrations of an anti-TRBV9 antibody are performed at a dose of 9 mg/kg at least every 12 months.

In some embodiments of the invention, the first administration to a subject in need thereof of an anti-TRBV9 antibody is performed at a dose of 4.5 mg/kg, the second administration of an anti-TRBV9 antibody is performed at a dose of 9 mg/kg after 3 months, the third administration of an anti-TRBV9 antibody is performed at a dose of 9 mg/kg after 6 months; or after 9 months; or after 12 months; and the subsequent administrations of an anti-TRBV9 antibody are performed at a dose of 9 mg/kg every 12 months .

In some embodiments of the invention, the first administration to a subject in need thereof of an anti-TRBV9 antibody at a dose of 4.5 mg/kg, the second administration of an anti-TRBV9 antibody at a dose of 9 mg/kg after 6 months, the third administration of an anti-TRBV9 antibody at a dose of 9 mg/kg after 6 months; or after 9 months; or after 12 months; and the subsequent administrations of an anti-TRBV9 antibody at a dose of 9 mg/kg every 12 months.

In some embodiments of the invention, the first administration to a subject in need thereof of an anti-TRBV9 antibody at a dose of 4.5 mg/kg, the second administration of an anti-TRBV9 antibody at a dose of 9 mg/kg after 9 months, the third administration of an anti-TRBV9 antibody at a dose of 9 mg/kg after 9 months; or after 12 months; and the subsequent administrations of an anti-TRBV9 antibody at a dose of 9 mg/kg every 12 months.

In some embodiments of the invention, the first administration to a subject in need thereof of an anti-TRBV9 antibody at a dose of **4.5** mg/kg, the second administration of an anti-TRBV9 antibody at a dose of 9 mg/kg after 12 months, the third administration of an anti-TRBV9 antibody at a dose of 9 mg/kg after 12 months, and the subsequent administrations of an anti-TRBV9 antibody at a dose of 9 mg/kg every 12 months.

In some embodiments of the invention, the first administration to a subject in need thereof of an anti-TRBV9 antibody is performed at a dose of 5 mg/kg, the second administration of an anti-TRBV9 antibody is performed at a dose of 10 mg/kg after at least 3 months, the third administration of an anti-TRBV9 antibody is performed at a dose of 10 mg/kg after at least 6 months, and the subsequent administrations of an anti-TRBV9 antibody are performed at a dose of 10 mg/kg at least every 12 months.

In some embodiments of the invention, the first administration to a subject in need thereof of an anti-TRBV9 antibody is performed at a dose of 5 mg/kg, the second administration of an anti-TRBV9 antibody is performed at a dose of 10 mg/kg after 3 months, the third administration of an anti-TRBV9 antibody is performed at a dose of 10 mg/kg after 6 months; or after 9 months; or after 12 months; and the subsequent administrations of an anti-TRBV9 antibody are performed at a dose of 10 mg/kg every 12 months.

In some embodiments of the invention, the first administration to a subject in need thereof of an anti-TRBV9 antibody at a dose of 5 mg/kg, the second administration of an anti-TRBV9 antibody at a dose of 10 mg/kg after 6 months, the third administration of an anti-TRBV9 antibody at a dose of 10 mg/kg after 6 months; or after 9 months; or after 12 months; and the subsequent administrations of an anti-TRBV9 antibody at a dose of 10 mg/kg every 12 months.

In some embodiments of the invention, the first administration to a subject in need thereof of an anti-TRBV9 antibody at a dose of 5 mg/kg, the second administration of an anti-TRBV9 antibody at a dose of 10 mg/kg after 9 months, the third administration of an anti-TRBV9 antibody at a dose of 10 mg/kg after 9 months; or after 12 months; and the subsequent administrations of an anti-TRBV9 antibody at a dose of 10 mg/kg every 12 months.

In some embodiments of the invention, the first administration to a subject in need thereof of an anti-TRBV9 antibody at a dose of 5 mg/kg, the second administration of an anti-TRBV9 antibody at a dose of 10 mg/kg after 12 months, the third administration of an anti-TRBV9 antibody at a dose of 10 mg/kg after 12 months, and the subsequent administrations of an anti-TRBV9 antibody at a dose of 10 mg/kg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration to a subject in need thereof of an anti-TRBV9 antibody is performed at a dose of 75-300 mg, the second administration of an anti-TRBV9 antibody at the main dose of 325-675 mg after at least 3 months, the third administration of an anti-TRBV9 antibody at the main dose of 325-675 mg after at least 6 months, and the subsequent administrations of an anti-TRBV9 antibody at the main dose of 325-675 mg at least every 12 months.

In some embodiments of the invention, the use comprises the first administration to a subject in need thereof of an anti-TRBV9 antibody at a dose of 75-300 mg, the second administration of an anti-TRBV9 antibody at the main dose of 325-675 mg after 3 months, the third administration of an anti-TRBV9 antibody at the main dose of 325-675 mg after 6 months; or after 9 months; or after 12 months; and the subsequent administrations of an anti-TRBV9 antibody at the main dose of 325-675 mg every 12 months.

In some embodiments of the invention, the use comprises the first administration to a subject in need thereof of an anti-TRBV9 antibody at a dose of 75-300 mg, the second administration of an anti-TRBV9 antibody at the main dose of 325-675 mg [] 6 months, the third administration of an anti-TRBV9 antibody at the main dose of 325-675 mg after 6 months; or after 9 months; or after 12 months; and the subsequent administrations of an anti-TRBV9 antibody at the main dose of 325-675 mg every 12 months.

In some embodiments of the invention, the use comprises the first administration to a subject in need thereof of an anti-TRBV9 antibody at a dose of 75-300 mg, the second administration of an anti-TRBV9 antibody at the main dose of 325-675 mg after 9 months, the third administration of an anti-TRBV9 antibody at the main dose of 325-675 mg after 9 months; or after 12 months; and the subsequent administrations of an anti-TRBV9 antibody at the main dose of 325-675 mg every 12 months.

In some embodiments of the invention, the use comprises the first administration to a subject in need thereof of an anti-TRBV9 antibody at a dose of 75-300 mg, the second administration of an anti-TRBV9 antibody at the main dose of 325-675 mg after 12 months, the third administration of an anti-TRBV9 antibody at the main dose of 325-675 mg after 12 months, and the subsequent administrations of an anti-TRBV9 antibody at the main dose of 325-675 mg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration to a subject in need thereof of an anti-TRBV9 antibody is performed at a dose of 100-275 mg, the second administration of an anti-TRBV9 antibody at the main dose of 350-650 mg after at least 3 months, the third administration of an anti-TRBV9 antibody at the main dose of 350-650 mg after at least 6 months, and the subsequent administrations of an anti-TRBV9 antibody at the main dose of 350-650 mg at least every 12 months.

In some embodiments of the invention, the use comprises the first administration to a subject in need thereof of an anti-TRBV9 antibody at a dose of 100-275 mg, the second administration of an anti-TRBV9 antibody at the main dose of 350-650 mg after 3 months, the third administration of an anti-TRBV9 antibody at the main dose of 350-650 mg after 6 months; or after 9 months; or after 12 months; and the subsequent administrations of an anti-TRBV9 antibody at the main dose of 350-650 mg every 12 months.

In some embodiments of the invention, the use comprises the first administration to a subject in need thereof of an anti-TRBV9 antibody at a dose of 100-275 mg, the second administration of an anti-TRBV9 antibody at the main dose of 350-650 mg after 6 months, the third administration of an anti-TRBV9 antibody at the main dose of 350-650 mg after 6 months; or after 9 months; or after 12 months; and the subsequent administrations of an anti-TRBV9 antibody at the main dose of 350-650 mg every 12 months.

In some embodiments of the invention, the use comprises the first administration to a subject in need thereof of an anti-TRBV9 antibody at a dose of 100-275 mg, the second administration of an anti-TRBV9 antibody at the main dose of 350-650 mg after 9 months, the third administration of an anti-TRBV9 antibody at the main dose of 350-650 mg after 9 months; or after 12 months; and the subsequent administrations of an anti-TRBV9 antibody at the main dose of 350-650 mg every 12 months.

In some embodiments of the invention, the use comprises the first administration to a subject in need thereof of an anti-TRBV9 antibody at a dose of 100-275 mg, the second administration of an anti-TRBV9 antibody at the main dose of 350-650 mg after 12 months, the third administration of an anti-TRBV9 antibody at the main dose of 350-650 mg after 12 months, and the subsequent administrations of an anti-TRBV9 antibody at the main dose of 350-650 mg every 12 months.

In one aspect, the present invention relates to use of an anti-TRBV9 antibody for the treatment of a disease or disorder mediated by T-lymphocytes bearing the TRBV9 segment within the T-cell receptor, comprising the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after at least 3 months, the third administration of an anti-TRBV9 antibody at the main dose of 350-400 mg; or 475- 525 mg; or 600-650 mg after at least 6 months, and the subsequent administrations of an anti-TRBV9 antibody at the main dose of 350-400 mg; or 475- 525 mg; or 600-650 mg at least every 12 months.

In some embodiments of the invention, the use comprises (i) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 3 months, the third administration of an anti-TRBV9 antibody at the main dose of 350-400 mg; or 475- 525 mg; or 600-650 mg after 6 months, and the subsequent administrations of an anti-TRBV9 antibody at the main dose of 350-400 mg; or 475- 525 mg; or 600-650 mg every 12 months, or
(ii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 3 months, the third administration of an anti-TRBV9 antibody at the main dose of 350-400 mg; or 475- 525 mg; or 600-650 mg after 9 months, and the subsequent administrations of an anti-TRBV9 antibody at the main dose of 350-400 mg; or 475- 525 mg; or 600-650 mg every 12 months, or
(iii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 3 months, the third administration of an anti-TRBV9 antibody at the main dose of 350-400 mg; or 475- 525 mg; or 600-650 mg after 12 months, and the subsequent administrations of an anti-TRBV9 antibody at the main dose of 350-400 mg; or 475- 525 mg; or 600-650 mg every 12 months, or
(iv) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 6 months, the third administration of an anti-TRBV9 antibody at the main dose of 350-400 mg; or 475- 525 mg; or 600-650 mg after 6 months, and the subsequent administrations of an anti-TRBV9 antibody at the main dose of 350-400 mg; or 475- 525 mg; or 600-650 mg every 12 months, or
(v) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 6 months, the third administration of an anti-TRBV9 antibody at the main dose of 350-400 mg; or 475- 525 mg; or 600-650 mg after 9 months, and the subsequent administrations of an anti-TRBV9 antibody at the main dose of 350-400 mg; or 475- 525 mg; or 600-650 mg every 12 months, or
(vi) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 6 months, the third administration of an anti-TRBV9 antibody at the main dose of 350-400 mg; or 475- 525 mg; or 600-650 mg after 12 months, and the subsequent administrations of an anti-TRBV9 antibody at the main dose of 350-400 mg; or 475- 525 mg; or 600-650 mg every 12 months, or
(vii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 9 months, the third administration of an anti-TRBV9 antibody at the main dose of 350-400 mg; or 475- 525 mg; or 600-650 mg after 9 months, and the subsequent administrations of an anti-TRBV9 antibody at the main dose of 350-400 mg; or 475- 525 mg; or 600-650 mg every 12 months, or
(viii) the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 9 months, the third administration of an anti-TRBV9 antibody at the main dose of 350-400 mg; or 475- 525 mg; or 600-650 mg after 12 months, and the subsequent administrations of an anti-TRBV9 antibody at the main dose of 350-400 mg; or 475- 525 mg; or 600-650 mg every 12 months, or
(ix)the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after 12 months, the third administration of an anti-TRBV9 antibody at the main dose of 350-400 mg; or 475- 525 mg; or 600-650 mg after 12 months, and the subsequent administrations of an anti-TRBV9 antibody at the main dose of 350-400 mg; or 475- 525 mg; or 600-650 mg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration to a subject in need thereof of an anti-TRBV9 antibody is performed at a dose of 115-135 mg; or 240-260 mg, the second administration of an anti-TRBV9 antibody at the main dose of 365-385 mg; or 490- 510 mg; or 615-635 mg after at least 3 months, the third administration of an anti-TRBV9 antibody at the main dose of 365-385 mg; or 490-510 mg; or 615-635 mg after at least 6 months, and the subsequent administrations of an anti-TRBV9 antibody at the main dose of 365-385 mg; or 490- 510 mg; or 615-635 mg at least every 12 months.

In some embodiments of the invention, the use comprises (i) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 3 months, the third administration of an anti-TRBV9 antibody at the main dose of 365-385 mg; or 490- 510 mg; or 615-635 mg after 6 months, and the subsequent administrations of an anti-TRBV9 antibody at the main dose of 365-385 mg; or 490- 510 mg; or 615-635 mg every 12 months, or
(ii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 3 months, the third administration of an anti-TRBV9 antibody at the main dose of 365-385 mg; or 490- 510 mg; or 615-635 mg after 9 months, and the subsequent administrations of an anti-TRBV9 antibody at the main dose of 365-385 mg; or 490- 510 mg; or 615-635 mg every 12 months, or
(iii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 3 months, the third administration of an anti-TRBV9 antibody at the main dose of 365-385 mg; or 490- 510 mg; or 615-635 mg after 12 months, and the subsequent administrations of an anti-TRBV9 antibody at the main dose of 365-385 mg; or 490- 510 mg; or 615-635 mg every 12 months, or
(iv) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 6 months, the third administration of an anti-TRBV9 antibody at the main dose of 365-385 mg; or 490- 510 mg; or 615-635 mg after 6 months, and the subsequent administrations of an anti-TRBV9 antibody at the main dose of 365-385 mg; or 490- 510 mg; or 615-635 mg every 12 months, or
(v) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 6 months, the third administration of an anti-TRBV9 antibody at the main dose of 365-385 mg; or 490- 510 mg; or 615-635 mg after 9 months, and the subsequent administrations of an anti-TRBV9 antibody at the main dose of 365-385 mg; or 490- 510 mg; or 615-635 mg every 12 months, or
(vi) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 6 months, the third administration of an anti-TRBV9 antibody at the main dose of 365-385 mg; or 490- 510 mg; or 615-635 mg after 12 months, and the subsequent administrations of an anti-TRBV9 antibody at the main dose of 365-385 mg; or 490- 510 mg; or 615-635 mg every 12 months, or
(vii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 9 months, the third administration of an anti-TRBV9 antibody at the main dose of 365-385 mg; or 490- 510 mg; or 615-635 mg after 9 months, and the subsequent administrations of an anti-TRBV9 antibody at the main dose of 365-385 mg; or 490- 510 mg; or 615-635 mg every 12 months, or
(viii) the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 9 months, the third administration of an anti-TRBV9 antibody at the main dose of 365-385 mg; or 490- 510 mg; or 615-635 mg after 12 months, and the subsequent administrations of an anti-TRBV9 antibody at the main dose of 365-385 mg; or 490- 510 mg; or 615-635 mg every 12 months, or
(ix)the first administration to a subject in need thereof of an anti-TRBV9 antibody at a dose of 115-135 mg; or 240-260 mg, the second administration of an anti-TRBV9 antibody at the main dose of 365-385 mg; or 490- 510 mg; or 615-635 mg after 12 months, the third administration of an anti-TRBV9 antibody at the main dose of 365-385 mg; or 490- 510 mg; or 615-635 mg after 12 months, and the subsequent administrations of an anti-TRBV9 antibody at the main dose of 365-385 mg; or 490- 510 mg; or 615-635 mg every 12 months.

In one aspect, the present invention relates to use of an anti-TRBV9 antibody for the treatment of a disease or disorder mediated by T-lymphocytes bearing the TRBV9 segment within the T-cell receptor, wherein the first administration to a subject in need thereof of an anti-TRBV9 antibody is performed at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after at least 3 months, the third administration of an anti-TRBV9 antibody at the main dose of 370 mg; or 375 mg; or 380 mg; or 495 mg; or 500 mg; or 505 mg; or 620 mg; or 625 mg; or 630 mg after at least 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg at least every 12 months.

In one aspect, the present invention relates to use of an anti-TRBV9 antibody for the treatment of a disease or disorder mediated by T-lymphocytes bearing the TRBV9 segment within the T-cell receptor, wherein (i) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 3 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(ii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 3 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 9 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(iii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 3 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 12 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(iv) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 6 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(v) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 6 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 9 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(vi) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 6 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 12 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(vii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 9 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 9 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(viii) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 9 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 12 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months, or
(ix) the first administration to a subject in need thereof of an anti-TRBV9 antibody at a dose of 120 mg; or 125 mg; or 130 mg; or 245 mg; or 250 mg; or 255 mg , the second administration of an anti-TRBV9 antibody at the main dose of 370 mg; or 375 mg; or 380 mg; or 495 mg; or 500 mg; or 505 mg; or 620 mg; or 625 mg; or 630 mg after 12 months, the third administration of an anti-TRBV9 antibody at the main dose of 370 mg; or 375 mg; or 380 mg; or 495 mg; or 500 mg; or 505 mg; or 620 mg; or 625 mg; or 630 mg after 12 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg every 12 months.

In some embodiments of the invention, the use comprises the first administration of an anti-TRBV9 antibody at a dose of 120 mg, the second administration of an anti-TRBV9 antibody at a dose of 370 mg after at least 3 months, the third administration of an anti-TRBV9 antibody at a dose of 370 mg after at least 6 months, and the subsequent administrations of an anti-TRBV9 antibody at a dose of 370 mg at least every 12 months.

In some embodiments of the invention, the use comprises the first administration of an anti-TRBV9 antibody at a dose of 120 mg, the second administration of an anti-TRBV9 antibody at a dose of 370 mg after 3 months; the third administration of an anti-TRBV9 antibody at a dose of 370 mg after 6 months; or after 9 months; or after 12 months; and the subsequent administrations of an anti-TRBV9 antibody at a dose of 370 mg every 12 months.

In some embodiments of the invention, the use comprises the first administration of an anti-TRBV9 antibody at a dose of 120 mg, the second administration of an anti-TRBV9 antibody at a dose of 370 mg after 6 months; the third administration of an anti-TRBV9 antibody at a dose of 370 mg after 6 months; or after 9 months; or after 12 months; and the subsequent administrations of an anti-TRBV9 antibody at a dose of 370 mg every 12 months.

In some embodiments of the invention, the use comprises the first administration of an anti-TRBV9 antibody at a dose of 120 mg, the second administration of an anti-TRBV9 antibody at a dose of 370 mg after 9 months; the third administration of an anti-TRBV9 antibody at a dose of 370 mg after 9 months; or after 12 months; and the subsequent administrations of an anti-TRBV9 antibody at a dose of 370 mg every 12 months.

In some embodiments of the invention, the use comprises the first administration of an anti-TRBV9 antibody at a dose of 120 mg, the second administration of an anti-TRBV9 antibody at a dose of 370 mg after 12 months, the third administration of an anti-TRBV9 antibody at a dose of 370 mg after 12 months, and the subsequent administrations of an anti-TRBV9 antibody at a dose of 370 mg every 12 months.

In some embodiments of the invention, the use comprises the first administration of an anti-TRBV9 antibody at a dose of 125 mg, the second administration of an anti-TRBV9 antibody at a dose of 375 mg after at least 3 months, the third administration of an anti-TRBV9 antibody at a dose of 375 mg after at least 6 months, and the subsequent administrations of an anti-TRBV9 antibody at a dose of 375 mg at least every 12 months.

In some embodiments of the invention, the use comprises the first administration of an anti-TRBV9 antibody at a dose of 125 mg, the second administration of an anti-TRBV9 antibody at a dose of 375 mg after 3 months; the third administration of an anti-TRBV9 antibody at a dose of 375 mg after 6 months; or after 9 months; or after 12 months; and the subsequent administrations of an anti-TRBV9 antibody at a dose of 375 mg every 12 months.

In some embodiments of the invention, the use comprises the first administration of an anti-TRBV9 antibody at a dose of 125 mg, the second administration of an anti-TRBV9 antibody at a dose of 375 mg after 6 months; the third administration of an anti-TRBV9 antibody at a dose of 375 mg after 6 months; or after 9 months; or after 12 months; and the subsequent administrations of an anti-TRBV9 antibody at a dose of 375 mg every 12 months.

In some embodiments of the invention, the use comprises the first administration of an anti-TRBV9 antibody at a dose of 125 mg, the second administration of an anti-TRBV9 antibody at a dose of 375 mg after 9 months; the third administration of an anti-TRBV9 antibody at a dose of 375 mg after 9 months; or after 12 months; and the subsequent administrations of an anti-TRBV9 antibody at a dose of 375 mg every 12 months.

In some embodiments of the invention, the use comprises the first administration of an anti-TRBV9 antibody at a dose of 125 mg, the second administration of an anti-TRBV9 antibody at a dose of 375 mg after 12 months, the third administration of an anti-TRBV9 antibody at a dose of 375 mg after 12 months, and the subsequent administrations of an anti-TRBV9 antibody at a dose of 375 mg every 12 months.

In some embodiments of the invention, the use comprises the first administration of an anti-TRBV9 antibody at a dose of 130 mg, the second administration of an anti-TRBV9 antibody at a dose of 380 mg after at least 3 months, the third administration of an anti-TRBV9 antibody at a dose of 380 mg after at least 6 months, and the subsequent administrations of an anti-TRBV9 antibody at a dose of 380 mg at least every 12 months.

In some embodiments of the invention, the use comprises the first administration of an anti-TRBV9 antibody at a dose of 130 mg, the second administration of an anti-TRBV9 antibody at a dose of 380 mg after 3 months; the third administration of an anti-TRBV9 antibody at a dose of 380 mg after 6 months; or after 9 months; or after 12 months; and the subsequent administrations of an anti-TRBV9 antibody at a dose of 380 mg every 12 months.

In some embodiments of the invention, the use comprises the first administration of an anti-TRBV9 antibody at a dose of 130 mg, the second administration of an anti-TRBV9 antibody at a dose of 380 mg after 6 months; the third administration of an anti-TRBV9 antibody at a dose of 380 mg after 6 months; or after 9 months; or after 12 months; and the subsequent administrations of an anti-TRBV9 antibody at a dose of 380 mg every 12 months.

In some embodiments of the invention, the use comprises the first administration of an anti-TRBV9 antibody at a dose of 130 **mg,** the second administration of an anti-TRBV9 antibody at a dose of 380 mg after 9 months; the third administration of an anti-TRBV9 antibody at a dose of 380 mg after 9 months; or after 12 months; and the subsequent administrations of an anti-TRBV9 antibody at a dose of 380 mg every 12 months.

In some embodiments of the invention, the use comprises the first administration of an anti-TRBV9 antibody at a dose of 130 mg, the second administration of an anti-TRBV9 antibody at a dose of 380 mg after 12 months, the third administration of an anti-TRBV9 antibody at a dose of 380 mg after 12 months, and the subsequent administrations of an anti-TRBV9 antibody at a dose of 380 mg every 12 months.

In some embodiments of the invention, the use comprises the first administration of an anti-TRBV9 antibody at a dose of 120 mg, the second administration of an anti-TRBV9 antibody at a dose of 370 mg after at least 3 months, the third administration of an anti-TRBV9 antibody at a dose of 370 mg after at least 6 months, and the subsequent administrations of an anti-TRBV9 antibody at a dose of 370 mg at least every 12 months based on subject's body weight from 30 to 50 kg.

In some embodiments of the invention, the use comprises the first administration of an anti-TRBV9 antibody at a dose of 120 mg, the second administration of an anti-TRBV9 antibody at a dose of 370 mg after 3 months; the third administration of an anti-TRBV9 antibody at a dose of 370 mg after 6 months; or after 9 months; or after 12 months; and the subsequent administrations of an anti-TRBV9 antibody at a dose of 370 mg every 12 months based on subject's body weight from 30 to 50 kg.

In some embodiments of the invention, the use comprises the first administration of an anti-TRBV9 antibody at a dose of 120 mg, the second administration of an anti-TRBV9 antibody at a dose of 370 mg after 6 months; the third administration of an anti-TRBV9 antibody at a dose of 370 mg after 6 months; or after 9 months; or after 12 months; and the subsequent administrations of an anti-TRBV9 antibody at a dose of 370 mg every 12 months based on subject's body weight from 30 to 50 kg.

In some embodiments of the invention, the use comprises the first administration of an anti-TRBV9 antibody at a dose of 120 mg, the second administration of an anti-TRBV9 antibody at a dose of 370 mg after 9 months; the third administration of an anti-TRBV9 antibody at a dose of 370 mg after 9 months; or after 12 months; and the subsequent administrations of an anti-TRBV9 antibody at a dose of 370 mg every 12 months based on subject's body weight from 30 to 50 kg.

In some embodiments of the invention, the use comprises the first administration of an anti-TRBV9 antibody at a dose of 120 mg, the second administration of an anti-TRBV9 antibody at a dose of 370 mg after 12 months, the third administration of an anti-TRBV9 antibody at a dose of 370 mg after 12 months, and the subsequent administrations of an anti-TRBV9 antibody at a dose of 370 mg every 12 months based on subject's body weight from 30 to 50 kg.

In some embodiments of the invention, the use comprises the first administration of an anti-TRBV9 antibody at a dose of 125 mg, the second administration of an anti-TRBV9 antibody at a dose of 375 mg after at least 3 months, the third administration of an anti-TRBV9 antibody at a dose of 375 mg after at least 6 months, and the subsequent administrations of an anti-TRBV9 antibody at a dose of 375 mg at least every 12 months based on subject's body weight from 30 to 50 kg.

In some embodiments of the invention, the use comprises the first administration of an anti-TRBV9 antibody at a dose of 125 mg, the second administration of an anti-TRBV9 antibody at a dose of 375 mg after 3 months; the third administration of an anti-TRBV9 antibody at a dose of 375 mg after 6 months; or after 9 months; or after 12 months; and the subsequent administrations of an anti-TRBV9 antibody at a dose of 375 mg every 12 months based on subject's body weight from 30 to 50 kg.

In some embodiments of the invention, the use comprises the first administration of an anti-TRBV9 antibody at a dose of 125 mg, the second administration of an anti-TRBV9 antibody at a dose of 375 mg after 6 months; the third administration of an anti-TRBV9 antibody at a dose of 375 mg after 6 months; or after 9 months; or after 12 months; and the subsequent administrations of an anti-TRBV9 antibody at a dose of 375 mg every 12 months based on subject's body weight from 30 to 50 kg.

In some embodiments of the invention, the use comprises the first administration of an anti-TRBV9 antibody at a dose of 125 mg, the second administration of an anti-TRBV9 antibody at a dose of 375 mg after 9 months; the third administration of an anti-TRBV9 antibody at a dose of 375 mg after 9 months; or after 12 months; and the subsequent administrations of an anti-TRBV9 antibody at a dose of 375 mg every 12 months based on subject's body weight from 30 to 50 kg.

In some embodiments of the invention, the use comprises the first administration of an anti-TRBV9 antibody at a dose of 125 mg, the second administration of an anti-TRBV9 antibody at a dose of 375 mg after 12 months, the third administration of an anti-TRBV9 antibody at a dose of 375 mg after 12 months, and the subsequent administrations of an anti-TRBV9 antibody at a dose of 375 mg every 12 months based on subject's body weight from 30 to **50 kg.**

In some embodiments of the invention, the use comprises the first administration of an anti-TRBV9 antibody at a dose of 130 **mg,** the second administration of an anti-TRBV9 antibody at a dose of 380 mg after at least 3 months, the third administration of an anti-TRBV9 antibody at a dose of 380 mg after at least 6 months, and the subsequent administrations of an anti-TRBV9 antibody at a dose of 380 mg at least every 12 months based on subject's body weight from 30 to 50 kg.

In some embodiments of the invention, the use comprises the first administration of an anti-TRBV9 antibody at a dose of 130 mg, the second administration of an anti-TRBV9 antibody at a dose of 380 mg after 3 months; the third administration of an anti-TRBV9 antibody at a dose of 380 mg after 6 months; or after 9 months; or after 12 months; and the subsequent administrations of an anti-TRBV9 antibody at a dose of 380 mg every 12 months based on subject's body weight from 30 to 50 kg.

In some embodiments of the invention, the use comprises the first administration of an anti-TRBV9 antibody at a dose of 130 mg, the second administration of an anti-TRBV9 antibody at a dose of 380 mg after 6 months; the third administration of an anti-TRBV9 antibody at a dose of 380 mg after 6 months; or after 9 months; or after 12 months; and the subsequent administrations of an anti-TRBV9 antibody at a dose of 380 mg every 12 months based on subject's body weight from 30 to 50 kg.

In some embodiments of the invention, the use comprises the first administration of an anti-TRBV9 antibody at a dose of 130 mg, the second administration of an anti-TRBV9 antibody at a dose of 380 mg after 9 months; the third administration of an anti-TRBV9 antibody at a dose of 380 mg after 9 months; or after 12 months; and the subsequent administrations of an anti-TRBV9 antibody at a dose of 380 mg every 12 months based on subject's body weight from 30 to 50 kg.

In some embodiments of the invention, the use comprises the first administration of an anti-TRBV9 antibody at a dose of 130 mg, the second administration of an anti-TRBV9 antibody at a dose of 380 mg after 12 months, the third administration of an anti-TRBV9 antibody at a dose of 380 mg after 12 months, and the subsequent administrations of an anti-TRBV9 antibody at a dose of 380 mg every 12 months based on subject's body weight from 30 to 50 kg.

In some embodiments of the invention, the use comprises the first administration of an anti-TRBV9 antibody at a dose of 245 mg, the second administration of an anti-TRBV9 antibody at a dose of 495 mg after at least 3 months, the third administration of an anti-TRBV9 antibody at a dose of 495 mg after at least 6 months, and the subsequent administrations of an anti-TRBV9 antibody at a dose of 495 mg at least every 12 months.

In some embodiments of the invention, the use comprises the first administration of an anti-TRBV9 antibody at a dose of 245 mg, the second administration of an anti-TRBV9 antibody at a dose of 495 mg after 3 months; the third administration of an anti-TRBV9 antibody at a dose of 495 mg after 6 months; or after 9 months; or after 12 months; and the subsequent administrations of an anti-TRBV9 antibody at a dose of 495 mg every 12 months.

In some embodiments of the invention, the use comprises the first administration of an anti-TRBV9 antibody at a dose of 245 mg, the second administration of an anti-TRBV9 antibody at a dose of 495 mg after 6 months; the third administration of an anti-TRBV9 antibody at a dose of 495 mg after 6 months; or after 9 months; or after 12 months; and the subsequent administrations of an anti-TRBV9 antibody at a dose of 495 mg every 12 months.

In some embodiments of the invention, the use comprises the first administration of an anti-TRBV9 antibody at a dose of 245 mg, the second administration of an anti-TRBV9 antibody at a dose of 495 mg after 9 months; the third administration of an anti-TRBV9 antibody at a dose of 495 mg after 9 months; or after 12 months; and the subsequent administrations of an anti-TRBV9 antibody at a dose of 495 mg every 12 months.

In some embodiments of the invention, the use comprises the first administration of an anti-TRBV9 antibody at a dose of 245 mg, the second administration of an anti-TRBV9 antibody at a dose of 495 mg after 12 months, the third administration of an anti-TRBV9 antibody at a dose of 495 mg after 12 months, and the subsequent administrations of an anti-TRBV9 antibody at a dose of 495 mg every 12 months.

In some embodiments of the invention, the use comprises the first administration of an anti-TRBV9 antibody at a dose of 250 mg, the second administration of an anti-TRBV9 antibody at a dose of 500 mg after at least 3 months, the third administration of an anti-TRBV9 antibody at a dose of 500 mg after at least 6 months, and the subsequent administrations of an anti-TRBV9 antibody at a dose of 500 mg at least every 12 months.

In some embodiments of the invention, the use comprises the first administration of an anti-TRBV9 antibody at a dose of 250 mg, the second administration of an anti-TRBV9 antibody at a dose of 500 mg after 3 months; the third administration of an anti-TRBV9 antibody at a dose of 500 mg after 6 months; or after 9 months; or after 12 months; and the subsequent administrations of an anti-TRBV9 antibody at a dose of 500 mg every 12 months.

In some embodiments of the invention, the use comprises the first administration of an anti-TRBV9 antibody at a dose of 250 mg, the second administration of an anti-TRBV9 antibody at a dose of 500 mg after 6 months; the third administration of an anti-TRBV9 antibody at a dose of 500 mg after 6 months; or after 9 months; or after 12 months; and the subsequent administrations of an anti-TRBV9 antibody at a dose of 500 mg every 12 months.

In some embodiments of the invention, the use comprises the first administration of an anti-TRBV9 antibody at a dose of 250 mg, the second administration of an anti-TRBV9 antibody at a dose of 500 mg after 9 months; the third administration of an anti-TRBV9 antibody at a dose of 500 mg after 9 months; or after 12 months; and the subsequent administrations of an anti-TRBV9 antibody at a dose of 500 mg every 12 months.

In some embodiments of the invention, the use comprises the first administration of an anti-TRBV9 antibody at a dose of 250 mg, the second administration of an anti-TRBV9 antibody at a dose of 500 mg after 12 months, the third administration of an anti-TRBV9 antibody at a dose of 500 mg after 12 months, and the subsequent administrations of an anti-TRBV9 antibody at a dose of 500 mg every 12 months.

In some embodiments of the invention, the use comprises the first administration of an anti-TRBV9 antibody at a dose of 255 mg, the second administration of an anti-TRBV9 antibody at a dose of 505 mg after at least 3 months, the third administration of an anti-TRBV9 antibody at a dose of 505 mg after at least 6 months, and the subsequent administrations of an anti-TRBV9 antibody at a dose of 505 mg at least every 12 months.

In some embodiments of the invention, the use comprises the first administration of an anti-TRBV9 antibody at a dose of 255 mg, the second administration of an anti-TRBV9 antibody at a dose of 505 mg after 3 months; the third administration of an anti-TRBV9 antibody at a dose of 505 mg after 6 months; or after 9 months; or after 12 months; and the subsequent administrations of an anti-TRBV9 antibody at a dose of 505 mg every 12 months.

In some embodiments of the invention, the use comprises the first administration of an anti-TRBV9 antibody at a dose of 255 mg, the second administration of an anti-TRBV9 antibody at a dose of 505 mg after 6 months; the third administration of an anti-TRBV9 antibody at a dose of 505 mg after 6 months; or after 9 months; or after 12 months; and the subsequent administrations of an anti-TRBV9 antibody at a dose of 505 mg every 12 months.

In some embodiments of the invention, the use comprises the first administration of an anti-TRBV9 antibody at a dose of 255 mg, the second administration of an anti-TRBV9 antibody at a dose of 505 mg after 9 months; the third administration of an anti-TRBV9 antibody at a dose of 505 mg after 9 months; or after 12 months; and the subsequent administrations of an anti-TRBV9 antibody at a dose of 505 mg every 12 months.

In some embodiments of the invention, the use comprises the first administration of an anti-TRBV9 antibody at a dose of 255 mg, the second administration of an anti-TRBV9 antibody at a dose of 505 mg after 12 months, the third administration of an anti-TRBV9 antibody at a dose of 505 mg after 12 months, and the subsequent administrations of an anti-TRBV9 antibody at a dose of 505 mg every 12 months.

In some embodiments of the invention, the use comprises the first administration of an anti-TRBV9 antibody at a dose of 245 mg, the second administration of an anti-TRBV9 antibody at a dose of 495 mg after at least 3 months, the third administration of an anti-TRBV9 antibody at a dose of 495 mg after at least 6 months, and the subsequent administrations of an anti-TRBV9 antibody at a dose of 495 mg at least every 12 months based on subject's body weight from 51 to 89 kg.

In some embodiments of the invention, the use comprises the first administration of an anti-TRBV9 antibody at a dose of 245 mg, the second administration of an anti-TRBV9 antibody at a dose of 495 mg after 3 months; the third administration of an anti-TRBV9 antibody at a dose of 495 mg after 6 months; or after 9 months; or after 12 months; and the subsequent administrations of an anti-TRBV9 antibody at a dose of 495 mg every 12 months based on subject's body weight from 51 to 89 kg.

In some embodiments of the invention, the use comprises the first administration of an anti-TRBV9 antibody at a dose of 245 mg, the second administration of an anti-TRBV9 antibody at a dose of 495 mg after 6 months; the third administration of an anti-TRBV9 antibody at a dose of 495 mg after 6 months; or after 9 months; or after 12 months; and the subsequent administrations of an anti-TRBV9 antibody at a dose of 495 mg every 12 months based on subject's body weight from 51 to 89 kg.

In some embodiments of the invention, the use comprises the first administration of an anti-TRBV9 antibody at a dose of 245 mg, the second administration of an anti-TRBV9 antibody at a dose of 495 mg after 9 months; the third administration of an anti-TRBV9 antibody at a dose of 495 mg after 9 months; or after 12 months; and the subsequent administrations of an anti-TRBV9 antibody at a dose of 495 mg every 12 months based on subject's body weight from 51 to 89 kg.

In some embodiments of the invention, the use comprises the first administration of an anti-TRBV9 antibody at a dose of 245 mg, the second administration of an anti-TRBV9 antibody at a dose of 495 mg after 12 months, the third administration of an anti-TRBV9 antibody at a dose of 495 mg after 12 months, and the subsequent administrations of an anti-TRBV9 antibody at a dose of 495 mg every 12 months based on subject's body weight from 51 to 89 kg.

In some embodiments of the invention, the use comprises the first administration of an anti-TRBV9 antibody at a dose of 250 mg, the second administration of an anti-TRBV9 antibody at a dose of 500 mg after at least 3 months, the third administration of an anti-TRBV9 antibody at a dose of 500 mg after at least 6 months, and the subsequent administrations of an anti-TRBV9 antibody at a dose of 500 mg at least every 12 months based on subject's body weight from 51 to 89 kg.

In some embodiments of the invention, the use comprises the first administration of an anti-TRBV9 antibody at a dose of 250 mg, the second administration of an anti-TRBV9 antibody at a dose of 500 mg after 3 months; the third administration of an anti-TRBV9 antibody at a dose of 500 mg after 6 months; or after 9 months; or after 12 months; and the subsequent administrations of an anti-TRBV9 antibody at a dose of 500 mg every 12 months based on subject's body weight from 51 to 89 kg.

In some embodiments of the invention, the use comprises the first administration of an anti-TRBV9 antibody at a dose of 250 mg, the second administration of an anti-TRBV9 antibody at a dose of 500 mg after 6 months; the third administration of an anti-TRBV9 antibody at a dose of 500 mg after 6 months; or after 9 months; or after 12 months; and the subsequent administrations of an anti-TRBV9 antibody at a dose of 500 mg every 12 months based on subject's body weight from 51 to 89 kg.

In some embodiments of the invention, the use comprises the first administration of an anti-TRBV9 antibody at a dose of 250 mg, the second administration of an anti-TRBV9 antibody at a dose of 500 mg after 9 months; the third administration of an anti-TRBV9 antibody at a dose of 500 mg after 9 months; or after 12 months; and the subsequent administrations of an anti-TRBV9 antibody at a dose of 500 mg every 12 months based on subject's body weight from 51 to 89 kg.

In some embodiments of the invention, the use comprises the first administration of an anti-TRBV9 antibody at a dose of 250 mg, the second administration of an anti-TRBV9 antibody at a dose of 500 mg after 12 months, the third administration of an anti-TRBV9 antibody at a dose of 500 mg after 12 months, and the subsequent administrations of an anti-TRBV9 antibody at a dose of 500 mg every 12 months based on subject's body weight from 51 to 89 kg.

In some embodiments of the invention, the use comprises the first administration of an anti-TRBV9 antibody at a dose of 255 mg, the second administration of an anti-TRBV9 antibody at a dose of 505 mg after at least 3 months, the third administration of an anti-TRBV9 antibody at a dose of 505 mg after at least 6 months, and the subsequent administrations of an anti-TRBV9 antibody at a dose of 505 mg at least every 12 months based on subject's body weight from 51 to 89 kg.

In some embodiments of the invention, the use comprises the first administration of an anti-TRBV9 antibody at a dose of 255 mg, the second administration of an anti-TRBV9 antibody at a dose of 505 mg after 3 months; the third administration of an anti-TRBV9 antibody at a dose of 505 mg after 6 months; or after 9 months; or after 12 months; and the subsequent administrations of an anti-TRBV9 antibody at a dose of 505 mg every 12 months based on subject's body weight from 51 to 89 kg.

In some embodiments of the invention, the use comprises the first administration of an anti-TRBV9 antibody at a dose of 255 mg, the second administration of an anti-TRBV9 antibody at a dose of 505 mg after 6 months; the third administration of an anti-TRBV9 antibody at a dose of 505 mg after 6 months; or after 9 months; or after 12 months; and the subsequent administrations of an anti-TRBV9 antibody at a dose of 505 mg every 12 months based on subject's body weight from 51 to 89 kg.

In some embodiments of the invention, the use comprises the first administration of an anti-TRBV9 antibody at a dose of 255 mg, the second administration of an anti-TRBV9 antibody at a dose of 505 mg after 9 months; the third administration of an anti-TRBV9 antibody at a dose of 505 mg after 9 months; or after 12 months; and the subsequent administrations of an anti-TRBV9 antibody at a dose of 505 mg every 12 months based on subject's body weight from 51 to 89 kg.

In some embodiments of the invention, the use comprises the first administration of an anti-TRBV9 antibody at a dose of 255 mg, the second administration of an anti-TRBV9 antibody at a dose of 505 mg after 12 months, the third administration of an anti-TRBV9 antibody at a dose of 505 mg after 12 months, and the subsequent administrations of an anti-TRBV9 antibody at a dose of 505 mg every 12 months based on subject's body weight from 51 to 89 kg.

In some embodiments of the invention, the use comprises the first administration of an anti-TRBV9 antibody at a dose of 245 mg, the second administration of an anti-TRBV9 antibody at a dose of 620 mg after at least 3 months, the third administration of an anti-TRBV9 antibody at a dose of 620 mg after at least 6 months, and the subsequent administrations of an anti-TRBV9 antibody at a dose of 620 mg at least every 12 months.

In some embodiments of the invention, the use comprises the first administration of an anti-TRBV9 antibody at a dose of 245 mg, the second administration of an anti-TRBV9 antibody at a dose of 620 mg after 3 months; the third administration of an anti-TRBV9 antibody at a dose of 620 mg after 6 months; or after 9 months; or after 12 months; and the subsequent administrations of an anti-TRBV9 antibody at a dose of 620 mg every 12 months.

In some embodiments of the invention, the use comprises the first administration of an anti-TRBV9 antibody at a dose of 245 mg, the second administration of an anti-TRBV9 antibody at a dose of 620 mg after 6 months; the third administration of an anti-TRBV9 antibody at a dose of 620 mg after 6 months; or after 9 months; or after 12 months; and the subsequent administrations of an anti-TRBV9 antibody at a dose of 620 mg every 12 months.

In some embodiments of the invention, the use comprises the first administration of an anti-TRBV9 antibody at a dose of 245 mg, the second administration of an anti-TRBV9 antibody at a dose of 620 mg after 9 months; the third administration of an anti-TRBV9 antibody at a dose of 620 mg after 9 months; or after 12 months; and the subsequent administrations of an anti-TRBV9 antibody at a dose of 620 mg every 12 months.

In some embodiments of the invention, the use comprises the first administration of an anti-TRBV9 antibody at a dose of 245 mg, the second administration of an anti-TRBV9 antibody at a dose of 620 mg after 12 months, the third administration of an anti-TRBV9 antibody at a dose of 620 mg after 12 months, and the subsequent administrations of an anti-TRBV9 antibody at a dose of 620 mg every 12 months.

In some embodiments of the invention, the use comprises the first administration of an anti-TRBV9 antibody at a dose of 250 mg, the second administration of an anti-TRBV9 antibody at a dose of 625 mg after at least 3 months, the third administration of an anti-TRBV9 antibody at a dose of 625 mg after at least 6 months, and the subsequent administrations of an anti-TRBV9 antibody at a dose of 625 mg at least every 12 months.

In some embodiments of the invention, the use comprises the first administration of an anti-TRBV9 antibody at a dose of 250 mg, the second administration of an anti-TRBV9 antibody at a dose of 625 mg after 3 months; the third administration of an anti-TRBV9 antibody at a dose of 625 mg after 6 months; or after 9 months; or after 12 months; and the subsequent administrations of an anti-TRBV9 antibody at a dose of 625 mg every 12 months.

In some embodiments of the invention, the use comprises the first administration of an anti-TRBV9 antibody at a dose of 250 mg, the second administration of an anti-TRBV9 antibody at a dose of 625 mg after 6 months; the third administration of an anti-TRBV9 antibody at a dose of 625 mg after 6 months; or after 9 months; or after 12 months; and the subsequent administrations of an anti-TRBV9 antibody at a dose of 625 mg every 12 months.

In some embodiments of the invention, the use comprises the first administration of an anti-TRBV9 antibody at a dose of 250 mg, the second administration of an anti-TRBV9 antibody at a dose of 625 mg after 9 months; the third administration of an anti-TRBV9 antibody at a dose of 625 mg after 9 months; or after 12 months; and the subsequent administrations of an anti-TRBV9 antibody at a dose of 625 mg every 12 months.

In some embodiments of the invention, the use comprises the first administration of an anti-TRBV9 antibody at a dose of 250 mg, the second administration of an anti-TRBV9 antibody at a dose of 625 mg after 12 months, the third administration of an anti-TRBV9 antibody at a dose of 625 mg after 12 months, and the subsequent administrations of an anti-TRBV9 antibody at a dose of 625 mg every 12 months.

In some embodiments of the invention, the use comprises the first administration of an anti-TRBV9 antibody at a dose of 255 mg, the second administration of an anti-TRBV9 antibody at a dose of 630 mg after at least 3 months, the third administration of an anti-TRBV9 antibody at a dose of 630 mg after at least 6 months, and the subsequent administrations of an anti-TRBV9 antibody at a dose of 630 mg at least every 12 months.

In some embodiments of the invention, the use comprises the first administration of an anti-TRBV9 antibody at a dose of 255 mg, the second administration of an anti-TRBV9 antibody at a dose of 630 mg after 3 months; the third administration of an anti-TRBV9 antibody at a dose of 630 mg after 6 months; or after 9 months; or after 12 months; and the subsequent administrations of an anti-TRBV9 antibody at a dose of 630 mg every 12 months.

In some embodiments of the invention, the use comprises the first administration of an anti-TRBV9 antibody at a dose of 255 mg, the second administration of an anti-TRBV9 antibody at a dose of 630 mg after 6 months; the third administration of an anti-TRBV9 antibody at a dose of 630 mg after 6 months; or after 9 months; or after 12 months; and the subsequent administrations of an anti-TRBV9 antibody at a dose of 630 mg every 12 months.

In some embodiments of the invention, the use comprises the first administration of an anti-TRBV9 antibody at a dose of 255 mg, the second administration of an anti-TRBV9 antibody at a dose of 630 mg after 9 months; the third administration of an anti-TRBV9 antibody at a dose of 630 mg after 9 months; or after 12 months; and the subsequent administrations of an anti-TRBV9 antibody at a dose of 630 mg every 12 months.

In some embodiments of the invention, the use comprises the first administration of an anti-TRBV9 antibody at a dose of 255 mg, the second administration of an anti-TRBV9 antibody at a dose of 630 mg after 12 months, the third administration of an anti-TRBV9 antibody at a dose of 630 mg after 12 months, and the subsequent administrations of an anti-TRBV9 antibody at a dose of 630 mg every 12 months.

In some embodiments of the invention, the use comprises the first administration of an anti-TRBV9 antibody at a dose of 245 mg, the second administration of an anti-TRBV9 antibody at a dose of 620 mg after at least 3 months, the third administration of an anti-TRBV9 antibody at a dose of 620 mg after at least 6 months, and the subsequent administrations of an anti-TRBV9 antibody at a dose of 620 mg at least every 12 months based on subject's body weight from 90 to 150 kg.

In some embodiments of the invention, the use comprises the first administration of an anti-TRBV9 antibody at a dose of 245 mg, the second administration of an anti-TRBV9 antibody at a dose of 620 mg after 3 months; the third administration of an anti-TRBV9 antibody at a dose of 620 mg after 6 months; or after 9 months; or after 12 months; and the subsequent administrations of an anti-TRBV9 antibody at a dose of 620 mg every 12 months based on subject's body weight from 90 to 150 kg.

In some embodiments of the invention, the use comprises the first administration of an anti-TRBV9 antibody at a dose of 245 mg, the second administration of an anti-TRBV9 antibody at a dose of 620 mg after 6 months; the third administration of an anti-TRBV9 antibody at a dose of 620 mg after 6 months; or after 9 months; or after 12 months; and the subsequent administrations of an anti-TRBV9 antibody at a dose of 620 mg every 12 months based on subject's body weight from 90 to 150 kg.

In some embodiments of the invention, the use comprises the first administration of an anti-TRBV9 antibody at a dose of 245 mg, the second administration of an anti-TRBV9 antibody at a dose of 620 mg after 9 months; the third administration of an anti-TRBV9 antibody at a dose of 620 mg after 9 months; or after 12 months; and the subsequent administrations of an anti-TRBV9 antibody at a dose of 620 mg every 12 months based on subject's body weight from 90 to 150 kg.

In some embodiments of the invention, the use comprises the first administration of an anti-TRBV9 antibody at a dose of 245 mg, the second administration of an anti-TRBV9 antibody at a dose of 620 mg after 12 months, the third administration of an anti-TRBV9 antibody at a dose of 620 mg after 12 months, and the subsequent administrations of an anti-TRBV9 antibody at a dose of 620 mg every 12 months based on subject's body weight from 90 to 150 kg.

In some embodiments of the invention, the use comprises the first administration of an anti-TRBV9 antibody at a dose of 250 mg, the second administration of an anti-TRBV9 antibody at a dose of 625 mg after at least 3 months, the third administration of an anti-TRBV9 antibody at a dose of 625 mg after at least 6 months, and the subsequent administrations of an anti-TRBV9 antibody at a dose of 625 mg at least every 12 months based on subject's body weight from 90 to 150 kg.

In some embodiments of the invention, the use comprises the first administration of an anti-TRBV9 antibody at a dose of 250 **mg,** the second administration of an anti-TRBV9 antibody at a dose of 625 mg after 3 months; the third administration of an anti-TRBV9 antibody at a dose of 625 mg after 6 months; or after 9 months; or after 12 months; and the subsequent administrations of an anti-TRBV9 antibody at a dose of 625 mg every 12 months based on subject's body weight from 90 to 150 kg.

In some embodiments of the invention, the use comprises the first administration of an anti-TRBV9 antibody at a dose of 250 mg, the second administration of an anti-TRBV9 antibody at a dose of 625 mg after 6 months; the third administration of an anti-TRBV9 antibody at a dose of 625 mg after 6 months; or after 9 months; or after 12 months; and the subsequent administrations of an anti-TRBV9 antibody at a dose of 625 mg every 12 months based on subject's body weight from 90 to 150 kg.

In some embodiments of the invention, the use comprises the first administration of an anti-TRBV9 antibody at a dose of 250 mg, the second administration of an anti-TRBV9 antibody at a dose of 625 mg after 9 months; the third administration of an anti-TRBV9 antibody at a dose of 625 mg after 9 months; or after 12 months; and the subsequent administrations of an anti-TRBV9 antibody at a dose of 625 mg every 12 months based on subject's body weight from 90 to 150 kg.

In some embodiments of the invention, the use comprises the first administration of an anti-TRBV9 antibody at a dose of 250 mg, the second administration of an anti-TRBV9 antibody at a dose of 625 mg after 12 months, the third administration of an anti-TRBV9 antibody at a dose of 625 mg after 12 months, and the subsequent administrations of an anti-TRBV9 antibody at a dose of 625 mg every 12 months based on subject's body weight from 90 to 150 kg.

In some embodiments of the invention, the use comprises the first administration of an anti-TRBV9 antibody at a dose of 255 mg, the second administration of an anti-TRBV9 antibody at a dose of 630 mg after at least 3 months, the third administration of an anti-TRBV9 antibody at a dose of 630 mg after at least 6 months, and the subsequent administrations of an anti-TRBV9 antibody at a dose of 630 mg at least every 12 months based on subject's body weight from 90 to 150 kg.

In some embodiments of the invention, the use comprises the first administration of an anti-TRBV9 antibody at a dose of 255 mg, the second administration of an anti-TRBV9 antibody at a dose of 630 mg after 3 months; the third administration of an anti-TRBV9 antibody at a dose of 630 mg after 6 months; or after 9 months; or after 12 months; and the subsequent administrations of an anti-TRBV9 antibody at a dose of 630 mg every 12 months based on subject's body weight from 90 to 150 kg.

In some embodiments of the invention, the use comprises the first administration of an anti-TRBV9 antibody at a dose of 255 mg, the second administration of an anti-TRBV9 antibody at a dose of 630 mg after 6 months; the third administration of an anti-TRBV9 antibody at a dose of 630 mg after 6 months; or after 9 months; or after 12 months; and the subsequent administrations of an anti-TRBV9 antibody at a dose of 630 mg every 12 months based on subject's body weight from 90 to 150 kg.

In some embodiments of the invention, the use comprises the first administration of an anti-TRBV9 antibody at a dose of 255 mg, the second administration of an anti-TRBV9 antibody at a dose of 630 mg after 9 months; the third administration of an anti-TRBV9 antibody at a dose of 630 mg after 9 months; or after 12 months; and the subsequent administrations of an anti-TRBV9 antibody at a dose of 630 mg every 12 months based on subject's body weight from 90 to 150 kg.

In some embodiments of the invention, the use comprises the first administration of an anti-TRBV9 antibody at a dose of 255 mg, the second administration of an anti-TRBV9 antibody at a dose of 630 mg after 12 months, the third administration of an anti-TRBV9 antibody at a dose of 630 mg after 12 months, and the subsequent administrations of an anti-TRBV9 antibody at a dose of 630 mg every 12 months based on subject's body weight from 90 to 150 kg.

In one aspect, the present invention relates to an anti-TRBV9 antibody for the treatment of a disease or disorder mediated by T-lymphocytes bearing the TRBV9 segment within the T cell receptor. The anti-TRBV9 antibody may be administered to a subject in need thereof at any dose described above, as well as using any dosing regimen described above.

The anti-TRBV9 antibody may be an antibody that specifically binds to the TRBV9 family beta chain. The anti-TRBV9 antibody may be a full-length antibody or antigen-binding fragment thereof that specifically binds to the TRBV9 family beta chain. The anti-TRBV9 antibody may be of different specificities (e.g. monospecific, bispecific antibody), different valencies (e.g. monovalent, bivalent, trivalent antibody), different formats (e.g. classical antibody, scFv, scFv-Fc, Minibody), different origins (e.g. murine, human, camel, chimeric antibody).

In some embodiments of the invention, the anti-TRBV9 antibody relates to an isolated monoclonal antibody.

In some embodiments of the invention, the anti-TRBV9 antibody relates to a monospecific antibody.

In some embodiments of the invention, the anti-TRBV9 antibody is a recombinant antibody.

In some embodiments of the invention, the anti-TRBV9 antibody comprises:
1) a heavy chain variable domain comprising:
   (a) HCDR1 having the amino acid sequence of SEQ ID NO: 1,
   (b) HCDR2 with the amino acid sequence of SEQ ID NO: 2, and
   (c) HCDR3 having an amino acid sequence selected from the group consisting of SEQ ID NO: 3, SEQ ID NO: 4, SEQ ID NO: 5 or SEQ ID NO: 6;
2) a light chain variable domain comprising:
   (a) LCDR1 having the amino acid sequence of SEQ ID NO: 7,
   (b) LCDR2 with the amino acid sequence of SEQ ID NO: 8, and
   (c) LCDR3 with the amino acid sequence of SEQ ID NO: 9.

In some embodiments of the invention, the anti-TRBV9 antibody comprises:
1) a heavy chain variable domain comprising:
   (a) HCDR1 having the amino acid sequence of SEQ ID NO: 1,
   (b) HCDR2 with the amino acid sequence of SEQ ID NO: 2, and
   (c) HCDR3 with the amino acid sequence of SEQ ID NO: 4;
2) a light chain variable domain comprising:
   (a) LCDR1 having the amino acid sequence of SEQ ID NO: 7,
   (b) LCDR2 with the amino acid sequence of SEQ ID NO: 8, and
   (c) LCDR3 with the amino acid sequence of SEQ ID NO: 9.

Antibodies according to the invention may be of any class (e.g. IgA, IgD, IgE, IgG, and IgM), or subclass (isotype) (e.g. IgG1, IgG2, IgG3, IgG4, IgA1 and IgA2).

In some embodiments of the invention, the anti-TRBV9 antibody is a full-length IgG antibody.

In some embodiments of the invention, the anti-TRBV9 antibody is of human IgG1, IgG2, IgG3 or IgG4 isotype.

In some embodiments of the invention, the anti-TRBV9 antibody comprises a heavy chain variable domain with the amino acid sequence of SEQ ID No: 14 and a light chain variable domain with the amino acid sequence of SEQ ID No: 17.

In some embodiments of the invention, the anti-TRBV9 antibody comprises a heavy chain variable domain with the amino acid sequence of SEQ ID No: 15 and a light chain variable domain with the amino acid sequence of SEQ ID No: 17.

In some embodiments of the invention, the anti-TRBV9 antibody is anti-TRBV9 antibody 180.

The anti-TRBV9 antibody 180 includes:
1) a heavy chain variable domain comprising:
   (a) HCDR1 having the amino acid sequence of SEQ ID NO: 1,
   (b) HCDR2 with the amino acid sequence of SEQ ID NO: 2, and
   (c) HCDR3 with the amino acid sequence of SEQ ID NO: 4;
2) a light chain variable domain comprising:
   (a) LCDR1 having the amino acid sequence of SEQ ID NO: 7,
   (b) LCDR2 with the amino acid sequence of SEQ ID NO: 8, and
   (c) LCDR3 with the amino acid sequence of SEQ ID NO: 9.

The anti-TRBV9 antibody 180 comprises a heavy chain variable domain with the amino acid sequence of SEQ ID No: 14 and a light chain variable domain with the amino acid sequence of SEQ ID No: 17.

The anti-TRBV9 antibody 180 comprises a heavy chain with the amino acid sequence of SEQ ID No: 22 and a light chain with the amino acid sequence of SEQ ID No: 25.

In some embodiments of the invention, the anti-TRBV9 antibody is an anti-TRBV9 antibody 43.

The anti-TRBV9 antibody 43 includes:
1) a heavy chain variable domain comprising:
   (a) HCDR1 having the amino acid sequence of SEQ ID NO: 1,
   (b) HCDR2 with the amino acid sequence of SEQ ID NO: 2, and
   (c) HCDR3 with the amino acid sequence of SEQ ID NO: 4;
2) a light chain variable domain comprising:
   (a) LCDR1 having the amino acid sequence of SEQ ID NO: 7,
   (b) LCDR2 with the amino acid sequence of SEQ ID NO: 8, and
   (c) LCDR3 with the amino acid sequence of SEQ ID NO: 9.

The anti-TRBV9 antibody 43 comprises a heavy chain variable domain with the amino acid sequence of SEQ ID No: 15 and a light chain variable domain with the amino acid sequence of SEQ ID No: 17.

The anti-TRBV9 antibody 43 comprises a heavy chain with the amino acid sequence of SEQ ID No: 23 and a light chain with the amino acid sequence of SEQ ID No: 25.

In some embodiments of the invention, the disease or disorder mediated by T-lymphocytes bearing a TRBV9 segment within the T cell receptor is selected from the group comprising: arthropathies, inflammatory bowel diseases, eye diseases, vasculitides, circulatory system diseases, kidney diseases, digestive system diseases, lymphoproliferative disorders.

In some embodiments of the invention, the disease or disorder mediated by T-lymphocytes bearing a TRBV9 segment within the T cell receptor is selected from the group comprising:
- arthropathies, in particular spondyloarthritides (radiographic axial spondyloarthritis (ankylosing spondylitis), nonradiographic axial spondyloarthritis, axial spondyloarthritis, peripheral spondyloarthritis, psoriatic arthritis, spondyloarthritis associated with inflammatory bowel diseases, reactive arthritis, undifferentiated peripheral spondyloarthritis), sacroiliitis associated with psoriasis, sacroiliitis associated with inflammatory bowel diseases, undifferentiated oligoarthropathy, juvenile spondylitis/enthesitis-related arthritis, juvenile ankylosing spondylitis (arthritis associated with enthesitis), juvenile arthritis, undifferentiated juvenile arthritis;
- inflammatory bowel diseases, in particular ulcerative colitis, Crohn's disease;
- eye diseases, in particular non-infectious uveitides, anterior uveitis;
- vasculitides, in particular Behcet's disease;
- circulatory system diseases, in particular aortitis, fibrosis of aortic and/or mitral valve leaflets with regurgitation, rhythm disturbances, conduction disturbances, left ventricular dysfunction, pericarditis, myocarditis;
- kidney diseases, in particular IgA nephropathy;
- digestive system diseases, in particular celiac disease;
- lymphoproliferative diseases, in particular T cell lymphoma, T cell leukemia.

In some embodiments of the invention, the disease or disorder mediated by T-lymphocytes bearing the TRBV9 segment within the T cell receptor is selected from the group comprising: spondyloarthritides, sacroiliitis associated with psoriasis, sacroiliitis associated with inflammatory bowel diseases, undifferentiated oligoarthropathy, juvenile spondylitis/enthesitis-related arthritis, juvenile ankylosing spondylitis (arthritis associated with enthesitis), juvenile arthritis, undifferentiated juvenile arthritis, ulcerative colitis, Crohn's disease, noninfectious uveitides, anterior uveitis, Behcet's disease, aortitis, fibrosis of aortic and/or mitral valve leaflets with regurgitation, rhythm disturbances, conduction disturbances, left ventricular dysfunction, pericarditis, myocarditis, IgA nephropathy, celiac disease, T cell lymphoma, T cell leukemia.

In some embodiments of the invention, the disease or disorder mediated by T-lymphocytes bearing the TRBV9 segment within the T cell receptor, for patients with no adequate response to standard therapy, is selected from the group comprising: spondyloarthritides, sacroiliitis associated with psoriasis, sacroiliitis associated with inflammatory bowel diseases, undifferentiated oligoarthropathy, juvenile spondylitis/enthesitis-related arthritis, juvenile ankylosing spondylitis (arthritis associated with enthesitis), juvenile arthritis, undifferentiated juvenile arthritis, ulcerative colitis, Crohn's disease, noninfectious uveitides, anterior uveitis, Behcet's disease, aortitis, fibrosis of aortic and/or mitral valve leaflets with regurgitation, rhythm disturbances, conduction disturbances, left ventricular dysfunction, pericarditis, myocarditis, IgA nephropathy, celiac disease, T cell lymphoma, T cell leukemia.

In some embodiments of the invention, the disease or disorder mediated by T-lymphocytes bearing the TRBV9 segment within the T cell receptor, for adult patients with no adequate response to standard therapy, is selected from the group comprising: spondyloarthritides, sacroiliitis associated with psoriasis, sacroiliitis associated with inflammatory bowel diseases, undifferentiated oligoarthropathy, juvenile spondylitis/enthesitis-related arthritis, juvenile ankylosing spondylitis (arthritis associated with enthesitis), juvenile arthritis, undifferentiated juvenile arthritis, ulcerative colitis, Crohn's disease, noninfectious uveitides, anterior uveitis, Behcet's disease, aortitis, fibrosis of aortic and/or mitral valve leaflets with regurgitation, rhythm disturbances, conduction disturbances, left ventricular dysfunction, pericarditis, myocarditis, IgA nephropathy, celiac disease, T cell lymphoma, T cell leukemia.

In some embodiments of the invention, spondyloarthritis is radiographic axial spondyloarthritis (ankylosing spondylitis), nonradiographic axial spondyloarthritis, axial spondyloarthritis, peripheral spondyloarthritis, psoriatic arthritis, spondyloarthritis associated with inflammatory bowel diseases, reactive arthritis, undifferentiated peripheral spondyloarthritis.

In some embodiments of the invention, spondyloarthritis is radiographic axial spondyloarthritis (ankylosing spondylitis) in patients with no adequate response to standard therapy, nonradiographic axial spondyloarthritis in patients with no adequate response to standard therapy, axial spondyloarthritis in patients with no adequate response to standard therapy, peripheral spondyloarthritis in patients with no adequate response to standard therapy, psoriatic arthritis in patients with no adequate response to standard therapy, spondyloarthritis associated with inflammatory bowel diseases in patients with no adequate response to standard therapy, reactive arthritis in patients with no adequate response to standard therapy, or undifferentiated peripheral spondyloarthritis in patients with no adequate response to standard therapy.

In some embodiments of the invention, spondyloarthritis is radiographic axial spondyloarthritis (ankylosing spondylitis) in adult patients with no adequate response to standard therapy, nonradiographic axial spondyloarthritis in adult patients with no adequate response to standard therapy, axial spondyloarthritis in adult patients with no adequate response to standard therapy, peripheral spondyloarthritis in adult patients with no adequate response to standard therapy, psoriatic arthritis in adult patients with no adequate response to standard therapy, spondyloarthritis associated with inflammatory bowel diseases in adult patients with no adequate response to standard therapy, reactive arthritis in adult patients with no adequate response to standard therapy, or undifferentiated peripheral spondyloarthritis in adult patients with no adequate response to standard therapy.

The anti-TRBV9 antibody may be administered as a single therapeutic agent or in combination with additional therapeutic agents as needed. Thus, in one embodiment, the present methods for treatment and/or prophylaxis are used in combination with administration of a therapeutically effective amount of other active agent. The other active agent may be administered before, during or following the administration of the pharmaceutical compositions according to the present invention. The other active agent may be administered as part of the present composition or, alternatively, as a separate formulation.

The anti-TRBV9 antibody may be administered in the form of a pharmaceutical composition.

The pharmaceutical compositions according to the present invention are typically suitable for parenteral administration as sterile formulations intended for administration to a human body through the breach in skin or mucosal barriers, bypassing the gastrointestinal tract by virtue of injection, infusion and implantation. In particular, it is contemplated that parenteral administration includes, inter alia, subcutaneous, intraperitoneal, intramuscular, intravenous, intraarterial, intrathecal, intraventricular, intraurethral, intracranial, intrasynovial, transdermal injection or infusion; and kidney dialytic infusion techniques. Preferred embodiments include intravenous and subcutaneous routes. Any method for administering peptides or proteins accepted in the art may be suitably employed for the composition of anti-TRBV9 antibody according to the present invention.

In some embodiments of the invention, said pharmaceutical composition of anti-TRBV9 antibody according to the present invention is intended for parenteral administration.

In some embodiments of the invention, said pharmaceutical composition of anti-TRBV9 antibody according to the present invention is intended for intramuscular, intravenous, or subcutaneous administration.

In some embodiments of the invention, said pharmaceutical composition of anti-TRBV9 antibody according to the present invention may be administered intravenously as an infusion.

The pharmaceutical composition of anti-TRBV9 antibody according to the present invention may be used after dilution. To this end, the required volume of the composition is transferred from a vial to an infusion container comprising a sterile 0.9% sodium chloride solution or a sterile 5% dextrose solution. The resulting solution is stirred by gently turning the infusion container over.

In some embodiments of the invention, the anti-TRBV9 antibody may be administered in the form of an aqueous pharmaceutical composition comprising:

| | |
|---|---|
| (i) anti-TRBV9 antibody; | |
| (ii) histidine buffer being histidine | a mixture of 0.517 mg/ml and 0.350 mg/ml; |
| histidine hydrochloride monohydrate | |
| (iii) osmotic agent being proline | |
| (iv) water for injection | 27 mg/ml; to 1 ml. |

In some embodiments of the invention, the anti-TRBV9 antibody is present at a concentration of 1.5 mg/ml, or 5.0 mg/ml, or 10.0 mg/ml, or 25.0 mg/ml, or 30.0 mg/ml, or 50.0 mg/ml, or 60.0 mg/ml, or 70.0 mg/ml, or 73.0 mg/ml, or 80.0 mg/ml, or 85.0 mg/ml, or 90.0 mg/ml, or 91.4 mg/ml, or 91.8 mg/ml, or 100.0 mg/ml, or 103.0 mg/ml, or 125.0 mg/ml, or 186.0 mg/ml, or 212.0 mg/ml.

In some embodiments of the invention, the anti-TRBV9 antibody may be administered in the form of an aqueous pharmaceutical composition comprising:

| | |
|---|---|
| (i) anti-TRBV9 antibody | 25 mg/ml; |
| (ii) histidine buffer being histidine | a mixture of 0.517 mg/ml and |
| histidine hydrochloride monohydrate | 0.350 mg/ml; |
| (iii) osmotic agent being proline | |
| (iv) water for injection | 27 mg/ml; to 1 ml. |

In some embodiments of the invention, the subject of treatment, or patient, is a mammal, preferably a human subject. Said subject may be either male or female, of any age.

### Implementation of the invention

The following examples are provided for better understanding of the invention. These examples are for purposes of illustration only and are not to be construed as limiting the scope of the invention in any manner.

All publications, patents, and patent applications cited in this specification are incorporated herein by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended embodiments.

The given formulations demonstrated excellent colloidal stability in the study of stability under accelerated storage (T = 37 °C), as well as a high monomer content followed by SE HPLC and stabilization of charge variant profile followed by IE HPLC.

The examples described below are given for the anti-TRBV9 antibody 180.

### Example 1. Study of specific activity following twice intravenous administration of anti-TRBV9 antibody 180 product to Macaca fascicularis on a collagen-induced arthritis model

The specific activity of the product was evaluated on a collagen-induced arthritis model in *Macaca fascicularis.* To induce the pathology, the animals were immunized twice with type II bovine collagen emulsion. Collagen was administered twice with a 28-day interval. The study was conducted on 12 male Macacas fascicularis. The anti-TRBV9 antibody 180 product was administered at doses of 0.2 and 1.0 mg/kg on days 29 and 50 of the study. The animals were observed for 78 days. Table 1 shows the groups of animals.

**Table 1- Groups of animals**

| Group no. | Animal qty | Product | Route of administratio n | Dose, mg/kg |
|---|---|---|---|---|
| 1 | 4 (♂) | anti-TRBV9 antibody 180 | intravenously | 0.2 |
| 2 | 4 (♂) | | | 1.0 |
| 3 | 4 (♂) | Placebo | | - |

Product efficacy was evaluated using the following indicators: tumor area percentage (TAP) in the metacarpophalangeal and metatarsophalangeal joints of all primate limbs, TRBV9 gene expression level in peripheral blood mononuclear cell fraction by RT PCR, alkaline phosphatase activity in the peripheral blood of primates, severity of histological changes in cartilage of the metacarpophalangeal and metatarsophalangeal joints according to the OARSI classification system [Pritzker, K., & Gay, S. (2006). Osteoarthritis cartilage histopathology: grading and staging // Osteoarthritis and cartilage. --2006. - Vol.14, No 1. - p.13-29].

The study results showed that, amid administration of anti-TRBV9 antibody 180, there were decreased values of TAP of the metacarpophalangeal and metatarsophalangeal joints of experimental animals in the 0.2 mg/kg and 1 mg/kg groups, indicating the anti-inflammatory activity of the product. The group receiving the maximum dose (1.0 mg/kg) showed the studied parameter at a statistically significantly lower level compared to the control group, starting from week 11 of the study.

The groups of animals that received anti-TRBV9 antibody 180 showed decreased relative number of TRBV9 transcripts in peripheral blood mononuclear cell fraction to 43% and 39% following the first administration and to 31% and 25% following the second administration of the product at doses of 0.2 mg/kg and 1.0 mg/kg, respectively.

The groups of animals that received the anti-TRBV9 antibody 180 product showed a tendency to decrease the level of alkaline phosphatase activity in blood serum. The maximum decrease in enzyme activity was observed in the group that received the anti-TRBV9 antibody 180 product at a dose of 1.0 mg/kg.

According to the OARSI classification system, histological changes in cartilage in the control group of animals corresponded to grade 3-5, and those in the groups of animals that received the anti-TRBV9 antibody 180 product corresponded to grade 2-3. The results of histological examination revealed that the anti-TRBV9 antibody 180 product promotes reduction of the damaged areas and the depth of cartilage cracks, as well as enhancement of proliferation processes regardless of the dose used.

Thus, the anti-TRBV9 antibody 180 product has an anti-inflammatory effect following being administered intravenously on a model of collagen-induced arthritis in *Macacas fascicularis. A* dose of the anti-TRBV9 antibody 180 product of 1.0 mg/kg is a pharmacologically active dose.

Example 2. Study of toxicity and local irritant action following repeated intravenous administration of anti-TRBV9 antibody 180 product to *Macacas fascicularis* for six weeks followed by a 20-week rest period.

The study of toxicity and local irritant action of the anti-TRBV9 antibody 180 product was carried out under repeated intravenous administration to Macacas fascicularis for 6 weeks. The recovery period was 20 weeks. The study was carried out on 40 animals (20 females, 20 males). The product was administered 1 time in 2 weeks at doses of 3 mg/kg, 10 mg/kg, 30 mg/kg. Table 2 shows the groups of animals.

**Table 2 - Groups of animals**

| Group no. | Animal qty | Product | Route of administration | Dose, mg/kg |
|---|---|---|---|---|
| 1 | 5 ♀ | anti-TRBV9 antibody | intravenously | 3 |
| | 5 ♂ | | | |
| 2 | 5 ♀ | | | 10 |
| | 5 ♂ | | | |
| 3 | 5 ♀ | | | 30 |
| | 5 ♂ | | | |
| 4 | 5 ♀ | Placebo | | - |
| | 5 ♂ | | | |

The study of toxicity involved evaluation of the following: general condition of animals (clinical examination), body weight dynamics, body temperature, hematological and biochemical parameters of blood serum, TRBV9⁺ T-lymphocyte content (pharmacodynamic marker), state of blood coagulation system, respiratory, cardiovascular and central nervous systems; pathomorphological studies were performed. The local irritant action was evaluated based on the results of macroscopic and histological examination of tissues at the site of administration and draining lymph nodes.

The study results showed that the anti-TRBV9 antibody 180 product at all test doses did not affect the main organs and organ systems of the primates following repeated intravenous administration for 6 weeks. The product did not exhibit a local irritant action. All groups of animals that received the product exhibited decreased levels of TRBV9⁺ T-lymphocytes versus the control group. No-observed-adverse-effect level (NOAEL) corresponded to the maximum test dose and was 30 mg/kg.

Example 3. Study of immunotoxicity following repeated intravenous administration of anti-TRBV9 antibody 180 product to Macacas fascicularis for six weeks followed by a 20-week rest period.

The study of immunotoxicity of the anti-TRBV9 antibody 180 product was carried out as part of the study of toxicity following repeated intravenous administration to Macacas fascicularis for 6 weeks. The recovery period was 20 weeks. The study was carried out on 40 animals (20 females, 20 males). The product was administered 1 time in 2 weeks at doses of 3 mg/kg, 10 mg/kg, 30 mg/kg. Table 3 shows the groups of animals.

**Table 3 - Groups of animals**

| Group no. | Animal qty | Product | Route of administration | Dose, mg/kg |
|---|---|---|---|---|
| 1 | 5 ♀ | anti-TRBV9 antibody | intravenously | 3 |
| | 5 ♂ | | | |
| 2 | 5 ♀ | | | 10 |
| | 5 ♂ | | | |
| 3 | 5 ♀ | | | 30 |
| | 5 ♂ | | | |
| 4 | 5 ♀ | Placebo | | - |
| | 5 ♂ | | | |

The study involved evaluation of the effect of the anti-TRBV9 antibody 180 product on the pattern of lymphocyte subpopulations (CD20⁺ B lymphocytes, CD3⁺ T lymphocytes, CD3⁺ CD4⁺ T helper cells, CD3⁺ CD8⁺ T killer cells, CD3⁻ CD16/56⁺ NK cells) and the levels of immunoglobulins A, G, M in the blood of the primates. Allergenic properties were studied by the dynamics of the level of immunoglobulin E; to assess the risk of delayed hypersensitivity, the proliferative activity of lymphocytes was evaluated.

The study results showed that the anti-TRBV9 antibody 180 product did not affect the pattern of lymphocyte subpopulations and the levels of immunoglobulins A, G, M. The evaluation of allergenic activity showed that the anti-TRBV9 antibody 180 product, following repeated intravenous administration, did not contribute to increased level of IgE and increased proliferative activity of lymphocytes. The resulting data indicate that the anti-TRBV9 antibody 180 product did not have a toxic effect on the immune system of Macacas fascicularis.

Example 4. Study of pharmacokinetics and immunogenicity following repeated intravenous administration of anti-TRBV9 antibody 180 product to *Macacas fascicularis* for six weeks followed by a 20-week rest period.

The study of pharmacokinetics and immunogenicity of the anti-TRBV9 antibody 180 product was carried out as part of the study of toxicity following repeated intravenous administration to Macacas fascicularis for 6 weeks. The recovery period was 20 weeks. The study was performed on 30 animals (15 females, 15 males). The product was administered 1 time in 2 weeks at doses of 3 mg/kg, 10 mg/kg, 30 mg/kg. Table 4 shows the groups of animals.

**Table 4 - Groups of animals**

| Group no. | Animal qty | Product | Route of administration | Dose, mg/kg |
|---|---|---|---|---|
| 1 | 5 ♀ | anti-TRBV9 antibody | intravenously | 3 |
| | 5 ♂ | | | |
| 2 | 5 ♀ | | | 10 |
| | 5 ♂ | | | |
| 3 | 5 ♀ | | | 30 |
| | 5 ♂ | | | |

The concentration of anti-TRBV9 antibody 180 and the level of antibodies binding the anti-TRBV9 antibody 180 product (BAbs) in the blood serum of primates were evaluated using a solid-phase enzyme-linked immunosorbent assay. Based on experimental data, the values of AUC₍₀₋₃₃₆₎, AUCₛₛ₍₆₇₂₋₁₀₀₈₎, C_{max,ss}, C_{min,ss}, C_{av,ss} and cumulation factor (R) were calculated. It should be noted that the values of the parameters characterizing the systemic exposure (AUC₍₀₋₃₃₆₎, AUCₛₛ₍₆₇₂₋₁₀₀₈₎, C_{av,ss}) were directly dependent on the dose used, which indicates the linearity of pharmacokinetics of the anti-TRBV9 antibody 180 product.

According to the results, BAbs against the anti-TRBV9 antibody 180 product were detected in 43% of experimental animals receiving the product at doses of 3, 10 and 30 mg/kg. The values of the cumulation factor (R) did not depend on the product dose used and were 1.53±0.36, 1.91±0.70 and 1.94± 0.71 following administration of doses of 3 mg/kg, 10 mg/kg and 30 mg/kg, respectively.

Example 5. An open-label single-arm study of pharmacodynamics (PD), pharmacokinetics (PK), safety and immunogenicity of the anti-TRBV9 antibody 180 product following a single administration thereof at graded doses to healthy volunteers.

Purpose: to study the pharmacodynamics, pharmacokinetics, safety and immunogenicity of the anti-TRBV9 antibody 180 product following a single intravenous administration thereof at graded doses to healthy volunteers.

Methodology: In terms of design, clinical study no. BCD-180-1 is an open-label single-arm study of pharmacodynamics, pharmacokinetics, tolerability, safety and immunogenicity of the anti-TRBV9 antibody 180 product following a single administration thereof at graded doses to healthy volunteers. The study involved the escalation of doses of the anti-TRBV9 antibody 180 product in 7 cohorts. Shown are the results of analysis of data from the main period (57 days) and follow-up period (361 days) of the clinical study.

The anti-TRBV9 antibody 180 product was administered to: 19 subjects.
In Cohort 1: 1 subject,
In Cohort 2: 3 subjects,
In Cohort 3: 3 subjects,
In Cohort 4: 3 subjects,
In Cohort 5: 3 subjects,
In Cohort 6: 3 subjects,
In Cohort 7: 3 subjects.

Diagnosis and main selection criteria: healthy HLA-B27+ and HLA-B27- male volunteers aged 18 to 45 years inclusive, who have not previously taken medications that affect the immune system, including other monoclonal antibody products with immunosuppressive effects, without a burdened allergic history, including indications for other significant adverse reactions following administration of any therapeutic products.

Inclusion of an HLA-B27+ subject in Cohort 1 was mandatory. Inclusion of at least one subject with HLA-B27+ in each of the further cohorts (2-7) was mandatory. Table 5 describes the cohorts of subjects.

**Table 5 - Cohorts of subjects**

| Cohort | Dose of anti-TRBV9 antibody product | Number of subjects included |
|---|---|---|
| 1 | 0.03 mg/kg | 1 |
| 2 | 0.3 mg/kg | 3 |
| 3 | 1 mg/kg | 3 |
| 4 | 2 mg/kg | 3 |
| 5 | 3 mg/kg | 3 |
| 6 | 5 mg/kg | 3 |
| 7 | 7 mg/kg | 3 |

Duration of administration of the studied product: the studied anti-TRBV9 antibody 180 product was administered to subjects once.

Evaluation criteria:
Endpoints for evaluating pharmacokinetics:
   - Cₘₐₓ - the maximum concentration of the product;
   - Tₘₐₓ - time to Cₘₐₓ;
   - T_{1/2} - the half-life of the product;
   - Kₑₗ - the constant of the elimination rate of the product;
   - Cl - total clearance;
   - V_{d} - volume of product distribution;
   - Cₘᵢₙ - the minimum concentration of the product following administration thereof;
   - AUC₀₋₁₃₄₄ - area under concentration-time curve from the moment of product administration to Day 57 (1344 hours);
   - AUC₀₋₂₀₁₆ - area under concentration-time curve from the moment of product administration to Day 85 (2016 hours);
   - AUC_{0-∞} - area under concentration-time curve from the moment of product administration to infinity.
Endpoints for evaluating pharmacodynamics:
   - Eₘᵢₙ - minimum value of the effect (Eₘᵢₙ - minimum value of relative abundance of TRBV9+ T-lymphocytes in human peripheral blood. Units - %);
   - Tₘᵢₙ - time to Eₘᵢₙ.
Endpoints for evaluating safety:
   - Proportion of subjects with adverse reactions;
   - Proportion of subjects with serious adverse reactions;
   - Proportion of subjects with adverse reactions of grade 3 or above according to CTCAE 5.0 classification;
   - Proportion of subjects who prematurely discontinued participation in the study due to the development of adverse reactions.
Endpoints for immunogenicity evaluation:
   - Proportion of BAb- and NAb-positive subjects;
   - Titer of binding and/or neutralizing antibodies.

### Results of pharmacodynamics evaluation.

The pharmacodynamics of the anti-TRBV9 antibody 180 product was evaluated based on the determination of the relative number (relative abundance) of T-lymphocytes comprising the TRBV9 gene by quantitative PCR. The TRBV9 gene abundance was determined versus the TRBC (T Cell Receptor Beta Constant) gene. As an additional method for evaluating pharmacodynamics, the relative number of T-lymphocytes expressing the TRBV9 protein was determined by flow cytometry. The absolute number of TRBV9+CD8+ and TRBV9+CD4+ T-lymphocytes was calculated based on the absolute number of lymphocytes determined in a clinical blood test and the relative number of T-lymphocyte subpopulations determined by flow cytometry.

Determination of relative number of T-lymphocytes comprising the TRBV9 gene by quantitative PCR.

Following a single intravenous administration of the anti-TRBV9 antibody 180 product, all cohorts showed a decrease in the mean values observed of the relative abundance of TRBV9+ T lymphocytes.

In the 0.03 mg/kg Cohort, the values of the studied parameter were below the lower limit of quantification (LLOQ) (i.e., less than 1% of the number of TRBC) on Visit 2 (Day 4) and on Visit 3 (Day 8). On Visit 4 (Day 15), an increase in the relative abundance of TRBV9+ T-lymphocytes was observed, indicating the beginning of repopulation of the target clone of lymphocytes in a given subject. Mean relative abundance of TRBV9+ T-lymphocytes, comparable to the baseline, was achieved on Visit 7 (day 57) .

On Visit 1 (Day 2), the 0.3 mg/kg Cohort showed an increase in the mean values of the studied parameter relative to the baseline; on Visit 2 (Day 4) and on Visit 3 (Day 8), it showed a gradual decrease thereof to zero ones, and on subsequent visits, it showed a subsequent gradual increase in the mean values of the parameter to the level that does not reach the baseline. The beginning of repopulation of TRBV9+ T-lymphocytes was observed on Visit 4 (Day 15), and the mean parameter value approximately comparable to the baseline was achieved on Visit 10 (Day 253).

In the 1 mg/kg Cohort, the mean values of the studied parameter reached zero values on Visit 3 (Day 8) and on Visit 4 (Day 15), as well as on Visit 6 (Day 43). On Visit 5 (Day 29) and on Visit 7 (Day 57), there was an increase in the mean values of the parameter to a level not reaching the baseline. Starting from Visit 7 (Day 57), there were fluctuations in the mean parameter values, with achievement of a level slightly higher than the baseline on the last visit, Visit 11 (Day 361). In the 2 mg/kg Cohort, following administration of the anti-TRBV9 antibody 180 product, a decrease in the mean values of relative abundance of TRBV9+ T lymphocytes was observed. During the observation period, the mean value of relative abundance of TRBV9+ T-lymphocytes did not reach the parameter baseline.

In the 3 mg/kg Cohort, the mean values of relative abundance of TRBV9+ T-lymphocytes decreased on Visit 1 (Day 2), reached zero level on Visit 1 (Day 3) and were maintained at this level until the end of the main period. The beginning of the increase in the mean value of relative abundance of TRBV9+ T-lymphocytes was reported on Visit 8 (Day 85). On Visit 9 (Day 169), in this cohort, pharmacodynamics evaluation data was available for only one subject who stood out with a pronounced increase in the relative abundance of TRBV9+ T-lymphocytes to 7.67 at this point. On this visit, this subject had an increase in the leukocyte count to 9.7x10⁹/l, which was reported as an adverse event (AE), "leukocytosis", whereas the levels of lymphocytes, neutrophils, monocytes were within the reference values. It is not possible to evaluate the further dynamics of the parameters in this subject because of subject's early termination of participation after a protocol violation.

In the 5 mg/kg and 7 mg/kg Cohorts, the dynamics of the level of the pharmacodynamic marker was identical showing a rapid decrease in the relative abundance of TRBV9+ T-lymphocytes within 24 hours following infusion of the anti-TRBV9 antibody 180 product. In the 5 mg/kg Cohort, the beginning of increase in the mean value of relative abundance of TRBV9+ T-lymphocytes was observed on Visit 8 (Day 85). Further, the individual values of the parameter in two out of three subjects in this cohort remained below LLOQ on Visit 8. Achievement of baseline of the mean parameter value was observed on Visit 11 (Day 361). In the 7 mg/kg Cohort, the beginning of increase in the mean value of relative abundance of TRBV9+ T-lymphocytes was observed on Visit 8 (Day 85); this parameter did not reach the baseline before the end of the study.

Table 6 shows descriptive statistics of the values of relative abundance of TRBV9+ T-lymphocytes in peripheral blood for cohorts.

HLA-B27- and HLA-B27+ subjects showed similar dynamics of the mean value of relative abundance of TRBV9+ T-lymphocytes throughout the study.

Based on the resulting data, the pharmacodynamic parameters Emin (the minimum value of the relative abundance of TRBV9+ T-lymphocytes in peripheral blood) and Tmin (time to Emin) were calculated.

In all cohorts, the minimum value of relative abundance of TRBV9+ T-lymphocytes in peripheral blood, Emin, was zero. The fastest achievement of Emin, i.e. the lowest Tmin values, was observed in the 3 mg/kg, 5 mg/kg and 7 mg/kg Cohorts, in which the median Tmin was 27.5 h, 27 h and 27.8 h, respectively.

Determination of number of lymphocytes expressing TRBV9 protein by flow cytometry.

Following a single intravenous injection of the anti-TRBV9 antibody 180 product, CD8+TRBV9+ T-lymphocyte depletion was reported in all cohorts. The beginning of increase in the CD8+TRBV9+ T-lymphocyte count relative to the total CD8+ T-lymphocyte count (and, accordingly, increase in the absolute value of the parameter) was observed on Visit 5 (Day 29) in the 0.03 mg/kg Cohort, on Visit 8 (Day 85) in the 0.3 mg/kg Cohort, on Visit 9 (Day 169) in the 1 mg/kg Cohort, on Visit 10 (Day 253) in the 2 mg/kg, 5 mg/kg and 7 mg/kg Cohorts. In all cohorts, the mean values of relative and absolute CD8+TRBV9+ T-lymphocyte count remained significantly lower than the baseline throughout the study.

Following a single intravenous injection of the anti-TRBV9 antibody 180 product, CD4+TRBV9+ T-lymphocyte depletion was reported in all cohorts. The level of CD4+TRBV9+ T lymphocytes remained below LLOQ in all cohorts throughout the study.

**Table 6. Descriptive statistics on values of relative abundance of TRBV9+ T-lymphocytes in peripheral blood for cohorts**

| Cohort Statistic s | Day 1, prior to admin istra tion | Day 2, 24 hours follo wing admin istra tion | Day 3, 48 hours follo wing admin istra tion | Day 4, 72 hours follo wing admin istra tion | Day 8, Day 8 follo wing admin istra tion | Day 15±2 | Day 29±2 | Day 43±2 | Day 57±2 | Day 85±2 | Day 169±2 | Day 253±2 | Day 361±2 |
|---|---|---|---|---|---|---|---|---|---|---|---|---|---|
| Anti-TRBV9 antibody 180 product 0.03 mg/kg | | | | | | | | | | | | | |
| N | 1 | 1 | 1 | 1 | 1 | 1 | 1 | 1 | 1 | 1 | 1 | 1 | 1 |
| Median, [L. - H. quartiles ] | 1.000 [1.00 - 1.00] | 0.910 [0.91 - 0.91] | 0.040 [0.04 - 0.04] | 0.000 [0.00 - 0.00] | 0.000 [0.00 - 0.00] | 0.030 [0.30 - 0.30] | 0.970 [0.97 - 0.97] | 0.840 [0.84 - 0.84] | 0.910 [0.91 - 0.91] | 1, 890 [1.89 - 1.89] | 2,370 [2.37 - 2.37] | 2,820 [2.82 - 2.82] | 1.810 [1.81 - 1.81] |
| Minimum-Maximum | 1.00-1.00 | 0.91-0.91 | 0.04-0.04 | 0.00-0.00 | 0.00-0.00 | 0.03-0.03 | 0.97-0.97 | 0.84-0.84 | 0.91-0.91 | 1,89-1,89 | 2,37-2, 37 | 2,82-2,82 | 1,81-1,81 |
| Anti-TRBV9 antibody 180 product 0.3 mg/kg | | | | | | | | | | | | | |
| N | 3 | 3 | 3 | 3 | 2 | 2 | 3 | 2 | 2 | 1 | 2 | 2 | 2 |
| Median, [L. - H. quartiles ] | 1.000 [1.00 - 1.00] | 1,090 [1.00 - 1.51] | 0.700 [0.49 - 1.08] | 0.000 [0.00 - 0.00] | 0.000 [0.00 - 0.00] | 0.140 [0.07 - 0.21] | 0.000 [0.00 - 0.22] | 0.175 [0.09 - 0.26] | 0.460 [0.23 - 0.69] | 0.000 [0.00 - 0.00] | 0.970 [0.61 - 1.34] | 0.995 [0.62 - 1.37] | 1.810 [1.31 - 2.32] |
| Minimum - Maximum | 1,00 - 1,00 | 0.91 1,92 | 0.28 1, 46 | 0,00 -0,00 | 0.00-0.00 | 0.00 -0.28 | 0.00 -0.44 | 0.00-0.35 | 0.00 -0.92 | 0,00 -0,00 | 0.24 -1,70 | 0.25 -1,74 | 0.80 -2,82 |
| Anti-TRBV9 antibody 180 product 1 mg/kg | | | | | | | | | | | | | |
| N | 3 | 3 | 3 | 3 | 3 | 3 | 3 | 3 | 3 | 3 | 3 | 2 | 2 |
| Median, [L. - H. quartiles ] | 1.000 [1.00 1.00] | 0.320 [0.16 0.98] | 0.210 [0.19 0.52] | 0.000 [0.00 0.34] | 0.000 [0.00 0.00] | 0.000 [0.00 0.00] | 0.280 [0.14 0.29] | 0.000 [0.00 0.00] | 0.250 [0.13 0.27] | 0.170 [0.13 0.20] | 0.290 [0.23 1.05] | 0.345 [0.28 0.41] | 1.310 [1.07 1.55] |
| Minimum - Maximum | 1,00 -1,00 | 0.00 -1, 64 | 0.16 -0.82 | 0.00 -0.68 | 0.00 -0.00 | 0,00 -0,00 | 0.00 -0.30 | 0,00 -0,00 | 0,00 -0,28 | 0.08 -0.23 | 0.17 -1,80 | 0.22 -0.47 | 0.83 -1,79 |
| Anti-TRBV9 antibody 180 product 2 mg/kg | | | | | | | | | | | | | |
| N | 3 | 3 | 3 | 3 | 3 | 2 | 2 | 3 | 3 | 3 | 3 | 3 | 3 |
| Median, [L. - H. quartiles ] | 1.000 [1.00 - 1.00] | 0.030 [0.02 - 0.44] | 0.000 [0.00 - 0.10] | 0.120 [0.06 - 0.16] | 0.000 [0.00 - 0.18] | 0.170 [0.09 - 0.26] | 0.090 [0.05 - 0.14] | 0.000 [0.00 - 0.10] | 0.130 [0.07 - 0.20] | 0.280 [0.14 - 0.30] | 0.170 [0.13 - 0.22] | 0.240 [0.22 - 0.32] | 0.790 [0.79 - 0.86] |
| Minimum - Maximum | 1.00-1.00 | 0.00 -0.84 | 0.00-0.20 | 0.00-0.20 | 0.00-0.36 | 0.00-0.34 | 0.00-0.18 | 0.00-0.20 | 0.00-0.27 | 0.00-0.32 | 0.09-0.27 | 0.20-0.40 | 0.79-0.93 |
| Anti-TRBV9 antibody 180 product 3 mg/kg | | | | | | | | | | | | | |
| N | 3 | 3 | 3 | 3 | 3 | 3 | 3 | 3 | 3 | 3 | 1 | 0 | 0 |
| Median, [L. - H. quartiles ] | 1.000 [1.00 1.00] | 0.000 [0.00 0.29] | 0.000 [0.00 0.00] | 0.000 [0.00 0.00] | 0.000 [0.00 0.00] | 0.000 [0.00 0.00] | 0.000 [0.00 0.00] | 0.000 [0.00 0.00] | 0.000 [0.00 0.00] | 0.660 [0.33 0.69] | 7.670 [7.67 7.67] | NA [NA] | NA [NA] |
| Minimum - Maximum | 1,00 -1,00 | 0.00-0.58 | 0,00-0,00 | 0, 00-0,00 | 0,00-0,00 | 0,00-0,00 | 0, 00-0,00 | 0,00-0,00 | 0,00-0,00 | 0.00-0.71 | 7, 67-7, 67 | NA | NA |
| Anti-TRBV9 antibody 180 product 5 mg/kg | | | | | | | | | | | | | |
| N | 3 | 3 | 3 | 3 | 3 | 3 | 3 | 3 | 3 | 3 | 3 | 3 | 3 |
| Median, [L. - H. quartiles ] | 1.000 [1.00 - 1.00] | 0.000 [0.00 - 0.00] | 0.000 [0.00 - 0.00] | 0.000 [0.00 - 0.00] | 0.000 [0.00 - 0.00] | 0.000 [0.00 - 0.00] | 0.000 [0.00 - 0.00] | 0.000 [0.00 - 0.00] | 0.000 [0.00 - 0.00] | 0.000 [0.00 - 0.05] | 0.140 [0.12 - 0.42] | 0.100 [0.09 - 0.39] | 1.050 [0.74 - 1.82] |
| Minimum - Maximum | 1.00-1.00 | 0,00-0,00 | 0, 00-0,00 | 0, 00-0,00 | 0,00-0,00 | 0,00-0,00 | 0, 00-0,00 | 0,00-0,00 | 0,00 -0,00 | 0.00 -0.09 | 0.09-0.70 | 0.07-0.67 | 0.43-2.59 |
| Anti-TRBV9 antibody 180 product 7 mg/kg | | | | | | | | | | | | | |
| N | 3 | 3 | 3 | 3 | 3 | 3 | 3 | 3 | 3 | 3 | 3 | 3 | 3 |
| Median, [L. - H. quartiles ] | 1.000 [1.00 - 1.00] | 0.000 [0.00 - 0.00] | 0.000 [0.00 - 0.00] | 0.000 [0.00 - 0.00] | 0.000 [0.00 - 0.00] | 0.000 [0.00 - 0.00] | 0.000 [0.00 - 0.00] | 0.000 [0.00 - 0.00] | 0.000 [0.00 - 0.00] | 0.290 [0.22 - 0.59] | 0.810 [0.50 - 1.16] | 0.530 [0.37 - 0.82] | 0.590 [0.59 - 0.95] |
| Minimum - Maximum | 1,00 - 1,00 | 0,00 -0,00 | 0,00-0,00 | 0,00 -0,00 | 0,00 -0,00 | 0,00 -0,00 | 0,00 -0,00 | 0,00-0,00 | 0,00-0,00 | 0.14 -0.89 | 0.19 -1.50 | 0.20-1,10 | 0.58-1,30 |
| *(N - number of observations at a certain point)* | | | | | | | | | | | | | |

Results of pharmacokinetics evaluation.

Pharmacokinetics was evaluated based on data on the concentration of the anti-TRBV9 antibody 180 product in the blood serum of subjects at time points determined by the protocol, followed by the calculation of standard pharmacokinetic parameters.

Cₘₐₓ, AUC₀₋₁₃₄₄, AUC_{0-∞} were shown to be dose-dependent: the values of the parameters increased with escalation of an administered dose. Other parameters were not directly dose-dependent. The half-life ranged from 21.7 hours in the 0.03 mg/kg Cohort to 401.8 hours in the 7 mg/kg Cohort, time to the maximum concentration ranged from 0.7 hours in the 0.03 mg/kg Cohort to 9.5 hours in the 3 mg/kg Cohort according to the results of the main study period (57 days); and from 29.78 hours in the 0.03 mg/kg Cohort to 405.76 h in the 3 mg/kg Cohort, time to the maximum concentration ranged from 0.68 h in the 0.03 mg/kg Cohort to 10.99 h in the 3 mg/kg Cohort according to the main period and follow-up period (1 year).

Results of immunogenicity evaluation.

Immunogenicity of the anti-TRBV9 antibody 180 product was determined by analyzing blood serum samples of subjects for the presence of binding antibodies (BAbs) and/or neutralizing antibodies (NAbs) and determining the titer thereof.

Within the analyzed period, BAbs to anti-TRBV9 antibody 180 were detected in 2 out of 18 (11.1%) subjects included in the immunogenicity analysis. In a subject in the 0.03 mg/kg Cohort, BAbs were detected twice: on Visit 6 (Day 43) and on Visit 7 (Day 57), with a titer of 5.4 and 4.3, respectively. In a subject in the 0.3 mg/kg Cohort, BAbs were detected on Visit 7 (Day 57) with a titer of 1.8. The analysis demonstrated the absence of NAbs in these subjects.

### Results of efficacy evaluation:

During the analyzed study period, an acceptable safety profile was observed in all cohorts. Administration of the product was tolerated by the subjects satisfactorily. During the analyzed period, all subjects in each cohort demonstrated at least one AE, including that associated with the study product. Serious AEs, AEs of grade 3 and higher according to CTCAE 5.0 were absent. Predominantly, AEs of grade 1 were observed; AEs of grade 2 were also reported. DLT (dose-limiting toxicity) was not observed. There were no cases of early withdrawal from the study for safety reasons.

**Table 7 - Endpoints for safety evaluation. Safety population**

| Endpoints | anti-TRBV9 antibodi es 1800.03 mg/kg (N = 1)n (%) | anti-TRBV9 antibodi es 1800.3 mg/kg (N = 3)n (%) | anti-TRBV9 antibodi es 1801 mg/kg (N = 3)n (%) | anti-TRBV9 antibodi es 1802 mg/kg (N = 3)n (% ) | anti-TRBV9 antibodi es 1803 mg/kg (N = 3)n (%) | anti-TRBV9 antibodi es 1805 mg/kg (N = 3)n (%) | anti-TRBV9 antibodi es 180 7 mg/kg (N = 3)n (%) | Tota 1 (N = 19 n (%) |
|---|---|---|---|---|---|---|---|---|
| Proportion of subjects with AEs, including serious AEs | 1 (100) | 3 (100) | 3 (100) | 3 (100) | 3 (100) | 3 (100) | 3 (100) | 19 (100 ) |
| Proportion of subjects with ARs | 1 (100) | 3 (100) | 3 (100) | 3 (100) | 3 (100) | 3 (100) | 3 (100) | 19 (100 ) |
| Proportion of subjects with SARs | 0 | 0 | 0 | 0 | 0 | 0 | 0 | 0 |
| Proportion of subjects with ARs of grade 3 or more according to CTCAE 5.0 | 0 | 0 | 0 | 0 | 0 | 0 | 0 | 0 |
| Proportion of subjects who prematurely discontinue d participati on in study due to development of ARs | 0 | 0 | 0 | 0 | 0 | 0 | 0 | 0 |
| Note: AEs - adverse events, ARs - adverse reactions, SARs - serious adverse reactions. AR is defined as an AE with a "certain", "probable" or "possible" degree of relation to the study product. | | | | | | | | |

The spectrum of AEs was analyzed both in the general population for safety evaluation (19 subjects) and with cohort breakdown.

Among AEs in organs and systems during the analyzed study period, the most common AEs observed (in more than 5 subjects out of 19, in descending order of frequency) were AEs represented by deviations of investigations; blood and lymphatic system disorders; injury, poisoning and procedural complications; metabolism and nutrition disorders. AEs related to other deviations were observed with a lower frequency.

AEs represented by deviations of investigations were reported in 17 out of 19 (89.5%) subjects. In the 1 mg/kg and 3 mg/kg Cohorts, these AEs were reported in 2 out of 3 (66.7%) subjects; in other cohorts, they were reported in all (100%) subjects. The most frequent AEs of this group (in descending order) were the following AEs: "increase in relative urine density" in 12 out of 19 (63%) subjects, "presence of protein in urine" in 5 out of 19 (26%) subjects, "deviation from normal urinalysis results" in 4 out of 19 (21%) subjects, "decreased blood creatinine levels" in 4 out of 19 (21.1%), "increased alanine aminotransferase levels" and "increased blood creatinine level" in 3 out of 19 subjects (15.8%). Other AEs represented by investigations were reported in 3 out of 19 (15.8%) subjects.

Blood and lymphatic system disorders were reported in 16 out of 19 (84%) subjects. AEs of this group were reported in all subjects in the 0.03 mg/kg, 1 mg/kg, 2 mg/kg and 3 mg/kg Cohorts; in the remaining cohorts, they were reported in 2 out of 3 subjects (66.7%). In the general population for safety evaluation, "leukocytosis", "neutrophilia" and "anemia" were reported most frequently, in 13 (68%), 12 (63%) and 7 (37%) subjects, respectively. Other AEs of this group were reported in 2 (11%) subjects or less.

Infusion reactions were reported in only 15 out of 19 (79%) subjects, whereas in the 1 mg/kg - 7 mg/kg Cohorts they were reported in all 3 (100%) subjects in each cohort. No infusion reactions were reported in the 0.03 mg/kg and 0.3 mg/kg Cohorts.

The most frequent manifestation of infusion reactions was fever reported in all 15 (79%) subjects with infusion reactions. Tachycardia was reported a little less frequently, in 12 (63%) cases; headache, nausea were reported in 10 (53%) cases. Vomiting was reported in 7 (37%) subjects. Infusion reactions were represented by hypotension, myalgia, fever, rash in 4 (21%) subjects and less. Weakness, abdominal cramping, discomfort in the chest or abdomen, erythema were reported in individual cases.

Metabolism and nutrition disorders were reported in 7 out of 19 (37%) subjects. These AEs were observed in no more than 2 (67%) subjects in each of the cohorts, with the exception of the 0.03 mg/kg and 2 mg/kg Cohorts, in which AEs of this class were not reported. Hypoproteinemia was reported in 6 (32%) cases; one case of hyperglycemia and one case of hyperproteinemia were reported.

AEs which were regarded by the researcher as product-associated AEs were reported in 100% of subjects in each of the cohorts. Most frequently, association with the administration of the studied product was reported for disorders of the blood and lymphatic system (in 16 (84%) subjects), investigations (in 14 (74%) subjects) and metabolic and nutritional disorders (in 7 (37%) subjects). All infusion reactions were associated with the administration of the study product according to the researchers (15 (79%)). Other AEs associated with the administration of the study product were reported in less than 6 (32%) subjects.

The reported AEs were generally expected for protein (monoclonal antibody)-based products, the mechanism of action of which is realized through the depletion of lymphocytes administered intravenously.

### Conclusion:

The results of the analysis allow to draw the following conclusions:
- The anti-TRBV9 antibody 180 product has a satisfactory safety profile:
   - no clear dependence of safety parameters on a dose when administering doses above 1 mg/kg,
   - no DLT, serious AEs and AEs of grade ≥3 (CTCAE 5.0),
   - most AEs were AEs of grade 1, less frequently AEs were AEs of grade 2,
   - the most frequent AEs were deviations of investigations and blood and lymphatic system disorders, and infusion reactions,
   - the most common symptoms of infusion reactions were: fever, headache, nausea, vomiting, tachycardia,
   - there were no cases of early withdrawal from the study for safety reasons.
   - Pharmacodynamics of the anti-TRBV9 antibody 180 product:
   - single administration of the anti-TRBV9 antibody 180 product at doses from 0.03 mg/kg to 7 mg/kg induces depletion of TRBV9+ lymphocytes in healthy volunteers,
   - doses of the anti-TRBV9 antibody 180 product of 5 mg/kg and 7 mg/kg provide for the deepest depletion of TRBV9+ lymphocytes during the analyzed period, 57 days following infusion.
   - Pharmacokinetics of the anti-TRBV9 antibody 180 product:
   - dose dependence of PK parameters (Cmax, AUC0-1344, AUC0-∞).

Example 6. Prediction of pharmacokinetics and pharmacodynamics profiles following single and multiple administration of anti-TRBV9 antibody 180 to healthy volunteers according to clinical study no. BCD-180-1 "Open-Label, Non-controlled Study of Pharmacodynamics, Pharmacokinetics, Safety, and Immunogenicity of Single Escalating Doses of BCD-180 (CJSC "BIOCAD", Russia) in Healthy Volunteers".

PK was evaluated according to the data of Phase I Clinical Study No. BCD-180-1 based on the determination of the concentration of a monoclonal antibody to TRBV9 in blood serum by enzyme-linked immunosorbent assay (ELISA).

The model was developed using data of flow cytometry conducted as part of PD evaluation in Phase I clinical study No. BCD-180-1. The number of lymphocytes bearing on the surface thereof a T-cell receptor of the TRBV9 family was determined.

### Model description:

The developed mathematical model is a QSP (Quantitative Systems Pharmacology) model and considers not only the effect of PK on PD, but also the effect of PD on PK [Azer, K.; Kaddi, C. D.; Barrett, J. S.; Bai, J. P. F.; McQuade, S. T.; Merrill, N. J.; Piccoli, B.; Neves-Zaph, S.; Marchetti, L.; Lombardo, R.; Parolo, S.; Immanuel, S. R. C.; Baliga, N. S. History and Future Perspectives on the Discipline of Quantitative Systems Pharmacology Modeling and Its Applications. Front. Physiol. 2021, 12, 637999. https://doi.org/10.3389/fphys.2021.637999].

Figure 1 shows the scheme of the main processes considered in the model. The model consists of three compartments as follows: central compartment, peripheral compartment and "lymphocyte depot". The central compartment describes the processes occurring in the blood following administration of the anti-TRBV9 antibody 180 product: intravenous infusion of the product, interaction of the anti-TRBV9 antibody 180 product with target cells, elimination of the product from the body. The peripheral compartment is an analogue of the part of the body to where the product is distributed in addition to blood. The lymphocyte depot is a compartment that represents the conditional volume of distribution of all TRBV9+ cells outside the blood. The interaction between the anti-TRBV9 antibody 180 product and target cells takes place only in the central compartment. Of the possible mechanisms of action evaluated in *in vitro* experiments and reported in "Evaluation of potentially possible mechanisms of action of anti-TRBV9 antibody 180 *in vitro*", the model implemented two main mechanisms: receptor internalization and antibody-dependent cellular cytotoxicity (ADCC). According to the report, they are the main mechanisms of action of the anti-TRBV9 antibody 180 product in blood that affect the PK and PD of the product. ADCC occurs through the formation of a receptor-antibody complex on the surface of TRBV9+ T-lymphocytes. Antibody-opsonized lymphocytes are eliminated from the body using effector cells - NK cells and CD16+monocytes [Lo Nigro, C.; Macagno, M.; Sangiolo, D.; Bertolaccini, L.; Aglietta, M.; Merlano, M. C. NK-Mediated Antibody-Dependent Cell-Mediated Cytotoxicity in Solid Tumors: Biological Evidence and Clinical Perspectives. Ann. Transl. Med 2019, 7 (5), 105-105. https://doi.org/10.21037/atm.2019.01.42; Yeap, W. H.; Wong, K. L.; Shimasaki, N.; Teo, E. C. Y.; Quek, J. K. S.; Yong, H. X.; Diong, C. P.; Bertoletti, A.; Linn, Y. C.; Wong, S. C. CD16 Is Indispensable for Antibody-Dependent Cellular Cytotoxicity by Human Monocytes. Sci Rep 2016, 6 (1), 34310. https://doi.org/10.1038/srep34310]. They bear, on the surface thereof, FcgRIIIa that is responsible for activation thereof. The number of effector cells in the model is constant. This assumption has been made because the number thereof exceeds that of target cells by orders of magnitude. The mathematical expression of ADCC considering changes in the number of target cells versus product concentration is based on the publication by Hoffman et al. [Hoffman, F.; Gavaghan, D.; Osborne, J.; Barrett, I. P.; You, T.; Ghadially, H.; Sainson, R.; Wilkinson, R. W.; Byrne, H. M. A Mathematical Model of Antibody-Dependent Cellular Cytotoxicity (ADCC). Journal of Theoretical Biology 2018, 436, 39-50. https://doi.org/10.1016/j.jtbi.2017.09.031].

### Conclusions

PK and PD of the ANTI-TRBV9 ANTIBODY 180 product were modeled based on data from the main period of Phase I Clinical Study no. BCD-180-1. Based on the modeling results, the prediction of PK and PD profiles following administration to subjects of the anti-TRBV9 antibody 180 product at doses ranging from 0.03 to 7 mg/kg was generated.

Model parameters were calibrated using optimization methods so that the model solutions were close to real data. For each cohort of Phase I Clinical Study no. BCD-180-1, a profile of anti-TRBV9 antibody 180 product concentration and the TRBV9+ T lymphocyte count was built, which predicts the available data from Phase I Clinical Study no. BCD-180-1. Overestimated predictions of concentrations for the 0.03 mg/kg and 0.3 mg/kg dose cohorts follow from the description of product clearance caused by the TMDD effect using Michaelis-Menten kinetics.

Based on the PK-PD modeling, numerical values and the timing of expected repopulation of target cells versus dose and dosing regimen were predicted.

The resulting data may serve as the basis for the following conclusions:
- when administered once every 6 months, the 5 mg/kg and 7 mg/kg doses of the anti-TRBV9 antibody 180 product provide the deepest depletion of target cells in blood;
- when using a step-by-step dosing schedule of the anti-TRBV9 antibody 180 product, a 12-week interval between the first administration of the product at half of the dose prescribed for a group, i.e. 2.5 mg/kg and 3.5 mg/kg for the 5 mg/kg and 7 mg/kg anti-TRBV9 antibody 180 groups, respectively, and the second infusion of the product at the full dose is contemplated to be optimal for the estimated repopulation time of target cells, based on PK and PD modeling;
- the results of PK and PD modeling of the anti-TRBV9 antibody 180 product (5 mg/kg or 7 mg/kg) demonstrate that the product's dosing regimen: administration of half of the dose at week 0, administration of the full dose at weeks 12, 36, 60, 84, 108, 132 and 156, provide deep depletion of TRBV9+ T-lymphocytes;
- the results of PD modeling of the anti-TRBV9 antibody 180 product, in particular the expected degree of repopulation of target cells following 60 weeks of therapy, make it possible to consider a rarer administration of the anti-TRBV9 antibody 180 product after week 60 of therapy, which seems more convenient for patients: administration of half of the dose of the anti-TRBV9 antibody 180 product on week 0, subsequent administration of the full dose of the product on weeks 12, 36, 84 and 132.

Example 7. An international multi-center, double-blind, randomized, placebo-controlled study of efficacy, safety, pharmacokinetics and pharmacodynamics of anti-TRBV9 antibody 180 product in patients with axial spondyloarthritis.

### Study tasks:

Primary task:
   - To determine the efficacy parameters of two doses of the anti-TRBV9 antibody 180 product and compare them with those of placebo;
Secondary tasks:
   - To study and compare to placebo parameters the profile of adverse reactions following using two doses of the anti-TRBV9 antibody 180 product;
   - To determine the immunogenicity of the anti-TRBV9 antibody 180 product (generation of binding (BAbs) and neutralizing antibodies (NAbs) to the anti-TRBV9 antibody 180 product and determination of titer thereof)
   - To determine the main parameters of pharmacokinetics of the anti-TRBV9 antibody 180 product;
   - To determine the main parameters of pharmacodinamics of the anti-TRBV9 antibody 180 product;

### Study design:

Clinical study no. BCD-180-2/ELEFTA is a multi-center, double-blind, randomized placebo-controlled clinical study of efficacy, safety, immunogenicity, pharmacokinetics and pharmacodynamics of two doses of anti-TRBV9 antibody 180 product compared with placebo in patients with active radiographic axial spondyloarthritis (axSpA), phase II.

The study includes HLA-B27+ patients with active radiographic axSpA (ankylosing spondylitis, AS) with ineffective previous treatment with nonsteroidal anti-inflammatory drugs (NSAIDs), who have not previously received biological therapy and targeted basic anti-inflammatory drugs (BAIDs).

The study includes the following periods:
Screening period (no more than 28 days);
Main period (week 0 - week 36);
Continuation therapy and follow-up period (week 37 - week 160).
The subjects were randomized 2:2:1 in 3 groups:
   - anti-TRBV9 antibody 180 product 5 mg/kg;
   - anti-TRBV9 antibody 180 product 7 mg/kg;
   - Placebo.

Main period (week 0 - week 36)
The main period starts on week 0 and will continue until week 36 inclusive. Within this period, the therapy is blinded for subjects and researchers (except for one or more authorized researchers).

The first infusion of the anti-TRBV9 antibody 180 product is carried out using ½ (half) of the dose prescribed for the group, subsequent infusions is carried out using the full dose prescribed for the group.

Subjects of the 5 mg/kg anti-TRBV9 antibody 180 and 7 mg/kg anti-TRBV9 antibody 180 groups will receive infusions of the anti-TRBV9 antibody 180 product on weeks 0, 12, 36 and a placebo infusion on week 24 in order to maintain blinding.

Subjects of the placebo group will receive placebo infusions on weeks 0, 12 and anti-TRBV9 antibody 180 product infusions on weeks 24 and 36.

The therapy continuation and follow-up period will start on week 37 and continue until week 160 inclusive. Within this period, the therapy will be blinded for subjects and researchers (except for one or more authorized researchers).

In the 5 mg/kg and 7 mg/kg anti-TRBV9 antibody 180 groups, the dosing regimen of the anti-TRBV9 antibody 180 product is determined by the presence/absence of the criterion for inactive radiographic axSpA in a subject. The compliance of subject's condition with the criterion for inactive radiographic axSpA is assessed on week 60. In the present clinical study, the criterion for inactive radiographic axSpA is ASDAS-CRP <1.3, i.e.:
- Subjects with ASDAS-CRP <1.3 on weeks 48 and 60 are considered to have achieved the status of inactive radiographic axSpA.
   In each of the 5 mg/kg anti-TRBV9 antibody 180 and 7 mg/kg anti-TRBV9 antibody 180 groups, subjects meeting the above criterion for inactive radiographic axSpA is subjected to another randomization (approximately in the ratio of 1:1) in two groups for the use of two different dosing regimens (once every 6 months and once every 12 months).
- Subjects of the 5 mg/kg anti-TRBV9 antibody 180 "A" and 7 mg/kg anti-TRBV9 antibody 180 "A" groups will receive infusions of anti-TRBV9 antibody 180 at a dose prescribed for the group in a once/12 months dosing regimen: on weeks 84 and 132; in order to maintain blinding, the subjects will receive placebo infusions on weeks 60, 108 and 156.
- Subjects of the 5 mg/kg anti-TRBV9 antibody 180 "B" and 7 mg/kg anti-TRBV9 antibody 180 "B" groups will receive infusions of the anti-TRBV9 antibody 180 product at a dose prescribed for the group in a once/6 months dosing regimen: on weeks 60, 84, 108, 132 and 156.
- In each of the 5 mg/kg / 7 mg/kg anti-TRBV9 antibody 180 groups, subjects who do not meet said criterion for inactive radiographic axSpA (5 mg/kg anti-TRBV9 antibody 180 "C" and 7 mg/kg anti-TRBV9 antibody 180 "C" groups) will continue to receive the anti-TRBV9 antibody 180 product at a dose prescribed for the group once every 6 months on weeks 60, 84, 108, 132 and 156.
- Subjects of the Placebo group will receive infusions of the anti-TRBV9 antibody 180 product at a dose of 5 mg/kg on weeks 60, 84, 108, 132 and 156.

Studied population and main selection criteria for study inclusion

The study includes HLA-B27+ patients with active radiographic axSpA (AS) with ineffective previous treatment with NSAIDs, who have not previously received biological therapy and targeted BAIDs.
Number of study subjects
Randomize: at least 250 subjects.
Mode of administration

The anti-TRBV9 antibody 180 product/placebo was administered to subjects as an intravenous infusion in a predetermined volume based on the visit, at a predetermined rate and prior premedication, by an authorized medical officer of the research site in the settings of a specialized medical institution. Anti-TRBV9 antibody 180/placebo was prepared for administration to a subject in the research site by an unblinded authorized researcher.
Duration of therapy: 156 weeks.

### Evaluation criteria.

| Study tasks | Study endpoints |
|---|---|
| To determine the efficacy parameters of two doses of the anti-TRBV9 antibody 180 product and compare them with those of placebo | Primary endpoint: |
| | Proportion of subjects who reached ASAS40 on week 24. |
| | Secondary endpoints: |
| | •Proportion of subjects who achieved disease activity status according to ASDAS-CRP: |
| | -inactive <1.3, |
| | -moderate activity (≥1.3 - <2.1), |
| | -high activity (≥2.1 - ≤3.5), |
| | -very high activity (>3.5); |
| | •Proportion of subjects who achieved ASDAS-CII (clinically important improvement) defined as a decrease from baseline in the ASDAS score ≥1.1; |
| | •Proportion of subjects who achieved ASDAS-MI (major improvement) defined as a decrease from baseline in the ASDAS score ≥2.0; |
| | •Change from baseline in the ASDAS-CRP score; |
| | •Proportion of subjects who achieved a clinical response defined as an improvement in the BASDAI score by at least 50% compared to the baseline; |
| | •Change from the baseline in the BASDAI score; |
| | •Proportion of subjects who achieved ASAS40; |
| | •Proportion of subjects who achieved ASAS20; |
| | •Proportion of subjects who achieved ASAS5/6; |
| | •Proportion of subjects who achieved ASAS partial remission; |
| | •Change from the baseline in the ASAS NSAID score; |
| | •Change from the baseline in the value of chest excursion; |
| | •Change from the baseline in the BASMI score; |
| | •Change from the baseline in the BASFI score; |
| | •Change from the baseline in swollen joint count (out of 44); |
| | •Change from the baseline in the MASES score; |
| | •Change from the baseline in the assessment of total back pain (BASDAI No. 2); |
| | •Change from the baseline in the assessment of nighttime back pain; |
| | •Change from the baseline in the patient's assessment of disease activity; |
| | ▪Change from the baseline in the assessment of quality of life according to the EQ-5D-3L questionnaire; |
| | •Change from the baseline in the score of SF-36 physical component summary; |
| | •Change from the baseline in the score of SF-36 mental component summary; |
| | ▪Change from the baseline in the WPAI score; |
| | ▪Change from the baseline in the ASAS HI score; |
| | •Change from the baseline in CRP concentration; |
| | •Change from the baseline in ESR level; |
| | •Change from the baseline in the SPARCC score; |
| | •Change from the baseline in the mSASSS score; |
| | •Proportion of subjects without signs of radiographic progression of the disease. |
| To study and compare to placebo parameters the profile of adverse reactions following using two doses of the anti-TRBV9 antibody 180 product | Endpoints for safety evaluation |
| | • Proportion of subjects with adverse reactions; |
| | • Proportion of subjects with serious adverse reactions; |
| | • Proportion of subjects with adverse reactions of grade 3 or above according to CTCAE 5.0 classification; |
| | • Proportion of subjects who prematurely discontinued participation in the study due to the development of adverse reactions; |
| | • Proportion of subjects with adverse events of special interest. |
| To determine the immunogenicity of the anti-TRBV9 antibody 180 product (generation of binding (BAbs) and neutralizing antibodies (NAbs) to the anti-TRBV9 antibody 180 product and determination of titer thereof) | Endpoints for immunogenicity evaluation: |
| | • Proportion of BAb- and NAb-positive subjects; |
| | • BAbs and/or NAbs titer. |
| To determine the main parameters of pharmacokinetics of the anti-TRBV9 antibody 180 product | Endpoints for pharmacokinetics evaluation: Cₘₐₓ, AUC_{0-12w}, C_{predose}, Tₘₐₓ, T_{1/2}, Kₑₗ, Cl, V_{d}, AUC_{0-∞}. |
| To determine the main parameters of pharmacodinamics of the anti-TRBV9 antibody 180 product | Endpoints for pharmacokinetics evaluation: Eₘᵢₙ, Tₘᵢₙ, E_{predose}. |

### Investigational hypothesis

The following null hypothesis will be tested: there are no differences between the groups of different doses of the anti-TRBV9 antibody 180 product (5 mg/kg and 7 mg/kg) and placebo versus the following alternative hypothesis: at least one of the doses of the anti-TRBV9 antibody 180 product is superior over placebo on week 24 according to the primary endpoint of efficacy (Proportion of subjects who achieved ASAS40 on week 24). The study outcome will be considered positive if the null hypothesis is rejected.

According to the protocol, after 30% of total randomized subjects completed 24 weeks of the study, an efficacy and safety gap assessment for the studied therapy was carried out

Evaluation criteria within the analyzed period.

At each evaluation point within the analyzed period:
- Proportion of subjects who achieved disease activity status according to ASDAS-CRP
   ∘ inactive <1.3,
   ∘ moderate activity (≥1.3 - <2.1),
   ∘ high activity (≥2.1 - ≤3.5),
   ∘ very high activity (>3.5);
- Proportion of subjects who achieved ASAS40;
- Proportion of subjects who achieved ASAS20;
- Proportion of subjects who achieved ASAS5/6;
- Proportion of subjects who achieved ASAS partial remission;
- Change from the baseline in the BASDAI score;
- Change from the baseline in the value of chest excursion;
- Change from the baseline in the BASMI score;
- Change from the baseline in the BASFI score;
- Change from the baseline in swollen joint count (out of 44);
- Change from the baseline in the MASES score;
- Change from the baseline in the assessment of total back pain (BASDAI No. 2);
- Change from the baseline in the assessment of nighttime back pain;
- Change from the baseline in the patient's assessment of disease activity;
- Change from the baseline in CRP concentration;
- Change from the baseline in ESR level.

### Endpoints for safety evaluation

- Proportion of subjects with adverse reactions;
- Proportion of subjects with serious adverse reactions;
- Proportion of subjects with adverse reactions of grade 3 or above according to CTCAE 5.0 classification;
- Proportion of subjects who prematurely discontinued participation in the study due to the development of adverse reactions;
- Proportion of subjects with adverse events of special interest.

Preliminary safety data were obtained for 252 subjects who received at least one infusion of the studied anti-TRBV9 antibody 180 product/placebo, out of 269 randomized subjects:
in the 5 mg/kg anti-TRBV9 antibody 180 group: 104 subjects,
in the 7 mg/kg anti-TRBV9 antibody 180 group: 99 subjects,
in the Placebo group: 49 subjects.

Also provided are preliminary efficacy data for 73 subjects who completed 24 weeks of the study, out of 269 randomized subjects:
in the 5 mg/kg anti-TRBV9 antibody 180 group: 25 subjects,
in the 7 mg/kg anti-TRBV9 antibody 180 group: 28 subjects,
in the Placebo group: 20 subjects.

14 subjects discontinued the study.

The results of the interim data analysis, according to the primary efficacy parameter, demonstrate that the proportion of subjects who achieved ASAS40 on week 24 in the 7 mg/kg anti-TRBV9 antibody 180 treatment group was 2 times greater than that in the group of subjects who received placebo (60.7% and 30.0%, respectively), exceeding the allowable differences in this parameter (24%) between groups in sample size calculation. Accordingly, on week 24, 12/25 (48.0%) subjects in the 5 mg/kg anti-TRBV9 antibody 180 group, 17/28 (60.7%) subjects in the 7 mg/kg anti-TRBV9 antibody 180 group, and 6/20 (30.0%) subjects in the placebo group/5 mg/kg achieved ASAS 40 (Table 8).

For such efficacy parameters as the proportion of subjects who achieved ASAS20 and the proportion of subjects who achieved ASAS5/6, as well as for change from baseline in BASMI and BASFI scores, change from baseline in assessment of total back pain (BASDAI No. 2), change from baseline in assessment of nighttime back pain, as well as the main laboratory parameter reflecting disease activity, change from baseline in CRP concentration, anti-TRBV9 antibody 180 at a dose of 7 mg/ kg demonstrated higher efficacy on week 24.

Considering secondary efficacy parameters, the results also give grounds to the efficacy of anti-TRBV9 antibody 180 at the both studied doses, 5 mg/kg and 7 mg/kg (Table 8).

**Table 8. Main results of interim efficacy analysis. mITT population**

| Efficacy parameter | anti-TRBV9 antibodies 180 5 mg/kg (N = 25) n (%) | anti-TRBV9 antibodies 180 7 mg/kg (N = 28) n (%) | Placebo / anti-TRBV9 antibodies 180 5 mg/kg (N = 20) n (%) | Total (N = 73) n (%) |
|---|---|---|---|---|
| ASAS40 (week 24) | 12 (48,0) | 17 (60,7) | 6 (30,0) | 35 (47,9) |
| Disease activity status | | | | |
| Inactive (ASDAS-CRP <1.3) | 3 (12,0) | 2 (7,1) | 1 (5,0) | 6 (8,2) |
| Moderate activity (ASDAS-CRP >= 1.3 and ASDAS-CRP < 2.1) | 7 (28,0) | 10 (35,7) | 6 (30,0) | 23 (31,5) |
| High activity (ASDAS-CRP >= 2.1 and ASDAS-CRP <= 3.5) | 9 (36,0) | 10 (35,7) | 5 (25,0) | 24 (32,9) |
| Very high activity (ASDAS-CRP > 3.5) | 6 (24,0) | 5 (17,9) | 7 (35,0) | 18 (24,7) |
| ASAS20 (week 24) | 12 (48,0) | 16 (57,1) | 4 (20,0) | 32 (43,8) |
| ASAS partial remission (week 24) | 5 (20,0) | 4 (14,3) | 2 (10,0) | 11 (15,1) |
| Change from the baseline in the BASDAI score (week 24) | | | | |
| Median | -3, 60 | -2.80 | -1.90 | -2.80 |
| [L. - H. quartile] | [-4.60, - 2.00] | [-4.10, - 1.65] | [-4.05, - 0.80] | [-4.20, -1.70] |
| Change from the baseline in the value of chest excursion (week 24) | | | | |
| Median | 0.00 | 0,50 | 0.00 | 0.20 |
| [L. - Up. quartile] | [-0.50, 1.00] | [0.00, 1.00] | [-0.50, - 0.80] | [-0.50, 1.00] |
| Change from the baseline in the BASMI score (week 24) | | | | |
| Median | -0.40 | -0,60 | -0,20 | -0.40 |
| [L. - Up. quartile] | [-0.80, 0.00] | [-1.00, - 0.40] | [-0.60, - 0.00] | [-0.80, 0.00] |
| Change from the baseline in the BASFI score (week 24) | | | | |
| Median | -1.90 | -2,15 | -1,55 | -1.90 |
| [L. - Up. quartile] | [-3.50, - 0.60] | [-3.65,-0.50] | [-2.65, - 0.50] | [-3.50, -0.40] |
| Change from the baseline in swollen joint count (out of 44) (week 24) | | | | |
| Median | 0.0 | 0.0 | 0.0 | 0.0 |
| [L. - Up. quartile] | [-2.0, 0.0] | [-1.0, 0.0] | [-1.0,- 0.0] | [-1.0, 0.0] |
| Change from the baseline in the MASES score (week 24) | | | | |
| Median | -2.0 | -1.0 | -1.0 | -1.0 |
| [L. - Up. quartile] | [-4.0, 0.0] | [-3.0, 0.0] | [-2.0, -0.0] | [-3.0, 0.0] |
| Change from the baseline in the assessment of total back pain (BASDAI No. 2) (week 24) | | | | |
| Median | -3.0 | -4.0 | -2,5 | -3.0 |
| [L. - Up. quartile] | [-5.0, - 2.0] | [-5.0, - 2.0] | [-4.5, -1.0] | [-5.0, - 1.0] |
| Change from the baseline in the assessment of nighttime back pain (week 24) | | | | |
| Median | -2.0 | -4,5 | -3.0 | -3.0 |
| [L. - Up. quartile] | [-4.0, - 1.0] | [-5.5, - 1.0] | [-4.0, -0.0] | [-5.0, - 1.0] |
| Change from the baseline in the patient's assessment of disease activity (week 24) | | | | |
| Median | -3.0 | -3.0 | -2.0 | -3.0 |
| [L. - Up. quartile] | [-4.0, - 2.0] | [-5.0, - 1.0] | [-4.0,-1.5] | [-5.0, - 1.0] |
| Change from the baseline in CRP concentration (week 24) | | | | |
| Median | -5.220 | -10,090 | -0,720 | -5.220 |
| [L. - Up. quartile] | [-15.63, - 0.25] | [-16.94, - 0.32] | [-14.91,-2.04] | [-15.63, 0.03] |
| Change from the baseline in ESR level (week 24) | | | | |
| Median, | -14, 0 | -4.0 | -1.0 | -10,0 |
| [L. - Up. quartile] | [-28.0, - 2] | [-15.0, 0.0] | [-25.0, -8.0] | [-25.0, 2.0] |

Accordingly, the evaluation of the interim analysis data on week 24 of the study showed that the proportion of subjects who achieved the primary efficacy parameter ASAS40 in the 7 mg/kg anti-TRBV9 antibody 180 treatment group was 2 times greater than that in the group of subjects who received placebo; also, data of secondary efficacy parameters (the proportion of patients who achieved ASAS20 on week 24, change from baseline in chest excursion on week 24, change from baseline in the BASFI score on week 24, change from baseline in assessment of total back pain (BASDAI No. 2) on week 24, change from baseline in assessment of total nighttime back pain on week 24, change from baseline in patient's assessment of disease activity on week 24, change from baseline in CRP concentration on week 24) give grounds to efficacy of anti-TRBV9 antibody 180 in the both studied doses, 5 mg/kg and 7 mg/kg.

Accordingly, according to the interim data evaluation, the patients with axial spondyloarthritis amid the use of anti-TRBV9 antibody 180 for 24 weeks showed clinical changes in the course of the disease, as well as a decrease in functional limitations.

### Safety.

In the present study, the interim analysis also included evaluation of safety parameters in patients with axial spondyloarthritis. Preliminary safety data were obtained for 252 subjects who received at least one infusion of the studied anti-TRBV9 antibody 180 product/placebo, out of 269 randomized subjects: 104 subjects in the 5 mg/kg anti-TRBV9 antibody 180 group, 99 subjects in the 7 mg/kg anti-TRBV9 antibody 180 group, 49 subjects in the placebo group. The groups were comparable in basic demographic and anthropometric characteristics.

In view of the fact that subjects randomized to the Placebo group began receiving the anti-TRBV9 antibody 180 product on week 24 of the study, the data analysis on safety endpoints includes division into AEs that occurred before week 24 (the period of the study during which the subjects of the Placebo group received placebo) and AEs that occurred on or after week 24 (when the subjects of the Placebo group received anti-TRBV9 antibody 180).

During the period until week 24, a total of 160/252 (63.5%) of subjects included in the safety analysis had any AEs (Table 9). The predominated ARs were ARs of grades 1-2. During the period until week 24, grade 3 ARs were reported in less than 5% of subjects in the group: 4/99 (4.0%) subjects in the 5 mg/kg anti-TRBV9 antibody 180 group and 5/104 (4.8%) subjects in the 7 mg/kg anti-TRBV9 antibody 180 group. Serious ARs were reported in 3/99 (3.0%) subjects in the 5 mg/kg anti-TRBV9 antibody 180 group and in 1/104 (1.0%) subjects in the 7 mg/kg anti-TRBV9 antibody 180 group.
Serious ARs were reported in 3/99 (3.0%) subjects in the 5 mg/kg anti-TRBV9 antibody 180 group and in 2/104 (1.9%) subjects in the 7 mg/kg anti-TRBV9 antibody 180 group.

The following AEs of special interest were subject to reporting: infusion reactions, first-time diagnosis or exacerbation of extra-skeletal manifestations of axSpA (acute uveitis, IBDs (Crohn's disease, ulcerative colitis), psoriasis, cardiac conduction disorders, aortitis, etc., IgA nephropathy). The most frequently reported AEs (≥5% of subjects included in the safety analysis) were AEs related to the following classes of organ systems: injuries, intoxications and procedural complications, general disorders and reactions at the injection site, investigations, and skin and subcutaneous tissue disorders.

**Table 9. Adverse events that occurred before week 24. Safety population**

| AE categories | anti-TRBV9 antibodies 180 5 mg/kg (N = 99) n (%) | anti-TRBV9 antibodies 1807 mg/kg (N = 104) n (%) | Placebo / anti-TRBV9 antibodies 180 5 mg/kg (N = 49) n (%) | Total (N = 252) n (%) |
|---|---|---|---|---|
| Any AEs | 72 (72,7) | 75 (72,1) | 13 (26,5) | 160 (63,5) |
| Any ARs | 67 (67,7) | 71 (68,3) | 5 (10,2) | 143 (56,7) |
| Serious ARs | 3 (3,0) | 1 (1.0) | 0 | 4 (1.6) |
| ARs of grade 3 and higher according to CTCAE 5.0 | 4 (4.0) | 5 (4.8) | 0 | 9 (3.6) |
| ARs that caused early termination of participation in the study | 3 (3,0) | 2 (1.9) | 0 | 5 (2.0) |
| AEs of special interest | 49 (49.5) | 54 (51.9) | 1 (2.0) | 104 (41.3) |
| Note: AE - adverse event, AR - adverse reaction. The table includes adverse events that occurred before the start of product infusion on week 24. | | | | |

During the period on or after week 24 of the study, ARs were reported in 1/104 (1.0%) subjects in the 7 mg/kg anti-TRBV9 antibody 180 group and in 7/49 (14.3%) subjects in the Placebo/5 mg/kg anti-TRBV9 antibody 180 group. No serious ARs were reported during this period. ARs of grade 3 or higher according to CTCAE 5.0 were reported in 1/49 (2.0%) subject in the Placebo/5 mg/kg anti-TRBV9 antibody 180 group. AEs of special interest were registered in 6/49 (12.2%) subjects in the Placebo/5 mg/kg anti-TRBV9 antibody 180 group. AEs that could be the reason for early termination of participation in the study were not reported during this period.

**Table 10. Adverse events that occurred on or after week 24. Safety population**

| AE categories | anti-TRBV9 antibodies 180 5 mg/kg (N = 99) n (%) | anti-TRBV9 antibodies 180 7 mg/kg (N = 104) n (%) | Placebo / anti-TRBV9 antibodies 180 5 mg/kg (N = 49) n (%) | Total(N = 252) n (%) |
|---|---|---|---|---|
| Any AEs | 0 | 1 (1.0) | 7 (14,3) | 8 (3.2) |
| Any ARs | 0 | 1 (1.0) | 7 (14,3) | 8 (3.2) |
| Serious ARs | 0 | 0 | 0 | 0 |
| ARs of grade 3 and higher according to CTCAE 5.0 | 0 | 0 | 1 (2.0) | 1 (0,4) |
| ARs that caused early termination of participation in the study | 0 | 0 | 0 | 0 |
| AEs of special interest | 0 | 0 | 6 (12,2) | 6 (2,4) |
| Note: AE - adverse event, AR - adverse reaction. The table includes adverse events that occurred on or after the start of product infusion on week 24. | | | | |

All injuries, poisoning and procedural complications during the analyzed period were represented by infusion reactions. During the period before week 24, in most cases, infusion reactions corresponded to grade 1 or 2 according to CTCAE 5.0. Infusion reactions of grade 3 were reported in 1/99 (1.0%) and 4/104 (3.8%) subjects in the groups receiving 5 mg/kg and 7 mg/kg, respectively. The most common manifestations of infusion reactions were fever, headache, chills, nausea, and vomiting.

During the period from week 24, these ARs for the anti-TRBV9 antibody 180 product were reported in 6/49 (12.2%) subjects in the placebo/5 mg/kg group: grade 1 in 1/49 (12.2%) subject, grade 2 in 4/49 (8.2%) subjects, and grade 3 in 1/49 (12.2%) subject. Manifestations of infusion reactions of grades 1-2 included fever, vomiting, headache, other symptoms were observed in individual cases. The symptom of the infusion reaction of grade 3 was redness.

General disorders and reactions at the injection site were reported for the anti-TRBV9 antibody 180 product during the period before week 24 in 4/99 (4.0%) subjects in the 5 mg/kg group and in 12/104 (11.5%) subjects in the 7 mg/kg group. These ARs were represented by pyrexia of grades 1-2, hyperthermia of grade 1, and asthenia of grade 1.

During the period before week 24, deviations in investigations for the anti-TRBV9 antibody 180 product were reported in 7/99 (7.1%) subjects in the 5 mg/kg group, 6/104 (5.8%) subjects in the 7 mg/kg group and 2/49 (4.1%) subjects in the Placebo/5 mg/kg group. These ARs were represented by ARs of grades 1-2. According to the results of an interim data evaluation, no cases of AEs of grade 4 and fatal AEs were reported in the study of the anti-TRBV9 antibody 180 product. Most ARs corresponded to ARs of grades 1 and 2.

The proportion of subjects with severe ARs (grade 3 or higher) reported before week 24 for the anti-TRBV9 antibody 180 product was 4.0% (4/99) in the 5 mg/kg group and 4.8% (5/104) in the 7 mg/kg group; the proportion of subjects with severe ARs reported on or after week 24 was 2.0% (1/49) in the Placebo/5 mg/kg group.

All registered severe ARs corresponded to ARs of grade 3. The severe ARs reported before week 24 for the anti-TRBV9 antibody 180 product included infusion reaction, anemia, neutropenia, dermatitis, hypertension. During the period on or after week 24, 1 severe AR was reported, which was represented by an infusion reaction of grade 3 in 1/49 (2.0%) subject in the Placebo/5 mg/kg group.

Thus, based on an interim safety and efficacy evaluation of the present clinical study of the use of anti-TRBV9 antibody 180 in patients with axial spondyloarthritis, it can be concluded that the use of anti-TRBV9 antibody 180 at the both studied doses, 5 mg/kg and 7 mg/kg, demonstrate an acceptable safety profile and tolerability; the both doses were characterized by comparable safety parameters when evaluated by endpoints and AR profile.

### Example 8. Study of flat doses of anti-TRBV9 antibody 180 product.

Clinical study (CS) no. BCD-180-3/LEVENTA investigates flat doses of the anti-TRBV9 antibody 180 product by intravenous infusion. The flat dose of the anti-TRBV9 antibody 180 product is determined based on the subject's body weight, and differs between the first and subsequent infusions. The first infusion of the anti-TRBV9 antibody 180 product is carried out at the initial dose (first dose) determined based on the subject's body weight, subsequent infusions are carried out at the full dose (main dose) provided for a given weight category (Table ).

**Table 11. Flat doses of anti-TRBV9 antibody 180 product**

| **Subject's body weight** | **Initial dose** | **Full dose** |
|---|---|---|
| ≤50 kg (from 30 to 50 kg) | 125 mg | 375 mg |
| From 51 to 89 kg | 250 mg | 500 mg |
| ≥ 90 kg (from 90 to 150 kg) | 250 mg | 625 mg |

The flat doses of the anti-TRBV9 antibody 180 product were selected based on the results of an interim efficacy and safety analysis performed as part of clinical study no. BCD-180-2/ELEFTA, and were justified by the results of population pharmacokinetic modeling of the anti-TRBV9 antibody 180 product, which allowed comparing the pharmacokinetics of the anti-TRBV9 antibody 180 product following administering same at said flat doses and following administering at a dose of 7 mg/kg.

Results of the interim data analysis of clinical trial no. BCD-180-2/ELEFTA are shown in Example 7. Based on the resulting data, a 7 mg/kg dose of the anti-TRBV9 antibody 180 product was selected for further clinical development.

### Modeling of pharmacokinetic profiles for flat and patient weight-based doses of anti-TRBV9 antibody 180 product following multiple intravenous administration

The analysis aimed at comparing the pharmacokinetics (PK) of the anti-TRBV9 antibody 180 product following administering flat doses as shown in Table and following administering a 7 mg/kg dose based on population pharmacokinetic modeling of the anti-TRBV9 antibody 180 product.

The analysis included generation of a mathematical model describing PK of the anti-TRBV9 antibody 180 product in a population of patients with axSpA, comparison of severity of adverse events (AEs) and infusion reactions vs exposure values of the anti-TRBV9 antibody 180 product, generation of a mathematical model establishing the relationship between PK and PD, comparison of Cₘₐₓ and Cₘᵢₙ following the first administration of the anti-TRBV9 antibody 180 product at the initial dose, following the second and third administrations of the anti-TRBV9 antibody 180 product at the full dose, and comparison of AUC following the first administration (0 - infusion 2) and AUC for all 3 administrations up to 60 weeks (0 - week 60) using different dosing regimens.

The analysis used interim data from CS no. BCD-180-2/ELEFTA. PK was evaluated based on determination of concentration of the anti-TRBV9 antibody 180 product in blood serum by enzyme-linked immunosorbent assay. PD was investigated using a validated technique for determining the abundance of the T-lymphocyte TRBV9 gene relative to the constant TRBC gene of peripheral blood mononuclear cells by real-time PCR.

To determine the level of exposure of the anti-TRBV9 antibody 180 product affecting the severity of AEs, including infusion reactions, real PK profiles of a number of patients receiving the anti-TRBV9 antibody 180 product at doses of 5 mg/kg and 7 mg/kg were compared with the AEs and infusion reactions reported in a given group. AEs were compared to AUC, infusion reactions were compared to the maximum concentration of the anti-TRBV9 antibody 180 product. Comparison of data revealed no clear dependence of frequency and severity of AEs and infusion reactions vs product exposure.

Based on the final population pharmacokinetics model of the anti-TRBV9 antibody 180 product, virtual patients were generated with the consideration of interindividual variability in parameters in order to compare flat doses of the anti-TRBV9 antibody 180 product and a 7 mg/kg dose.

The PK profile was predicted for all 20000 patients up to week 60, i.e. 3 administrations of the product (week 0, week 12 and week 36). According to the obtained predictions, statistics for product exposure parameters were calculated depending on the administration of flat doses of anti-TRBV9 antibody 180 and a 7 mg/kg dose (Table ) and on the administration of different doses and the weight of a virtual patient (Table ,Table 2).

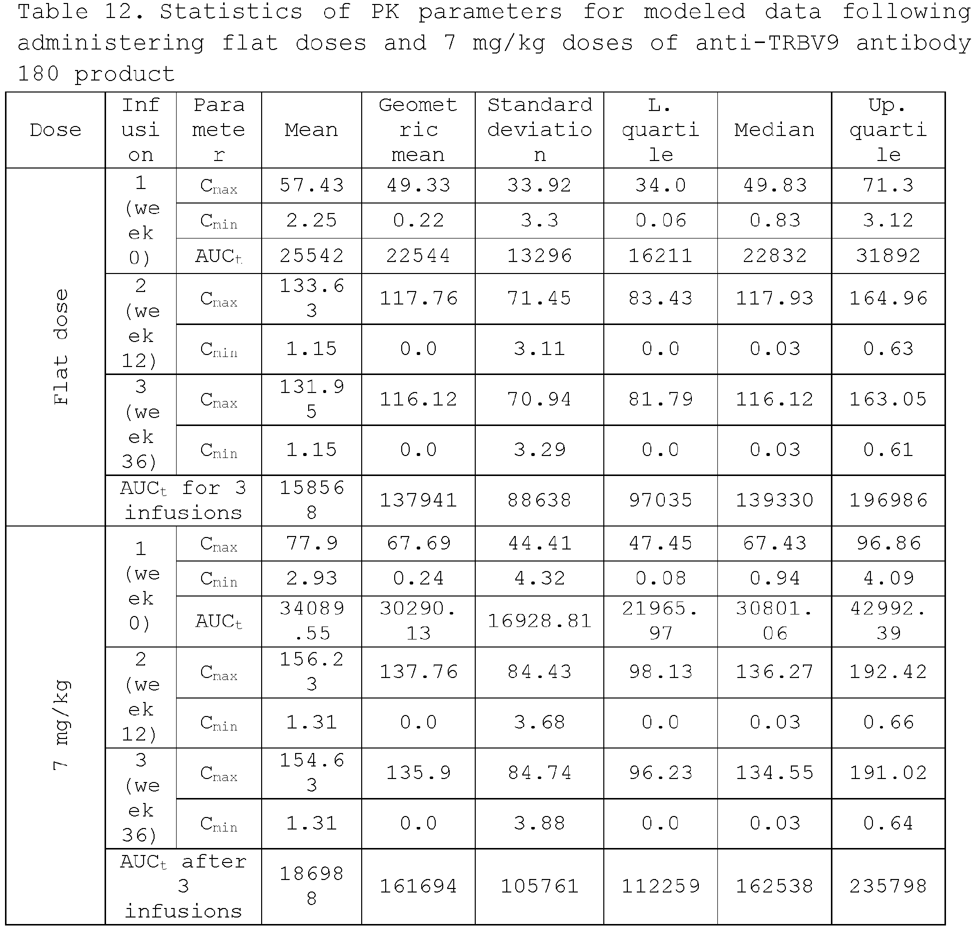

**Table 13. Statistics of PK parameters of modeled data following administering flat doses of anti-TRBV9 antibody 180 product in different weight cohorts**

| Weig ht | Infu sion | Para mete r | Mean | Geomet ric mean | Standard deviatio n | L. quarti le | Median | Up. quarti le |
|---|---|---|---|---|---|---|---|---|
| ≤ 50 kg (N = 1030 ) | 1 (wee k 0) | Cₘₐₓ | 47.07 | 41.04 | 25.89 | 28.91 | 41.87 | 58.32 |
| | | Cₘᵢₙ | 1. 49 | 0.13 | 2.3 | 0.03 | 0.49 | 1.87 |
| | | AUCₜ | 19477 | 17455 | 9171 | 13113 | 17709 | 24141 |
| | 2 (wee k 12) | Cₘₐₓ | 140.6 3 | 123.86 | 74.56 | 87.58 | 126.37 | 173.27 |
| | | Cₘᵢₙ | 0.9 | 0.0 | 2.92 | 0.0 | 0.02 | 0.35 |
| | 3 (wee k 36) | Cₘₐₓ | 139.3 7 | 122.75 | 73.88 | 87.51 | 124.75 | 171.26 |
| | | Cₘᵢₙ | 0.93 | 0.0 | 3.29 | 0.0 | 0.02 | 0.34 |
| | AUCₜ for 3 infusions | | 14757 2 | 129273 | 79811 | 93827 | 132045 | 180768 |
| 51-89 kg (N = 3510 ) | 1 (wee k 0) | Cₘₐₓ | 70.59 | 61.58 | 39.43 | 43.26 | 61.55 | 86.78 |
| | | Cₘᵢₙ | 2.69 | 0.25 | 3.93 | 0.08 | 0.98 | 3.74 |
| | | AUCₜ | 31132 | 27731 | 15399 | 20387 | 28222 | 38708 |
| | 2 (wee k 12) | Cₘₐₓ | 141.9 1 | 125.5 | 75.54 | 89.58 | 124.62 | 172.48 |
| | | Cₘᵢₙ | 1.17 | 0.0 | 3.27 | 0.0 | 0.03 | 0.6 |
| | 3 (wee k 36) | Cₘₐₓ | 139.7 7 | 123.27 | 75.15 | 87.04 | 122.68 | 170.65 |
| | | Cₘᵢₙ | 1.15 | 0.0 | 3.36 | 0.0 | 0.03 | 0.58 |
| | AUCₜ for 3 infusions | | 17065 1 | 148021 | 96351 | 104212 | 148762 | 212879 |
| ≥ 90 kg (N = 5460 ) | 1 (wee k 0) | Cₘₐₓ | 50.93 | 44.29 | 28.42 | 30.58 | 44.63 | 64.1 |
| | | Cₘᵢₙ | 2.1 | 0.23 | 2.96 | 0.07 | 0.83 | 3.01 |
| | | AUCₜ | 23093 | 20710 | 11067 | 15225 | 21016 | 28706 |
| | 2 (wee k 12) | Cₘₐₓ | 126.9 8 | 111.96 | 67.36 | 79.21 | 112.18 | 158.35 |
| | | Cₘᵢₙ | 1.18 | 0.0 | 3.04 | 0.0 | 0.04 | 0.72 |
| | 3 (wee k 36) | Cₘₐₓ | 125.5 1 | 110.58 | 66.85 | 78.06 | 110.55 | 156.73 |
| | | Cₘᵢₙ | 1.19 | 0.0 | 3.24 | 0.0 | 0.04 | 0.7 |
| | AUCₜ after 3 infusions | | 15287 4 | 133450 | 84105 | 93646 | 133872 | 190632 |

**Table 2. Statistics of PK parameters of modeled data following administering anti-TRBV9 antibody 180 at 7 mg/kg dose in different weight cohorts**

| Weig ht | Infu sion | Para mete r | Mean | Geomet ric mean | Standard deviatio n | L. quarti le | Median | Up. quarti le |
|---|---|---|---|---|---|---|---|---|
| ≤ 50 kg (N = 890) | 1 (wee k 0) | Cₘₐₓ | 61.13 | 53.28 | 34.26 | 37.52 | 53.28 | 77.43 |
| | | Cₘᵢₙ | 1.93 | 0.13 | 2.97 | 0.03 | 0.46 | 2.65 |
| | | AUCₜ | 24747 | 22249 | 11621 | 16401 | 22952 | 30322 |
| | 2 (wee k 12) | Cₘₐₓ | 123.2 6 | 108.73 | 66.53 | 77.58 | 107.85 | 153.83 |
| | | Cₘᵢₙ | 0.81 | 0.0 | 2.2 | 0.0 | 0.01 | 0.37 |
| | 3 (wee k 36) | Cₘₐₓ | 122.6 8 | 107.68 | 67.55 | 75.91 | 107.07 | 154.4 |
| | | Cₘᵢₙ | 0.79 | 0.0 | 2.19 | 0.0 | 0.01 | 0.36 |
| | AUCₜ after 3 infusions | | 13427 6 | 117873 | 71627 | 83124 | 120841 | 165170 |
| 51- 89 kg (N = 3750 ) | 1 (wee k 0) | Cmax | 71.17 | 62.25 | 39.79 | 44.5 | 62.24 | 87.42 |
| | | Cₘᵢₙ | 2.48 | 0.21 | 3.69 | 0.07 | 0.78 | 3.4 |
| | | AUCₜ | 30446 | 27324 | 14553 | 20165 | 27661 | 37962 |
| | 2 (wee k 12) | Cₘₐₓ | 143.1 4 | 126.87 | 76.34 | 91.55 | 125.97 | 174.93 |
| | | Cₘᵢₙ | 1.09 | 0.0 | 3.14 | 0.0 | 0.02 | 0.5 |
| | 3 (wee k 36) | Cₘₐₓ | 141.7 7 | 125.23 | 76.74 | 89.9 | 123.98 | 173.13 |
| | | Cₘᵢₙ | 1.09 | 0.0 | 3.3 | 0.0 | 0.02 | 0.49 |
| | AUCₜ after 3 infusions | | 16608 4 | 145359 | 90335 | 102532 | 145670 | 206778 |
| ≥ 90 kg (N = 5360 ) | 1 (wee k 0) | Cₘₐₓ | 85.4 | 74.69 | 47.35 | 52.4 | 74.27 | 106.27 |
| | | Cₘᵢₙ | 3.42 | 0.3 | 4.82 | 0.1 | 1.21 | 4.99 |
| | | A_{UCt} | 38190 | 34265 | 17987 | 25298 | 34935 | 47795 |
| | 2 (wee k 12) | Cₘₐₓ | 170.8 6 | 151.79 | 89.3 | 109.09 | 150.01 | 209.56 |
| | | Cₘᵢₙ | 1.55 | 0.0 | 4.18 | 0.0 | 0.04 | 0.89 |
| | 3 (wee k 36) | Cₘₐₓ | 168.9 4 | 149.58 | 89.62 | 106.43 | 147.85 | 208.42 |
| | | Cₘᵢₙ | 1.55 | 0.0 | 4.43 | 0.0 | 0.04 | 0.86 |
| | AUCₜ after 3 infusions | | 21036 5 | 183590 | 114002 | 129205 | 185090 | 265053 |

PK parameters (Cₘₐₓ, Cₘᵢₙ and AUC) were compared following administering a flat dose and a 7 mg/kg dose.

Tables 12-14 above show that the PK parameters of the anti-TRBV9 antibody 180 product in the group of patients administered with a flat dose are comparable with those of the anti-TRBV9 antibody 180 product in the group of patients administered with a 7 mg/kg dose, both in the patient population in general and in weight cohorts in particular.

The results may serve as the basis for the following conclusions:
1) The maximum and minimum concentrations following each administration of the product show similar results across the group of patients administered with a flat dose of the product and the group of patients administered with a 7 mg/kg dose of the product, as well as across groups taking into account weight cohorts.
2) AUC after the first administration and AUC after 3 administrations up to 60 weeks show similar results across the group of patients administered with a flat dose of the product and the group of patients administered with a 7 mg/kg dose, as well as across groups taking into account weight cohorts.
3) Inaccuracies in the prediction of pharmacokinetic profiles by the model will not affect the safety of the clinical study, since the severity of adverse events and infusion reactions does not depend on product exposure.

Thus, phase II CS no. BCD-180-2/ELEFTA resulted in data indicating clinical efficacy of the anti-TRBV9 antibody 180 product and superiority of the test anti-TRBV9 antibody 180 product at a dose of 7 mg/kg over placebo in the parameter "Proportion of subjects who reached ASAS40 on week 24" in the target population of patients with active axSpA. An interim safety analysis confirmed an acceptable safety profile and tolerability of the anti-TRBV9 antibody 180 product at both studied doses, 5 mg/kg and 7 mg/kg. The results of the analysis using mathematical modeling of pharmacokinetic parameters of the anti-TRBV9 antibody 180 product following using same at a dose of 7 mg/ kg and at a flat dose demonstrate similar exposure parameters.

Administration of the anti-TRBV9 antibody 180 product at a dose of 7 mg/ kg causes complete depletion of TRBV9+ T-lymphocytes in the absolute majority of subjects. Thus, with proven identical exposure of the anti-TRBV9 antibody 180 product at flat doses and at a dose of 7 mg/kg, a significant depletion of TRBV9+ T lymphocytes will also be observed.

## Claims

1. A method of treatment of a disease or disorder mediated by T-lymphocytes bearing the TRBV9 segment within the T-cell receptor, comprising administering to a subject in need thereof an anti-TRBV9 antibody at a dose of 0.03-10 mg/kg or 75-675 mg.

2. A method of treatment according to claim 1 of a disease or disorder mediated by T-lymphocytes bearing the TRBV9 segment within the T-cell receptor, comprising administering to a subject in need thereof an anti-TRBV9 antibody at a dose of 0.03-7 mg/kg.

3. A method of treatment according to claim 1 of a disease or disorder mediated by T-lymphocytes bearing the TRBV9 segment within the T-cell receptor, comprising administering to a subject in need thereof an anti-TRBV9 antibody at a dose of 100-650 mg.

4. The method according to claim 1, wherein the anti-TRBV9 antibody is administered at a dose of 1-7 mg/kg.

5. The method according to claim 1, wherein the anti-TRBV9 antibody is administered at a dose of 3-7 mg/kg.

6. The method according to claim 1, wherein the anti-TRBV9 antibody is administered at a dose of 5-7 mg/kg.

7. The method according to claim 1, wherein the anti-TRBV9 antibody is administered at a dose of 75-175 mg; or 200-300 mg; or 325-425 mg; or 450-650 mg; or 575-675 mg.

8. The method according to claim 1, wherein the anti-TRBV9 antibody is administered at a dose of 100-150 mg; or 225-275 mg; or 350-400 mg; or 475- 525 mg; or 600-650mg.

9. The method according to claim 1, wherein the anti-TRBV9 antibody is administered at a dose of 115-135 mg; or 240-260 mg; or 365-385 mg; or 490- 510 mg; or 615-635mg.

10. The method according to claim 1, wherein the anti-TRBV9 antibody is administered at a dose of 1 mg/kg, or 2 mg/kg, or 3 mg/kg, or 4 mg/kg, or 5 mg/kg, or 6 mg/kg, or 7 mg/kg;
or at a dose of 120 mg; or 125 mg; or 130 mg; or 245 mg; or 250 mg; or 255 mg; or 370 mg; or 375 mg; or 380 mg; or 495 mg; or 500 mg; or 505 mg; or 620 mg; or 625 mg; or 630 mg.

11. The method according to any one of claims 1-10, wherein the anti-TRBV9 antibody is administered at least every 3-12 months.

12. The method according to any one of claims 1-10, wherein the anti-TRBV9 antibody is administered at least every 6-12 months.

13. The method according to any one of claims 1-10, wherein the anti-TRBV9 antibody is administered at least every 3 months; or the anti-TRBV9 antibody is administered at least every 6 months; or
the anti-TRBV9 antibody is administered at least every 9 months; or
the anti-TRBV9 antibody is administered at least every 12 months.

14. A method of treatment of a disease or disorder mediated by T-lymphocytes bearing the TRBV9 segment within the T-cell receptor, comprising (i) the first administration to a subject in need thereof of an anti-TRBV9 antibody at a dose equal to half of the main dose, 0.5-5 mg/kg, the second administration of an anti-TRBV9 antibody at the main dose of 1-10 mg/kg after at least 3 months, and the subsequent administrations of an anti-TRBV9 antibody at the main dose of 1-10 mg/kg at least every 6 months; or (ii) the first administration to a subject in need thereof of an anti-TRBV9 antibody at a dose of 75-300 mg, the second administration of an anti-TRBV9 antibody at the main dose of 325-675 mg after at least 3 months, and the subsequent administrations of an anti-TRBV9 antibody at the main dose of 325-675 mg at least every 6 months.

15. The method of treatment according to claim 14 of a disease or disorder mediated by T-lymphocytes bearing the TRBV9 segment within the T-cell receptor, comprising (i) the first administration to a subject in need thereof of an anti-TRBV9 antibody at a dose equal to half of the main dose, 0.5-3.5 mg/kg, the second administration of an anti-TRBV9 antibody at the main dose of 1-7 mg/kg after at least 3 months, and the subsequent administrations of an anti-TRBV9 antibody at the main dose of 1-7 mg/kg at least every 6 months; or (ii) the first administration to a subject in need thereof of an anti-TRBV9 antibody at a dose of 100-275 mg, the second administration of an anti-TRBV9 antibody at the main dose of 350-650 mg after at least 3 months, the third administration of an anti-TRBV9 antibody at the main dose of 350-650 mg/kg after at least 6 months, and the subsequent administrations of an anti-TRBV9 antibody at the main dose of 350-650 mg at least every 12 months.

16. The method according to claim 14, comprising (i) the first administration of an anti-TRBV9 antibody at a dose of 0.5 mg/kg, the second administration of an anti-TRBV9 antibody at a dose of 1 mg/kg after at least 3 months, and the subsequent administrations of an anti-TRBV9 antibody at a dose of 1 mg/kg at least every 6 months; or
(ii) the first administration of an anti-TRBV9 antibody at a dose of 1 mg/kg, the second administration of an anti-TRBV9 antibody at a dose of 2 mg/kg after at least 3 months, and the subsequent administrations of an anti-TRBV9 antibody at a dose of 2 mg/kg at least every 6 months; or
(iii) the first administration of an anti-TRBV9 antibody at a dose of 1.5 mg/kg, the second administration of an anti-TRBV9 antibody at a dose of 3 mg/kg after at least 3 months, and the subsequent administrations of an anti-TRBV9 antibody at a dose of 3 mg/kg at least every 6 months; or
(iv) the first administration of an anti-TRBV9 antibody at a dose of 2.5 mg/kg, the second administration of an anti-TRBV9 antibody at a dose of 5 mg/kg after at least 3 months, and the subsequent administrations of an anti-TRBV9 antibody at a dose of 5 mg/kg at least every 6 months; or
(v) the first administration of an anti-TRBV9 antibody at a dose of 3.5 mg/kg, the second administration of an anti-TRBV9 antibody at a dose of 7 mg/kg after at least 3 months, and the subsequent administrations of an anti-TRBV9 antibody at a dose of 7 mg/kg at least every 6 months.

17. The method according to claim 14, comprising the first administration of an anti-TRBV9 antibody at a dose of 100-150 mg; or 225-275 mg, the second administration of an anti-TRBV9 antibody at the main dose of 350-400 mg; or 475- 525 mg; or 600-650 mg after at least 3 months, and the subsequent administrations of an anti-TRBV9 antibody at the main dose of 350-400 mg; or 475- 525 mg; or 600-650 mg at least every 6 months.

18. The method according to claim 14, comprising the first administration of an anti-TRBV9 antibody at a dose of 115-135 mg; or 240-260 mg, the second administration of an anti-TRBV9 antibody at the main dose of 365-385 mg; or 490- 510 mg; or 615-635 mg after at least 3 months, and the subsequent administrations of an anti-TRBV9 antibody at the main dose of 365-385 mg; or 490- 510 mg; or 615-635 mg at least every 6 months.

19. The method according to claim 14, comprising the first administration of an anti-TRBV9 antibody at a dose of 120 mg; or 125 mg; or 130 mg; or 245 mg; or 250 mg; or 255 mg, the second administration of an anti-TRBV9 antibody at the main dose of 370 mg; or 375 mg; or 380 mg; or 495 mg; or 500 mg; or 505 mg; or 620 mg; or 625 mg; or 630 mg after at least 3 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg at least every 6 months.

20. A method of treatment of a disease or disorder mediated by T-lymphocytes bearing the TRBV9 segment within the T-cell receptor, comprising (i) the first administration of an anti-TRBV9 antibody to a subject in need thereof at a dose equal to half of the main dose, 0.5-5 mg/kg, the second administration of an anti-TRBV9 antibody at the main dose of 1-10 mg/kg after at least 3 months, the third administration of an anti-TRBV9 antibody at the main dose of 1-10 mg/kg after at least 6 months, and the subsequent administrations of an anti-TRBV9 antibody at the main dose of 1-10 mg/kg at least every 12 months; or
(ii) the first administration to a subject in need thereof of an anti-TRBV9 antibody at a dose of 75-300 mg, the second administration of an anti-TRBV9 antibody at the main dose of 325-675 mg after at least 3 months, the third administration of an anti-TRBV9 antibody at the main dose of 325-675 mg after at least 6 months, and the subsequent administrations of an anti-TRBV9 antibody at the main dose of 325-675 mg at least every 12 months.

21. The method of treatment according to claim 20 of a disease or disorder mediated by T-lymphocytes bearing the TRBV9 segment within the T-cell receptor, comprising (i) the first administration of an anti-TRBV9 antibody to a subject in need thereof at a dose equal to half of the main dose, 0.5-3.5 mg/kg, the second administration of an anti-TRBV9 antibody at the main dose of 1-7 mg/kg after at least 3 months, the third administration of an anti-TRBV9 antibody at the main dose of 1-7 mg/kg after at least 6 months, and the subsequent administrations of an anti-TRBV9 antibody at the main dose of 1-7 mg/kg at least every 12 months; or
(ii) the first administration to a subject in need thereof of an anti-TRBV9 antibody at a dose of 100-275 mg, the second administration of an anti-TRBV9 antibody at the main dose of 350-650 mg after at least 3 months, the third administration of an anti-TRBV9 antibody at the main dose of 350-650 mg after at least 6 months, and the subsequent administrations of an anti-TRBV9 antibody at the main dose of 350-650 mg at least every 12 months.

22. The method according to claim 20, comprising (i) the first administration of an anti-TRBV9 antibody at a dose of 0.5 mg/kg, the second administration of an anti-TRBV9 antibody at a dose of 1 mg/kg after at least 3 months, the third administration of an anti-TRBV9 antibody at a dose of 1 mg/kg after at least 6 months, and the subsequent administrations of an anti-TRBV9 antibody at a dose of 1 mg/kg at least every 12 months; or
(ii) the first administration of an anti-TRBV9 antibody at a dose of 1 mg/kg, the second administration of an anti-TRBV9 antibody at a dose of 2 mg/kg after at least 3 months, the third administration of an anti-TRBV9 antibody at a dose of 2 mg/kg after at least 6 months, and the subsequent administrations of an anti-TRBV9 antibody at a dose of 2 mg/kg at least every 12 months; or
(iii) the first administration of an anti-TRBV9 antibody at a dose of 1.5 mg/kg, the second administration of an anti-TRBV9 antibody at a dose of 3 mg/kg after at least 3 months, the third administration of an anti-TRBV9 antibody at a dose of 3 mg/kg after at least 6 months, and the subsequent administrations of an anti-TRBV9 antibody at a dose of 3 mg/kg at least every 12 months; or
(iv) the first administration of an anti-TRBV9 antibody at a dose of 2.5 mg/kg, the second administration of an anti-TRBV9 antibody at a dose of 5 mg/kg after at least 3 months, the third administration of an anti-TRBV9 antibody at a dose of 5 mg/kg after at least 6 months, and the subsequent administrations of an anti-TRBV9 antibody at a dose of 5 mg/kg at least every 12 months; or
(v) the first administration to a subject in need thereof of an anti-TRBV9 antibody at a dose of 3.5 mg/kg, the second administration of an anti-TRBV9 antibody at a dose of 7 mg/kg after at least 3 months, the third administration of an anti-TRBV9 antibody at a dose of 7 mg/kg after at least 6 months, and the subsequent administrations of an anti-TRBV9 antibody at a dose of 7 mg/kg at least every 12 months.

23. The method according to claim 20 comprising the first administration to a subject in need thereof of an anti-TRBV9 antibody at a dose of 100-150 mg; or 225-275 mg, the second administration of an anti-TRBV9 antibody at the main dose of 350-400 mg; or 475- 525 mg; or 600-650 mg after at least 3 months, the third administration of an anti-TRBV9 antibody at the main dose of 350-400 mg; or 475- 525 mg; or 600-650 mg after at least 6 months, and the subsequent administrations of an anti-TRBV9 antibody at the main dose of 350-400 mg; or 475- 525 mg; or 600-650 mg at least every 12 months.

24. The method according to claim 20, comprising the first administration of an anti-TRBV9 antibody at a dose of 115-135 mg; or 240-260 mg, the second administration of an anti-TRBV9 antibody at the main dose of 365-385 mg; or 490- 510 mg; or 615-635 mg after at least 3 months, and the subsequent administrations of an anti-TRBV9 antibody at the main dose of 365-385 mg; or 490- 510 mg; or 615-635 mg at least every 6 months.

25. The method according to claim 20 comprising the first administration of an anti-TRBV9 antibody at a dose of 120 mg; or 125 mg; or 130 mg; or 245 mg; or 250 mg; or 255 mg, the second administration of an anti-TRBV9 antibody at the main dose of 370 mg; or 375 mg; or 380 mg; or 495 mg; or 500 mg; or 505 mg; or 620 mg; or 625 mg; or 630 mg after at least 3 months, the third administration of an anti-TRBV9 antibody at the main dose of 370 mg; or 375 mg; or 380 mg; or 495 mg; or 500 mg; or 505 mg; or 620 mg; or 625 mg; or 630 mg after at least 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg at least every 12 months.

26. The method according to any one of claims 1, 14, 20, wherein the anti-TRBV9 antibody comprises:
1) a heavy chain variable domain comprising:
(a) HCDR1 with the amino acid sequence of SEQ ID NO: 1,
(b) HCDR2 with the amino acid sequence of SEQ ID NO: 2, and
(c) HCDR3 with an amino acid sequence selected from the group: SEQ ID NO: 3, SEQ ID NO: 4, SEQ ID NO: 5 or SEQ ID NO: 6;
2) a light chain variable domain comprising:
(a) LCDR1 with the amino acid sequence of SEQ ID NO: 7,
(b) LCDR2 with the amino acid sequence of SEQ ID NO: 8, and
(c) LCDR3 with the amino acid sequence of SEQ ID NO: 9.

27. The method according to claim 26, wherein the anti-TRBV9 antibody comprises:
1) a heavy chain variable domain comprising:
(a) HCDR1 with the amino acid sequence of SEQ ID NO: 1,
(b) HCDR2 with the amino acid sequence of SEQ ID NO: 2, and
(c) HCDR3 with the amino acid sequence of SEQ ID NO: 4;
2) a light chain variable domain comprising:
(a) LCDR1 with the amino acid sequence of SEQ ID NO: 7,
(b) LCDR2 with the amino acid sequence of SEQ ID NO: 8, and
(c) LCDR3 with the amino acid sequence of SEQ ID NO: 9

28. The method according to claim 26, wherein the anti-TRBV9 antibody is a full-length antibody and is of human IgG1, IgG2, IgG3 or IgG4 isotype.

29. The method according to claim 26, wherein the anti-TRBV9 antibody comprises a heavy chain variable domain with the amino acid sequence of SEQ ID No: 14 and a light chain variable domain with the amino acid sequence of SEQ ID No: 17; or
a heavy chain variable domain with the amino acid sequence of SEQ ID No: 15 and a light chain variable domain with the amino acid sequence of SEQ ID No: 17.

30. The method according to claim 26, wherein the anti-TRBV9 antibody comprises a heavy chain with the amino acid sequence of SEQ ID No: 22 and a light chain with the amino acid sequence of SEQ ID No: 25; or
a heavy chain with the amino acid sequence of SEQ ID No: 23 and a light chain with the amino acid sequence of SEQ ID No: 25.

31. The method according to any one of claims 1, 14, 20, wherein the disease or disorder mediated by T-lymphocytes bearing the TRBV9 segment within the T cell receptor is selected from the group comprising: arthropathies, inflammatory bowel diseases, eye diseases, vasculitides, circulatory system diseases, kidney diseases, digestive system diseases, lymphoproliferative disorders.

32. The method according to any one of claims 1, 14, 20, wherein the disease or disorder mediated by T-lymphocytes bearing the TRBV9 segment within the T cell receptor is selected from the group comprising: spondyloarthritides, sacroiliitis associated with psoriasis, sacroiliitis associated with inflammatory bowel diseases, undifferentiated oligoarthropathy, juvenile spondylitis/enthesitis-related arthritis, juvenile ankylosing spondylitis (arthritis associated with enthesitis), juvenile arthritis, undifferentiated juvenile arthritis, ulcerative colitis, Crohn's disease, noninfectious uveitides, anterior uveitis, Behcet's disease, aortitis, fibrosis of aortic and/or mitral valve leaflets with regurgitation, rhythm disturbances, conduction disturbances, left ventricular dysfunction, pericarditis, myocarditis, IgA nephropathy, celiac disease, T cell lymphoma, T cell leukemia.

33. The method according to claim 32, wherein spondyloarthritis is radiographic axial spondyloarthritis (ankylosing spondylitis), nonradiographic axial spondyloarthritis, axial spondyloarthritis, peripheral spondyloarthritis, psoriatic arthritis, spondyloarthritis associated with inflammatory bowel diseases, reactive arthritis, undifferentiated peripheral spondyloarthritis.

34. The method according to claim 32, wherein spondyloarthritis is radiographic axial spondyloarthritis (ankylosing spondylitis) in patients with no adequate response to standard therapy, nonradiographic axial spondyloarthritis in patients with no adequate response to standard therapy, axial spondyloarthritis in patients with no adequate response to standard therapy, peripheral spondyloarthritis in patients with no adequate response to standard therapy, psoriatic arthritis in patients with no adequate response to standard therapy, spondyloarthritis associated with inflammatory bowel diseases in patients with no adequate response to standard therapy, reactive arthritis in patients with no adequate response to standard therapy, or undifferentiated peripheral spondyloarthritis in patients with no adequate response to standard therapy.

35. Use of an anti-TRBV9 antibody for the treatment of a disease or disorder mediated by T-lymphocytes bearing the TRBV9 segment within the T-cell receptor, wherein the anti-TRBV9 antibody is administered to a subject in need thereof at a dose of 0.03-10 mg/kg or at a dose of 75-675 mg.

36. The use according to claim 35 of an anti-TRBV9 antibody for the treatment of a disease or disorder mediated by T-lymphocytes bearing the TRBV9 segment within the T-cell receptor, wherein the anti-TRBV9 antibody is administered to a subject in need thereof at a dose of 0.03-7 mg/kg.

37. The use according to claim 35 of an anti-TRBV9 antibody for the treatment of a disease or disorder mediated by T-lymphocytes bearing the TRBV9 segment within the T-cell receptor, wherein the anti-TRBV9 antibody is administered to a subject in need thereof at a dose of 100-650 mg.

38. The use according to claim 35, wherein the anti-TRBV9 antibody is administered at a dose of 1-7 mg/kg.

39. The use according to claim 35, wherein the anti-TRBV9 antibody is administered at a dose of 3-7 mg/kg.

40. The use according to claim 35, wherein the anti-TRBV9 antibody is administered at a dose of 5-7 mg/kg.

41. The use according to claim 35, wherein the anti-TRBV9 antibody is administered either at a dose of 75-175 mg; or 200-300 mg; or 325-425 mg; or 450-650 mg; or 575-675 mg.

42. The use according to claim 35, wherein the anti-TRBV9 antibody is administered at a dose of 100-150 mg; or 225-275 mg; or 350-400 mg; or 475- 525 mg; or 600-650mg.

43. The use according to claim 35, wherein the anti-TRBV9 antibody is administered at a dose of 115-135 mg; or 240-260 mg; or 365-385 mg; or 490- 510 mg; or 615-635mg.

44. The use according to claim 35, wherein the anti-TRBV9 antibody is administered at a dose of 1 mg/kg, or 2 mg/kg, or 3 mg/kg, or 4 mg/kg, or 5 mg/kg, or 6 mg/kg, or 7 mg/kg;
or 120 mg; or 125 mg; or 130 mg; or 245 mg; or 250 mg; or 255 mg; or 370 mg; or 375 mg; or 380 mg; or 495 mg; or 500 mg; or 505 mg; or 620 mg; or 625 mg; or 630 mg.

45. The use according to any one of claims 35-44, wherein the anti-TRBV9 antibody is administered at least every 3-12 months.

46. The use according to any one of claims 35-44, wherein the anti-TRBV9 antibody is administered at least every 6-12 months.

47. The use according to any one of claims 35-44, wherein the anti-TRBV9 antibody is administered at least every 3 months; or the anti-TRBV9 antibody is administered at least every 6 months; or
the anti-TRBV9 antibody is administered at least every 9 months; or
the anti-TRBV9 antibody is administered at least every 12 months.

48. Use of an anti-TRBV9 antibody for the treatment of a disease or disorder mediated by T-lymphocytes bearing the TRBV9 segment within the T-cell receptor, wherein (i) the first administration to a subject in need thereof of an anti-TRBV9 antibody is performed at a dose equal to half of the main dose, 0.5-5 mg/kg, the second administration of an anti-TRBV9 antibody is performed at the main dose of 1-10 mg/kg after at least 3 months, and the subsequent administrations of an anti-TRBV9 antibody are performed at the main dose of 1-10 mg/kg at least every 6 months; or (ii) the first administration to a subject in need thereof of an anti-TRBV9 antibody is performed at a dose of 75-300 mg, the second administration of an anti-TRBV9 antibody at the main dose of 325-675 mg after at least 3 months, and the subsequent administrations of an anti-TRBV9 antibody at the main dose of 325-675 mg at least every 6 months.

49. The use according to claim 48 of an anti-TRBV9 antibody for the treatment of a disease or disorder mediated by T-lymphocytes bearing the TRBV9 segment within the T-cell receptor, wherein (i) the first administration to a subject in need thereof of an anti-TRBV9 antibody is performed at a dose equal to half of the main dose, 0.5-3.5 mg/kg, the second administration of an anti-TRBV9 antibody is performed at the main dose of 1-7 mg/kg after at least 3 months, and the subsequent administrations of an anti-TRBV9 antibody are performed at the main dose of 1-7 mg/kg at least every 6 months; or (ii) the first administration of an anti-TRBV9 antibody is performed at a dose of 100-275 mg, the second administration of an anti-TRBV9 antibody at the main dose of 350-650 mg after at least 3 months, and the subsequent administrations of an anti-TRBV9 antibody at the main dose of 350-650 mg at least every 6 months.

50. The use according to claim 48, wherein (i) the first administration of an anti-TRBV9 antibody is performed at a dose of 0.5 mg/kg, the second administration of an anti-TRBV9 antibody is performed at a dose of 1 mg/kg after at least 3 months, and the subsequent administrations of an anti-TRBV9 antibody are performed at a dose of 1 mg/kg at least every 6 months; or
(ii) the first administration of an anti-TRBV9 antibody is performed at a dose of 1 mg/kg, the second administration of an anti-TRBV9 antibody is performed at a dose of 2 mg/kg after at least 3 months, and the subsequent administrations of an anti-TRBV9 antibody are performed at a dose of 2 mg/kg at least every 6 months; or
(iii) the first administration of an anti-TRBV9 antibody is performed at a dose of 1.5 mg/kg, the second administration of an anti-TRBV9 antibody is performed at a dose of 3 mg/kg after at least 3 months, and the subsequent administrations of an anti-TRBV9 antibody are performed at a dose of 3 mg/kg at least every 6 months; or
(iv) the first administration of an anti-TRBV9 antibody is performed at a dose of 2.5 mg/kg, the second administration of an anti-TRBV9 antibody is performed at a dose of 5 mg/kg after at least 3 months, and the subsequent administrations of an anti-TRBV9 antibody are performed at a dose of 5 mg/kg at least every 6 months; or
(v) the first administration of an anti-TRBV9 antibody is performed at a dose of 3.5 mg/kg, the second administration of an anti-TRBV9 antibody is performed at a dose of 7 mg/kg after at least 3 months, and the subsequent administrations of an anti-TRBV9 antibody are performed at a dose of 7 mg/kg at least every 6 months.

51. The use according to claim 48, comprising the first administration of an anti-TRBV9 antibody at a dose of 100-150 mg; or 225-275 mg, the second administration of an anti-TRBV9 antibody at the main dose of 350-400 mg; or 475- 525 mg; or 600-650 mg after at least 3 months, and the subsequent administrations of an anti-TRBV9 antibody at the main dose of 350-400 mg; or 475- 525 mg; or 600-650 mg at least every 6 months.

52. The use according to claim 48, comprising the first administration of an anti-TRBV9 antibody at a dose of 115-135 mg; or 240-260 mg, the second administration of an anti-TRBV9 antibody at the main dose of 365-385 mg; or 490- 510 mg; or 615-635 mg after at least 3 months, and the subsequent administrations of an anti-TRBV9 antibody at the main dose of 365-385 mg; or 490- 510 mg; or 615-635 mg at least every 6 months.

53. The use according to claim 48, comprising the first administration to a subject in need thereof of an anti-TRBV9 antibody at a dose of 120 mg; or 125 mg; or 130 mg; or 245 mg; or 250 mg; or 255 mg, the second administration of an anti-TRBV9 antibody at the main dose of 370 mg; or 375 mg; or 380 mg; or 495 mg; or 500 mg; or 505 mg; or 620 mg; or 625 mg; or 630 mg after at least 3 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg at least every 6 months.

54. Use of an anti-TRBV9 antibody for the treatment of a disease or disorder mediated by T-lymphocytes bearing the TRBV9 segment within the T-cell receptor, wherein (i) the first administration to a subject in need thereof of an anti-TRBV9 antibody is performed at a dose equal to half of the main dose, 0.5-5 mg/kg, the second administration of an anti-TRBV9 antibody is performed at the main dose of 1-10 mg/kg after at least 3 months, the third administration of an anti-TRBV9 antibody is performed at the main dose of 1-10 mg/kg after at least 6 months, and the subsequent administrations of an anti-TRBV9 antibody are performed at the main dose of 1-10 mg/kg at least every 12 months; or (ii) the first administration to a subject in need thereof of an anti-TRBV9 antibody is performed at a dose of 75-300 mg, the second administration of an anti-TRBV9 antibody at the main dose of 325-675 mg after at least 3 months, the third administration of an anti-TRBV9 antibody at the main dose of 325-675 mg/kg after at least 6 months, and the subsequent administrations of an anti-TRBV9 antibody at the main dose of 325-675 mg at least every 12 months.

55. The use according to claim 54 of an anti-TRBV9 antibody for the treatment of a disease or disorder mediated by T-lymphocytes bearing the TRBV9 segment within the T-cell receptor, wherein (i) the first administration to a subject in need thereof of an anti-TRBV9 antibody is performed at a dose equal to half of the main dose, 0.5-3.5 mg/kg, the second administration of an anti-TRBV9 antibody is performed at the main dose of 1-7 mg/kg after at least 3 months, the third administration of an anti-TRBV9 antibody is performed at the main dose of 1-7 mg/kg after at least 6 months, and the subsequent administrations of an anti-TRBV9 antibody are performed at the main dose of 1-7 mg/kg at least every 12 months; or (ii) the first administration to a subject in need thereof of an anti-TRBV9 antibody is performed at a dose of 100-275 mg, the second administration of an anti-TRBV9 antibody at the main dose of 350-650 mg after at least 3 months, the third administration of an anti-TRBV9 antibody at the main dose of 350-650 mg/kg after at least 6 months, and the subsequent administrations of an anti-TRBV9 antibody at the main dose of 350-650 mg at least every 12 months.

56. The use according to claim 54, wherein (i) the first administration of an anti-TRBV9 antibody is performed at a dose of 0.5 mg/kg, the second administration of an anti-TRBV9 antibody is performed at a dose of 1 mg/kg after at least 3 months, the third administration of an anti-TRBV9 antibody is performed at a dose of 1 mg/kg after at least 6 months, and the subsequent administrations of an anti-TRBV9 antibody are performed at a dose of 1 mg/kg at least every 12 months; or
(ii) the first administration of an anti-TRBV9 antibody is performed at a dose of 1 mg/kg, the second administration of an anti-TRBV9 antibody is performed at a dose of 2 mg/kg after at least 3 months, the third administration of an anti-TRBV9 antibody is performed at a dose of 2 mg/kg after at least 6 months, and the subsequent administrations of an anti-TRBV9 antibody are performed at a dose of 2 mg/kg at least every 12 months; or
(iii) the first administration of an anti-TRBV9 antibody is performed at a dose of 1.5 mg/kg, the second administration of an anti-TRBV9 antibody is performed at a dose of 3 mg/kg after at least 3 months, the third administration of an anti-TRBV9 antibody is performed at a dose of 3 mg/kg after at least 6 months, and the subsequent administrations of an anti-TRBV9 antibody are performed at a dose of 3 mg/kg at least every 12 months; or
(iv) the first administration of an anti-TRBV9 antibody is performed at a dose of 2.5 mg/kg, the second administration of an anti-TRBV9 antibody is performed at a dose of 5 mg/kg after at least 3 months, the third administration of an anti-TRBV9 antibody is performed at a dose of 5 mg/kg after at least 6 months, and the subsequent administrations of an anti-TRBV9 antibody are performed at a dose of 5 mg/kg at least every 12 months; or
(v) the first administration to a subject in need thereof of an anti-TRBV9 antibody is performed at a dose of 3.5 mg/kg, the second administration of an anti-TRBV9 antibody is performed at a dose of 7 mg/kg after at least 3 months, the third administration of an anti-TRBV9 antibody is performed at a dose of 7 mg/kg after at least 6 months, and the subsequent administrations of an anti-TRBV9 antibody are performed at a dose of 7 mg/kg at least every 12 months.

57. The use according to claim 54 comprising the first administration to a subject in need thereof of an anti-TRBV9 antibody performed at a dose of 100-150 mg; or 225-275 mg, the second administration of an anti-TRBV9 antibody at the main dose of 350-400 mg; or 475- 525 mg; or 600-650 mg after at least 3 months, the third administration of an anti-TRBV9 antibody at the main dose of 350-400 mg; or 475- 525 mg; or 600-650 mg after at least 6 months, and the subsequent administrations of an anti-TRBV9 antibody at the main dose of 350-400 mg; or 475- 525 mg; or 600-650 mg at least every 12 months.

58. The use according to claim 54, the first administration to a subject in need thereof of an anti-TRBV9 antibody is performed at a dose of 115-135 mg; or 240-260 mg, the second administration of an anti-TRBV9 antibody at the main dose of 365-385 mg; or 490-510 mg; or 615-635 mg after at least 3 months, the third administration of an anti-TRBV9 antibody at the main dose of 365-385 mg; or 490-510 mg; or 615-635 mg after at least 6 months, and the subsequent administrations of an anti-TRBV9 antibody at the main dose of 365-385 mg; or 490- 510 mg; or 615-635 mg at least every 12 months.

59. The use according to claim 54, the first administration to a subject in need thereof of an anti-TRBV9 antibody is performed at a dose of 120 mg; or 125 mg; or 130 mg; or 245 mg; or 250 mg; or 255 mg, the second administration of an anti-TRBV9 antibody at the main dose of 370 mg; or 375 mg; or 380 mg; or 495 mg; or 500 mg; or 505 mg; or 620 mg; or 625 mg; or 630 mg after at least 3 months, the third administration of an anti-TRBV9 antibody at the main dose of 370 mg; or 375 mg; or 380 mg; or 495 mg; or 500 mg; or 505 mg; or 620 mg; or 625 mg; or 630 mg after at least 6 months, and the subsequent administrations of an anti-TRBV9 antibody at the main dose of 370 mg; or 375 mg; or 380 mg; or 495 mg; or 500 mg; or 505 mg; or 620 mg; or 625 mg; or 630 mg at least every 12 months.

60. The use according to any one of claims 35, 48, 54, wherein the anti-TRBV9 antibody comprises:
1) a heavy chain variable domain comprising:
(a) HCDR1 with the amino acid sequence of SEQ ID NO: 1,
(b) HCDR2 with the amino acid sequence of SEQ ID NO: 2, and
(c) HCDR3 with an amino acid sequence selected from the group: SEQ ID NO: 3, SEQ ID NO: 4, SEQ ID NO: 5 or SEQ ID NO: 6;
2) a light chain variable domain comprising:
(a) LCDR1 with the amino acid sequence of SEQ ID NO: 7,
(b) LCDR2 with the amino acid sequence of SEQ ID NO: 8, and
(c) LCDR3 with the amino acid sequence of SEQ ID NO: 9.

61. The use according to claim 60, wherein the anti-TRBV9 antibody comprises:
1) a heavy chain variable domain comprising:
(a) HCDR1 with the amino acid sequence of SEQ ID NO: 1,
(b) HCDR2 with the amino acid sequence of SEQ ID NO: 2, and
(c) HCDR3 with the amino acid sequence of SEQ ID NO: 4;
2) a light chain variable domain comprising:
(a) LCDR1 with the amino acid sequence of SEQ ID NO: 7,
(b) LCDR2 with the amino acid sequence of SEQ ID NO: 8, and
(c) LCDR3 with the amino acid sequence of SEQ ID NO: 9

62. The use according to claim 60, wherein the anti-TRBV9 antibody is a full-length antibody and is of human IgG1, IgG2, IgG3 or IgG4 isotype.

63. The use according to claim 60, wherein the anti-TRBV9 antibody comprises a heavy chain variable domain with the amino acid sequence of SEQ ID No: 14 and a light chain variable domain with the amino acid sequence of SEQ ID No: 17; or a heavy chain variable domain with the amino acid sequence of SEQ ID No: 15 and a light chain variable domain with the amino acid sequence of SEQ ID No: 17.

64. The use according to claim 60, wherein the anti-TRBV9 antibody comprises a heavy chain with the amino acid sequence of SEQ ID No: 22 and a light chain with the amino acid sequence of SEQ ID No: 25; or a heavy chain with the amino acid sequence of SEQ ID No: 23 and a light chain with the amino acid sequence of SEQ ID No: 25.

65. The use according to any one of claims 35, 48, 54, wherein the disease or disorder mediated by T-lymphocytes bearing the TRBV9 segment within the T cell receptor is selected from the group comprising: arthropathies, inflammatory bowel diseases, eye diseases, vasculitides, circulatory system diseases, kidney diseases, digestive system diseases, lymphoproliferative disorders.

66. The use according to any one of claims 35, 48, 54, wherein the disease or disorder mediated by T-lymphocytes bearing the TRBV9 segment within the T cell receptor is selected from the group comprising: spondyloarthritides, sacroiliitis associated with psoriasis, sacroiliitis associated with inflammatory bowel diseases, undifferentiated oligoarthropathy, juvenile spondylitis/enthesitis-related arthritis, juvenile ankylosing spondylitis (arthritis associated with enthesitis), juvenile arthritis, undifferentiated juvenile arthritis, ulcerative colitis, Crohn's disease, noninfectious uveitides, anterior uveitis, Behcet's disease, aortitis, fibrosis of aortic and/or mitral valve leaflets with regurgitation, rhythm disturbances, conduction disturbances, left ventricular dysfunction, pericarditis, myocarditis, IgA nephropathy, celiac disease, T cell lymphoma, T cell leukemia.

67. The use according to claim 66, wherein spondyloarthritis is radiographic axial spondyloarthritis (ankylosing spondylitis), nonradiographic axial spondyloarthritis, axial spondyloarthritis, peripheral spondyloarthritis, psoriatic arthritis, spondyloarthritis associated with inflammatory bowel diseases, reactive arthritis, undifferentiated peripheral spondyloarthritis.

68. The use according to claim 66, wherein spondyloarthritis is radiographic axial spondyloarthritis (ankylosing spondylitis) in patients with no adequate response to standard therapy, nonradiographic axial spondyloarthritis in patients with no adequate response to standard therapy, axial spondyloarthritis in patients with no adequate response to standard therapy, peripheral spondyloarthritis in patients with no adequate response to standard therapy, psoriatic arthritis in patients with no adequate response to standard therapy, spondyloarthritis associated with inflammatory bowel diseases in patients with no adequate response to standard therapy, reactive arthritis in patients with no adequate response to standard therapy, or undifferentiated peripheral spondyloarthritis in patients with no adequate response to standard therapy.
